# EUROPEAN PATENT APPLICATION

(11) **EP 4 039 696 A1**
(43) Date of publication of application: **10.08.2022**
(21) Application number: 20859528.0
(22) Date of filing: 27.08.2020
(51) Int. Cl.: C07K 14/435, C07K 19/00, A61K 38/17, A61K 39/005, A61K 39/10, A61K 39/12, A61K 39/35, A61K 39/205, A61K 39/215, C12N 15/12, C12N 15/62, C12N 15/63, G01N 33/566, G01N 33/569

(54) **PROTEIN RECEPTACLE, POLYNUCLEOTIDE, VECTOR, EXPRESSION CASSETTE, CELL, METHOD FOR PRODUCING THE RECEPTACLE, METHOD OF IDENTIFYING PATHOGENS OR DIAGNOSING DISEASES, USE OF THE RECEPTACLE AND DIAGNOSTIC KIT**

(30) Priority: 27.08.2019 BR 102019017792
(71) Applicant: Fundação Oswaldo Cruz, 21040-360 Rio de Janeiro, RJ (BR)
(72) Inventor: PROVANCE, JR., David William, 21940-140 Rio de Janeiro - RJ (BR); DURANS, Andressa, da Matta, 22251-080 Rio de Janeiro - RJ (BR); PÊGO, Paloma, Napleão, 21910-200 Rio de Janeiro - RJ (BR); DE SIMONE, Salvatore Giovanni, 22640-180 Rio de Janeiro - RJ (BR)
(74) Representative: Grünecker Patent- und Rechtsanwälte PartG mbB
(86) International application number: PCT/BR2020/050341
(87) International publication number: WO 2021/035325

(57) **Abstract**

The present invention relates to a protein receptacle capable of receiving several exogenous polyamino acid sequences, concomitantly, for expression in various systems and for different uses. The present invention relates to polynucleotides capable of generating the aforementioned protein receptacle. The present invention also relates to vector and expression cassette comprising the aforementioned polynucleotide. The present invention further relates to the cell comprising the aforementioned expression vector or cassette. The present invention further relates to a method for producing said protein receptacle and for pathogen identification or disease diagnosis *in vitro.* The present invention further relates to the use of said protein receptacle and kit comprising said protein receptacle for diagnostic purposes or as vaccine compositions.

## Description

### FIELD OF INVENTION

The present invention falls within the field of application of Chemistry, Pharmacy, Medicine, Biotechnology and, more specifically, in the area of preparations for biomedical purposes. The present invention relates to a protein receptacle capable of receiving several exogenous polyamino acid sequences, concomitantly, for expression in various systems and for different uses. The present invention relates to polynucleotides capable of generating protein receptacles mentioned above. The present invention also relates to vector and expression cassette comprising the aforementioned polynucleotide(s). The present invention further relates to a cell comprising the aforementioned vector or expression cassette. The present invention further relates to a method for producing said protein receptacle and for pathogen identification or disease diagnosis *in vitro.* The present invention further relates to the use of said protein receptacle and kit comprising said protein receptacle for diagnostic purposes or as vaccine compositions.

### BACKGROUND OF THE INVENTION

This document has several references throughout the text, which are indicated within parentheses. The information published in these references is included here in order to better describe the state of the art to which the present invention belongs.

The green fluorescent protein (GFP), produced by a cnidarian, the jellyfish *Aequorea victoria* emits high fluorescence in the green zone of the visible spectrum(Prasher et al., Gene 15, 111 (2): 229-33, 1992(1]) and, classically, has been used as a marker of gene expression and localization, and, thus, known as reporter protein. After the first observation that the GFP protein could emit fluorescence, several uses were described for the protein.

The use of GFP as a reporter protein has been patented for different uses and in different systems. Patent application US2018298058 uses GFP as a protein production and purification system. Patent application CN108303539 uses GFP as an input for cancer detection testing; application CN108220313 presents a high-throughput GFP fusion and expression method. Application CN108192904 claims a GFP fusion protein capable of inserting itself into biological membranes; application CN107703219 already emphasizes the use of GFP in the metabolomic study of mesen-chymal cells. Still, patent application US2018016310 presents variations of "superfolder" fusion GFP. There are several patents, such as these examples: US6054321, US6096865, US6027881, US6025485 that have mutated GFPs to increase fluorescence expression or modify fluorescence wavelength peaks. Thus, it is clear that the GFP protein, since its description, has been used and patented for various uses as a reporter protein.

Reporter molecules are often used in biological systems to monitor gene expression. The GFP protein brought great innovation in this scenario by dispensing with the use of any substrate or cofactor, that is, it does not require the addition of any other reagent in order to be visualized, as occurs for most other reporter proteins. Another advantage presented by GFP is its ability to show autofluorescence, not requiring fluorescent markers that, for various causes, do not have the appropriate sensitivity or specificity for its use.

In light of this characteristic, its self-production of detectable green fluorescence, GFP has been widely used to study gene expression and protein localization, and is considered one of the most promising reporter proteins in the literature.

In its use as a reporter protein, the gene encoding GFP can be employed in the production of fusion proteins, i.e. a particular gene of interest is fused to the gene encoding GFP. The fused gene cassette can be inserted into a living system, allowing expression of the fused genes and monitoring of the intracellular localization of that protein of interest (Santos-Beneit & Errington, Archives Microbiology, 199 (6): 875-880, 2017; Belardinelli & Jackson, Tuberculosis (Edinb), 105: 13-17, 2017; Wakabayashi et al, International Journal Food Microbiology, 19, 291: 144-150, 2018; Caì et al, Viruses, 20; 10 (11), 2018).

The GFP protein has also been useful as a framework for peptide presentation or even peptide libraries, both in yeast and mammalian cell systems (Kamb et al., Proc. Natl. Acad Sci. USA, 95: 7508-7513, 1998; WO 2004005322). The GFP protein, as a framework protein for the presentation of random peptides, can be used to define the characteristics of a peptide library.

Advances in the development of new variations of GFP seek to achieve improvements in the properties of the protein in order to produce new reagents useful for a wide range of research purposes. New versions of GFP have been developed, via mutations, containing DNA sequences optimized for increased production in human cellular systems, i.e., humanized GFP proteins (Cormack, et al., Gene 173, 33-38, 1996; Haas, et al., Current Biology 6, 315-324, 1996; Yang, et al., Nucleic Acids Research 24, 4592-4593, 1996).

In one of these versions, the enhanced green fluorescent protein, the "enhanced green fluorescent protein" (eGFP) was described (Heim & Cubitt, Nature, 373, pp. 663-664, 1995).

GFP, encoded by the gfp10 gene originating from a cnidarian, the jellyfish *Aequorea victoria,* is a protein of 238 amino acids. The protein has the ability to absorb blue light (with a main excitation peak at 395 nm) and emit green light, from a chromophore in the center of the protein (main emission peak at 509 nm) (Morin & Hastings, Journal Cell Physiology, 77(3): 313-8, 1971; Prasher etal, Gene 15, 111 (2): 229-33, 1992). The chromophore is composed of six peptides and starts at amino acid 64, and is derived from the primary amino acid sequence by cyclization and oxidation of serine, tyrosine and glycine (at positions 65, 66 and 67) (Shimomura, 104 (2) ,1979; Cody et al., Biochemistry, 32 (5): 1212-8, 1993). The light emitted by GFP is independent of the cell biological species where it is expressed and does not require any type of substrate, cofactors or additional gene products from *A. victoria* (Chalfie et al., Science, 263 (5148): 802-5, 1994). This property of GFP allows its fluorescence to be detected in living cells other than *A. victoria,* provided that it can be processed in the cell's protein expression system (Ormo et al, Science 273: 1392-1395, 1996;. Yang et al, Nature Biotech 14: 1246-1251, 1996).

The basic structure of a GFP consists of eleven antiparallel pleated beta chains, which are intertwined to form a tertiary structure in the shape of a beta barrel. Each chain is connected to the next by a domain of handle that projects to the top and bottom surface of the barrel, interacting with the environment. By convention, each string e loop can be identified by a number in order to better describe the protein.

Targeted mutation experiments have shown that several biochemical properties of the GFP protein arise from this barrel structure. And therefore, amino acid changes in the primary structure are responsible for accelerating protein folding, reducing the aggregation of translation products, and increasing the stability of the protein in solution.

A particular loop moves into the cavity of the protein barrel, forming an alpha-helix that is responsible for the fluorescent properties of the protein (Crone et al, GFP-Based Biosensors, InTech, 2013). Some interventions in the protein structure can interfere with the ability to emit fluorescence. Mutations in certain amino acids can change from the intensity of fluorescence emission to the wavelength, changing the emitted color. Mutation of Tyr66, an internal residue participating in the fluorescent chromophore, can generate a large number of fluorescent protein variants with the structure of the altered chromophore or the surrounding environment. These changes interfere with the absorption and emission of light at different wavelengths, producing a wide range of distinct emitted colors (Heim & Tsien, Current Biology, 6 (2): 178-82, 1996).

Changes in pH can also interfere with fluorescence intensity. At physiological pH, GFP exhibits maximum absorption at 395 nm, while at 475 nm it absorbs less light. However, increasing the pH to about 12.0 causes the absorption maximum to occur in the 475 nm range, and at 395 nm it has decreased absorption (Ward et al, Photochemistry and Photobiology, 35(6): 803-808, 1982).

The compact structure of the protein core allows GFP to be highly stable even under adverse conditions, such as treatment by proteases, making the protein extremely useful as a reporter protein in general.

There are different versions of the GFP, always seeking to improve the protein by adding new functionality or removing some limitation. The eGFP presents itself as an enhancement that allows for greater flexibility of the protein in the face of modifications to the F64L and S65T amino acids(Heim et al, Nature 373: 663-664, 1995; Li et al, Journal Biology Chemistry, 272 (45): 28545-9, 1997). This enhancement allows GFP to achieve both its expected three-dimensional shape and its ability to express fluorescence, even when harboring heterologous sequences in its protein sequence (Pedelacq et al, Nat Biotechnology, 24 (1): 79-88, 2006).

GFAb, is a version of the modified protein that accepts the exogenous sequences in two loop domains. In its development, several rounds of direct evolution were required to select three mutated protein clones that supported the insertion of exogenous peptides into the two proximal regions, namely Glu-172-Asp-173 and Asp-102-Asp-103. The authors using unmutated proteins proved that the insertion of two exogenous peptides prevented GFP fluorescence production and protein expression on the yeast cell surface. Simultaneous insertion, in only two regions, was possible after a series of mutations and selections, but still resulting in great loss of the inherent activities of GFP, sometimes making its production or expression of the insert impossible (Pavoor et al, PNAS 106 (29): 11895-11900,2009).

Mutants circularly permuted to the N- and C-terminals have also demonstrated that eGFP is amenable to manipulation in the coding sequence without compromising the structural aspects of the protein core (Topell et al., FEBS Letters 457(2): 283-289, 1999). However, analysis of 20 circularly permuted protein variants demonstrated the proteins' low tolerance to insertion of a new terminus and, for the most part, that they lose the ability to form the chromophore. This fact indicates that manipulation of the protein sequence can drastically interfere with its characteristics or even its cellular expression.

Several attempts have been made to simultaneously insert multiple epitopes into the loop regions of GFP with the goal of achieving use of the protein for specific binding reactions to a target. However, all these efforts have shown limited success in light of the structural sensitivity of GFP and its chromophore.

Other mutant proteins of the GFP protein show improved versions that emit other types of fluorescent light spectrum.

For example, Heim et al (Proc Natl Acad Sci USA, 91 (26): 12501-4, 1994) described a mutant protein that emits blue fluorescence by containing a histidine instead of a tyrosine at amino acid 66. Heim et al(Nature, 373 (6516): 663-4, 1995) subsequently also described a mutant GFP protein, by substitution of a serine for a threonine at amino acid 65, which has a spectrum very similar to that obtained from *Renilla reniformis,* which has a 10-fold higher extinction coefficient per monomer than the wavelength peak of the native GFP from *Aequorea.* Other patent documents describe mutant GFP proteins showing light emission spectra other than green, such as blue, red (US 5625048, WO 2004005322).

Also, other GFP mutant proteins have excitation spectra optimized for use specifically in certain argon laser flow cytometer (FACS) equipment (US 5804387). There is still a description of mutant GFP proteins modified to be better expressed in plant cell systems (WO1996027675). The patent document US5968750 presents a humanized GFP that has been adapted to be expressed in mammalian cells, including human. Humanized GFP incorporates preferential codons for reading into human cell gene expression systems.

In the state of the art, it is clear that GFP is capable of harboring genes at its 5' or 3' ends without interfering with expression, three-dimensional tangling, and fluorescence production, as can be seen in the patent documents listed above. Additionally, GFP has been used as a carrier for peptide display or even peptide libraries *in vivo.* In the case of peptide libraries, GFP can assist in the presentation of random peptides, and thus in defining the characteristics of the peptide library (Kamb et al., Proc. Natl. Acad. Sci. USA, 95:7508-7513, 1998; WO 2004005322).

For example, Abedi et al (1998, Nucleic Acids Res. 26: 623-300) inserted peptides into GFP proteins of *Aequorea victoria* in regions of exposed loops and demonstrated that GFP molecules retain autofluorescence when expressed in yeast *and Escherichia coli.* The authors further demonstrated that the fluorescence of a GFP frame can be used to monitor peptide diversity, as well as the presence or expression of a given peptide in a given cell. However, the fluorescence rate of the GFP framework molecules is relatively low compared to natural GFP. Kamb and Abedi (US 6025485) prepared libraries of GFP arrays from enhanced green fluorescent protein (eGFP) in order to amplify fluorescence intensity.

Additionally, Peele et al (Chem. & Bio. 8: 521-534, 2001) tested peptide libraries using eGFP as a framework with different structural biases in mammalian cells. Anderson *et al* further amplified the fluorescence intensity by inserting peptides into the GFP loops with tetraglycine ligands (US20010003650). Happe *et al* described a humanized GFP that can be expressed in large quantities in mammalian cell systems, tolerates peptide insertions, and preserves autofluorescence (WO 2004005322).

However, there is still a need in the technological field for GFP molecule frameworks that not only display fluorescence at appropriate intensities, but that can also be expressed at high levels in cellular systems.

There is variability in tolerance, among GFP molecules, for peptide presentation while retaining autofluorescence. Thus, there is a need in the state of the art to develop GFPs that can be expressed at high levels and tolerate insertions, while preserving autofluorescence.

In addition to the ability to support gene expression at the ends, GFP may allow the insertion of epitopes into the surface loops of the molecule exposed to the medium. Several attempts have been made to simultaneously insert multiple peptides into the loop regions of GFP that could allow the protein to be used for target-specific binding reactions. However, all of these efforts have had limited success in light of the structural sensitivity of GFP and its chromophore.

Pavoor and colleagues undertook efforts to develop a protein modified to accept exogenous sequences in two loop domains. In its development, several rounds of direct evolution were necessary to select three mutated protein clones that supported the insertion of exogenous peptides in the two proximal regions, namely, Glu-172-Asp-173 and Asp-102-Asp-103 . The authors using unmutated proteins proved that the insertion of two exogenous peptides prevented GFP fluorescence production and protein expression on the yeast cell surface. Simultaneous insertion, in only two regions, was possible after a series of mutations and selections, but still resulted in a great loss of the inherent activities of GFP, sometimes making its production or expression of the insert impossible (Pavoor et al, PNAS 106 (29): 11895-11900, 2009).

Abedi et al (Nucleic Acids Research 26(2): 623-30, 1998) showed 10 positions of the protein, in loop regions, of which 8 positions between-β-sheets, for peptide expression. The chimeric protein would be useful for experiments requiring intracellular expression, and therefore fluorescence uninterruptedness would be a limiting factor. In this study, only three chimeric proteins (those with insertion sites at amino acids 157-158 172-173 and 194-195) showed fluorescence (dimmed to a quarter of the original); and only two insertion sites (studied separately) could harbor peptides without loss of fluorescence. The authors of the paper further concluded that "it is curious how GFP is so sensitive to structural perturbations even if it is in β-sheets."

Li et al (Photochemistry and Photobiology, 84(1): 111-9, 2008) present a study of the chimeric protein (red fluorescent protein - RFP), pointing out in this protein six genetically distinct sites located in three different loops where sequences of five residues can be inserted without interfering with the ability of the protein to be fluorescent. However, the authors have not demonstrated the concomitant use of these sites for insertion of different peptides.

Patent application WO02090535 presents a fluorescent GFP with non-simultaneous peptide insertions into 5 different loops of the protein. The patent application, in its descriptive report, indicates the possibility of inserting peptides in more than one loop of the protein at the same time, increasing the complexity of the library and allowing presentation on the same face of the protein. However, the patent application does not prove this possibility, since it only presents insertion assays of peptides, one at a time, in 5 different protein loops. It is worth noting that the text further emphasizes that loops 1 and 5 do not present themselves as good insertion sites, because peptide insertion at these sites prevented protein expression. Still other patent documents present variations of GFP aiming at the expression of peptides in the protein's loops, however, these studies do not prove the feasibility of simultaneous expression of more than 4 peptides in different insertion sites in the GFP protein loops without loss of any of its essential characteristics (WO02090535, US2003224412, WO200134824).

### SUMMARY OF THE INVENTION

To solve the problems mentioned above, the present invention will provide significant advantages, since the receptacle proteins can express a large number of different polyamino acid sequences, being characterized as multivalent receptacle proteins, expanding their use for vaccine composition purposes, an input for research and technological development or disease diagnosis.

There is a real need in the state of the art to develop receptacle proteins that not only exhibit adequate fluorescence intensities, but can also be expressed in large quantities in production cell systems and, furthermore, tolerate the concomitant presentation of multiple exogenous polyamino acid sequences while still exhibiting detectable autofluorescence.

In one aspect, the present invention relates to a protein receptacle capable of presenting several exogenous polyamino acid sequences concurrently at more than four different sites on the receptacle protein.

In another aspect, the invention relates to polynucleotides capable of generating the aforementioned protein receptacle.

In another aspect, the invention relates to a vector comprising the aforementioned polynucleotide.

In another aspect, the invention relates to expression cassette comprising the aforementioned polynucleotide.

In another aspect, the invention relates to a method for producing said protein receptacle and for pathogen identification or disease diagnosis *in vitro.*

In another aspect, the present invention relates to the use of said protein receptacle for diagnostic purposes or as vaccine compositions.

In another aspect, the present invention relates to kit comprising said protein receptacle for diagnostic purposes or as vaccine compositions.

### BRIEF DESCRIPTION OF THE FIGURES

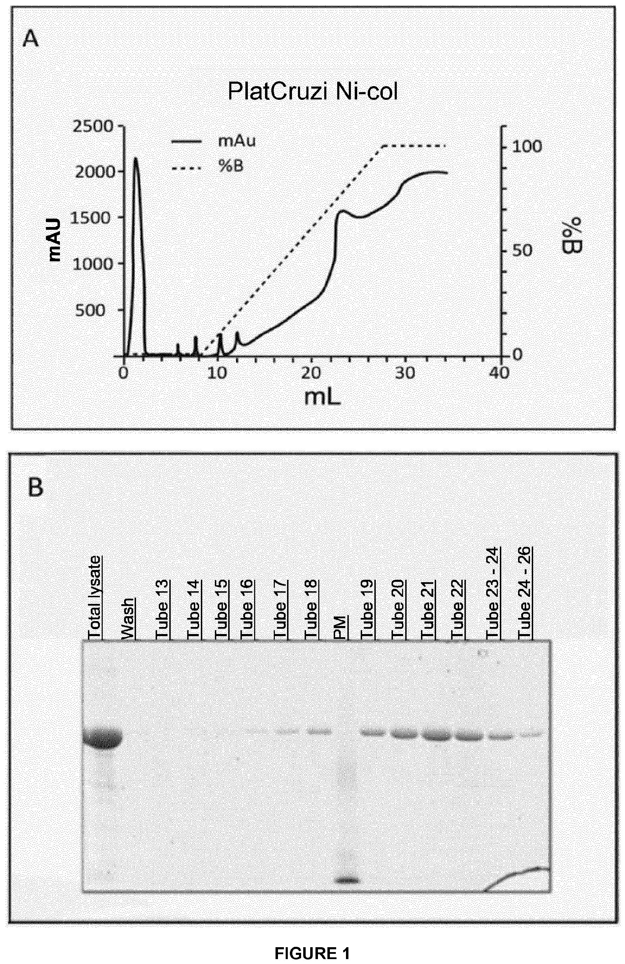

**Figure 1** - Purification of PlatCruzi protein by affinity chromatography. (A) Elution profile of the receptacle protein on a nickel column using a *Äktapurifier* liquid chromatography system. (B) Analysis by polyacrylamide gel electrophoresis (SDS PAGE) of the collected 13-26 eluates. Elution was performed with buffer B and in ascending order. PM - Molecular Weight Marker.

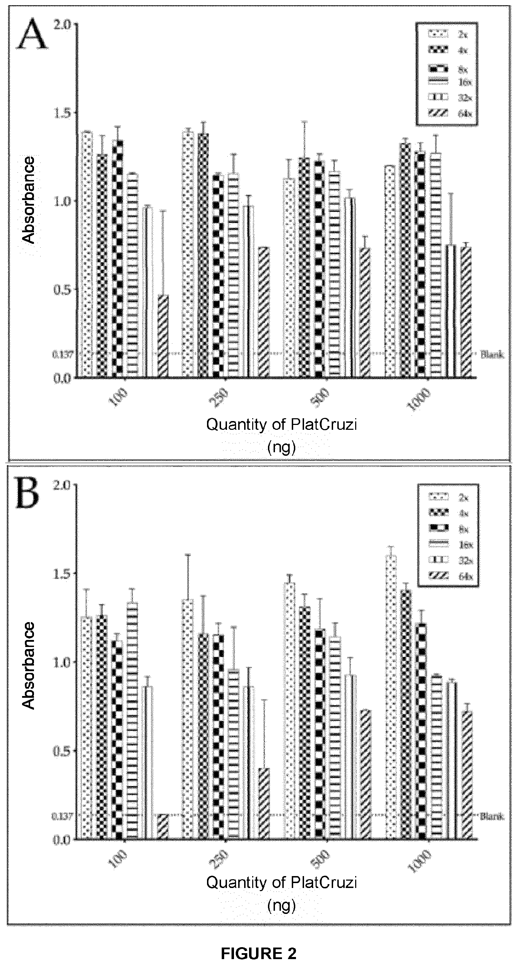

**Figure 2** - Reactivity of PlatCruzi, by ELISA, with sera from *Trypanosoma cruzi* International Standard Biological References provided by WHO. (A) Pool of patient sera recognizing TcI strains, called IS 09/188. (B) Pool of patient sera recognizing Tell strains, called IS 09/186.

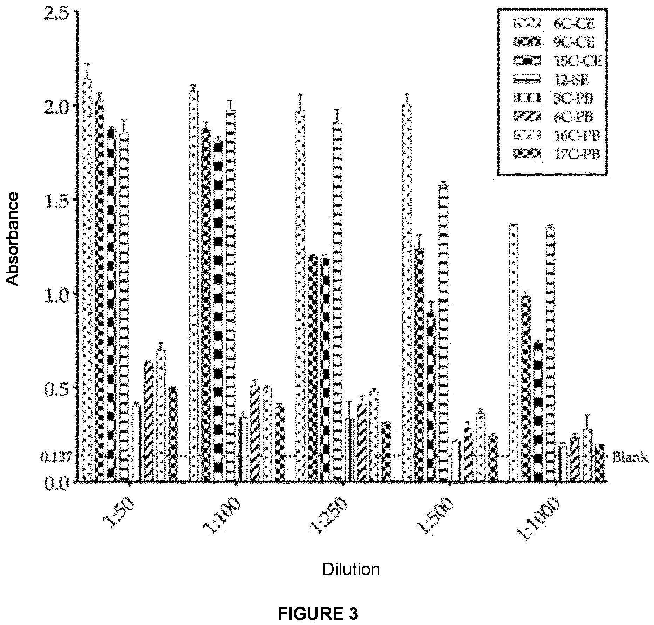

**Figure 3** - Determination of antibody titer of sera from patients with chronic Chagas disease using PlatCruzi receptacle protein as antigen. Sera were provided by the LACENS and a concentration of 500 ng/well and serum dilution 1: 50 - 1: 1000 were used in ELISAS.

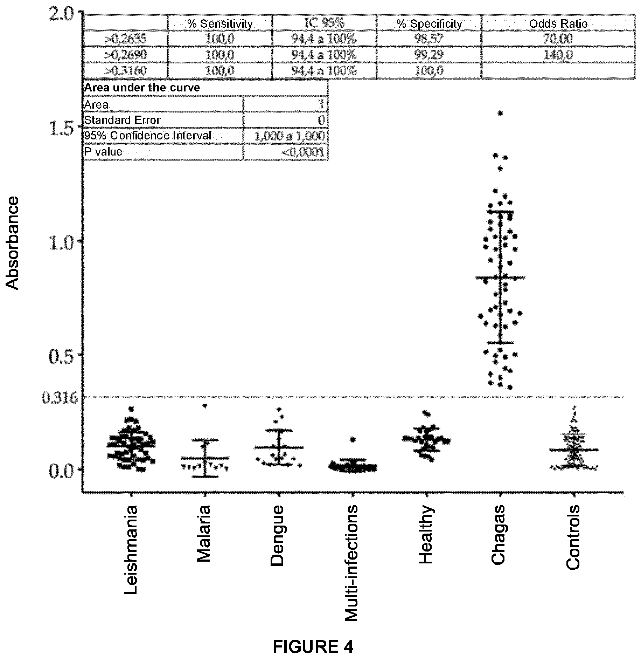

**Figure 4** - Performance of the PlatCruzi antigen by ELISA against serum from patients with different diseases. PlatCruzi antigen was used at a concentration of 500 ng/well and sera diluted 1:250.

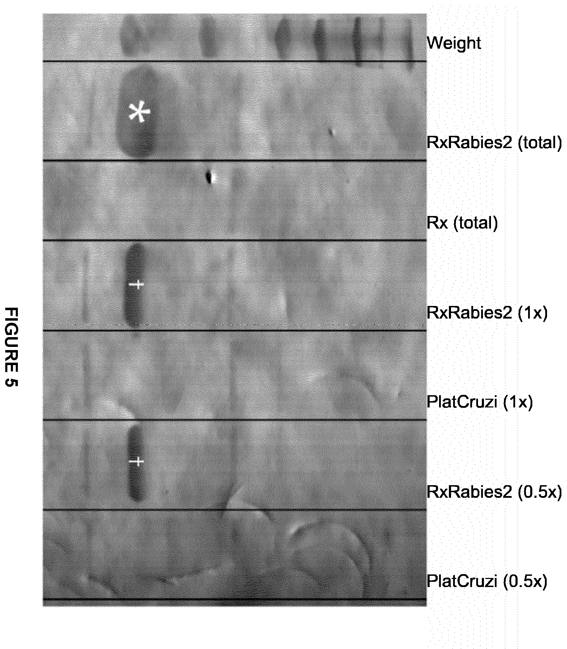

**Figure 5** - Detection of rabies virus-specific epitopes by rabbit anti-RxRabies2 serum. Rabbit antibodies immunized with RxRabies2 were purified by RxRabies2 affinity chromatography and used as primary antibody, by *Western blot,* to detect RxRabies2 in a crude (^{∗}; column 2) or semi-purified (†) extract at two different concentrations of 1x and 0.5x (columns 4 and 6, respectively). Negative controls: Rx receptacle protein (column 3) and PlatCruzi (in two concentrations: 1x and 0.5x in columns 5 and 7, respectively).

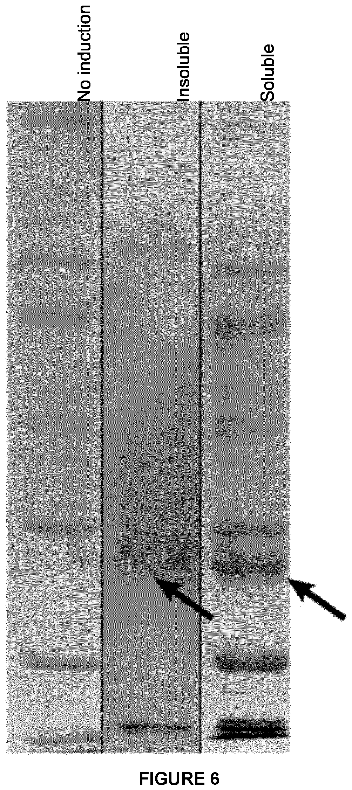

**Figure 6** - Analysis of RxHoIgG3 protein by polyacrylamide gel electrophoresis (SDS PAGE). A, soluble extract of *E.coli* not producing RxHoIgG3. B, Aqueous insoluble fraction of RxHoIgG3-producing bacteria. C, Soluble fraction of RxHoIgG3-producing bacteria. The arrows indicate the position of the RxHoIgG3 protein.

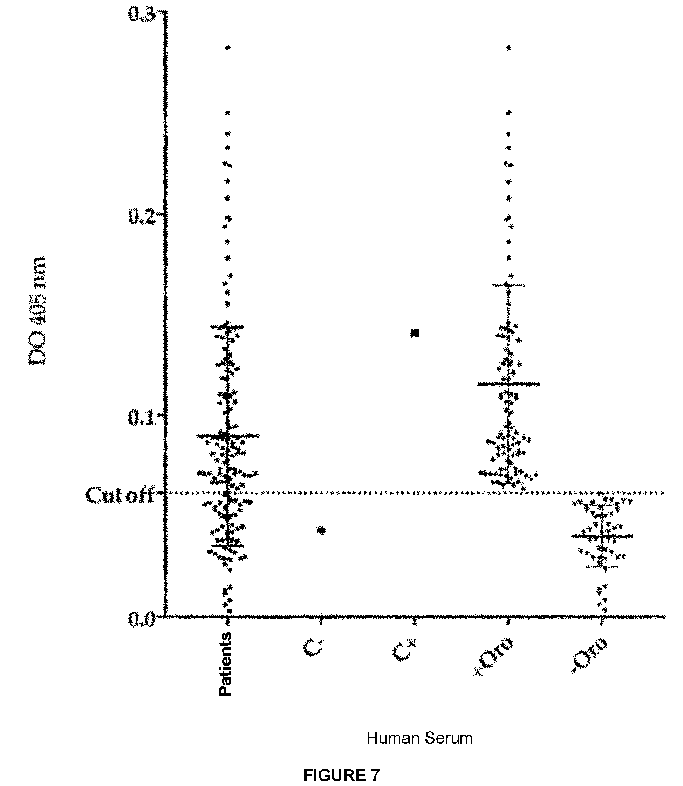

**Figure 7** - Detection of IgM anti-RxOro antibodies by ELISA. C-: negative control (serum from patient without Oropouche virus infection); C+: standard positive serum for Oropouche virus infection. Patients: suspected cases of Oropouche virus infection. Oro+: positive for Oropouche infection (detection of IgM anti-RxOro antibodies). Oro- (negative control): No IgM reaction for RxOro. Protein concentration: 0,288µg/µL. *Cutoff*: 0.0613.

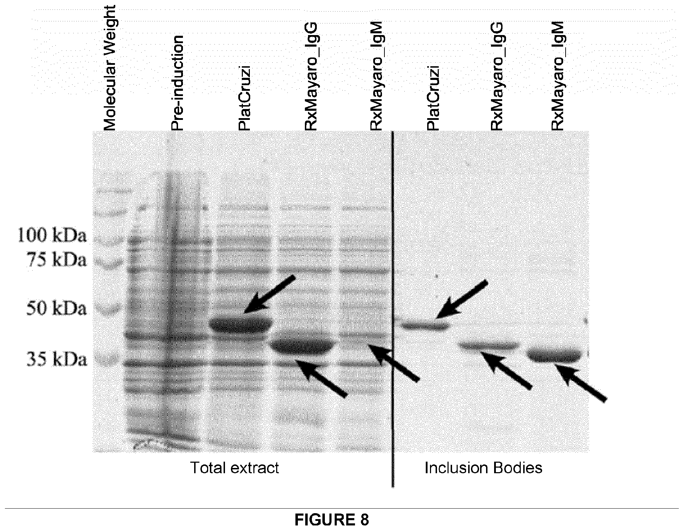

**Figure 8** - Polyacrylamide gel electrophoresis (SDS-PAGE) representing the production of the PlatCruzi, RxMayaro_IgG and RxMayaro_IgM proteins. Vertical columns 1 to 8, indicate: 1) molecular weight; 2) total bacterial extract without induction of recombinant protein; 3) total bacterial extract after induction of PlatCruzi production; 4) total bacterial extract after induction of RxMayaro_IgG production; 5) total bacterial extract after induction of RxMayaro_IgM production; 6) insoluble bacterial proteins after induction of PlatCruzi production; 7) insoluble bacterial proteins after induction of RxMayaro_IgG production; 8) insoluble bacterial proteins after induction of RxMayaro IgM production. The arrows point to the bands representing PlatCruzi (columns 3 and 6), RxMayaroIgG (columns 4 and 7) and RxMayaroIgM (columns 5 and 8).

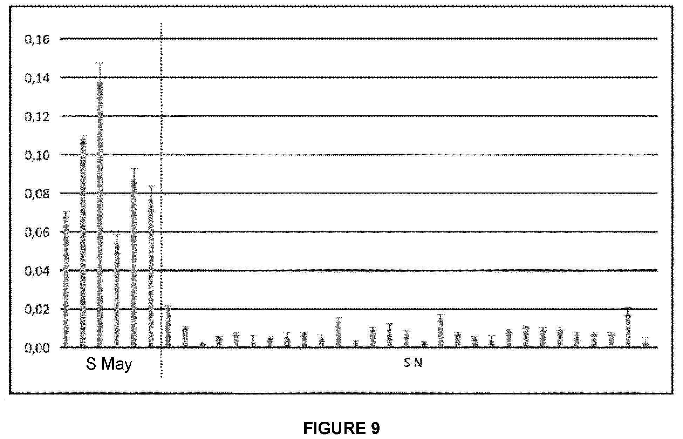

**Figure 9** - Reactivity of sera from Mayaro virus positive patients (S MAY) and healthy individuals (S N), by ELISA, using the RxMayaro IgG protein. Revealing was performed with anti-IgG immunoglobulin conjugated to alkaline phosphatase enzyme*(Cutoff*= 0.0210).

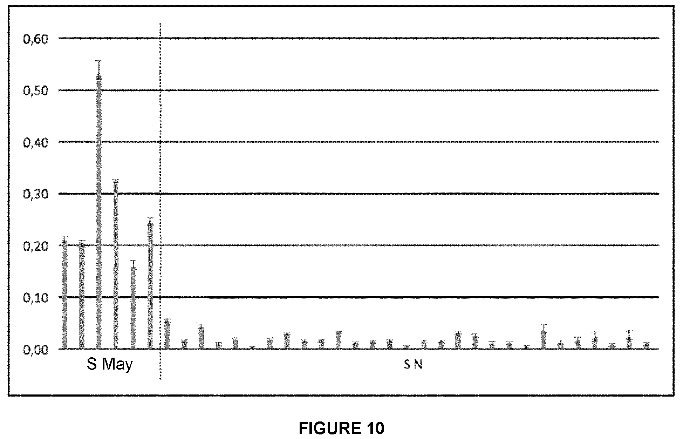

**Figure 10** - Reactivity of sera from individuals considered healthy (S N) and positive for Mayaro virus (S MAY), by ELISA, using the RxMayaro_IgM protein. Revealing was performed with anti-IgM immunoglobulin conjugated to alkaline phosphatase enzyme. *(Cut off =* 0.0547).

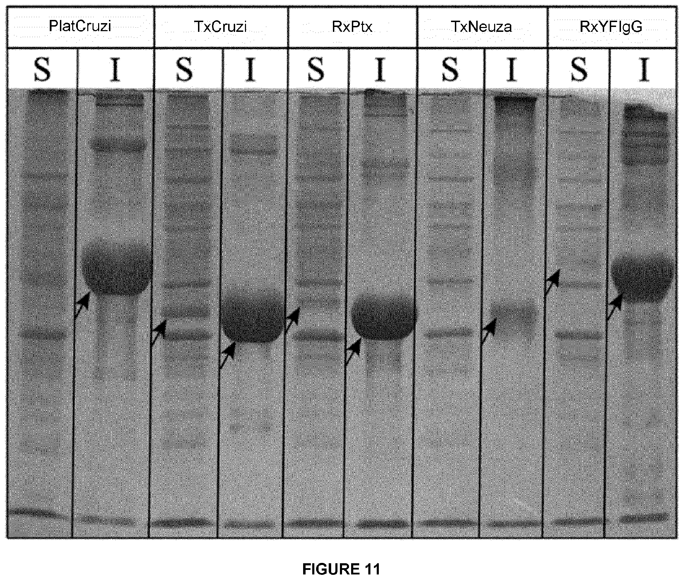

**Figure 11** - Polyacrylamide gel electrophoresis (SDS-PAGE) showing the yield ofinsoluble (I) and soluble (S) proteins from PlatCruzi, TxCruzi, RxPtx, TxNeuza, and RxYFIgG. Columns 1 through 10 contain: 1) insoluble proteins of bacteria induced to produce PlatCruzi; 2) soluble proteins of bacteria induced to produce PlatCruzi; 3) insoluble proteins of bacteria induced to produce TxCruzi; 4) soluble proteins of bacteria induced to produce TxCruzi; 5) insoluble proteins of bacteria induced to produce RxPtx; 6) soluble proteins of bacteria induced to produce RxPtx; 7) insoluble proteins of bacteria induced to produce TxNeuza; 8) soluble proteins of bacteria induced to produce TxNeuza; 9) insoluble proteins of bacteria induced to produce RxYFIgG; 10) soluble proteins of bacteria induced to produce RxYFIgG. The arrows point to the bands representing PlatCruzi (columns 1 and 2), TxCruzi (columns 3 and 4), RxPtx (columns 5 and 6), TxNeuza (columns 7 and 8) and RxYFIgG (columns 9 and 10).

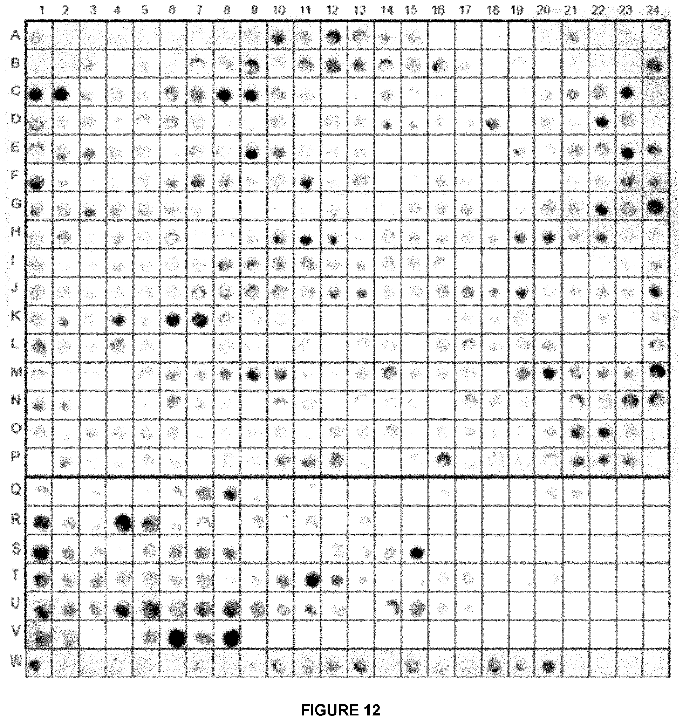

**Figure 12** - Pictorial cellulose membrane with polyamino acid of SARS-CoV-2 reacting with IgM antibodies from Covid-19 positive patient sera, as spots of various shades of gray within regions enclosed by a grid in the form of a checkerboard. Each square encompasses a reacting spot of the cellulose membrane region in which a distinct polypeptide sequence has been synthesized in linear form covalently bound to the membrane surface. The relationship between the physical position in the membrane and the sequence of the polyamino acid is listed in Table 18. The combined polyamino acid sequences represent the encoded sequence of the spike protein SARS-CoV-2 (S1: aa 1-1273, A7-K19), protein ORF3a (OF3: aa 1-275, K22-N2), membrane glycoprotein (M: aa 1-222, N5-O23); ORF6 (OF6: aa 1-61, P2-P12); ORF7 protein (OF7: aa1-121, P15-Q13), ORF8 protein (OF8: aa 1-121, Q16-R17), nucleocapsid protein (N: aa1-419, R20-V17), envelope protein (E: aa 1 -75, W1-W13), ORF10 protein regions (OF10: aa 1-38, W15-W20). Each polyamino acid has a length of 15 amino acids and an adjacent, continuous overlap of 10 amino acids.

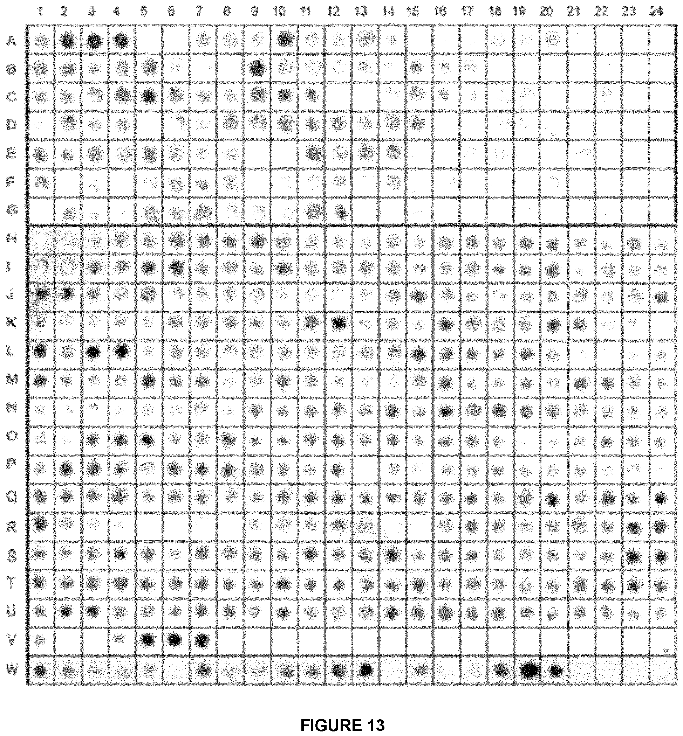

**Figure 13** - Pictorial cellulose polyamino acid membrane of SARS-CoV-2 reacted with IgG antibodies from Covid19 patient sera, as spots of various shades of gray visualized within regions bounded in the form of a grid. Each square encompasses a reacting spot of the cellulose membrane region in which a distinct polypeptide sequence has been synthesized in linear form covalently bound to the membrane surface. The relationship between the physical positions in the membrane and the sequences of the polyamino acids is listed in Table 19. The combined polyamino acid sequences represent the encoded sequence of the SARS-CoV-2 ORF3a protein (ORF3: aa 1-275, A7-C11), membrane glycoprotein (G: aa 1-61, C14-E8); ORF6 protein (ORF6: aa 1-61, E11-E21); ORF7 protein (OF7: aa1-121, E24-F22), ORF8 protein (ORF8: aa 1-121, G1-G23), spike protein (S: aa 1-1273, H1-R13), nucleocapsid protein (N: aa1 -419, R16-V1), envelope protein (E: aa 1-75, W1-W13), ORF10 (ORF10: aa 1-38, W15-W20). Each polyamino acid has a length of 15 amino acids and an adjacent, continuous overlap of 10 amino acids.

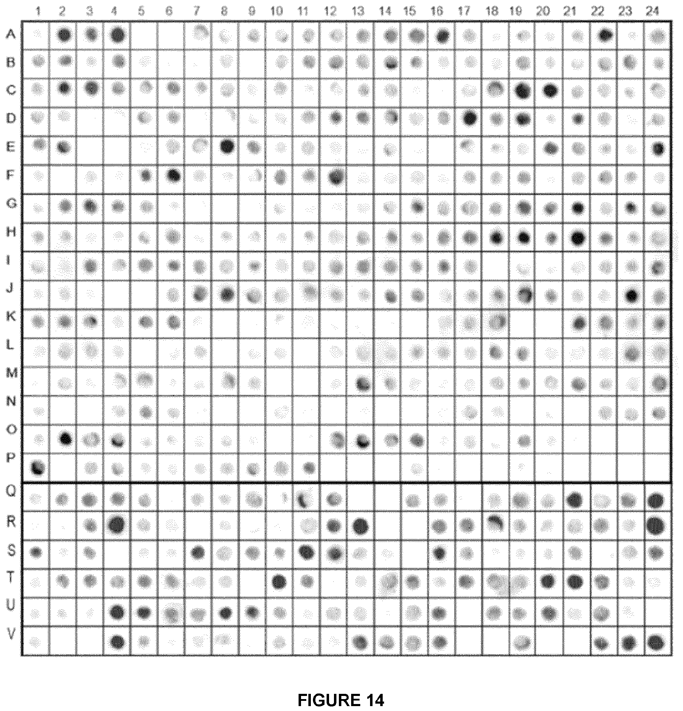

**Figure 14** - Pictorial cellulose membrane with polyamino acid of SARS-CoV-2 reacting with IgA antibodies, from Covid19 patient serum as spots of various shades of gray visualized within delimited regions in the form of a grid pattern. Each square encompasses a reaction spot of the cellulose membrane region in which a distinct polypeptide sequence has been synthesized in linear form covalently bound to the membrane surface. The relationship between the physical positions in the membrane and the sequence of the polyamino acid is listed in Table 20. These combined polyamino acid sequences represent the encoded sequence of the spike protein of SARS-CoV-2 (S: aa1-1273, A6-K18), ORF3a (ORF3: aa 1-275, K21-N1), membrane glycoprotein (M: aa 1-61, N4-O22); ORF6 (ORF6: aa 1-61, P1-P11); ORF7 (ORF7: aa1-121, P14-Q12), ORF8 (ORF8: aa 1-121, Q15-R13), Nucleocapsid (N: aa1-419, R16-V1), Protein der Energie (E: aa 1-75 .), Regs V4-V16), ORF10 (ORF10: aa 1-38, V19-V24). Each polyamino acid has a length of 15 amino acids and an adjacent, continuous overlap of 10 amino acids.

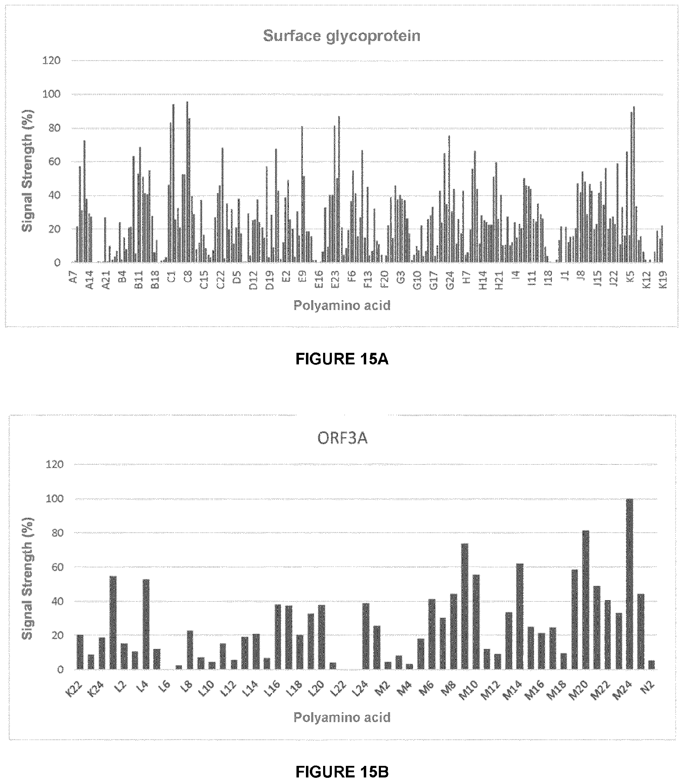

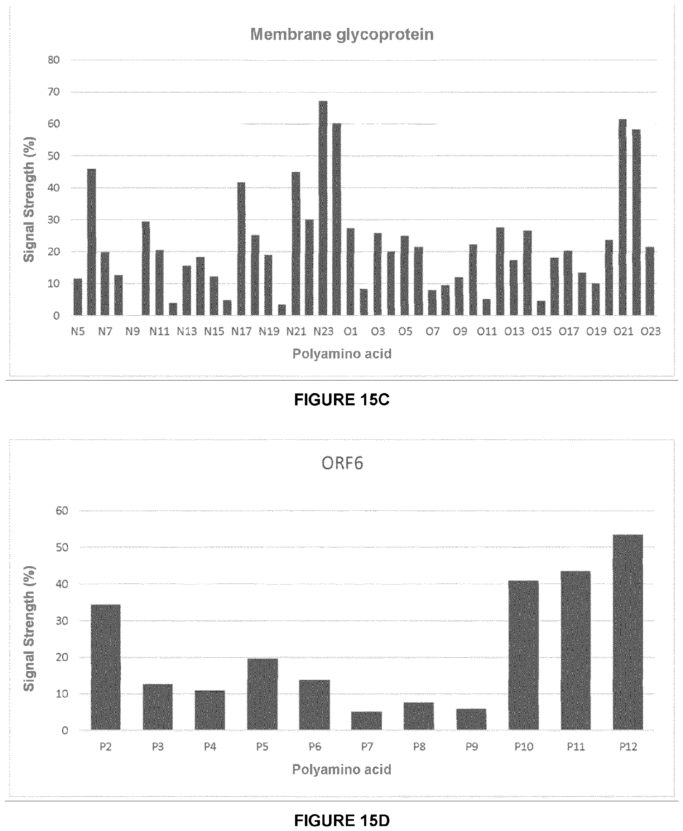

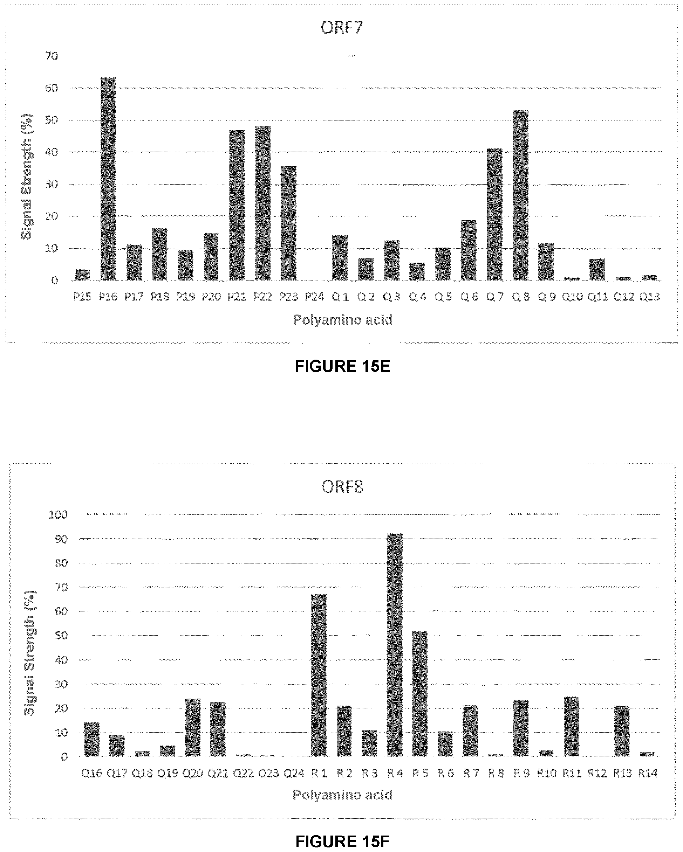

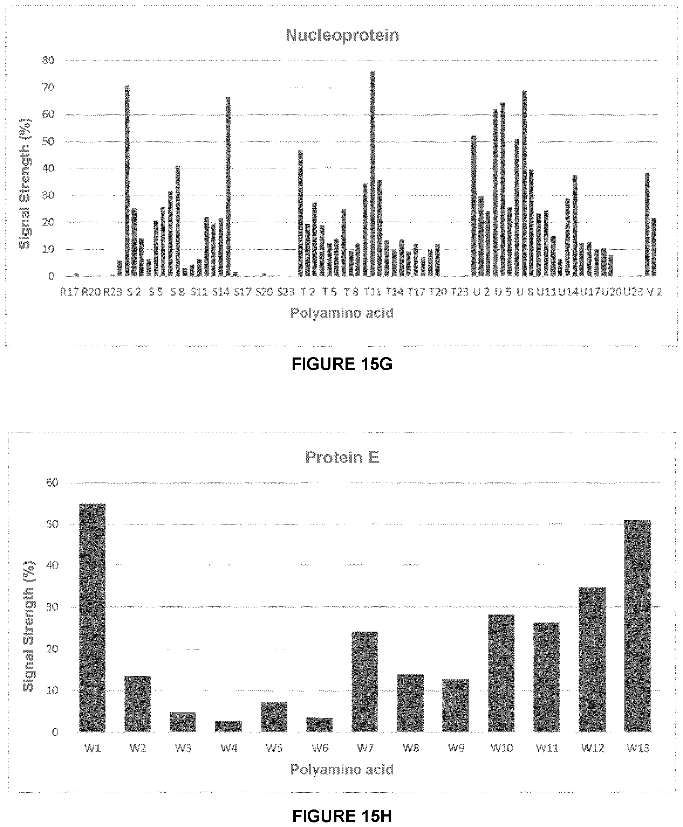

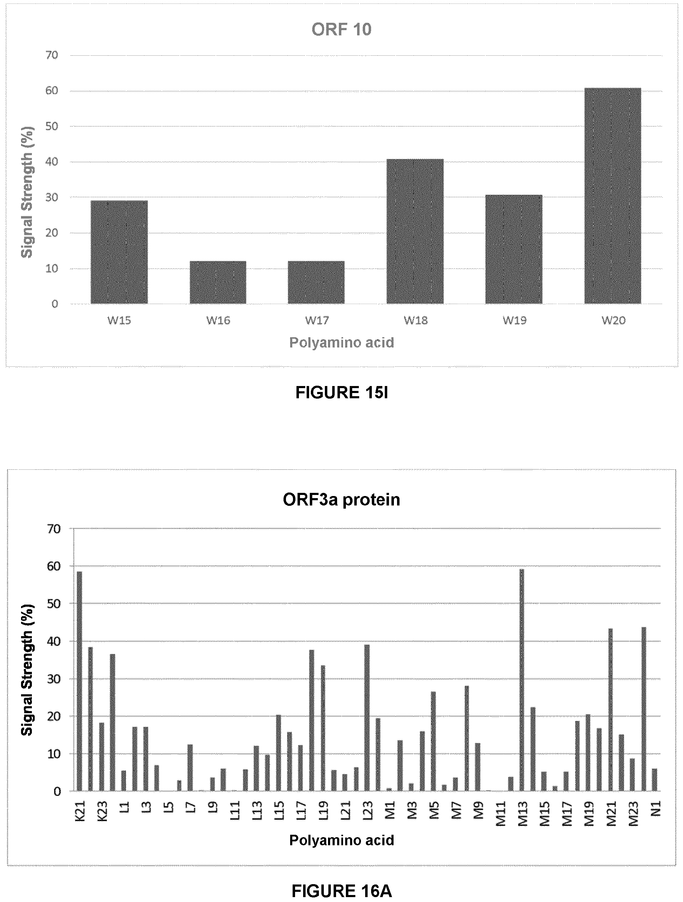

**Figure 15** - Reactivity of sera from patients with COVID, revealed with alkaline phosphatase-labeled anti-human IgM antibodies, to SARS-CoV-2 peptides synthesized on cellulose membrane (Figure 12). 15A, surface glycoprotein; 15B, ORF 3a; 15C, membrane glycoprotein; 15D, ORF 6; 15E, ORF 7; 15F, ORF 8; 15G, nucleoprotein; 15H, protein E; 151, ORF 10.

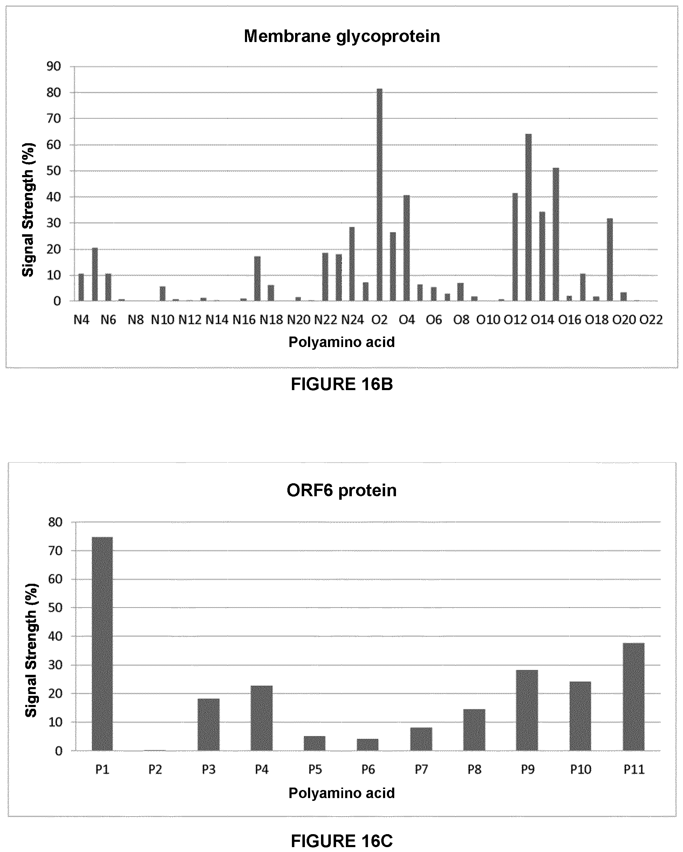

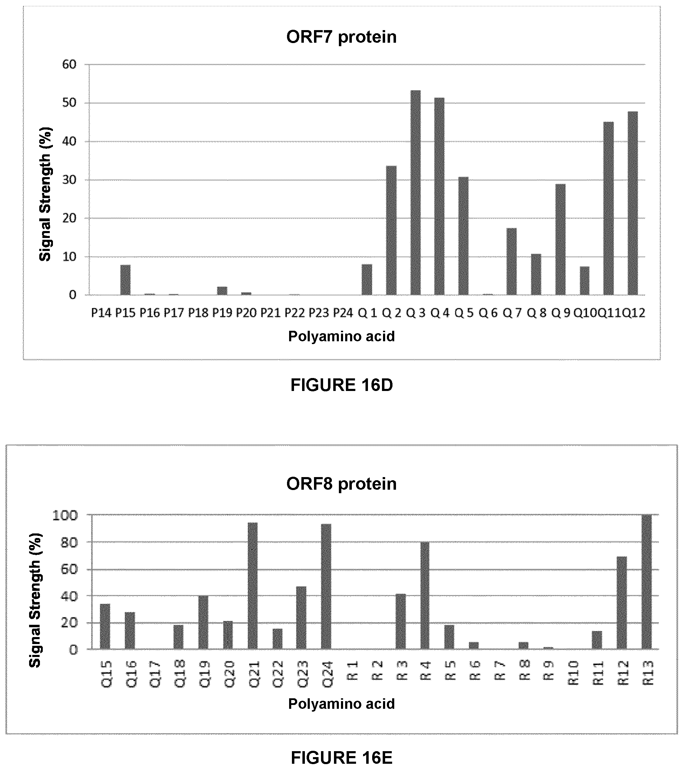

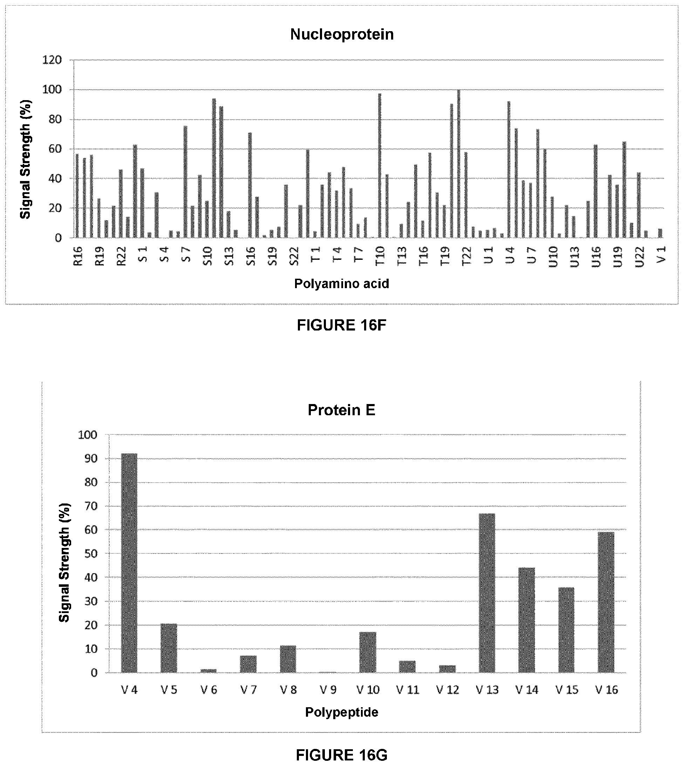

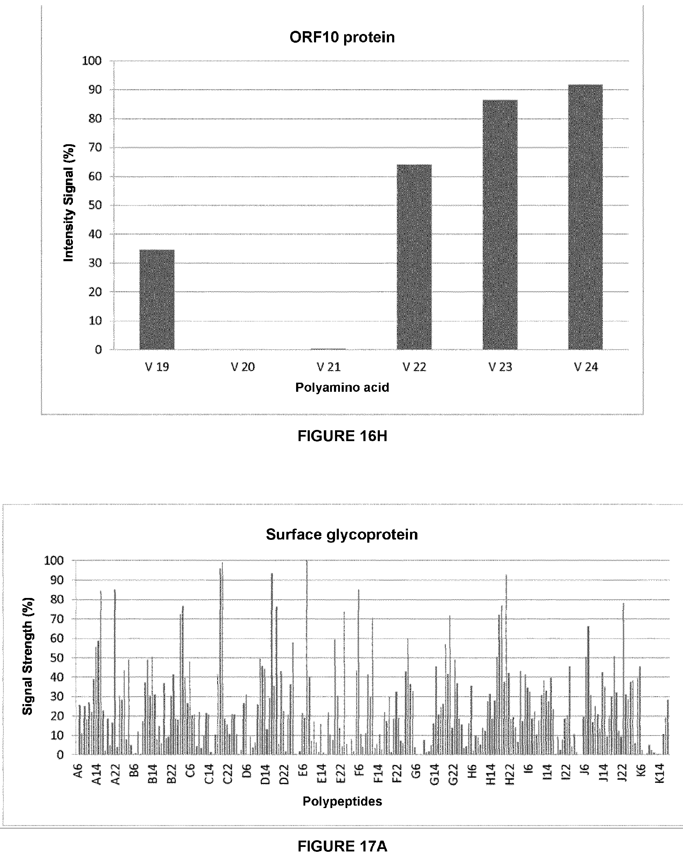

**Figure 16** - Reactivity of sera from patients with COVID, revealed with alkaline phosphatase-labeled anti-human IgG antibodies, to SARS-CoV-2 peptides synthesized on cellulose membrane (Figure 13). 16A ORF 3a; 16B: membrane glycoprotein; 16C: ORF 6; 16D: ORF7; 16E: ORF 8; 16F: nucleoprotein; 16G: E protein; 16H: ORF 10.

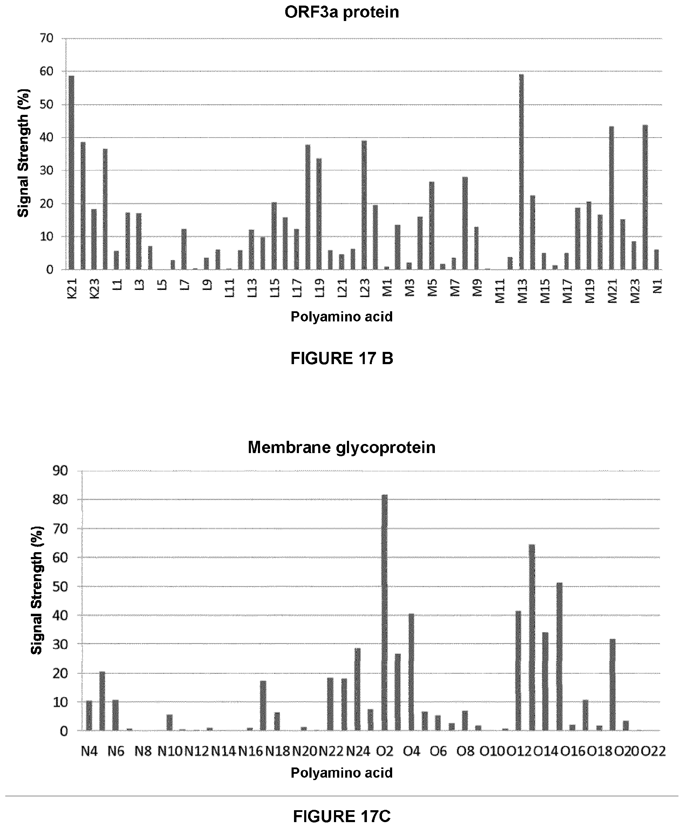

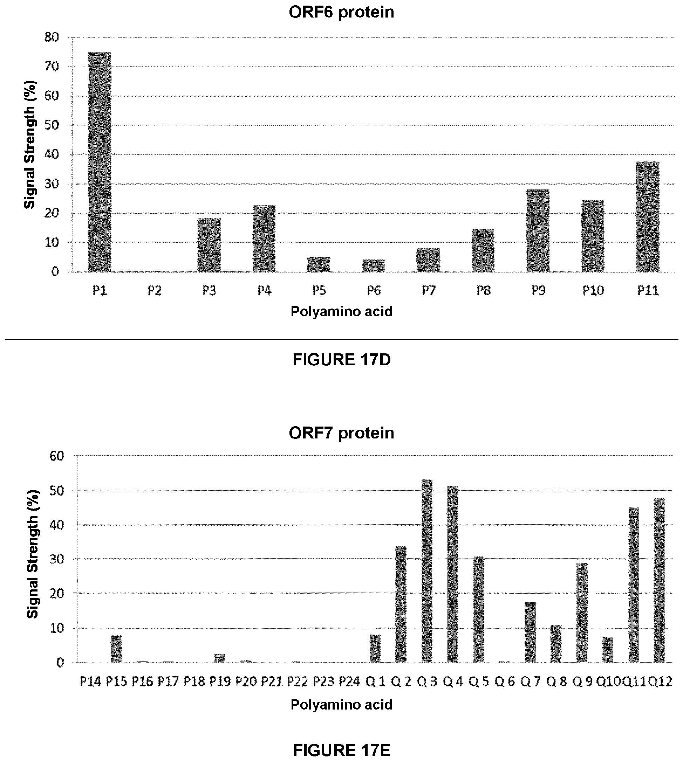

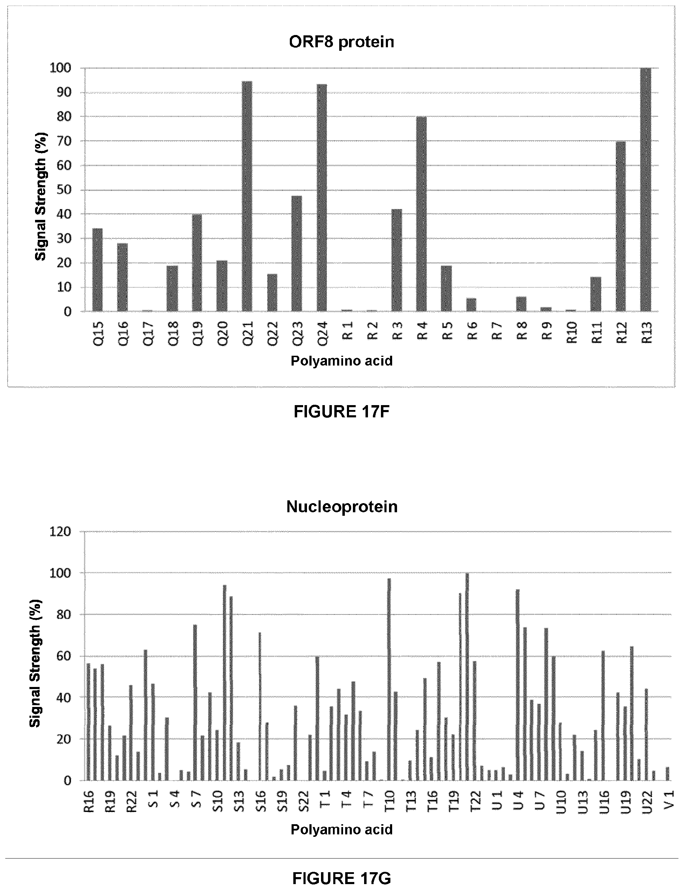

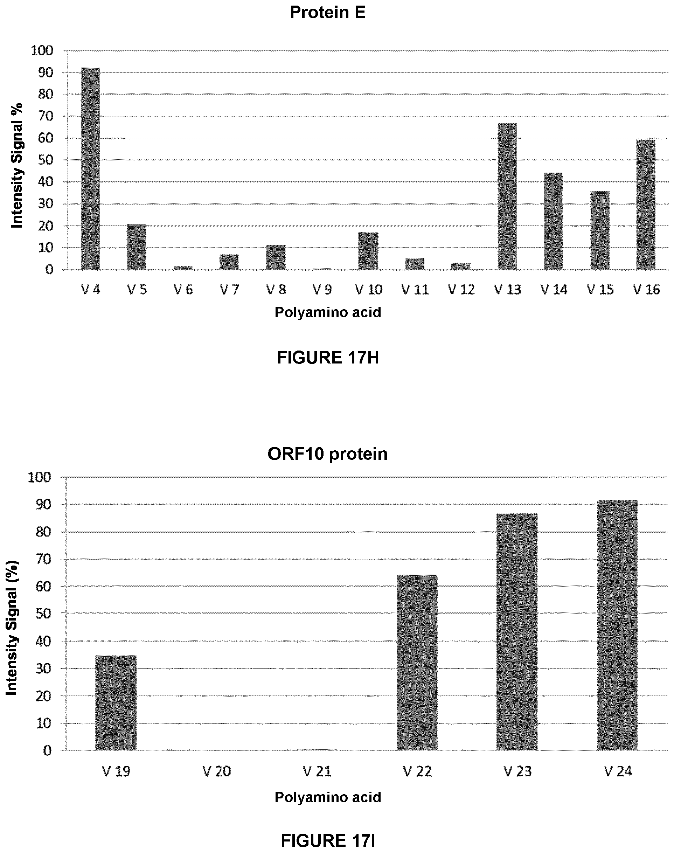

**Figure 17** - Reactivity of sera from patients with COVID, revealed with alkaline phosphatase-labeled anti-human IgG antibodies, to SARS-CoV-2 peptides synthesized on cellulose membrane (Figure 14). 17A, surface glycoprotein; 17B, ORF 3a; 17C, membrane glycoprotein; 17D, ORF 6; 17E, ORF 7; 17F ORF 8; 17G, nucleoprotein; 17H, protein E; 171, ORF 10.

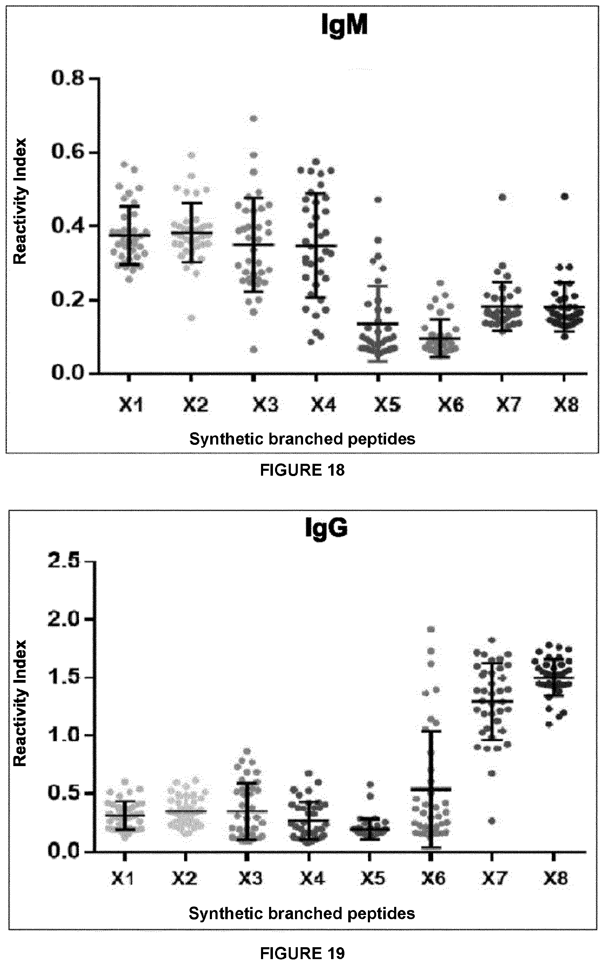

**Figure 18** - ELISA of serum from hospitalized patients (n=36) (group 3) with branched synthetic peptides (SARS-X1-SARS-X8) revealed with anti-IgM secondary antibodies.
**Figure 19** - ELISA of serum from hospitalized patients (n=36) with branched synthetic peptides (SARS-X1-SARS-X8) revealed with anti-IgG secondary antibodies.

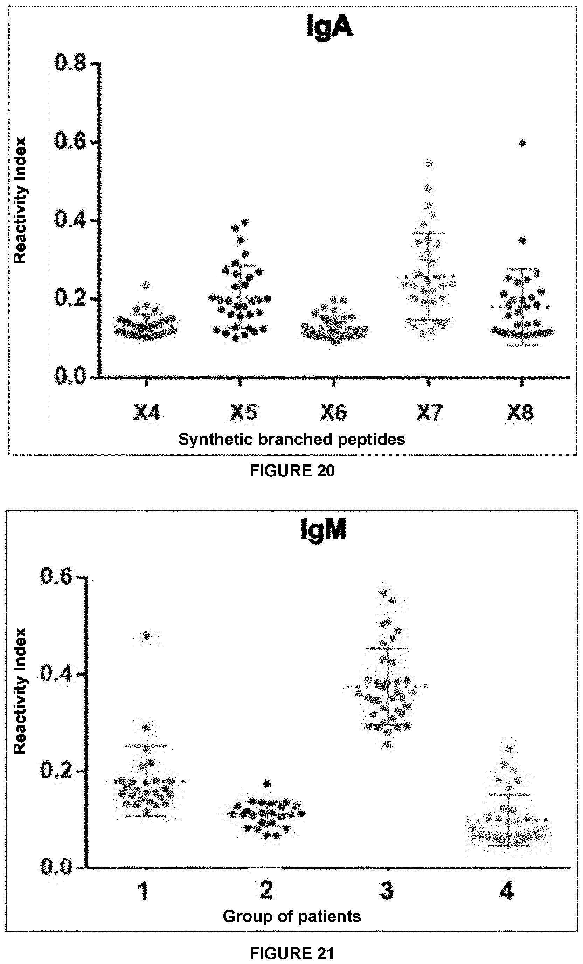

**Figure 20** - ELISA of serum from hospitalized patients (n=36) with branched synthetic peptides (SARS-X4-SARS-X8) revealed with anti-IgA secondary antibodies.
**Figure 21** - ELISA of serum from four patient groups (group 1: asymptomatic, 2: suspected; 3: hospitalized, and 4: immunoprotected) with the SARS-X3 branched synthetic peptide, revealed with anti-IgM secondary antibodies.

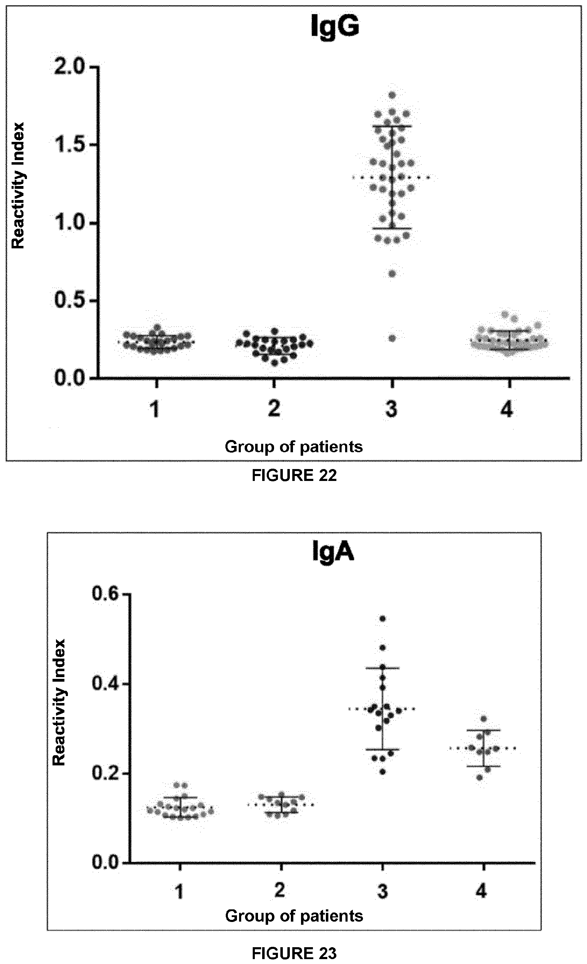

**Figure 22** - ELISA of serum from four groups of patients (group 1: asymptomatic, 2: suspected; 3: hospitalized, and 4: immunoprotected) with the SARS-X8 branched synthetic peptide, revealed with anti-IgG secondary antibodies.
**Figure 23** - ELISA of serum from four groups of patients (group 1: asymptomatic, 2: suspected; 3: hospitalized, and 4: immunoprotected) with the synthetic peptide SARS-X7, revealed with anti-IgA secondary antibodies.

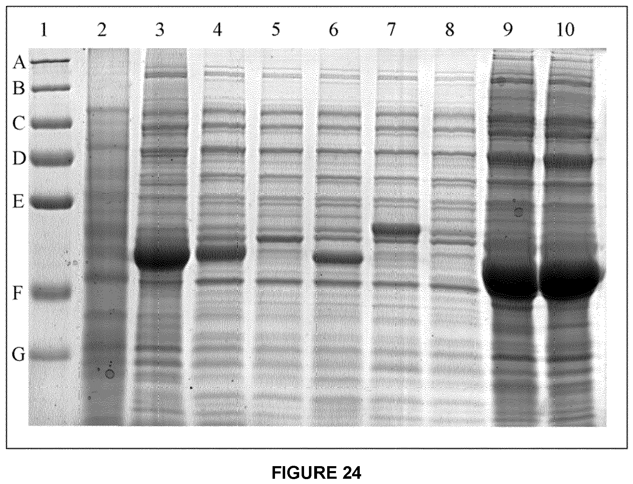

**Figure 24** - Polyacrylamide gel (SDS- PAGE) subjected to electrophoresis demonstrating the production of Ag-Covid19, Ag-COVID 19 proteins with a tail of six histidines Tx-SARS-IgM, Tx-SARS2-IgG, Tx-SARS2-G/M, Tx-SARS2-IgA, Tx-SARS2-Universal and Tx-SARS2-G5. The vertical columns 1 to 10, indicate: 1) molecular weight; 2) total bacterial extract without induction of recombinant protein; 3) total bacterial extract after induction of Ag-COVID 19 production; 4) total bacterial extract after induction of Ag-COVID19 protein production with a six histidine tail; 5) total bacterial extract after induction of Tx-SARS2-IgM protein production; 6) total bacterial extract after induction of Tx-SARS2-IgG protein production; 7) total bacterial extract after induction of Tx-SARS2-G/M protein production; 8) total bacterial extract after induction of Tx-SARS2-IgA protein production; 9) total bacterial extract after induction of Tx-SARS2-Universal protein production and 10) total bacterial extract after induction of Tx-SARS2- production The letters stand for the molecular weight standard of A) 250 kDa; B) 130 kDa; C) 100 kDa; D) 70 kDa; E) 55 kDa; F) 35 kDa and G) 25 kDa.

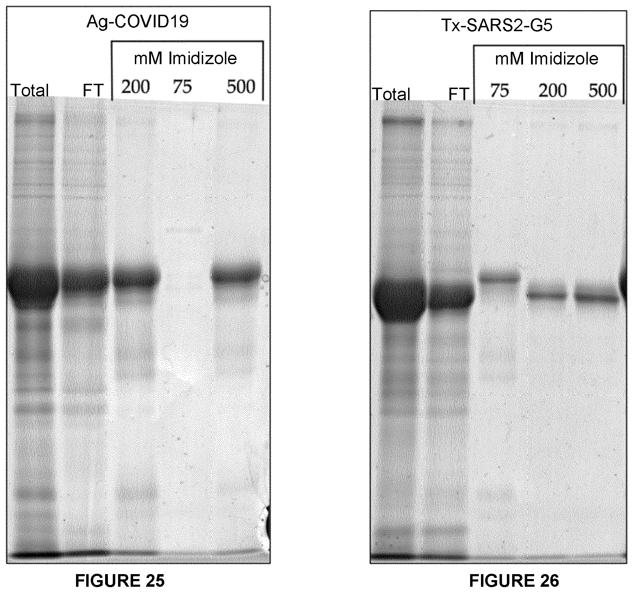

**Figure 25** - Polyacrylamide gel (SDS- PAGE) subjected to electrophoresis demonstrating the purification of Ag-COVID19 protein by affinity chromatography. (Total) Profile of a total bacterial extract after induction of production; (FT) Profile of proteins that did not bind on a nickel column; (200) Elution profile of Ag-COVID19 protein after addition of 200 mM Imidizole; (75) Elution profile of Ag-COVID19 protein after addition of 75 mM Imidizole and (500) Elution profile of Ag-COVID 19 protein after addition of 500 mM Imidizole.
**Figure 26** - Polyacrylamide gel (SDS- PAGE) submitted to electrophoresis demonstrating the purification of Tx-SARS2-G5 protein by affinity chromatography. (Total) Profile of a total bacterial extract after induction of production; (FT) Profile of proteins that do not bind on a nickel column; (200) Elution profile of Tx-SARS2-G5 protein after addition of 200 mM Imidizole; (75) Elution profile of Tx-SARS2-G5 protein after addition of 75 mM Imidizole and (500) Elution profile of Tx-SARS2-G5 protein after addition of 500 mM Imidizole.

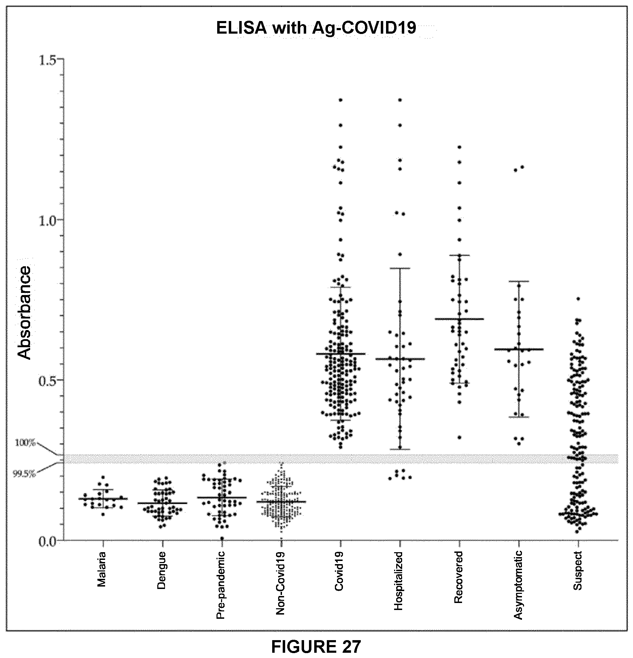

**Figure 27** - ELISA of serum from seven groups of patients infected with malaria, dengue fever or SARS-CoV-2 and still admitted (hospitalized), recovered, suspected or asymptomatic. As a control, a set of sera from healthy people, collected prior to the pandemic, was used. Ag-COVID19 protein was used in the ELISA and the binding antibodies were revealed by secondary human anti-IgG antibodies.

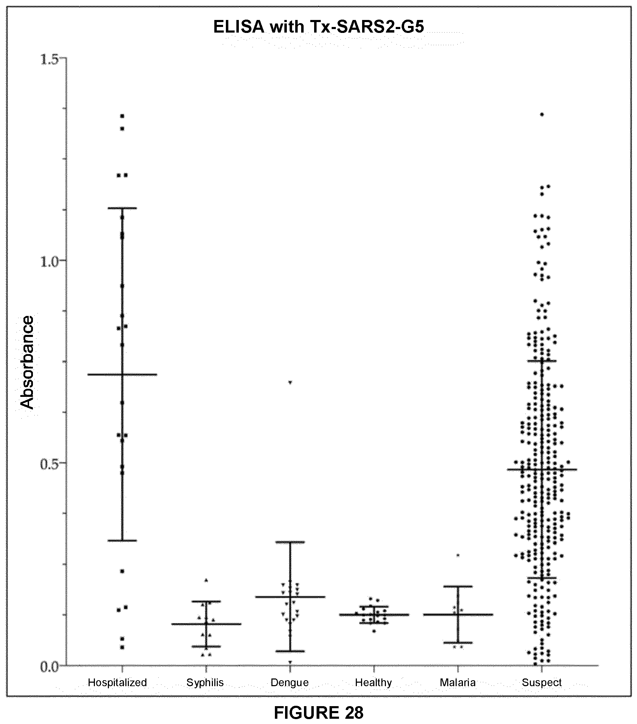

**Figure 28** - ELISA assay from serum of six groups of patients, with syphilis, malaria, dengue or SARS-CoV2, hospitalized or suspected. As a control, a set of sera from healthy people, collected prior to the pandemic, was used. Tx-SARS2-G5 protein was used in the ELISA and the binding antibodies were revealed by human anti-IgG secondary antibodies.

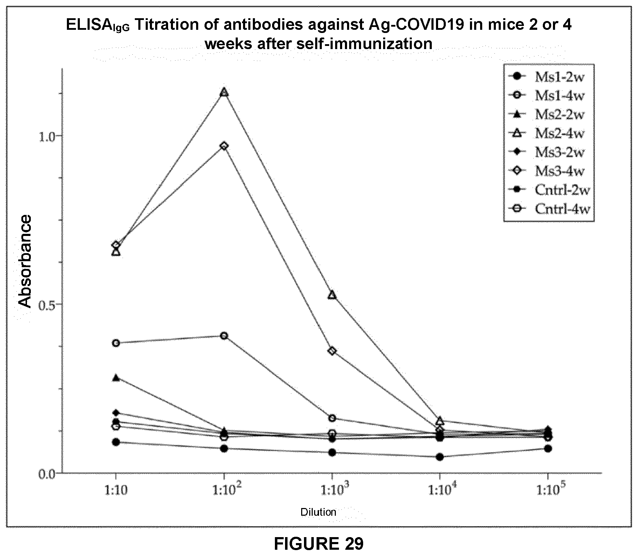

**Figure 29** - Antibody titration by ELISA against Ag-COVID19 in mice two or four weeks after their immunization with Ag-COVID19 protein.

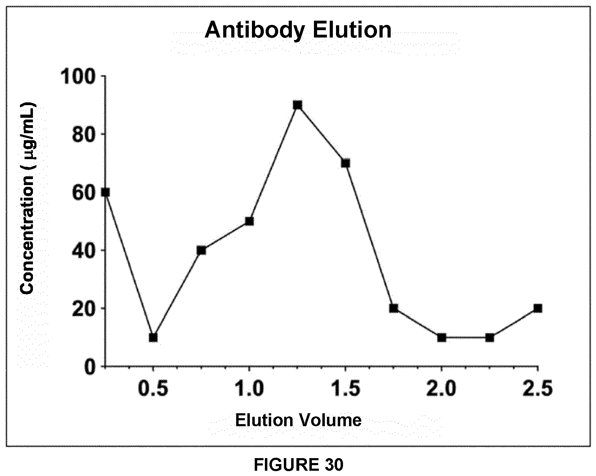

**Figure 30** - Antibody purification using Ag-COVID19 protein.

### DETAILED DESCRIPTION OF THE INVENTION

While the present invention may be susceptible to different embodiments, preferred embodiments are shown in the drawings and in the following detailed discussion with the understanding that the present description should be considered an exemplification of the principles of the invention and is not intended to limit the present invention to what has been illustrated and described herein.

Throughout this document some abbreviations will be used. Below is a list of abbreviations:
Regarding nitrogenous bases:
   C = cytosine; A = adenosine; T = thymidine; G = guanosine
   Regarding amino acids:
I = isoleucine; L = leucine; V = valine; F = phenylalanine; M = methionine; C = cysteine; A = alanine; G = glycine; P = proline; T = threonine; S = serine; Y = tyrosine; W = tryptophan; Q = glutamine; N = asparagine; H = histidine; E = glutamic acid; D = aspartic acid; K = lysine; R = arginine.

### Protein receptacle

The present invention is directed to the production and use of a protein receptacle, based on the sequence of a green fluorescent protein, herein called GFP, in a variety of methods and compositions that exploit the ability of said protein receptacle to concurrently present several different or identical exogenous polyamino acid sequences at more than four different protein sites, and furthermore, to exhibit adequate fluorescence intensities, to be efficiently expressed in cellular protein production systems, and to be useful as a reagent for research, diagnosis, or in vaccine compositions.

In a first embodiment, the invention relates to a stable protein structure that supports, at different sites, the insertion of four or more exogenous polyamino acid sequences simultaneously. In another embodiment, the protein receptacle comprises the amino acid sequence shown in SEQ ID NO: 1. In another embodiment, the protein receptacle comprises the amino acid sequence shown in SEQ ID NO: 3. In another embodiment, the protein receptacle comprises the amino acid sequence shown in SEQ ID NO: 77. In another embodiment, the protein receptacle presents insertion sites for the exogenous polyamino acid sequences in protein loops facing the external environment. In another embodiment, the insertion of the exogenous polyamino acid sequences simultaneously does not interfere with the production conditions of the receptor protein. In another embodiment, the protein receptacle contains exogenous polyamino acid sequences simultaneously for use in vaccine compositions, in diagnostics, or in the development of laboratory reagents. In another embodiment, the exogenous polyamino acid sequences did not lose their immunogenic characteristics when simultaneously inserted into the protein loops of the receptacle protein. In another embodiment, the protein receptacle comprises the exogenous polyamino acid sequences SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15 and SEQ ID NO: 16, simultaneously. In another embodiment, the protein receptacle comprises the amino acid sequence shown in SEQ ID NO:. 18. In another embodiment, the protein receptacle comprises the exogenous polyamino acid sequences SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28 and SEQ ID NO: 29, simultaneously.

In another embodiment, the protein receptacle comprises the amino acid sequence shown in SEQ ID NO: 20. In another embodiment, the protein receptacle comprises several copies of the exogenous polyamino acid sequence simultaneously, SEQ ID NO: 30. In another embodiment, the protein receptacle comprises the amino acid sequence shown in SEQ ID NO: 31. In another embodiment, the protein receptacle comprises the exogenous polyamino acid sequences SEQ ID NO: 35, SEQ ID NO: 36, SEQ ID NO: 37, SEQ ID NO: 38, SEQ ID NO: 39 and SEQ ID NO: 40, simultaneously. In another embodiment, the protein receptacle comprises the amino acid sequence shown in SEQ ID NO:. 33. In another embodiment, the protein receptacle comprises the exogenous polyamino acid sequences SEQ ID NO: 41, SEQ ID NO: 42, SEQ ID NO: 43 and SEQ ID NO: 44, simultaneously. In another embodiment, the protein receptacle comprises the amino acid sequence shown in SEQ ID NO: 45. In another embodiment, the protein receptacle comprises the exogenous polyamino acid sequences SEQ ID NO: 47, SEQ ID NO: 48, SEQ ID NO: 49 and SEQ ID NO: 50, simultaneously. In another embodiment, the protein receptacle comprises the amino acid sequence shown in SEQ ID NO: 51. In another embodiment, the protein receptacle comprises the exogenous polyamino acid sequences SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 95 and SEQ ID NO: 96, simultaneously. In another embodiment, the protein receptacle comprises the amino acid sequence shown in SEQ ID NO: 64. In another embodiment, the protein receptacle comprises the exogenous polyamino acid sequences SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74 and SEQ ID NO: 97, simultaneously. In another embodiment, the protein receptacle comprises the amino acid sequence shown in SEQ ID NO: 75. In another embodiment, the protein receptacle comprises the exogenous polyamino acid sequences, simultaneously, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87 and SEQ ID NO: 98. In another embodiment, the protein receptacle comprises the amino acid sequence shown in SEQ ID NO: 88.

In another embodiment, the protein receptacle comprises the exogenous polyamino acid sequences SEQ ID NO: 7, SEQ ID NO: 9, SEQ ID NO: 10, SEQ ID NO: 12, SEQ ID NO: 14, SEQ ID NO: 15, SEQ ID NO: 16, SEQ ID NO: 92, SEQ ID NO: 93, SEQ ID NO: 94 and SEQ ID NO: 99, simultaneously. In another embodiment, the protein receptacle comprises the amino acid sequence shown in SEQ ID NO: 90. In another embodiment, the protein receptacle comprises the exogenous polyamino acid sequences as defined in SEQ ID NO: 100, 124, 125, and 126, simultaneously; or SEQ ID NO:101, 127, 128, 129, 130, 131, 132, 133, 134, and 135, simultaneously; or SEQ ID NO: 136, 137, 138, 139, 140, 141, 142, simultaneously; or SEQ ID NO: 129, 133, 135, 137, 140, 141, 142, 143, 144, 146, 147, and 148, simultaneously; or SEQ ID NO: 103, 149, 150, 151, 152, 153, 154, 155, simultaneously; or SEQ ID NO: 104, 156, 157, 158, 159, 160, 161, 162, and 163, simultaneously; or SEQ ID NO: 136, 139, 140, 141, 142, 143, 144, 146 and 147, simultaneously. In another embodiment, the protein receptacle comprises any of the amino acid sequences shown in SEQ ID NO: 334-341.

Another embodiment of the present invention relates to the efficient expression of said protein receptacle, based on the sequence of a GFP, carrying one or two, or more than two, three or four, or more than ten exogenous polyamino acid sequences, concomitantly, at ten different sites on the receptacle protein, in a cellular system. Specifically, the present invention relates to the efficient expression of said protein receptacle presenting exogenous polyamino acid sequences at up to ten different protein sites simultaneously. More specifically, the present invention relates to the efficient expression of said receptacle carrying said exogenous polyamino acid sequences, inserted into different protein sites, without, however, losing its inherent characteristics, such as, autofluorescence.

The invention is also directed to the production and use of the protein receptacles, "Platform", "Rx" and "Tx", and their amino acid sequences (described in SEQ ID NO: 1, SEQ ID NO: 3 and SEQ ID NO: 77, respectively), of their nucleotide sequences (described in SEQ ID NO: 2, SEQ ID NO: 4 and SEQ ID NO: 78, respectively) and their amino acid sequences including the exogenous polyamino acid sequences of choice (described in SEQ ID NO: 18, SEQ ID NO: 20, SEQ ID NO: 31, SEQ ID NO:.
33, SEQ ID NO: 45, SEQID NO: 51, SEQ ID NO: 64, SEQ ID NO: 75, SEQ ID NO: 88 and SEQ ID NO: 90).

The receptacle protein can also undergo modifications necessary for the development of its uses, through the insertion of accessory elements. Sequences can be added to the receptacle protein that aid in the purification process, such as, but not limited to, polyhistidine tail, chitin-binding protein, maltose-binding protein, calmodulin-binding protein, strep-tag, and GST. Sequences for stabilization such as thiorodixin can still be incorporated into the receptor protein. Sequences that aid in any antibody detection process, such as V5, Myc, HA, Spot, FLAG sequences, can also be added to the receptacle protein.

Further, for purposes of this invention, sequences with at least about 85%, more preferably at least about 90%, 95%, 96%, 97%, 98% or 99% identity with the protein and polyamino acid receptors described herein are included, as measured by well-known sequence identity assessment algorithms such as FASTA, BLAST or Gap.

Sequences acting as target cleavage sites (catalytic site) for proteases may still be added to the protein receptacle, allowing the main protein to be separated from the above accessory elements, included strictly for the purpose of optimizing the production or purification of the protein, but not contributing to the suggested end use. These sequences, containing sites that serve as targets for proteases, can be inserted anywhere and include, but are not limited to, thrombin, factor Xa, enteropeptidase, PreScission, TEV (Kosobokova et al., Biochemistry, 8: 187-200, 2015). Yet another accessory sequence marking proteases can be AviTag, which allows specific biotinylation at a single point during or after protein expression. In this sense, it is possible to create tagged proteins by combining different elements (Wood, Current Opinion in Structural Biology, 26: 54-61, 2014).

The isolated receptacle protein can be further modified *in vitro* for different uses.

### Polynucleotide

In a first embodiment, the invention relates to a polynucleotide comprising any one of SEQ ID NO: 2, 4, 78, 17, 19, 32, 34, 46, 52, 63, 76, 89, 91, 326-333 and their degenerate sequences, capable of generating, respectively, the polypeptides defined by SEQ ID NO: 1, 3, 77, 18, 20, 31, 33, 45, 51, 64, 75, 88, 90, 334-341.

This invention also provides an isolated receptacle protein, produced by any expression system, from a DNA molecule, comprising a regulatory element containing the nucleotide sequence encoding the receptacle protein of choice.

The DNA sequence encoding for the receptacle protein differs from the DNA sequence of forms of GFP that occur in nature, in terms of the identity or location of one or more amino acid residues, either by deletion, addition, or substitution of amino acids. But, they still preserve some or all of the characteristics inherent to forms that occur in nature, such as, but not limited to, fluorescence production, characteristic three-dimensional shape, ability to be expressed in different systems, and ability to receive exogenous peptides.

The DNA sequence encoding for the receptacle protein of the present invention includes: the incorporation of preferred codons for expression by certain expression systems; the insertion of cleavage sites for restriction enzymes; the insertion of optimized sequences for facilitating expression vector construction; the insertion of facilitator sequences to house the polyamino acid sequences of choice to be introduced into the receptacle protein. All these strategies are already well known in the state of the art.

Additionally, the invention further provides added genetic elements of the nucleotide sequences encoding the receptacle proteins, such as in the sequences described in SEQ ID NO: 2, SEQ ID NO: 4 and SEQ ID NO: 78. Yet additionally, it provides such elements containing nucleotide sequences encoding the added receptacle proteins of the DNA coding for the exogenous polyamino acid sequences of choice. Such genetic elements containing the sequences described in SEQ ID NO: 17, SEQ ID NO: 19, SEQ ID NO: 32, SEQ ID NO: 34, SEQ ID NO: 46, SEQ ID NO: 52, SEQ ID NO: 63, SEQ ID NO: 76, SEQ ID NO: 89 and SEQ ID NO: 91.

Regulatory elements required for receptacle protein expression include promoter sequences for binding to RNA polymerase and translation start sequences for binding to the ribosome. For example, a bacterial expression vector must include a primer codon, a promoter suitable for the cellular system, and for translation initiation a Shine-Dalgarno sequence. Similarly, a eukaryotic expression vector includes a promoter, a start codon, a polyadenylation signal downstream process, and a termination codon. Such vectors can be obtained commercially, or built from already known state-of-the-art sequences.

### Vector

In a first embodiment, the invention relates to a vector comprising the polynucleotide as previously defined.

Transition from one plasmid to another vector can be achieved by altering the nucleotide sequence by adding or deleting restriction sites without modifying the amino acid sequence, which can be accomplished by nucleic acid amplification techniques.

### Expression Cassette

In a first embodiment, the invention relates to an expression cassette comprising the polynucleotide as previously defined.

Optimized expression in other systems can be achieved by altering the nucleotide sequence to add or delete restriction sites and also to optimize codons for alignment to the preferred codons of the new expression system, without, however, changing the final amino acid sequence.

### Cell

In a first embodiment, the invention relates to a cell comprising the vector or expression cassette as previously defined.

The invention further provides cells containing nucleotide sequences encoding the receptacle protein, or the receptacle protein added from the DNA encoding for the exogenous polyamino acid sequences of choice, to act as expression systems for the receptacle protein. The cells can be bacterial, fungal, yeast, insect, plant or even animal cells.

The DNA sequences encoding the receptacle proteins added from the coding DNA for the exogenous polyamino acid sequences of choice can also be inserted into viruses that can be used for expression and production of the receptacle protein as delivery systems, such as baculovirus, adenovirus, adenovirus-associated viruses, alphavirus, herpes virus, pox virus, retrovirus, lentivirus, but not limited to these.

There is a wide variety of methods for introducing exogenous genetic material into cells, all already well known in the state of the art. For example, exogenous DNA material can be introduced into a cell by calcium phosphate precipitation technology. Other technologies, such as technologies using electroporation, lipofection, microinjection, retroviral vectors, and other viral vector systems, such as adenovirus-associated viral systems may be used for development of this invention.

This invention provides a living organism comprising at least cells containing a DNA molecule containing a regulatory element for expression of the sequence encoding the receptacle protein. The invention is applicable for production of receptacle proteins in vertebrate animals, non-vertebrate animals, plants and microorganisms.

Expression of the receptacle proteins can be performed in, but not limited to, *Escherichia coli* cells, *Bacillus subtilis, Saccharomyces cerevisiae, Pichia pastoris, Pichia methanolica, Candida boidinii, Pichia angusta* , mammalian cells such as CHO cells, HEK293 cells or insect cells such as Sf9 cells. All known state-of-the-art prokaryotic or eukaryotic protein expression systems are capable of producing the receptacle proteins.

In one development of the present invention, a virus or bacteriophage, carrying the coding sequence for the receptacle protein, can infect a particular type of bacterial or eukaryotic cell and provide expression of the receptacle protein in that cellular system. Infection can be easily observed by detecting the expression of the receptacle protein. Similarly, a eukaryotic plant or animal cell virus carrying the sequence encoding the receptacle protein can infect a specific cell type and lead to expression of the receptacle protein in the eukaryotic cell system.

### Method for producing the protein receptacle

In a first embodiment, the invention relates to a method for producing the protein receptacle, comprising introducing into competent cells of interest the polynucleotide as previously defined; performing culture of the competent cells and performing isolation of the protein receptacle containing the exogenous polyamino acids of choice. In another embodiment, the protein receptacle is not interfered with by the insertion of various exogenous polyamino acid sequences.

Receptor proteins can also be produced by multiple synthetic biology systems. The generation of fully synthetic genes is linked to basically three linkage-based synthesis systems, widely described in the state of the art.

This invention provides methods for producing the receptacle protein using a protein expression system comprising: introducing into competent cells of interest the DNA sequence encoding for the receptacle protein plus the DNA encoding for the exogenous polyamino acid sequences of choice, culturing these cells under conditions favorable for producing the receptacle protein containing the polyamino acid sequences of choice, and isolating the receptacle protein containing the exogenous polyamino acids of choice.

The invention further provides techniques for producing the receptacle proteins containing the exogenous polyamino acids of choice. This invention presents an efficient method for expressing receptacle proteins containing the exogenous polyamino acids of choice which promotes the production of the protein of interest in large quantities. Methods for the production of the receptor proteins can be performed in different cellular systems, such as yeast, plants, plant cells, insect cells, mammalian cells, and transgenic animals. Each system can be used by incorporating a codon-optimized nucleic acid sequence, which can generate the desired amino acid sequence, into a plasmid appropriate for the particular cell system. This plasmid may contain elements, which confer a number of attributes on the expression system, which include, but are not limited to, sequences that promote retention and replication, selectable markers, promoter sequences for transcription, stabilizing sequences of the transcribed RNA, ribosome binding site.

Methods for isolating expressed proteins are well known in the state of the art, and in this regard, receptacle proteins can be easily isolated by any technique. The presence of the polyhistidine tail allows purification of the recombinant proteins after their expression in the bacterial system (Hochuli et al Bio/Technology 6: 1321-25; Bornhorst and Falke, Methods Enzymology 326:245-54).

The present invention further contemplates the choice and selection of exogenous polyamino acids. Receptor proteins can house different polyamino acid sequences from different sources, ranging from vertebrate animals including mammals, invertebrates, plants, microorganisms or viruses in order to promote their expression, presentation or use in different media. Different methods of polyamino acid sequence selection can be used, such as specific selection by binding affinity to antibodies or other binding proteins, by epitope mapping, or other techniques known in the state of the art.

Polyamino acid sequences are sequences of five to 30 amino acids that sensitively or specifically represent an organism, for any purpose described in this patent application. Polyamino acid sequences may represent, but not limited to, these examples: (i) linear B-cell epitopes; (ii) T-cell epitopes; (iii) neutralizing epitopes; (iv) protein regions specific to pathogenic or non-pathogenic sources; (v) regions proximal to active sites of enzymes that are not normally targets of an immune response. These epitope regions can be identified by a wide variety of methods that include, but are not limited to: spot synthesis analysis, random peptide libraries, phage display, software analysis, use of X-ray crystallography data, epitope databases, or other state-of-the-art methods.

Insertion of exogenous polyamino acid sequences into the receptacle proteins at previously identified sites surprisingly did not disrupt or interfere with the inherent characteristics of the protein. Eight introduction sites for exogenous polyamino acid sequences have been identified in the receptacle proteins (Kiss et al. Nucleic Acids Res 34: e132, 2006; Pavoor et al. Proc Natl Acad Sci U S A 106: 11895-900, 2009; Abedi et al, Nucleic Acids Res 26: 623-30, 1998; Zhong et al. Biomol Eng 21: 67-72, 2004).

These introduction sites can house one, two, or more different exogenous polyamino acid sequences *in tandem* at the same insertion site, greatly amplifying the expression of different polyamino acid sequences.

### Method of pathogen identification or in vitro disease diagnosis

In a first embodiment, the invention relates to a method for pathogen identification or *in vitro* disease diagnosis characterized in that it uses the receptacle protein as previously defined. In another embodiment, the method is for diagnosing Chagas disease, rabies, pertussis, yellow fever, Oropouche virus infections, Mayaro, IgE hypersensitivity, *D. pteronyssinus* allergy, or COVID-19.

### Use of the protein receptacle

In a first embodiment, the invention relates to the use of said protein receptacle as a laboratory reagent. In a further embodiment, the invention relates to the use of said protein receptacle for the production of a vaccine composition for immunization against Chagas disease, rabies, pertussis, yellow fever, Oropouche virus infections, Mayaro virus infections, IgE hypersensitivity, *D. pteronyssinus* allergy or COVID-19.

Also, the present invention relates to a system for concomitant expression of multiple polyamino acid sequences using a single protein receptacle, based on GFP, useful for different uses, such as as a reagent for research, for diagnosis or for vaccine compositions. Specifically, said expression system can act as a useful research reagent to purify antibodies by binding on epitopes. Additionally, said expression system can also act for use in immunological and/or molecular techniques for diagnosing chronic and infectious diseases. Also, said expression system may be useful for use in vaccine compositions containing multiple antigens for immunization of animals and humans.

Also an embodiment of the present invention is the method for concomitant production of multiple polyamino acid sequences using a single protein receptacle, based on GFP, useful for different uses, such as as a reagent for research, for diagnostics or for vaccine compositions.

The invention further presents several uses for the receptacle protein. These uses include, but are not limited to, the use of the protein receptacle (i) as a reporter molecule in cellular screening assays, including intracellular assays; (ii) as a protein for presentation of random or selected peptide libraries; (iii) as an antigen-presenting protein as a reagent for development of *in vitro* immunological diagnostic tests, in general, and for infectious, parasitic or other immunological diseases; (iv) as an antigen-presenting protein useful for selection, capture, screening or purification of binding substances, such as, antibodies; (v) as an antigen-presenting protein for vaccine composition; (vi) as a protein containing antibody sequences for binding to antigens; (vii) as an antigen-presenting protein with passive immunization activity.

Receptor proteins can be useful as a vaccine composition by, unusually, having:
(a) a large number, simultaneously, of immune response-inducing polyamino acid sequences, and
(b) a non-immune response-inducing protein core.

### Diagnostic kit

In a first embodiment, the invention relates to a diagnostic kit comprising the protein receptacle as previously defined.

Finally, the present invention is described in detail through the examples given below. It is necessary to stress that the invention is not limited to these examples, but that it also includes variations and modifications within the limits within which it can be developed. It is also worth mentioning that the access to all biological sequences of the Brazilian genetic heritage are registered in SISGEN, under the registration number AC53976.

### EXAMPLES

### EXAMPLE 1- Receptacle protein construction

The amino acid sequences between different examples of protein fluorescent green, eGFP (GenBank: L29345.1; UniProtKB - P42212), Cycle-3 (GenBank: CAH64883.1), SuperFolder (GenBank: AOH95453.1), Split (Cabantous et al. Science Reports 3: 2854, 2013), Superfast (Fisher & DeLisa. PLoS One 3: e2351, 2008) were used to construct new proteins for the present invention application.

Sequence alignments and comparisons were performed by Intaglio software (Purgatory Design, V3.9.4). From these data, several changes were made so that the receptacle proteins could achieve the required characteristics.

Alterations were made to create restriction enzyme action sites. The insertion of these sites was designed so that there would be no change in the physicochemical characteristics of the GFP protein and thus not affect the properties or qualities described in this patent application. Additionally, the insertion of these restriction sites adds further properties to the receptor proteins by allowing potential uses in genetic engineering methods and processes that would allow genetic manipulation of these proteins for incorporation of various peptides into different regions of the protein.

The nucleotide sequence of the GFP protein was manipulated to introduce or replace nucleotides in order to create new restriction enzyme sites. Thus, two new receptacle proteins were produced, the "Platform" protein and the "Rx" protein.

The changes in nucleotide sequences based on the eGFP protein, gave rise to the following amino acid changes in the receptacle proteins:
Platform" Protein
Position 16, amino acid I;
Position 28, amino acid F;
Position 30, amino acid R;
Position 39, amino acid I;
Position 43, amino acid S;
Position 72, amino acid S;
Position 99, amino acid Y;
Position 105, amino acid T;
Position 111, amino acid E;
Position 124, amino acid V;
Position 128, amino acid I;
Position 145, amino acid F;
Position 153, amino acid T;
Position 163, amino acid A;
Position 166, amino acid T;
Position 167, amino acid V;
Position 171, amino acid V;
Position 205, amino acid T;
Position 206, amino acid I;
Position 208, amino acid L.

Rx" protein
Position 16, amino acid V;
Position 28, amino acid S;
Position 30, amino acid R;
Position 39, amino acid I;
Position 43, amino acid T;
Position 72, amino acid A;
Position 99, amino acid S;
Position 105, amino acid K;
Position 111, amino acid V;
Position 124, amino acid V;
Position 128, amino acid T;
Position 145, amino acid F;
Position 153, amino acid T;
Position 163, amino acid A;
Position 166, amino acid T;
Position 167, amino acid V;
Position 171, amino acid V;
Position 205, amino acid T;
Position 206, amino acid V;
Position 208, amino acid S.

Alternative amino acid substitutions can still be selected, for all the receptacle proteins, at positions:
Position 39, amino acid N;
Position 72, amino acid S;
Position 99, amino acid S;
Position 105, amino acid Y or K;
Position 206, amino acid I;
Position 208, amino acid L.

The presence of some mutations can influence biochemical characteristics of the protein. The S30R mutation positively influences the protein's coiling characteristics; the Y145F and 1171V mutations prevent translation of undesirable intermediates; the A206V or I mutations reduce the possibility of aggregation of nascent proteins.

Other changes were made to the receptacle proteins "Platform" and "Rx" from the inclusion of new nucleotide codons to create new restriction sites, which can be seen in Table 1 (below):

**Table 1**

| Amino acid sequence variation | Substituted nucleotides | Nucleotides introduced | Restriction enzyme to be used |
|---|---|---|---|
| D102_D103insV | - | GTC | *AatII* |
| G116_D117insT | - | ACC | *KpnI* |
| L137_G138insK | - | AAG | *AfIII* |
| D191_P192insG | - | GGT | *RsrII* |
| E213_K214insL | - | CTC | *SacI* |

Additionally, the nucleotide sequence of the receptacle proteins "Platform" and "Rx" harbors two additional restriction sites for NdeI and NheI, at the amino 5' terminus of the protein, from the insertion of the sequence CATATGGTGGCTAGC (SEQ ID NO: 5) and another two restriction sites for EcoRI and XhoI, at the 3' carboxy terminus, from the insertion of the sequence GAATTCTAATGACTCGAG (SEQ ID NO: 6). In addition, two stop codons and a polyhistidine tail at the amino-terminus have been incorporated into the receptor proteins.

The amino acid sequence of the "Platform" protein can be seen in SEQ ID NO: 1 and its corresponding sequence in nucleotides is described in SEQ ID NO: 2.

The amino acid sequence of the "Rx" protein can be seen in SEQ ID NO: 3 and its corresponding sequence in nucleotides is described in SEQ ID NO: 4.

By creating restriction sites without altering the three-dimensional structure of the original protein, 10 new insertion sites for exogenous polyamino acid sequences were allowed to appear in the protein. Each new insertion site will be referred to here as position 1 to position 10.

The locations, in the nucleotide and amino acid sequences, of positions 1 to 10, of the receptacle proteins "Platform" and "Rx" are shown in Table 2 (below):

**Table 2**

| Position in the protein receptacle | Position in the amino acid sequence |
|---|---|
| 1 | MVAS |
| 2 | TTGKLPVP |
| 3 | FKDVDG |
| 4 | FEGTDTL |
| 5 | TDFKEDGNILKGHKL |
| 6 | DKQKN |
| 7 | ED |
| 8 | PIGDGPVLLPDN |
| 9 | SKDPNELKRD |
| 10 | DELYKEF |

From alignments of the amino acid sequences of different GFP proteins, a consensus amino acid sequence, designated CGP (Dai et al. Protein Engineering, Design and Selection 20(2): 69-79 2007).

This fluorescent protein although exhibiting high stability, was improved by directed evolution to exhibit greater stability relative to CGP (Kiss et al. Protein Engineering, Design & Selection 22(5): 313-23, 2009). However, the enhanced protein, due to the presence of three mutations, was prone to aggregation. Further mutations were also incorporated based on an analysis of its crystal structure resulting in the elimination of aggregation and production of the protein called Thermal Green Protein(TGP) (Close et al. Proteins 83(7): 1225-37, 2015). When used as a protein receptacle, the sequence is called "Tx". The amino acid sequence of the "Tx" protein can be seen in SEQ ID NO: 77 and its corresponding sequence in nucleotides is described in SEQ ID NO: 78.

The nucleotide and amino acid sequence locations of positions 1 to 13 of the "Tx" receptacle proteins are shown in Table 3 (below). At the amino and carboxy-terminal ends of the receptacle proteins, two polyamino acid sequences can be inserted consecutively at both ends. Thus, insertion sites 1a and 1b and 13a and 13b are characterized in the amino and carboxy-terminal regions, respectively.

**Table 3**

| Position in the protein receptacle | Position in the amino acid sequence |
|---|---|
| 1 | GAHASVIKPE |
| 2 | NG |
| 3 | YE |
| 4 | GAPLPFS |
| 5 | AFPE |
| 6 | EDQ |
| 7 | GD |
| 8 | NFPPNGPVMQKK |
| 9 | DG |
| 10 | EGGG |
| 11 | KKDVRLPDA |
| 12 | DKDYN |
| 13 | RYSG |

### EXAMPLE 2 - Construction of the PlatCruzi protein

The "Platform" receptacle protein was genetically engineered to harbor Trypanosoma cruzi epitopes, which herein we call the PlatCruzi platform. The gene corresponding to the PlatCruzi protein, here called the PlatCruzi gene, is described in the SEQ ID NO nucleotide sequence:17.

Polyamino acid sequences originating from *T. cruzi* were selected from the available state-of-the-art literature, considering experimental data on specificity and sensitivity for diagnostic tests for Chagas disease (Peralta JM et al. J Clin Microbiol 32: 971-974, 1994; Houghton RL et al. J Infect Dis 179: 1226-1234, 1999; Thomas et al. Clin Exp Immunol 123: 465-471, 2001; Rabello etal, 1999; Gruber & Zingales, Exp Parasitology, 76(1): 1-12, 1993; Lafaille et al, Molecular Biochemistry Parasitology, 35(2): 127-36, 1989). Ten polyamino acid sequences were selected for insertion into the ten insertion sites in the "Platform" protein, here called TcEp1 to TcEp10, as shown in Table 4 (below).

After the selection of the *T. cruzi* polyamino acid sequences, the synthetic gene corresponding to the PlatCruzi protein was produced by chemical synthesis, by the ligation gene synthesis methodology and inserted into plasmids for experimentation. The amino acid sequence corresponding to the PlatCruzi gene, containing the epitopes TcEp1 to TcEp10, is described in SEQ ID NO: 18.

**Table 4**

| Epitope | Position in the protein | Original epitope protein | Sequence | SEQ ID no. |
|---|---|---|---|---|
| TcEpl | 1 | KMP11 | KFAELLEQQKNAQFPGK | SEQ ID no. 7 |
| TcEp2 | 2 | TcE | KAAAAP A | SEQ ID no. 8 |
| TcEp3 | 3 | TcE | KAAIAP A | SEQ ID no. 9 |
| TcEp4 | 4 | PEP-2 | GDKPSPFGQAAAADK | SEQ ID no. 10 |
| TcEp5 | 5 | CRA | KQKAAEATK | SEQ ID no. 11 |
| TcEp6 | 6 | TcD-1 | AEPKPAEPKS | SEQ ID no. 12 |
| TcEp7 | 7 | TcD-2 | AEPKSAEPKP | SEQ ID no. 13 |
| TcEp8 | 8 | TcLo 1.2 | GTSEEGSRGGSSMPS | SEQ ID no. 14 |
| TcEp9 | 9 | B13 | SPFGQAAAGDK | SEQ ID no. 15 |
| TcEp10 | 10 | CRA | KQRAAEATK | SEQ ID no. 16 |

### EXAMPLE 3 - - Development of the PlatCruzi protein

The synthetic gene was introduced into pET28a plasmids using the restriction sites for the enzymes Ndel and Xhol by state-of-the-art molecular biology methods. In order to identify whether the synthetic gene matched the sequence designed for PlatCruzi, a DH5α strain of *Escherichia coli* was transformed and the plasmid material analyzed by restriction enzyme digestion techniques and then sequenced.

The sequencing method used was the enzymatic, dideoxy or chain termination method, which is based on the enzymatic synthesis of a complementary strand, the growth of which is stopped by the addition of a dideoxynucleotide (Sanger et al., Proceeding National Academy of Science, 74(12): 5463-5467, 1977). This methodology consists of the following steps: Sequencing reaction (DNA replication in 25 cycles in the thermocycler), DNA precipitation by isopropanol/ethanol, denaturation of the double strand (95 °C for 2 min) and reading of the nucleotide sequence was performed in the ABI 3730XL automated sequencer (ThermoFischer SCIENTIFIC) (Otto et al,. Genetics and Molecular Research 7: 861-871, 2008). The analyses of the obtained sequences were done with the help of the 4Peaks program (Nucleobytes; Mac OS X, 2004). The primers from the pET-28a vector (T7 Promoter and T7 Terminator) were used in the reaction.

The analyzed plasmid clone harboring the correct sequence for PlatCruzi was transferred to E. *coli,* strain BL21, in order to produce the PlatCruzi protein.

The BL21 strain can express T7 RNA polymerase when induced by Isopropyl β-D-1-thiogalactopyranoside (IPTG). The strain was grown overnight in LB medium and then reseeded in the same medium, with kanamycin (30 µg/ml) added, on a shaker at 200 rpm until it reached an optical turbidity density of 0.6-0.8 (600 nm). Then, IPTG (q.s.p. 1 mM) is added to the culture and the same culture conditions are maintained for another 3 h.

The culture was subjected to centrifugation and the pellet resuspended in urea buffer (100 mM NaH2PO4, 10 mM Tris-base, 8 M urea, pH 8.0). The solution was subjected to HisTrapTM affinity column chromatography, 1 mL, (GE Healthcare Life Sciences) which allows high-resolution purification of histidine-tagged proteins. The supernatant was applied to a nickel affinity column (HisTrapTM, 1 mL, GE Healthcare Life Sciences) at a flow rate of 0.5 mL per minute, previously equilibrated in buffer A (50 mM Tris-HCl, pH 8.0, 100 mM NaCl and 5 mM imidazole). After binding, the resin was washed with 10 mL of buffer A. The protein was eluted in a 100% gradient of buffer B (50 mM Tris-HCl, pH 8.0, 100 mM NaCl, and 500 mM imidazole) at a flow rate of 0.7 mL/min for 45 minutes. The purification of PlatCruzi was followed at 280 nm (black line) and is shown in Figure 1A. The percentage of imidazole is marked in red. The protein was eluted in a volume of approximately 19 to 25 ml.

Aliquots of the recombinant proteins (1 (µg/well) were subjected to SDS-containing polyacrylamide gel electrophoresis (SDS-PAGE) (Laemmli, Nature 227: 680-685, 1970). Concentration *gels(stacking gel)* and separation gels*(running gel)* were prepared at an acrylamide concentration of 4% and 11%, respectively (table 5, below). Samples were prepared under denaturing conditions in 62.5 mM Tris-HCl buffer, pH 6.8, 2% SDS, 5% β-mercaptoethanol, 10% glycerol and boiled at 95 °C for 5 min (Hames BD, Gel electrophoresis of proteins: a practical approach. 3. ed. Oxford. 1998). After electrophoresis, the proteins were detected by staining with comassie blue *R250* (Bio-Rad, USA).

The *KaleidoscopeTM Prestained Standards* marker was used as a molecular weight reference (Bio-Rad, USA). Figure 1B, shows the purification of PlatCruzi protein by affinity chromatography using a nickel-agarose column using a *Äktapurifier* liquid chromatography system.

**Table 5: Volume and concentration of reagents used to prepare the 4% sample concentration gel and for the 11% separation gel.**

| **4% concentration gel** | | **Separation Gel 11** | |
|---|---|---|---|
| **Reagents** | **Volume (mL)** | **Reagents** | **Volume (mL)** |
| H2O | 1.63 | H2O | 1.44 |
| 0.5M Tris-HCl, pH 6.8 | 0.833 | 1.5M Tris-HCl, pH8.8 | 1.4 |
| 30% Acrylamide/ 0.8% Bisacrylamide | 0.5 | 30% Acrylamide/ 0.8% Bisacrylamide | 2 |
| 10% SDS (sodium duodecyl sulfate) | 0.0333 | 10% SDS (sodium duodecyl sulfate) | 0.055 |
| 10% Ammonium Persulfate | 0.013 | 10% Ammonium Persulfate | 0.015 |
| 80% Glycerol TEMED | 0.333 0.0067 | 80% Glycerol TEMED | 0.55 0.0075 |

### EXAMPLE 4 - Development of an ELISA from PlatCruzi

The performance of PlatCruzi protein was evaluated against a panel of reference biological samples and individuals affected by *T. cruzi* infections. PlatCruzi protein, in carbonate/bicarbonate buffer solution (50 mM, pH 9.6), was added to a 96-well ELISA plate in the amounts of 0.1, 0.25, 0.5 and 1.0 µg/orifice at 4 °C for 12-18 h. The wells were washed with saline-phosphate buffer (PBS) solution added Tween 20 (PBS-T, 10 mM sodium phosphate - Na3PO4, 150 mM sodium chloride - NaCl and 0.05% Tween-20, pH 7.4) and then incubated with 1x PBS buffer containing 5% (weight/volume) dehydrated skim milk for 2 h at 37 °C.

The wells were then washed 3 times with PBS-T buffer and incubated with the reference biological samples TCl (IS 09/188) or TCII (IS 09/186) (World Health Organization) diluted 2,4, 8, 16, 32 and 64 times for 1 h at 37°C. After the incubation period, the wells were washed three times with PBS-T and then incubated with alkaline phosphatase-labeled human IgG antibody at a dilution of 1:5000 for 1 h at 37°C. The wells were washed again three times with PBS-T buffer and the substrate para-nitrophenylphosphate (PNPP, 1mg/mL, ThermoFischer SCIENTIFIC) was added. After 30 minutes and under shelter from light, the absorbance was measured in an ELISA plate reader at 405 nm.

The results showed satisfactory responses in all dilutions of TCI and TCII reference biological samples used regardless of the amount of PlatCruzi used (Figure 2A and 2B). The results strongly support the use of PlatCruzi for detection of *T. cruzi* infections caused by any of the six *DTUs(discrete typing units),* covering the entire geographic range of circulating *T*. *cruzi* strains. The same performance can be observed when using sera from Chagas disease patients, with low and high antibody detection titers (Figure 3).

Elisa plates containing 500ng (in 0.3M Urea, pH 8.0) of PlatCruzi were prepared as previously described and, after three washes with PBS-T, were incubated with sera from four patients with high anti-T. *cruzi* antibody indices (6C-CE, 9C-CE, 15C-CE, and 12-SE) and from four patients with low anti*-T.cruzi* antibody indices (3C-PB, 6C-PB, 16C-PB, and 17C-PB) at different dilutions: 1:50, 1:100, 1:250, 1:500, 1:1000 for 1 h at 37 °C. After this period, the wells were washed three times with PBS-T and incubated with alkaline phosphatase-labeled human IgG antibody at a dilution of 1:5000 for 1 h. The wells were washed again three times with PBS-T buffer and the substrate para-nitrophenylphosphate (PNPP, 1mg/mL, ThermoFischer SCIENTIFIC) was added. After 30 minutes, the absorbance was measured in an ELISA plate reader at 405 nm.

The results showed that for sera with low antibody titers, reading signals at dilutions 1:50, 1:100 and 1:250 were unequivocally above the threshold reached by the negative control, evidencing the potential of the PlatCruzi platform for detection of anti-T. *cruzi* antibodies in both high and low antibody patient sera.

The use of patient serum samples for experimental purposes was approved by the Ethics Committee of Fiocruz, as per authorization CEP/IOC - CAAE: 52892216.8.0000.5248.

### EXAMPLE 5 - Sensitivity and specificity of PlatCruzi ELISA

The Elisa plates developed in the previous example containing 500 ng (0.3 M Urea, pH 8.0) of PlatCruzi were incubated with 71 sera from patients diagnosed for *T.* cruzi, plus 18 sera from patients diagnosed for leishmaniasis (negative for T. *cruzi),* 20 sera diagnosed for dengue (negative for *T. cruzi),* and 39 negative sera (other infections and uninfected individuals) at a dilution of 1:250 for 1 h at 37 °C. After this period, the plates were subjected to washing and antibody labeling, developing and reading processes as previously performed.

The results pointed to excellent sensitivity and specificity using the PlatCruzi platform (Figure 4) from the receiver operating characteristic (ROC) curve correlation analysis. No false negative results were observed for the sera previously identified as positive for *T. cruzi;* as well as no false positives were observed for the other sera known to be negative for *T. cruzi,* including those positive for other infectious diseases. Both the sensitivity and specificity indices were 100%.

### EXAMPLE 6 - Development of RxRabies2 protein

The "Rx" protein was tested for its performance and ability to express epitopes from other microorganisms, including viruses. The literature points to a significant number of specific polyamino acid sequences that can be used as targets for neutralizing antibodies. However, small variations in the sequences observed between viral strains can interfere with neutralization. Thus, a thorough study of the best polyamino acid sequences to use requires a great deal of knowledge of the biology of the virus and the epidemiology of its interaction with its host.

Rabies virus polyamino acid sequences were selected from the available state-of-the-art literature, considering experimental data on specificity and sensitivity for the diagnosis of the disease caused by rabies virus (Kuzmina et al., J Antivir Antiretrovir 5:2: 37-43, 2013; Cai et al, Microbes Infect 12: 948-955, 2010).

Ten polyamino acid sequences were selected for insertion into the ten insertion sites in the Rx protein, here called RaEp1 to RaEp10, as below. Combining the sequence of these polyamino acid sequences with the Rx protein gave rise to the RxRabies2 protein. The gene corresponding to the RxRabies2 protein, here called the RxRabies2 gene, is described in the SEQ ID NO nucleotide sequence: 19. The amino acid sequence corresponding to the RxRabies2 gene which contains the polyamino acid sequences RaEp1 to RaEp10, is described in SEQ ID NO: 20.

**Table 6**

| Polyamino acid sequences | Position in the protein | Original epitope protein | Sequence | SEQ ID no. |
|---|---|---|---|---|
| RaEp 1 | 1 | antigenic site 1 | CKLKLCGVLGL | SEQ ID no. 21 |
| RaEp 2 | 2 | antigenic site 1 | CKLKLCGCSGL | SEQ ID no. 22 |
| RaEp 3 | 3 | antigenic site 1 | CKLKLCGVPGL | SEQ ID no. 23 |
| RaEp 4 | 4 | - | VDERGLYK | SEQ ID no. 24 |
| RaEp 5 | 5 | - | WVAMQTSN | SEQ ID no. 25 |
| RaEp 6 | 6 | antigenic site III | KSVRTWNEI | SEQ ID no. 26 |
| RaEp 8 | 8 | g5 antigenic site | LHDFHSD | SEQ ID no. 27 |
| RaEp 9 | 9 | g5 antigenic site | LHDFRSD | SEQ ID no. 28 |
| RaEp 10 | 10 | g5 antigenic site | LHDLHSD | SEQ ID no. 29 |

A synthetic gene containing a sequence coding for the Rx protein and sequences coding for the polyamino acid sequences described in Table 6 above has been synthesized.

The synthetic gene was introduced into pET28a plasmids using the restriction sites for the enzymes Ndel and Xhol by state-of-the-art molecular biology methods. In order to identify whether the synthetic gene matched the sequence designed for RxRabies2, a DH5α strain of *Escherichia coli* was transformed and the plasmid material analyzed by restriction enzyme digestion and then sequencing techniques, performed in the same way as described in PlatCruzi.

The analyzed plasmid harboring the correct sequence for RxRabies2 was transferred to E. *coli,* strain BL21, in order to produce the RxRabies2 protein. The BL21 strain can express T7 RNA polymerase when induced by Isopropyl β-D-1-thiogalactopyranoside (IPTG). The BL21 strain was grown overnight in LB medium and then reseeded in the same medium with kanamycin (30 µg/ml) on a shaker at 200 rpm until it reached an optical turbidity density of 0.6-0.8 (600 nm). Then, IPTG (q.s.p. 1 mM) is added to the culture and the same culture conditions are maintained for another 3 h at 37 °C.

The culture was subjected to centrifugation and the *pellet* resuspended in urea buffer (100 mM NaH2PO4, 10 mM Tris-base, 8 M urea, pH 8.0). The solution was subjected to HisTrapTM affinity column chromatography, 1 mL, GE Healthcare Life Sciences, which allows high-resolution purification of histidine-tagged proteins, at a flow rate of 0.5 mL per minute, previously equilibrated in buffer A (50 mM Tris-HCl, pH 8.0, 100 mM NaCl and 5 mM imidazole). After binding, the resin was washed with 10 mL of buffer A. The protein was eluted in a 100% gradient of buffer B (50 mM Tris-HCl, pH 8.0, 100 mM NaCl, and 500 mM imidazole) at a flow rate of 0.7 mL/min for 45 minutes.

RxRabies2 production was analyzed in three different culture volumes; 3, 25 and 50 ml. As seen in Table 7 (below), the expression of RxRabies2 was 123 µg / ml on average.

**Table 7**

| Protein | Amount of purified protein (µg/mL) | | | |
|---|---|---|---|---|
| | 3 mL culture | 25 mL culture | 50 mL culture | Average |
| RxRabies2 | 190 | 132 | 46 | 123 |

### EXAMPLE 7 - - RxRabies2 protein as vaccine composition

The RxRabies2 protein was produced as described in the previous example. 100 µg of protein was suspended in Freud's incomplete adjuvant (0.5 mL) and inoculated, intramuscularly, into the quadriceps of two 6-month-old male New Zealand rabbits. Rabbits were reinoculated seven and fourteen days after the initial inoculation with RxRabies protein suspended in PBS (100 µg/0.5 mL).

After 21 days of inoculation of the first dose of the vaccine composition, blood was collected from the animals. Plasma was collected by centrifugation and subjected to affinity purification by binding to Rx-Rabies2 protein adsorbed to the surface of a nitrocellulose membrane.

The nitrocellulose membrane containing the Rx-Rabies2 protein was prepared as described below in order to isolate it from potential contaminants. After electrophoresis, the proteins were transferred to a nitrocellulose membrane using state-of-the-art Western blot techniques.
preparation of the 11% SDS-PAGE gel, as described in the previous example and in Table 5;
application of 10 µg of the Rx-Rabies2 protein on an 11% SDS-PAGE gel (Table 5) and submission to an electrophoretic current of 100 volts for approximately 2 hours;
transfer of proteins to nitrocellulose membrane: proteins were transferred to nitrocellulose membranes using *Trans-Blot Cell* (Bio-Rad, USA) with transfer buffer (25 mM Tris base, 192 mM glycine and 20% methanol) for one hour at 100V;
staining with Ponceau S red (Ponceau S 0.1%, acetic acid 5%) to confirm the presence of the recombinant protein.

The membrane was cut so as to specifically obtain a piece with only the RxRabies2 protein;
the membranes were then decolored in distilled water and left in TBS (0.1%) for 12 to 18 hours*(overnight);*
incubation with blocking solution (25 mM Tris-HCl, 125 mM NaCl pH 7.4 (TBS) containing 0.05% (v/v) Tween 20 (TBS-T) and 5% (w/v) skim milk dehydrate) overnight, then the membranes were incubated in blocking solution again for one hour and then three washes with TBS-T for 5 minutes each and three more 5-minute washes with TBS.

Sera from the immunized rabbits were diluted 1:500 in TBS and 10ml were placed in contact with the nitrocellulose membrane segments containing the RxRabies2 protein for 1 h under stirring and then washed three times for 5 min with TBS-T and again washed 3 times for 5 min with TBS. Then, the specifically bound antibodies were released by adding 1 ml of 100 mM glycine (pH 3.0). The pH of the solution was raised to 7 by adding 100 µl of 1M Tris (pH 9.0) to purify the rabbit antibodies. The purification of antibodies that bind specifically to antigens is an important step in using these antibodies for therapy, as it allows for a drastic reduction in the amount to be administered while minimizing potential adverse effects.

Different extracts were used to demonstrate the specific ability of the produced rabbit antibodies to bind to RxRabies2. The following were used:
crude bacterial extract with Rx protein expression, obtained using the same conditions as mentioned in PlatCruzi;
rxRabies2 protein in the crude bacterial extract and purified at 1x and 0.5x concentrations;
purified Platcruzi protein at 1x and 0.5x concentrations.

The potential ligands were subjected to polyacrylamide gel electrophoresis (11% SDS-PAGE, Table 5), as described previously, and then transferred to nitrocellulose membrane and subjected to Western blot, the details of which have already been described for RxRabies2.

The membranes were incubated for one hour with the purified anti-RxRabies2 serum as described above, under agitation. Then three 5-minute washes with TBS-T and three 5-minute washes with TBS were done again. Subsequently, the membranes were incubated for one hour with the secondary anti-rabbit IgG antibodies with peroxidase diluted at 1:10,000. After incubation with the secondary antibody, three washes with TBS-T for 5 minutes and three washes with TBS for 5 minutes were performed. The development was performed with the aid of the SigmaFast^{™} DAB Peroxidase Substrate Tablet.

The results showed the specific binding of rabbit antibodies produced by inoculating RxRabies2 to ligands containing rabies virus 2 proteins. Figure 5 shows read only bands in lanes 2, 4 and 6 which contain the crude bacterial extract containing the RxRabies2 protein, and the purified and diluted RxRabies2 protein at 1x and 0.5x concentrations, respectively.

The specificity of the immune response can also be observed by the lack of banding in lines 3, 5 and 7, containing (i) the Rx receptacle protein without epitope introduction, (ii) the Platcruzi platform, diluted 1x and 0.5x, respectively. These results confirm that the immune response was restricted to rabies virus epitopes, indicating that the Rx protein per se is not immunogenic.

### EXAMPLE 8 - Development of RxHolgG3 protein

From mapping studies of polyamino acid sequences of horse immunoglobulins (De-Simone et al., Toxicon 78: 83-93, 2014; Wagner et al, Journal of Immunology 173: 3230-3242, 2004), a sequence of horse IgG3 polyamino acids recognized by human IgG and IgE, suitable for use in laboratory assays to diagnose horse serum allergy, was identified.

The polyamino acid sequence DVLFTWYVDGTEV(SEQ ID NO: 30) was incorporated into the Rx protein at positions 1, 5, 6, 8, 9 and 10, giving rise to the RxHolgG3 protein. The amino acid sequence of the RxHolgG3 protein is described in SEQ ID NO: 31. The nucleotide sequence of the RxHolgG3 protein is described in SEQ ID NO: 32. A synthetic gene containing a sequence coding for the Rx protein and the sequence coding for the polyamino acid sequence described above (SEQ ID NO: 30) has been synthesized.

The synthetic gene was introduced into pET28a plasmids using the restriction sites for the enzymes Ndel and Xhol by state-of-the-art molecular biology methods. In order to identify whether the synthetic gene matched the sequence designed for the RxHoIgG3 protein, a DH5α strain of *Escherichia coli* was transformed and the plasmid material analyzed by restriction enzyme digestion techniques and then sequenced, as already specified in PlatCruzi.

The analyzed plasmid harboring the correct sequence for RxHoIgG3 was transferred to E. *coli,* strain BL21, in order to produce the RxHoIgG3 protein. The BL21 strain can express T7 RNA polymerase when induced by Isopropyl β-D-1-thiogalactopyranoside (IPTG). The BL21 strain was grown overnight in LB medium and then reseeded in the same medium with kanamycin (30 µg/ml) on a shaker at 200 rpm until it reached an optical turbidity density of 0.6-0.8 (600 nm). Then, IPTG (q.s.p. 1 mM) is added to the culture and the same culture conditions are maintained for another 3 h.

The culture was subjected to centrifugation and the pellet resuspended in urea buffer (100 mM NaH2PO4, 10 mM Tris-base, 8 M urea, pH 8.0). The solution was chromatographed using a nickel affinity column (HisTrapTM, 1 mL, *GE Healthcare Life Sciences)* and flowed at 0.5 mL per minute, previously equilibrated in buffer A (50 mM Tris-HCl, pH 8.0, 100 mM NaCl and 5 mM imidazole). After binding, the resin was washed with 10 mL of buffer A. The protein was eluted in a 100% gradient of buffer B (50 mM Tris-HCl, pH 8.0, 100 mM NaCl, and 500 mM imidazole) at a flow rate of 0.7 mL/min for 45 minutes. The production of RxHolgG3 protein can be evidenced in the eluate after 11% SDS-PAGE electrophoresis (Table 5). The results are presented in Figure 6, and show the expression of RxHoIgG3 as a recombinant protein. No bands were detected in the uninduced soluble bacterial extract (Figure 6, column 1), and one each in the insoluble (column 2) and soluble (column 3) fractions.

### EXAMPLE 9 - - Development of RxOro protein

From a polyamino acid sequence mapping study of oropouche virus (strain Q71MJ4 and Q9J945, Uniprot) (Acrani et al, Journal of General Virology 96: 513-523, 2014 Tilston-Lunel et al, Journal of General Virology 96 (Pt 7): 1636-1650, 2015), we selected polyamino acid sequences from spot synthesis or peptide microarray techniques available in the state of the art, considering their diagnostic potential. Six polyamino acid sequences were selected for insertion into nine insertion points in the Rx protein, herein called OrEp 1 to OrEp 7, as shown in table 8 below:

**Table 8**

| Polyamino acid sequences | Position in the protein | Original epitope protein | Sequence | SEQ ID no. |
|---|---|---|---|---|
| OrEp 1 | 1 | G1 | YIEKDDSDALKALF | SEQ ID no. 35 |
| OrEp 2 | 3 | G2 | GNFMVLSVDD | SEQ ID no. 36 |
| OrEp 2 | 4 | G2 | GNFMVLSVDD | SEQ ID no. 36 |
| OrEp 3 | 5 | N | KTSRPMVDLTFGGVQ | SEQ ID no. 37 |
| OrEp 3 | 6 | N | KTSRPMVDL TFGGVQ | SEQ ID no. 37 |
| OrEp 4 | 7 | N | IFNDVPQRTTSTFDP | SEQ ID no. 38 |
| OrEp 4 | 8 | N | IFNDVPQRTTSTFDP | SEQ ID no. 38 |
| OrEp 5 | 9 | G2 | LYSDLFSKNLVTEY | SEQ ID no. 39 |
| OrEp 6 | 10 | G1 | YIEKDDSDALKALF | SEQ ID no. 40 |

The combination of these polyamino acid sequences described above with the Rx protein gave rise to the RxOro protein. The amino acid sequence corresponding to the RxOro gene, containing the polyamino acid sequences OrEp1 to OrEp6, is described in SEQ ID no. 33. The gene corresponding to the RxOro protein, herein referred to as the RxOro gene, is described in SEQ ID nucleotide sequence no. 34.

A synthetic RxOro gene was synthesized and introduced into pET28a plasmids using the restriction sites for the enzymes Ndel and Xhol by state-of-the-art molecular biology methods. In order to identify whether the synthetic gene matched the sequence designed for RxOro, a DH5α strain of *Escherichia coli* was transformed and the plasmid material analyzed by restriction enzyme digestion and subsequently sequencing techniques as described in PlatCruzi.

The analyzed plasmid harboring the correct sequence for the RxOro protein was transferred to E. *coli,* strain BL21. The BL21 strain can express T7 RNA polymerase when induced by Isopropyl β-D-1-thiogalactopyranoside (IPTG). The BL21 strain was grown overnight in LB medium and then reseeded in the same medium with kanamycin (30 µg/ml) on a shaker at 200 rpm until it reached an optical turbidity density of 0.6-0.8 (600 nm). Then, IPTG (q.s.p. 1 mM) is added to the culture and the same culture conditions are maintained for another 3 h.

The culture was subjected to centrifugation and the pellet resuspended in urea buffer (100 mM NaH2PO4, 10 mM Tris-base, 8 M urea, pH 8.0). The solution was chromatographed using a nickel affinity column (HisTrapTM, 1 mL, *GE Healthcare Life Sciences)* and flowed at 0.5 mL per minute, previously equilibrated in buffer A (50 mM Tris-HCl, pH 8.0, 100 mM NaCl and 5 mM imidazole). After binding, the resin was washed with 10 mL of buffer A. The protein was eluted in a 100% gradient of buffer B (50 mM Tris-HCl, pH 8.0, 100 mM NaCl, and 500 mM imidazole) at a flow rate of 0.7 mL/min for 45 minutes. RxOro production was examined in three different volumes of bacterial growth; 3,25 and 50 ml. The expression level of RxOro was 203 µg / ml on average and the results are shown in Table 9.

**Table 9**

| Protein | Amount of purified protein (µg/mL) | | | |
|---|---|---|---|---|
| | 3 mL culture | 25 mL culture | 50 mL culture | Average |
| RxOro | 407 | 164 | 40 | 204 |

### EXAMPLE 10 - Development of an ELISA from RxOro

The performance of the RxOro protein was evaluated against a panel of sera from individuals affected by Oropouche infections. RxOro protein, in solution (0.3 M Urea, pH 8.0), was added to a 96-well ELISA plate in the amount of 500 ng/orifice at 4 °C for 12-18 h. The wells were washed with saline-phosphate buffer (PBS) solution added Tween 20 (PBS-T, 10 mM sodium phosphate - Na3PO4, 150 mM sodium chloride - NaCl and 0.05% Tween-20, pH 7.4) and then incubated with 1x PBS buffer containing 5% (weight/volume) skim milk dehydrate for 2 h at 37 °C.

The wells were then washed 3 times with PBS-T buffer and incubated with 98 sera samples from patients suspected of Oropouche virus infection and 51 sera samples from healthy patients diluted 1:100, for 1 h at 37 °C. After the incubation period, the wells were washed three times with PBS-T and then incubated with alkaline phosphatase-labeled human IgG antibody at 1:5000 dilution for 1 h at 37°C.

The wells were washed again three times with PBS-T buffer and the substrate para-nitrophenylphosphate (PNPP, 1mg/mL, ThermoFischer SCIENTIFIC) was added. After 30 minutes and under shelter from light, the absorbance was measured in an ELISA plate reader at 405 nm.

The results pointed to excellent sensitivity and specificity using RxOro (Figure 7). The results strongly support the use of RxOro for detection of Oropouche virus infections.

The use of patient serum samples for experimental purposes was approved by the Ethics Committee of Fiocruz, as per authorization CEP/IOC - CAAE: 52892216.8.0000.5248.

### EXAMPLE 11 - - Development of RxMayaro_IgG protein

From a mapping study of polyamino acid sequences of Mayaro virus (strain Q8QZ73 and Q8QZ72, Uniprot) (Espósito et al., Genome Announcement 3: e01372-15, 2015), were selected polyamino acid sequences from the available state-of-the-art literature, considering their diagnostic potential. Four polyamino acid sequences were selected for insertion into nine insertion points in the Rx protein, here named MGEp1 to MGEp 4, as shown in Table 10 below:

**Table 10**

| Polyamino acid sequences | Position in the protein | Original epitope protein | Sequence | SEQ ID no. |
|---|---|---|---|---|
| MGEp 1 | 1 | nsP2 | KLSATDWSAI | SEQ ID no. 41 |
| MGEp 2 | 3 | Capsid | KPKPQPEK | SEQ ID no. 42 |
| MGEp 3 | 4 | nsP1 | KKMTPSDQI | SEQ ID no. 43 |
| MGEp 4 | 5 | nsP3 | VELPWPLETI | SEQ ID no. 44 |
| MGEp 2 | 6 | Capsid | KPKPQPEK | SEQ ID no. 42 |
| MGEp 1 | 7 | nsP2 | KLSATDWSAI | SEQ ID no. 41 |
| MGEp 4 | 8 | nsP3 | VELPWPLETI | SEQ ID no. 44 |
| MGEp 3 | 9 | nsP1 | KKMTPSDQI | SEQ ID no. 43 |
| MGEp 1 | 10 | nsP2 | KLSATDWSAI | SEQ ID no. 41 |

Combining the sequence of these polyamino acid sequences described above with the Rx protein gave rise to the RxMayaro_IgG protein. The amino acid sequence corresponding to the RxMayaro_IgG gene, containing the polyamino acid sequences MGEp 1 a MGEp 4 is described in SEQ ID no. 45. The gene corresponding to the RxMayaroIgG protein, herein referred to as the RxMayaro_IgG gene, is described in SEQ ID nucleotide sequence no. 46.

A synthetic RxMayaro_IgG gene was synthesized and introduced into pET28a plasmids using the restriction sites for the enzymes Ndel and Xhol via molecular biology methods known to the state of the art. In order to identify whether the synthetic gene matched the sequence designed for RxMayaro_IgG, a DH5α strain of *Escherichia coli* was transformed and the plasmid material analyzed by restriction enzyme digestion techniques and then sequenced, as cited in PlatCruzi.

The analyzed plasmid harboring the correct sequence for the RxMayaro_IgG protein was transferred into E. *coli,* strain BL21. The BL21 strain can express T7 RNA polymerase when induced by Isopropyl β-D-1-thiogalactopyranoside (IPTG). The BL21 strain was grown overnight in LB medium and then reseeded in the same medium with kanamycin (30 µg/ml) on a shaker at 200 rpm until it reached an optical turbidity density of 0.6-0.8 (600 nm). Then, IPTG (q.s.p. 1 mM) is added to the culture and the same culture conditions are maintained for another 3 h.

The culture was subjected to centrifugation and the *pellet* resuspended in urea buffer (100 mM NaH2PO4, 10 mM Tris-base, 8 M urea, pH 8.0). The solution was chromatographed on a nickel affinity column (HisTrapTM, 1 mL, *GE Healthcare Life Sciences)* at a flow rate of 0.5 mL per minute, previously equilibrated in buffer A (50 mM Tris-HCl, pH 8.0, 100 mM NaCl and 5 mM imidazole). After binding, the resin was washed with 10 mL of buffer A. The protein was eluted in a 100% gradient of buffer B (50 mM Tris-HCl, pH 8.0, 100 mM NaCl, and 500 mM imidazole) at a flow rate of 0.7 mL/min for 45 minutes. Production of the RxMayaro_IgG protein can be evidenced in the eluate from performing 11% SDS-PAGE electrophoresis (Table 5). The RxMayaro _IgG protein was also examined by SDS-PAGE to confirm its production and determine its distribution between the soluble and insoluble fractions. As seen in Figure 8, columns 4 and 7 (arrows) show that RxMayaro IgG is produced as soluble and insoluble.

RxMayaro_IgG production was examined at three different growth volumes: 3, 25 and 50 ml. As seen in Table 11 (below), the expression level of RxMayaro IgG was 130 µg / ml on average.

**Table 11**

| Protein | Amount of purified protein (µg/mL) | | | |
|---|---|---|---|---|
| | 3 mL culture | 25 mL culture | 50 mL culture | Media |
| RxMayaro_IgG | 157 | 168 | 64 | 130 |

### EXAMPLE 12 - Development of ELISA from RxMayaro_IgG

The performance of the RxMayaroIgG protein was evaluated against a panel of sera from individuals affected by Mayaro virus infections. RxMayaroIgG protein, in solution (0.3 M Urea, pH 8.0), was added to a 96-hole ELISA plate in the amount of 500 ng/orifice at 4 °C for 12-18 h. The wells were washed with saline-phosphate buffer (PBS) solution added Tween 20 (PBS-T, 10 mM sodium phosphate - Na3PO4, 150 mM sodium chloride - NaCl and 0.05% Tween-20, pH 7.4) and then incubated with 1x PBS buffer containing 5% (weight/volume) dehydrated skim milk for 2 h at 37 °C.

The wells were then washed three times with PBS-T buffer and incubated with 6 samples of sera from patients suspected of Mayaro virus infection and 29 samples of sera from healthy patients diluted 1:100, for 1 h at 37 °C. After the incubation period, the wells were washed three times with PBS-T and then incubated with alkaline phosphatase-labeled human IgG antibody at a dilution of 1:5000, for 1 h at 37 °C. The wells were washed again three times with PBS-T buffer and the substrate para-nitrophenylphosphate (PNPP, 1mg/mL, ThermoFischer SCIENTIFIC) was added. After 30 minutes and under shelter from light, the absorbance was measured in an ELISA plate reader at 405 nm.

The results pointed to excellent sensitivity and specificity using RxMayaro_IgG (Figure 9). The results strongly support the use of RxMayaro_IgG for detection of Mayaro virus infections.

The use of patient serum samples for experimental purposes was approved by the Ethics Committee of Fiocruz, as per authorization CEP/IOC - CAAE: 52892216.8.0000.5248.

### EXAMPLE 13 - - Development of RxMayaro_IgM protein

From a mapping study of polyamino acid sequences of Mayaro virus (strain Q8QZ73 and Q8QZ72, Uniprot) (Espósito et al., Genome Announcement 3: e01372-15, 2015), were selected polyamino acid sequences from the available state-of-the-art literature, considering their diagnostic potential. Four polyamino acid sequences were selected for insertion into nine insertion points in the Rx protein, herein named MMEp1 to MMEp 4, as shown in table 12 (below):

**Table 12**

| Polyamino acid sequences | Position in the protein | Original epitope protein | Sequence | SEQ ID no. |
|---|---|---|---|---|
| MMEp1 | 1 | nsP1 | HRIRLLLQS | SEQ ID no. 47 |
| MMEp2 | 3 | E2 | SYRTGAERV | SEQ ID no. 48 |
| MMEp3 | 4 | nsP2 | NGVKQTVDV | SEQ ID no. 49 |
| MmEp4 | 5 | E1 | QSRTLDSRD | SEQ ID no. 50 |
| MMEp 4 | 6 | E1 | QSRTLDSRD | SEQ ID no. 50 |
| MMEp1 | 7 | nsP1 | HRIRLLLQS | SEQ ID no. 47 |
| MMEp 2 | 8 | E2 | SYRTGAERV | SEQ ID no. 48 |
| MMEp3 | 9 | nsP2 | NGVKQTVDV | SEQ ID no. 49 |
| MMEp1 | 10 | nsP1 | HRIRLLLQS | SEQ ID no. 47 |

Combining the sequence of these polyamino acid sequences described above with the Rx protein gave rise to the RxMayaro_IgM protein. The amino acid sequence corresponding to the RxMayaro_IgM gene, containing the polyamino acid sequences MMEp 1 a MMEp 4 is described in SEQ ID no. 51. The gene corresponding to the RxMayaro_IgM protein, herein referred to as the RxMayaro _IgM gene, is described in SEQ ID nucleotide sequence no. 52.

A synthetic RxMayaro _IgM gene was synthesized and introduced into pET28a plasmids using the restriction sites for the enzymes Ndel and Xhol via molecular biology methods known to the state of the art.

In order to identify whether the synthetic gene matched the sequence designed for RxMayaro _IgM, a DH5α strain of *Escherichia coli* was transformed and the plasmid material analyzed by restriction enzyme digestion techniques and then sequenced, as previously described in PlatCruzi.

The analyzed plasmid harboring the correct sequence for the RxMayaro_IgM protein was transferred into *E*. *coli,* strain BL21. The BL21 strain can express T7 RNA polymerase when induced by Isopropyl β-D-1-thiogalactopyranoside (IPTG). The BL21 strain was grown overnight in LB medium and subsequently reseeded in the same medium, with kanamycin (30 µg/ml) added, on a shaker at 200 rpm until it reached an optical turbidity density of 0.6-0.8 (600 nm). Then, IPTG (q.s.p. 1 mM) is added to the culture and the same culture conditions are maintained for another 3 h.

The culture was subjected to centrifugation and the *pellet* resuspended in urea buffer (100 mM NaH2PO4, 10 mM Tris-base, 8 M urea, pH 8.0). The solution was chromatographed on a nickel affinity column (HisTrapTM, 1 mL, *GE Healthcare Life Sciences*) at a flow rate of 0.5 mL per minute, previously equilibrated in buffer A (50 mM Tris-HCl, pH 8.0, 100 mM NaCl and 5 mM imidazole). After binding, the resin was washed with 10 mL of buffer A. The protein was eluted in a 100% gradient of buffer B (50 mM Tris-HCl, pH 8.0, 100 mM NaCl, and 500 mM imidazole) at a flow rate of 0.7 mL/min for 45 minutes.

The production of RxMayaro IgM protein can be evidenced in the eluate by performing SDS-PAGE electrophoresis and its distribution can be observed between the soluble and insoluble fractions. As can be seen in Figure 8, columns 5 and 8 (arrows) show that RxMayaro_IgM is produced as soluble and insoluble.

RxMayaro_IgM production was also examined at three different growth volumes: 3, 25 and 50 ml. As seen in Table 13 (below), the expression level of RxMayaroIgM was 205 µg / ml on average.

**Table 13**

| Protein | Amount of purified protein (µg/mL) | | | |
|---|---|---|---|---|
| | 3 mL culture | 25 M1 Culture | 50 mL culture | Average |
| RxMayaro_IgM | 200 | 172 | 242 | 205 |

### EXAMPLE 14 - Development of an ELISA based on RxMayaro_IgM

The performance of the RxMayaroIgM protein was evaluated against a panel of sera from individuals affected by Mayaro virus infections. RxMayaro_IgM protein, in solution (0.3 M Urea, pH 8.0), was added to a 96-hole ELISA plate in the amount of 500 ng/orifice at 4 °C for 12-18 h. The wells were washed with saline-phosphate buffer (PBS) solution added Tween 20 (PBS-T, 10 mM sodium phosphate - Na3PO4, 150 mM sodium chloride - NaCl and 0.05% Tween-20, pH 7.4) and then incubated with 1× PBS buffer containing 5% (weight/volume) dehydrated skim milk for 2 h at 37 °C.

The wells were then washed three times with PBS-T buffer and incubated with 6 sera samples from patients suspected of Mayaro virus infection and 29 sera samples from healthy patients diluted 1:100, for 1 h at 37 °C. After the incubation period, the wells were washed three times with PBS-T and then incubated with alkaline phosphatase-labeled human IgM antibody at a dilution of 1:5000, for 1 h at 37 °C. The wells were washed again three times with PBS-T buffer and the substrate para-nitrophenylphosphate (PNPP, 1mg/mL, ThermoFischer SCIENTIFIC) was added. After 30 minutes and under shelter from light, the absorbance was measured in an ELISA plate reader at 405 nm.

The results pointed to excellent sensitivity and specificity using RxMayaro_IgM (Figure 10). The results strongly support the use of RxMayaro_IgM for detection of Mayaro virus infections.

The use of patient serum samples for experimental purposes was approved by the Ethics Committee of Fiocruz, as per authorization CEP/IOC - CAAE: 52892216.8.0000.5248.

### EXAMPLE 15 - Protein RxPtx development

From a mapping study of polyamino acid sequences of the bacterial toxin protein *Bordetella pertussis*(P04977; P04978; P04979; P0A3R5 and P04981: Uniprot), causing pertussis, polyamino acid sequences were selected from the available state-of-the-art literature, considering their diagnostic potential. Ten polyamino acid sequences were selected for insertion into nine insertion sites in the Rx protein, here called PtxEp1 to PtxEp 10, as shown in Table 14 below. In this example, two epitopes were located at position 1 using a spacer (SEQ ID NO: 95: SYWKGS) among them. Two epitopes were also inserted at position 10 using another spacer (SEQ ID NO: 96: EAAKEAAK). The purpose of introducing these spacers is to generate an inert physical space between consecutive polyaminoacids, thus helping to prevent binding competition between adjacent antibodies.

**Table 14**

| Polyamino acid sequences | Position in the protein | Original epitope protein | Sequence | SEQ ID no. |
|---|---|---|---|---|
| PtxEp 1 | 1 | Ptx-S1 | PYTSRRSVASIVGT | SEQ ID no. 53 |
| Spacer | Between PtxEp1 and 2 | AT | SYWKGS | SEQ ID no. 95 |
| PtxEp 2 | 1 | Ptx-S3 | QYYDYEDATF | SEQ ID no. 54 |
| PtxEp 3 | 2 | Ptx-S4 | GPKQL TFEGK | SEQ ID no. 55 |
| PtxEp 4 | 3 | Ptx-S2 | DATFETYALT | SEQ ID no. 56 |
| PtxEp 5 | 5 | Ptx-S5 | LTVEDSPYP | SEQ ID no. 57 |
| PtxEp 6 | 6 | Ptx-S1 | ALATYQSEY | SEQ ID no. 58 |
| PtxEp 7 | 8 | Ptx-S3 | PGIVIPPKALFTQQQ | SEQ ID no. 59 |
| PtxEp 8 | 9 | Ptx-S1 | AVEAERAGR | SEQ ID no. 60 |
| PtxEp 9 | 10 | Ptx-S1 | TTTEYSNAR | SEQ ID no. 61 |
| Spacer | Between PtxEp9 and 10 | AT | EAAKEAAK | SEQ ID no. 96 |
| PtxEp10 | 10 | Ptx-S1 | ERAGEAMVL VYYES | SEQ ID no. 62 |

Combining the sequence of these polyamino acid sequences described above with the Rx protein gave rise to the protein RxPtx. The amino acid sequence corresponding to the gene RxPtx gene, containing the epitopes PtxEp 1 a PtxEp 10 is described in SEQ ID NO: 64. The gene corresponding to the RxPtx gene, herein referred to as the RxPtx gene is described in the SEQ ID NO nucleotide sequence: 63.

A synthetic gene RxPtx was synthesized and introduced into pET28a plasmids using the restriction sites for the enzymes Ndel and Xhol by state-of-the-art molecular biology methods. In order to identify whether the synthetic gene matched the sequence designed for RxPtx a DH5α strain of *Escherichia coli* was transformed and the plasmid material analyzed by restriction enzyme digestion and subsequently sequencing techniques, as previously described in PlatCruzi.

The analyzed plasmid harboring the correct sequence for the RxPtx protein protein was transferred to *E*. *coli,* strain BL21. The BL21 strain can express T7 RNA polymerase when induced by Isopropyl β-D-1-thiogalactopyranoside (IPTG). The BL21 strain was grown overnight in LB medium and subsequently reseeded in the same medium, with kanamycin (30 µg/ml) added, on a shaker at 200 rpm until it reached an optical turbidity density of 0.6-0.8 (600 nm). Then, IPTG (q.s.p. 1 mM) is added to the culture and the same culture conditions are maintained for another 3 h.

The culture was subjected to centrifugation and the *pellet* resuspended in 2 mL of PBS with CelLytic (0.5×) for one hour at 4 °C. After another centrifugation, the supernatant was collected and the pellet was resuspended in 8 M urea solution (pH 8.0) in the same volume as the supernatant. Equal volumes were loaded on the SDS-PAGE gel (11%, Table 5).

The RxPtx protein was examined by SDS-PAGE electrophoresis to confirm its production and determine its distribution between the soluble and insoluble fractions. As seen in Figure 11, columns 5 and 6 (arrows) show that RxPtx is produced as soluble and insoluble with a higher proportion in the insoluble fraction.

### EXAMPLE 16 - Development of RxYFIgG protein

From a mapping study of polyamino acid sequences of the yellow fever virus (strain 17DD and the sequences in the p03314-Uniprot archive)polyamino acid sequences were selected from the available state-of-the-art literature, considering their diagnostic potential. Ten polyamino acid sequences were selected for insertion into nine insertion sites in the Rx protein, here called YFIgGEp1 to YFIgGEp10, as shown in Table 15 below. Two epitopes were located at position 10 using a spacer (SEQ ID NO: 97: TSYWKGS) between them. The spacer has the function of creating a physical space between consecutive epitopes, helping to preserve the interaction with the antibodies.

**Table 15**

| Polyamino acid sequences | Position in the protein | Original epitope protein | Sequence | SEQ ID no. |
|---|---|---|---|---|
| YFIgGEp 1 | 1 | NS4B | SPWSWPDLDLKPGA | SEQ ID no. 65 |
| YFIgGEp 2 | 3 | NS2A | DGNCDGRGKSTRST | SEQ ID no. 66 |
| YFIgGEp 3 | 4 | NS1 | VFSPGRKNGSFIID | SEQ ID no. 67 |
| YFIgGEp 4 | 5 | NS4B | HVQDCDESVLTRLE | SEQ ID no. 68 |
| YFIgGEp 5 | 6 | NS1 | DCDGSILGAAVNGK | SEQ ID no. 69 |
| YFIgGEp 6 | 7 | NS1 | FTTRVYMDA | SEQ ID no. 70 |
| YFIgGEp 7 | 8 | NS1 | RDSDDDWLNKYSYYP | SEQ ID no. 71 |
| YFIgGEp 8 | 9 | NS1 | ESEMFMPRSIGGPV | SEQ ID no. 72 |
| YFIgGEp 9 | 10 | NS4B | AEAEMVIHHQHVQD | SEQ ID no. 73 |
| Spacer | Between YFIgGEp9 and 10 | | TSYWKGS | SEQ ID no. 97 |
| YFIgGEp 10 | 10 | NS1 | LEHEMWRSRADEINAIFEE | SEQ ID no. 74 |

Combining the sequence of these polyamino acids described above with the Rx protein gave rise to the protein RxYFIgG protein. The amino acid sequence corresponding to the gene RxYFIgG containing the epitopes YFIgGEp 1 a YFIgGEp 10 is described in SEQ ID NO: 75. The gene corresponding to the RxYFIgG protein gene, herein called the RxYFIgG gene is described in SEQ ID nucleotide sequence no. 76.

A synthetic gene RxYFIgG was synthesized and introduced into pET28a plasmids using the restriction sites for the enzymes Ndel and Xhol by state-of-the-art molecular biology methods.

In order to identify whether the synthetic gene matched the sequence designed for RxYFIgG, a DH5α strain of *Escherichia coli* was transformed and the plasmid material analyzed by restriction enzyme digestion and subsequently sequencing techniques, as previously described in PlatCruzi.

The analyzed plasmid harboring the correct sequence for RxYFIgG was transferred to *E. coli,* strain BL21, in order to produce the RxYFIgG protein. The BL21 strain can express T7 RNA polymerase when induced by Isopropyl β-D-1-thiogalactopyranoside (IPTG). The BL21 strain was grown overnight in LB medium at 37 °C and subsequently reseeded in the same medium with kanamycin (30 µg/ml) on a shaker at 200 rpm until an optical turbidity density of 0.6-0.8 (600 nm) was reached. Then IPTG (q.s.p. 1 mM) is added to the culture and the same culture conditions are maintained for another 3 h at 37 °C.

The culture was centrifuged and the pellet resuspended in 2 mL PBS with CelLytic in 2 mL of PBS with CelLytic (0.5×) for one hour at 4 °C. After another centrifugation, the supernatant was collected and the pellet was resuspended in 8 M urea solution (pH 8.0) in the same volume as the supernatant. Equal volumes were loaded on the SDS-PAGE gel (11%, Table 5). The protein RxYFIgG was examined by SDS-PAGE electrophoresis to confirm its production and to determine its distribution between the soluble and insoluble fractions. As seen in Figure 11, columns 9 and 10 (arrows) show that RxYFIgG is produced as both soluble and insoluble, with a higher proportion in the insoluble fraction.

### EXAMPLE 17 - Development of TxNeuza protein

The receptacle protein "Tx" has been genetically manipulated to harbor epitopes of t-cell epitopes from *Dermatophogoides pteronyssinus* a leading cause of respiratory allergy in humans, which we refer to here as the TxNeuza platform. The gene corresponding to the TxNeuza protein, here called the TxNeuza gene, is described in the SEQ ID NO nucleotide sequence: 89.

From a mapping study of T-cell polyamino acid sequences from *D*. *pteronyssinus*,polyamino acid sequences were selected from the available state-of-the-art literature considering their diagnostic potential for allergies caused by *D. pteronyssinus* (Hinz et al., Clin Exp Allergy 45: 1601-1612, 2015; Oseroff et al, Clin Exp Allergy 47:577-592, 2017). Nine polyamino acid sequences were selected for insertion into nine insertion sites in the Tx protein, here named NeuzaEp1 to NeuzaEp9, as shown in Table 16 below. In this example, two epitopes were located at position 12 using a spacer (SEQ ID NO: 98: GGSG) among them.

**Table 16**

| Polyamino acid sequences | Position in the protein | Original epitope protein | Sequence | SEQ ID no. |
|---|---|---|---|---|
| NeuzaEp1 | 12 | Derp1 | DLRQMRTVTPIRMQGGCGSC | SEQ ID no. 79 |
| NeuzaEp2 | 10 | Derp1 | GCGSCWAFSGVAATESAYLA | SEQ ID no. 80 |
| NeuzaEp3 | 7 | Derp1 | QESYYRYVAREQSCR | SEQ ID no. 81 |
| NeuzaEp4 | 2 | Derp1 | HAVNIVGYSNAQGVD | SEQ ID no. 82 |
| NeuzaEp5 | 9 | Derp2 | CHGSEPCIIHRGKPFQLEAV | SEQ ID no. 83 |
| NeuzaEp6 | 6 | Derp2 | YDIKYTWNVPKIAPKSENVVV | SEQ ID no. 84 |
| NeuzaEp7 | 12 | Derp2 | NTKTAKIEIKASIDG | SEQ ID no. 85 |
| NeuzaEp8 | 5 | Derp2 | GVLACAIATHAKIRD | SEQ ID no. 86 |
| NeuzaEp9 | 1 | Derp23 | PKDPHKFYICSNWEAVHKDC | SEQ ID no. 87 |
| Spacer | Between NeuzaEp1 and 7 | | GGSG | SEQ ID no. 98 |

Combining the sequence of these epitopes described above with the Tx protein gave rise to the TxNeuza protein. The amino acid sequence corresponding to the TxNeuza gene, containing the epitopes NeuzaEp 1 a NeuzaEp9 is described in SEQ ID NO: 88.

A synthetic gene TxNeuza was synthesized and introduced into pET28a plasmids using the restriction sites for the enzymes Ndel and Xhol by state-of-the-art molecular biology methods. In order to identify whether the synthetic gene matched the sequence designed for TxNeuza a DH5α strain of *Escherichia coli* was transformed and the plasmid material analyzed by restriction enzyme digestion and subsequently sequencing techniques, as previously described in PlatCruzi.

The analyzed plasmid harboring the correct sequence for TxNeuza was transferred to *E*. *coli,* strain BL21, in order to produce the TxNeuza protein. The BL21 strain can express T7 RNA polymerase when induced by Isopropyl β-D-1-thiogalactopyranoside (IPTG). The BL21 strain was grown overnight in LB medium at 37 °C and subsequently reseeded in the same medium with kanamycin (30 µg/ml) on a shaker at 200 rpm until an optical turbidity density of 0.6-0.8 (600 nm) was reached. Then IPTG (q.s.p. 1 mM) is added to the culture and the same culture conditions are maintained for another 3 h at 37 °C.

The culture was centrifuged and the *pellet* resuspended in 2 mL PBS with CelLytic in 2 mL of PBS with CelLytic (0.5×) for one hour at 4 °C. After another centrifugation, the supernatant was collected and the pellet was resuspended in 8 M urea solution (pH 8.0) in the same volume as the supernatant. Equal volumes were loaded on the SDS-PAGE gel (11%, Table 5). The TxNeuza protein was examined by SDS-PAGE to confirm its production and determine its distribution between the soluble and insoluble fractions. As seen in Figure 11, columns 7 and 8 (arrows) show that the TxNeuza protein is produced as insoluble.

### EXAMPLE 18 - Development of TxCruzi protein

*T. cruzi* polyamino acid sequences were selected from the available state-of-the-art literature, considering experimental specificity and sensitivity data for diagnostic tests for Chagas disease (Balouz, et al., Clin Vaccine Immunol 22, 304-312, 2015; Alvarez, et al., Infect Immun 69, 7946-7949, 2001; Fernandez-Villegas, et al., J Antimicrob Chemother 71, 2005-2009,2016; Thomas, et al., Clin Vaccine Immunol 19, 167-173, 2012). Ten polyamino acid sequences were selected for insertion into the ten insertion sites in the "Tx" protein, here called TcEp 1, TcEp 3, TcEp 4, TcEp 6, TcEp 8, TcEp 9, TcEp 10, TcEp 11, TcEp 12, and TcEp 13, as shown in Table 17 below. Two epitopes were located at position 12 using a spacer (SEQ ID NO: 99: GGASG) among them.

**Table 17**

| Polyamino acid sequences | Position in the protein | Original epitope protein | Sequence | SEQ ID no. |
|---|---|---|---|---|
| TcEp1 | 1 | KMP11 | KFAELLEQQKNAQFPGK | SEQ ID no. 7 |
| TcEp11 | 4 | SAPA | DSSAHSTPSTPA | SEQ ID no. 92 |
| TcEp4 | 6 | PEP-2 | GDKPSPFGQAAAADK | SEQ ID no. 10 |
| TcEp12 | 7 | TcCA-2 | FGQAAAGDKPS | SEQ ID no. 93 |
| TcEp6 | 8 | TcD-1 | AEPKPAEPKS | SEQ ID no. 12 |
| TcEp13 | 9 | TSSA | TSSTPPSGTENKPATG | SEQ ID no. 94 |
| TcEp8 | 10 | TcLo 1.2 | GTSEEGSRGGSSMPS | SEQ ID no. 14 |
| TcEp9 | 11 | B13 | SPFGQAAAGDK | SEQ ID no. 15 |
| TcEp3 TcEplO | 12 | TcE CRA | KAAIAPA KQRAAEATK | SEQ ID no. 9 SEQ ID no. 16 |
| Spacer | Between TcEp3 and 10 | | GGASG | SEQ ID no. 99 |

After the selection of the *T. cruzi* polyamino acid sequences, the synthetic gene corresponding to the TxCruzi protein was produced by chemical synthesis, by the ligation gene synthesis methodology and inserted into plasmids for experimentation. The nucleotide sequence corresponding to the TxCruzi gene, containing the epitopes TcEp 1, TcEp 3, TcEp 4, TcEp 6, TcEp 8, TcEp 9, TcEp 10, TcEp 11, TcEp 12 and TcEp 13, is described in SEQ ID no. 91.

Combining the sequence of these epitopes described above with the Tx protein gave rise to the TxCruzi protein. The amino acid sequence corresponding to the TxCruzi gene gene, containing the epitopes TcEp 1, TcEp 3, TcEp 4, TcEp 6, TcEp 8, TcEp 9, TcEp 10, TcEp 11, TcEp 12, and TcEp 13, is described in SEQ ID NO: 90.

The synthesized gene was introduced into pET28a plasmids using the restriction sites for the enzymes Xbal and Xhol by state-of-the-art molecular biology methods. In order to identify whether the synthetic gene matched the sequence designed for the TxCruzi protein, a DH5α strain of *Escherichia coli* was transformed and the plasmid material analyzed by restriction enzyme digestion techniques and then sequenced, as already specified in PlatCruzi.

The analyzed plasmid harboring the correct sequence for TxCruzi was transferred to *E. coli,* strain BL21, in order to produce the TxCruzi protein. The BL21 strain can express T7 RNA polymerase when induced by Isopropyl β-D-1-thiogalactopyranoside (IPTG). The BL21 strain was grown overnight in LB medium at 37 °C and subsequently reseeded in the same medium with kanamycin (30 µg/ml) on a shaker at 200 rpm until an optical turbidity density of 0.6-0.8 (600 nm) was reached. Then IPTG (q.s.p. 1 mM) is added to the culture and the same culture conditions are maintained for another 3 h at 37 °C.

The culture was subjected to centrifugation and the *pellet* resuspended in 2 mL of PBS with CelLytic (0.5×) for one hour at 4 °C. After another centrifugation, the supernatant was collected and the *pellet* was resuspended in 8 M urea solution (pH 8.0) in the same volume as the supernatant. Equal volumes were loaded on the SDS-PAGE gel (11%, Table 5).

The TxCruzi protein was examined by SDS-PAGE to confirm its production and to determine its distribution between the soluble and insoluble fractions. As seen in Figure 11, columns 3 and 4 (arrows) show that TxCruzi is produced as soluble and insoluble. Compared to PlatCruzi (columns 1 and 2), TxCruzi showed an improvement in the proportion of soluble protein produced, which can be attributed to the use of Tx as a receptacle protein.

### EXAMPLE 19 - Synthesis of SARS-CoV-2 peptide libraries on cellulose membranes and reactivity with sera from SARS-CoV-2 positive and negative individuals

Polypeptide libraries covering all protein-coding regions of ORF3a, ORF6, ORF7, ORF8, ORF10, N, M, S, E of SARS-CoV-2 virus were synthesized based on the genomic sequence of SARS-CoV-2 isolated in Wuhan city in China and published in the GenBank database (https://www.ncbi.nlm.nih.gov/nuccore/MN908947.3?report=genbank) and were annotated as follows:

Four polypeptides not encoded by SARS-CoV-2 virus were included in the peptide library relationships to represent positive controls for the reactivity of human sera. In Table 18, A1, V5 (IHLVNNESSEVIVHK, *Clostriduium tetani* peptide precursor), A2, V6 (GYPKDGNAFNNLDR, *Clostridium tetani*)*,* A3, V7 (KEVPALTAVETGATG, human polyvirus), A4, V8 (YPYDVPDYAGYPYD, triple hemagglutinin peptide) were used as such controls. In Tables 19 and 20 A1, V4 (IHLVNNESSEVIVHK, *Clostriduium tetani* peptide precursor), A2, V5 (GYPKDGNAFNNLDR, *Clostridium tetani*)*,* A3, V6 (KEVPALTAVETGATG, human polyvirus), A4, V8 (YPYDVPDYAGYPYD, triple hemagglutinin peptide) were used as such controls.

As negative controls, the peptide-free spot reactant was used in A5, A6, K20, K21, N3, N4, O24, P1, P13, P14, Q14, Q15, R15, R16, V3, V4, V9-V24, W14 in Table 18, and A5, K19, K20, N2, N3, O23, O24, P12, P13, Q13, Q14, R15, R15, V2, V3, V17, V18 in Table 19 and Table 20.

The relationship of the synthetic linear polyamino acids is shown in Table 18, Table 19, and Table 20.

**Table 18 - List of SARS-CoV-2 polyamino acids synthesized for mapping IgM-reactive epitopes from patient sera in Figure 12.**

| | | | | | |
|---|---|---|---|---|---|
| Spot | Polypeptides | B20 | FKNLREFVFKNIDGY | D16 | EVRQIAPGQTGKIAD |
| A1 | IHLVNNESSEVIVHK | B21 | EFVFKNIDGYFKIYS | D17 | APGQTGKIADYNYKL |
| A2 | GYPKDGNAFNNLDRI | B22 | NIDGYFKIYSKHTPI | D18 | GKIADYNYKLPDDFT |
| A3 | KEVPALTAVETGATN | B23 | FKIYSKHTPINLVRD | D19 | YNYKLPDDFTGCVIA |
| A4 | YPYDVPDYAGYPYDV | B24 | KHTPINLVRDLPQGF | D20 | PDDFTGCVIAWNSNN |
| A5 | | C1 | NLVRDLPQGFSALEP | D21 | GCVIAWNSNNLDSKV |
| A6 | | C2 | LPQGFSALEPLVDLP | D22 | WNSNNLDSKVGGNYN |
| A7 | MFVFLVLLPLVSSQC | C3 | SALEPLVDLPIGINI | D23 | LDSKVGGNYNYLYRL |
| A8 | VLLPLVSSQCVNLTT | C4 | LVDLPIGINITRFQT | D24 | GGNYNYLYRLFRKSN |
| A9 | VSSQCVNLTTRTQLP | C5 | IGINITRFQTLLALH | E1 | YLYRLFRKSNLKPFE |
| A10 | VNLTTRTQLPPAYTN | C6 | TRFQTLLALHRSYLT | E2 | FRKSNLKPFERDIST |
| A11 | RTQLPPAYTNSFTRG | C7 | LLALHRSYLTPGDSS | E3 | LKPFERDISTEIYQA |
| A12 | PAYTNSFTRGVYYPD | C8 | RSYLTPGDSSSGWTA | E4 | RDISTEIYQAGSTPC |
| A13 | SFTRGVYYPDKVFRS | C9 | PGDSSSGWTAGAAAY | E5 | EIYQAGSTPCNGVEG |
| A14 | VYYPDKVFRSSVLHS | C10 | SGWTAGAAAYYVGYL | E6 | GSTPCNGVEGFNCYF |
| A15 | KVFRSSVLHSTQDLF | C11 | GAAAYYVGYLQPRTF | E7 | NGVEGFNCYFPLQSY |
| A16 | SVLHSTQDLFLPFFS | C12 | YVGYLQPRTFLLKYN | E8 | FNCYFPLQSYGFQPT |
| A17 | TQDLFLPFFSNVTWF | C13 | QPRTFLLKYNENGTI | E9 | PLQSYGFQPTNGVGY |
| A18 | LPFFSNVTWFHAIHV | C14 | LLKYNENGTITDAVD | E10 | GFQPTNGVGYQPYRV |
| A19 | NVTWFHAIHVSGTNG | C15 | ENGTITDAVDCALDP | E11 | NGVGYQPYRVVVLSF |
| A20 | HAIHVSGTNGTKRFD | C16 | TDAVDCALDPLSETK | E12 | QPYRVVVLSFELLHA |
| A21 | SGTNGTKRFDNPVLP | C17 | CALDPLSETKCTLKS | E13 | VVLSFELLHAPATVC |
| A22 | TKRFDNPVLPFNDGV | C18 | LSETKCTLKSFTVEK | E14 | ELLHAPATVCGPKKS |
| A23 | NPVLPFNDGVYFAST | C19 | CTLKSFTVEKGIYQT | E15 | PATVCGPKKSTNLVK |
| A24 | FNDGVYFASTEKSNI | C20 | FTVEKGIYQTSNFRV | E16 | GPKKSTNLVKNKCVN |
| B1 | YFASTEKSNIIRGWI | C21 | GIYQTSNFRVQPTES | E17 | TNLVKNKCVNFNFNG |
| B2 | EKSNIIRGWIFGTTL | C22 | SNFRVQPTESIVRFP | E18 | NKCVNFNFNGLTGTG |
| B3 | IRGWIFGTTLDSKTQ | C23 | QPTESIVRFPNITNL | E19 | FNFNGLTGTGVLTES |
| B4 | FGTTLDSKTQSLLIV | C24 | IVRFPNITNLCPFGE | E20 | LTGTGVLTESNKKFL |
| B5 | DSKTQSLLIVNNATN | D1 | NITNLCPFGEVFNAT | E21 | VLTESNKKFLPFQQF |
| B6 | SLLIVNNATNVVIKV | D2 | CPFGEVFNATRFASV | E22 | NKKFLPFQQFGRDIA |
| B7 | NNATNVVIKVCEFQF | D3 | VFNATRFASVYAWNR | E23 | PFQQFGRDIADTTDA |
| B8 | WIKVCEFQFCNDPF | D4 | RFASVYAWNRKRISN | E24 | GRDIADTTDAVRDPQ |
| B9 | CEFQFCNDPFLGVYY | D5 | YAWNRKRISNCVADY | F1 | DTTDAVRDPQTLEIL |
| B10 | CNDPFLGVYYHKNNK | D6 | KRISNCVADYSVLYN | F2 | VRDPQTLEILDITPC |
| B11 | LGVYYHKNNKSWMES | D7 | CVADYSVLYNSASFS | F3 | TLEILDITPCSFGGV |
| B12 | HKNNKSWMESEFRVY | D8 | SVLYNSASFSTFKCY | F4 | DITPCSFGGVSVITP |
| B13 | SWMESEFRVYSSANN | D9 | SASFSTFKCYGVSPT | F5 | SFGGVSVITPGTNTS |
| B14 | EFRVYSSANNCTFEY | D10 | TFKCYGVSPTKLNDL | F6 | SVITPGTNTSNQVAV |
| B15 | SSANNCTFEYVSQPF | D11 | GVSPTKLNDLCFTNV | F7 | GTNTSNQVAVLYQDV |
| B16 | CTFEYVSQPFLMDLE | D12 | KLNDLCFTNVYADSF | F8 | NQVAVLYQDVNCTEV |
| B17 | VSQPFLMDLEGKQGN | D13 | CFTNVYADSFVIRGD | F9 | LYQDVNCTEVPVAIH |
| B18 | LMDLEGKQGNFKNLR | D14 | YADSFVIRGDEVRQI | F10 | NCTEVPVAIHADQLT |
| B19 | GKQGNFKNLREFVFK | D15 | VIRGDEVRQIAPGQT | F11 | PVAIHADQLTPTWRV |
| F12 | ADQLTPTWRVYSTGS | H8 | ARDLICAQKFNGLTV | J4 | TYVPAQEKNFTTAPA |
| F13 | PTWRVYSTGSNVFQT | H9 | CAQKFNGLTVLPPLL | J5 | QEKNFTTAPAICHDG |
| F14 | YSTGSNVFQTRAGCL | H10 | NGLTVLPPLLTDEMI | J6 | TTAPAICHDGKAHFP |
| F15 | NVFQTRAGCLIGAEH | H11 | LPPLLTDEMIAQYTS | J7 | ICHDGKAHFPREGVF |
| F16 | RAGCLIGAEHVNNSY | H12 | TDEMIAQYTSALLAG | J8 | KAHFPREGVFVSNGT |
| F17 | IGAEHVNNSYECDIP | H13 | AQYTSALLAGTITSG | J9 | REGVFVSNGTHWFVT |
| F18 | VNNSYECDIPIGAGI | H14 | ALLAGTITSGWTFGA | J10 | VSNGTHWFVTQRNFY |
| F19 | ECDIPIGAGICASYQ | H15 | TITSGWTFGAGAALQ | J11 | HWFVTQRNFYEPQII |
| F20 | IGAGICASYQTQTNS | H16 | WTFGAGAALQIPFAM | J12 | QRNFYEPQIITTDNT |
| F21 | CASYQTQTNSPRRAR | H17 | GAALQIPFAMQMAYR | J13 | EPQIITTDNTFVSGN |
| F22 | TQTNSPRRARSVASQ | H18 | IPFAMQMAYRFNGIG | J14 | TTDNTFVSGNCDVVI |
| F23 | PRRARSVASQSIIAY | H19 | QMAYRFNGIGVTQNV | J15 | FVSGNCDVVIGIVNN |
| F24 | SVASQSIIAYTMSLG | H20 | FNGIGVTQNVLYENQ | J16 | CDVVIGIVNNTVYDP |
| G1 | SIIAYTMSLGAENSV | H21 | VTQNVLYENQKLIAN | J17 | GIVNNTVYDPLQPEL |
| G2 | TMSLGAENSVAYSNN | H22 | LYENQKLIANQFNSA | J18 | TVYDPLQPELDSFKE |
| G3 | AENSVAYSNNSIAIP | H23 | KLIANQFNSAIGKIQ | J19 | LQPELDSFKEELDKY |
| G4 | AYSNNSIAIPTNFTI | H24 | QFNSAIGKIQDSLSS | J20 | DSFKEELDKYFKNHT |
| G5 | SIAIPTNFTISVTTE | I1 | IGKIQDSLSSTASAL | J21 | ELDKYFKNHTSPDVD |
| G6 | TNFTISVTTEILPVS | 12 | DSLSSTASALGKLQD | J22 | FKNHTSPDVDLGDIS |
| G7 | SVTTEILPVSMTKTS | 13 | TASALGKLQDVVNQN | J23 | SPDVDLGDISGINAS |
| G8 | ILPVSMTKTSVDCTM | 14 | GKLQDVVNQNAQALN | J24 | LGDISGINASVVNIQ |
| G9 | MTKTSVDCTMYICGD | I5 | WNQNAQALNTLVKQ | K1 | GINASWNIQKEIDR |
| G10 | VDCTMYICGDSTECS | I6 | AQALNTLVKQLSSNF | K2 | VVNIQKEIDRLNEVA |
| G11 | YICGDSTECSNLLLQ | 17 | TLVKQLSSNFGAISS | K3 | KEIDRLNEVAKNLNE |
| G12 | STECSNLLLQYGSFC | 18 | LSSNFGAISSVLNDI | K4 | LNEVAKNLNESLIDL |
| G13 | NLLLQYGSFCTQLNR | 19 | GAISSVLNDILSRLD | K5 | KNLNESLIDLQELGK |
| G14 | YGSFCTQLNRALTGI | I10 | VLNDILSRLDKVEAE | K6 | SLIDLQELGKYEQYI |
| G15 | TQLNRALTGIAVEQD | I11 | LSRLDKVEAEVQIDR | K7 | QELGKYEQYIKWPWY |
| G16 | ALTGIAVEQDKNTQE | 112 | KVEAEVQIDRLITGR | K8 | YEQYIKWPWYIWLGF |
| G17 | AVEQDKNTQEVFAQV | 113 | VQIDRLITGRLQSLQ | K9 | KWPWYIWLGFIAGLI |
| G18 | KNTQEVFAQVKQIYK | 114 | LITGRLQSLQTYVTQ | K10 | IWLGFIAGLIAIVMV |
| G19 | VFAQVKQIYKTPPIK | 115 | LQSLQTYVTQQLIRA | K11 | IAGLIAIVMVTIMLC |
| G20 | KQIYKTPPIKDFGGF | 116 | TYVTQQLIRAAEIRA | K12 | AIVMVTIMLCCMTSC |
| G21 | TPPIKDFGGFNFSQI | 117 | QLIRAAEIRASANLA | K13 | TIMLCCMTSCCSCLK |
| G22 | DFGGFNFSQILPDPS | 118 | AEIRASANLAATKMS | K14 | CMTSCCSCLKGCCSC |
| G23 | NFSQILPDPSKPSKR | 119 | SANLAATKMSECVLG | K15 | CSCLKGCCSCGSCCK |
| G24 | LPDPSKPSKRSFIED | 120 | ATKMSECVLGQSKRV | K16 | GCCSCGSCCKFDEDD |
| H1 | KPSKRSFIEDLLFNK | 121 | ECVLGQSKRVDFCGK | K17 | GSCCKFDEDDSEPVL |
| H2 | SFIEDLLFNKVTLAD | 122 | QSKRVDFCGKGYHLM | K18 | FDEDDSEPVLKGVKL |
| H3 | LLFNKVTLADAGFIK | 123 | DFCGKGYHLMSFPQS | K19 | DDSEPVLKGVKLHYT |
| H4 | VTLADAGFIKQYGDC | 124 | GYHLMSFPQSAPHGV | K20 | |
| H5 | AGFIKQYGDCLGDIA | J1 | SFPQSAPHGVVFLHV | K21 | |
| H6 | QYGDCLGDIAARDLI | J2 | APHGVVFLHVTYVPA | K22 | MDLFMRIFTIGTVTL |
| H7 | LGDIAARDLICAQKF | J3 | VFLHVTYVPAQEKNF | K23 | RIFTIGTVTLKQGEI |
| K24 | GTVTLKQGEIKDATP | M20 | FIYNKIVDEPEEHVQ | 016 | LSYYKLGASQRVAGD |
| L1 | KQGEIKDATPSDFVR | M21 | IVDEPEEHVQIHTID | 017 | LGASQRVAGDSGFAA |
| L2 | KDATPSDFVRATATI | M22 | EEHVQIHTIDGSSGV | 018 | RVAGDSGFAAYSRYR |
| L3 | SDFVRATATIPIQAS | M23 | IHTIDGSSGVVNPVM | 019 | SGFAAYSRYRIGNYK |
| L4 | ATATIPIQASLPFGW | M24 | GSSGVVNPVMEPIYD | O20 | YSRYRIGNYKLNTDH |
| L5 | PIQASLPFGWLIVGV | N1 | VNPVMEPIYDEPTTT | 021 | IGNYKLNTDHSSSSD |
| L6 | LPFGWLIVGVALLAV | N2 | EPIYDEPTTTTSVPL | O22 | LNTDHSSSSDNIALL |
| L7 | LIVGVALLAVFQSAS | N3 | | O23 | TDHSSSSDNIALLVQ |
| L8 | ALLAVFQSASKIITL | N4 | | O24 | |
| L9 | FQSASKIITLKKRWQ | N5 | MADSNGTITVEELKK | P1 | |
| L10 | KIITLKKRWQLALSK | N6 | GTITVEELKKLLEQW | P2 | MFHLVDFQVTIAEIL |
| L11 | KKRWQLALSKGVHFV | N7 | EELKKLLEQWNLVIG | P3 | DFQVTIAEILLIIMR |
| L12 | LALSKGVHFVCNLLL | N8 | LLEQWNLVIGFLFLT | P4 | IAEILLIIMRTFKVS |
| L13 | GVHFVCNLLLLFVTV | N9 | NLVIGFLFLTWICLL | P5 | LIIMRTFKVSIWNLD |
| L14 | CNLLLLFVTVYSHLL | N10 | FLFLTWICLLQFAYA | P6 | TFKVSIWNLDYIINL |
| L15 | LFVTVYSHLLLVAAG | N11 | WICLLQFAYANRNRF | P7 | IWNLDYIINLIIKNL |
| L16 | YSHLLLVAAGLEAPF | N12 | QFAYANRNRFLYIIK | P8 | YIINLIIKNLSKSLT |
| L17 | LVAAGLEAPFLYLYA | N13 | NRNRFLYIIKLIFLW | P9 | IIKNLSKSLTENKYS |
| L18 | LEAPFLYLYALVYFL | N14 | LYIIKLIFLWLLWPV | P10 | SKSLTENKYSQLDEE |
| L19 | LYLYALVYFLQSINF | N15 | LIFLWLLWPVTLACF | P11 | ENKYSQLDEEQPMEI |
| L20 | LVYFLQSINFVRIIM | N16 | LLWPVTLACFVLAAV | P12 | NKYSQLDEEQPMEID |
| L21 | QSINFVRIIMRLWLC | N17 | TLACFVLAAVYRINW | P13 | |
| L22 | VRIIMRLWLCWKCRS | N18 | VLAAVYRINWITGGI | P14 | |
| L23 | RLWLCWKCRSKNPLL | N19 | YRINWITGGIAIAMA | P15 | MKIILFLALITLATC |
| L24 | WKCRSKNPLLYDANY | N20 | ITGGIAIAMACLVGL | P16 | FLALITLATCELYHY |
| M1 | KNPLLYDANYFLCWH | N21 | AIAMACLVGLMWLSY | P17 | TLATCELYHYQECVR |
| M2 | YDANYFLCWHTNCYD | N22 | CLVGLMWLSYFIASF | P18 | ELYHYQECVRGTTVL |
| M3 | FLCWHTNCYDYCIPY | N23 | MWLSYFIASFRLFAR | P19 | QECVRGTTVLLKEPC |
| M4 | TNCYDYCIPYNSVTS | N24 | FIASFRLFARTRSMW | P20 | GTTVLLKEPCSSGTY |
| M5 | YCIPYNSVTSSIVIT | O1 | RLFARTRSMWSFNPE | P21 | LKEPCSSGTYEGNSP |
| M6 | NSVTSSIVITSGDGT | O2 | TRSMWSFNPETNILL | P22 | SSGTYEGNSPFHPLA |
| M7 | SIVITSGDGTTSPIS | O3 | SFNPETNILLNVPLH | P23 | EGNSPFHPLADNKFA |
| M8 | SGDGTTSPISEHDYQ | O4 | TNILLNVPLHGTILT | P24 | FHPLADNKFALTCFS |
| M9 | TSPISEHDYQIGGYT | O5 | NVPLHGTILTRPLLE | Q 1 | DNKFALTCFSTQFAF |
| M10 | EHDYQIGGYTEKWES | O6 | GTILTRPLLESELVI | Q 2 | LTCFSTQFAFACPDG |
| M11 | IGGYTEKWESGVKDC | O7 | RPLLESELVIGAVIL | Q 3 | TQFAFACPDGVKHVY |
| M12 | EKWESGVKDCVVLHS | O8 | SELVIGAVILRGHLR | Q 4 | ACPDGVKHVYQLRAR |
| M13 | GVKDCVVLHSYFTSD | O9 | GAVILRGHLRIAGHH | Q 5 | VKHVYQLRARSVSPK |
| M14 | VVLHSYFTSDYYQLY | O10 | RGHLRIAGHHLGRCD | Q 6 | QLRARSVSPKLFIRQ |
| M15 | YFTSDYYQLYSTQLS | O11 | IAGHHLGRCDIKDLP | Q 7 | SVSPKLFIRQEEVQE |
| M16 | YYQLYSTQLSTDTGV | 012 | LGRCDIKDLPKEITV | Q 8 | LFIRQEEVQELYSPI |
| M17 | STQLSTDTGVEHVTF | 013 | IKDLPKEITVATSRT | Q 9 | EEVQELYSPIFLIVA |
| M18 | TDTGVEHVTFFIYNK | 014 | KEITVATSRTLSYYK | Q10 | LYSPIFLIVAAIVFI |
| M19 | EHVTFFIYNKIVDEP | 015 | ATSRTLSYYKLGASQ | Q11 | FLIVAAIVFITLCFT |
| Q12 | AIVFITLCFTLKRKT | S 8 | TNSSPDDQIGYYRRA | U 4 | TDYKHWPQIAQFAPS |
| Q13 | IVFITLCFTLKRKTE | S 9 | DDQIGYYRRATRRIR | U 5 | WPQIAQFAPSASAFF |
| Q14 | | S10 | YYRRATRRIRGGDGK | U 6 | QFAPSASAFFGMSRI |
| Q15 | | S11 | TRRIRGGDGKMKDLS | U 7 | ASAFFGMSRIGMEVT |
| Q16 | MKFLVFLGIITTVAA | S12 | GGDGKMKDLSPRWYF | U 8 | GMSRIGMEVTPSGTW |
| Q17 | FLGIITTVAAFHQEC | S13 | MKDLSPRWYFYYLGT | U 9 | GMEVTPSGTWLTYTG |
| Q18 | TTVAAFHQECSLQSC | S14 | PRWYFYYLGTGPEAG | U10 | PSGTWLTYTGAIKLD |
| Q19 | FHQECSLQSCTQHQP | S15 | YYLGTGPEAGLPYGA | U11 | LTYTGAIKLDDKDPN |
| Q20 | SLQSCTQHQPYVVDD | S16 | GPEAGLPYGANKDGI | U12 | AIKLDDKDPNFKDQV |
| Q21 | TQHQPYVVDDPCPIH | S17 | LPYGANKDGIIWVAT | U13 | DKDPNFKDQVILLNK |
| Q22 | YVVDDPCPIHFYSKW | S18 | NKDGIIWVATEGALN | U14 | FKDQVILLNKHIDAY |
| Q23 | PCPIHFYSKWYIRVG | S19 | IWVATEGALNTPKDH | U15 | ILLNKHIDAYKTFPP |
| Q24 | FYSKWYIRVGARKSA | S20 | EGALNTPKDHIGTRN | U16 | HIDAYKTFPPTEPKK |
| R 1 | YIRVGARKSAPLIEL | S21 | TPKDHIGTRNPANNA | U17 | KTFPPTEPKKDKKKK |
| R 2 | ARKSAPLIELCVDEA | S22 | IGTRNPANNAAIVLQ | U18 | TEPKKDKKKKADETQ |
| R 3 | PLIELCVDEAGSKSP | S23 | PANNAAIVLQLPQGT | U19 | DKKKKADETQALPQR |
| R 4 | CVDEAGSKSPIQYID | S24 | AIVLQLPQGTTLPKG | U20 | ADETQALPQRQKKQQ |
| R 5 | GSKSPIQYIDIGNYT | T 1 | LPQGTTLPKGFYAEG | U21 | ALPQRQKKQQTVTLL |
| R 6 | IQYIDIGNYTVSCLP | T 2 | TLPKGFYAEGSRGGS | U22 | QKKQQTVTLLPAADL |
| R 7 | IGNYTVSCLPFTINC | T 3 | FYAEGSRGGSQASSR | U23 | TVTLLPAADLDDFSK |
| R 8 | VSCLPFTINCQEPKL | T 4 | SRGGSQASSRSSSRS | U24 | PAADLDDFSKQLQQS |
| R 9 | FTINCQEPKLGSLVV | T 5 | QASSRSSSRSRNSSR | V 1 | DDFSKQLQQSMSSAD |
| R10 | QEPKLGSLVVRCSFY | T 6 | SSSRSRNSSRNSTPG | V 2 | KQLQQSMSSADSTQA |
| R11 | GSLVVRCSFYEDFLE | T 7 | RNSSRNSTPGSSRGT | V 3 | |
| R12 | RCSFYEDFLEYHDVR | T 8 | NSTPGSSRGTSPARM | V 4 | |
| R13 | EDFLEYHDVRVVLDF | T 9 | SSRGTSPARMAGNGG | V 5 | IHLVNNESSEVIVHK |
| R14 | DFLEYHDVRVVLDFI | T10 | SPARMAGNGGDAALA | V 6 | GYPKDGNAFNNLDRI |
| R15 | | T11 | AGNGGDAALALLLLD | V 7 | KEVPALTAVETGATN |
| R16 | | T12 | DAALALLLLDRLNQL | V 8 | YPYDVPDYAGYPYDV |
| R17 | MSDNGPQNQRNAPRI | T13 | LLLLDRLNQLESKMS | V 9 | |
| R18 | PQNQRNAPRITFGGP | T14 | RLNQLESKMSGKGQQ | V 10 | |
| R19 | NAPRITFGGPSDSTG | T15 | ESKMSGKGQQQQGQT | V 11 | |
| R20 | TFGGPSDSTGSNQNG | T16 | GKGQQQQGQTVTKKS | V 12 | |
| R21 | SDSTGSNQNGERSGA | T17 | QQGQTVTKKSAAEAS | V 13 | |
| R22 | SNQNGERSGARSKQR | T18 | VTKKSAAEASKKPRQ | V 14 | |
| R23 | ERSGARSKQRRPQGL | T19 | AAEASKKPRQKRTAT | V 15 | |
| R24 | RSKQRRPQGLPNNTA | T20 | KKPRQKRTATKAYNV | V 16 | |
| S 1 | RPQGLPNNTASWFTA | T21 | KRTATKAYNVTQAFG | V 17 | |
| S 2 | PNNTASWFTALTQHG | T22 | KAYNVTQAFGRRGPE | V 18 | |
| S 3 | SWFTALTQHGKEDLK | T23 | TQAFGRRGPEQTQGN | V 19 | |
| S 4 | LTQHGKEDLKFPRGQ | T24 | RRGPEQTQGNFGDQE | V 20 | |
| S 5 | KEDLKFPRGQGVPIN | U 1 | QTQGNFGDQELIRQG | V 21 | |
| S 6 | FPRGQGVPINTNSSP | U 2 | FGDQELIRQGTDYKH | V 22 | |
| S 7 | GVPINTNSSPDDQIG | U 3 | LIRQGTDYKHWPQIA | V 23 | |
| V 24 | | W8 | ALRLCAYCCNIVNVS | W16 | VFAFPFTIYSLLLCR |
| W1 | MYSFVSEETGTLIVN | W9 | AYCCNIVNVSLVKPS | W17 | FTIYSLLLCRMNSRN |
| W2 | SEETGTLIVNSVLLF | W10 | IVNVSLVKPSFYVYS | W18 | LLLCRMNSRNYIAQV |
| W3 | TLIVNSVLLFLAFVV | W11 | LVKPSFYVYSRVKNL | W19 | MNSRNYIAQVDVVNF |
| W4 | SVLLFLAFVVFLLVT | W12 | FYVYSRVKNLNSSRV | W20 | RNYIAQVDVVNFNLT |
| W5 | LAFVVFLLVTLAILT | W13 | RVKNLNSSRVPDLLV | | |
| W6 | FLLVTLAILTALRLC | W14 | | | |
| W7 | LAILTALRLCAYCCN | W15 | MGYINVFAFPFTIYS | | |

**Table 19 - List of SARS-CoV-2 polyamino acids synthesized for mapping IgG-reactive epitopes from patient sera in Figure 13**

| | | | | | |
|---|---|---|---|---|---|
| Spot | Polypeptide | B4 | DSKTQSLLIVNNATN | C8 | PGDSSSGWTAGAAAY |
| A1 | IHLVNNESSEVIVHK | B5 | SLLIVNNATNVVIKV | C9 | SGWTAGAAAYYVGYL |
| A2 | GYPKDGNAFNNLDRI | B6 | NNATNVVIKVCEFQF | C10 | GAAAYYVGYLQPRTF |
| A3 | KEVPALTAVETGATN | B7 | VVIKVCEFQFCNDPF | C11 | YVGYLQPRTFLLKYN |
| A4 | YPYDVPDYAGYPYDV | B8 | CEFQFCNDPFLGVYY | C12 | QPRTFLLKYNENGTI |
| A5 | | B9 | CNDPFLGVYYHKNNK | C13 | LLKYNENGTITDAVD |
| A6 | MFVFLVLLPLVSSQC | B10 | LGVYYHKNNKSWMES | C14 | ENGTITDAVDCALDP |
| A7 | VLLPLVSSQCVNLTT | B11 | HKNNKSWMESEFRVY | C15 | TDAVDCALDPLSETK |
| A8 | VSSQCVNLTTRTQLP | B12 | SWMESEFRVYSSANN | C16 | CALDPLSETKCTLKS |
| A9 | VNLTTRTQLPPAYTN | B13 | EFRVYSSANNCTFEY | C17 | LSETKCTLKSFTVEK |
| A10 | RTQLPPAYTNSFTRG | B14 | SSANNCTFEYVSQPF | C18 | CTLKSFTVEKGIYQT |
| A11 | PAYTNSFTRGVYYPD | B15 | CTFEYVSQPFLMDLE | C19 | FTVEKGIYQTSNFRV |
| A12 | SFTRGVYYPDKVFRS | B16 | VSQPFLMDLEGKQGN | C20 | GIYQTSNFRVQPTES |
| A13 | VYYPDKVFRSSVLHS | B17 | LMDLEGKQGNFKNLR | C21 | SNFRVQPTESIVRFP |
| A14 | KVFRSSVLHSTQDLF | B18 | GKQGNFKNLREFVFK | C22 | QPTESIVRFPNITNL |
| A15 | SVLHSTQDLFLPFFS | B19 | FKNLREFVFKNIDGY | C23 | IVRFPNITNLCPFGE |
| A16 | TQDLFLPFFSNVTWF | B20 | EFVFKNIDGYFKIYS | C24 | NITNLCPFGEVFNAT |
| A17 | LPFFSNVTWFHAIHV | B21 | NIDGYFKIYSKHTPI | D1 | CPFGEVFNATRFASV |
| A18 | NVTWFHAIHVSGTNG | B22 | FKIYSKHTPINLVRD | D2 | VFNATRFASVYAWNR |
| A19 | HAIHVSGTNGTKRFD | B23 | KHTPINLVRDLPQGF | D3 | RFASVYAWNRKRISN |
| A20 | SGTNGTKRFDNPVLP | B24 | NLVRDLPQGFSALEP | D4 | YAWNRKRISNCVADY |
| A21 | TKRFDNPVLPFNDGV | C1 | LPQGFSALEPLVDLP | D5 | KRISNCVADYSVLYN |
| A22 | NPVLPFNDGVYFAST | C2 | SALEPLVDLPIGINI | D6 | CVADYSVLYNSASFS |
| A23 | FNDGVYFASTEKSNI | C3 | LVDLPIGINITRFQT | D7 | SVLYNSASFSTFKCY |
| A24 | YFASTEKSNIIRGWI | C4 | IGINITRFQTLLALH | D8 | SASFSTFKCYGVSPT |
| B1 | EKSNIIRGWIFGTTL | C5 | TRFQTLLALHRSYLT | D9 | TFKCYGVSPTKLNDL |
| B2 | IRGWIFGTTLDSKTQ | C6 | LLALHRSYLTPGDSS | D10 | GVSPTKLNDLCFTNV |
| B3 | FGTTLDSKTQSLLIV | C7 | RSYLTPGDSSSGWTA | D11 | KLNDLCFTNVYADSF |
| D12 | CFTNVYADSFVIRGD | F2 | TLEILDITPCSFGGV | G16 | AVEQDKNTQEVFAQV |
| D13 | YADSFVIRGDEVRQI | F3 | DITPCSFGGVSVITP | G17 | KNTQEVFAQVKQIYK |
| D14 | VIRGDEVRQIAPGQT | F4 | SFGGVSVITPGTNTS | G18 | VFAQVKQIYKTPPIK |
| D15 | EVRQIAPGQTGKIAD | F5 | SVITPGTNTSNQVAV | G19 | KQIYKTPPIKDFGGF |
| D16 | APGQTGKIADYNYKL | F6 | GTNTSNQVAVLYQDV | G20 | TPPIKDFGGFNFSQI |
| D17 | GKIADYNYKLPDDFT | F7 | NQVAVLYQDVNCTEV | G21 | DFGGFNFSQILPDPS |
| D18 | YNYKLPDDFTGCVIA | F8 | LYQDVNCTEVPVAIH | G22 | NFSQILPDPSKPSKR |
| D19 | PDDFTGCVIAWNSNN | F9 | NCTEVPVAIHADQLT | G23 | LPDPSKPSKRSFIED |
| D20 | GCVIAWNSNNLDSKV | F10 | PVAIHADQLTPTWRV | G24 | KPSKRSFIEDLLFNK |
| D21 | WNSNNLDSKVGGNYN | F11 | ADQLTPTWRVYSTGS | H1 | SFIEDLLFNKVTLAD |
| D22 | LDSKVGGNYNYLYRL | F12 | PTWRVYSTGSNVFQT | H2 | LLFNKVTLADAGFIK |
| D23 | GGNYNYLYRLFRKSN | F13 | YSTGSNVFQTRAGCL | H3 | VTLADAGFIKQYGDC |
| D24 | YLYRLFRKSNLKPFE | F14 | NVFQTRAGCLIGAEH | H4 | AGFIKQYGDCLGDIA |
| E1 | FRKSNLKPFERDIST | F15 | RAGCLIGAEHVNNSY | H5 | QYGDCLGDIAARDLI |
| E2 | LKPFERDISTEIYQA | F16 | IGAEHVNNSYECDIP | H6 | LGDIAARDLICAQKF |
| E3 | RDISTEIYQAGSTPC | F17 | VNNSYECDIPIGAGI | H7 | ARDLICAQKFNGLTV |
| E4 | EIYQAGSTPCNGVEG | F18 | ECDIPIGAGICASYQ | H8 | CAQKFNGLTVLPPLL |
| E5 | GSTPCNGVEGFNCYF | F19 | IGAGICASYQTQTNS | H9 | NGLTVLPPLLTDEMI |
| E6 | NGVEGFNCYFPLQSY | F20 | CASYQTQTNSPRRAR | H10 | LPPLLTDEMIAQYTS |
| E7 | FNCYFPLQSYGFQPT | F21 | TQTNSPRRARSVASQ | H11 | TDEMIAQYTSALLAG |
| E8 | PLQSYGFQPTNGVGY | F22 | PRRARSVASQSIIAY | H12 | AQYTSALLAGTITSG |
| E9 | GFQPTNGVGYQPYRV | F23 | SVASQSIIAYTMSLG | H13 | ALLAGTITSGWTFGA |
| E10 | NGVGYQPYRVVVLSF | F24 | SIIAYTMSLGAENSV | H14 | TITSGWTFGAGAALQ |
| E11 | QPYRVVVLSFELLHA | G1 | TMSLGAENSVAYSNN | H15 | WTFGAGAALQIPFAM |
| E12 | VVLSFELLHAPATVC | G2 | AENSVAYSNNSIAIP | H16 | GAALQIPFAMQMAYR |
| E13 | ELLHAPATVCGPKKS | G3 | AYSNNSIAIPTNFTI | H17 | IPFAMQMAYRFNGIG |
| E14 | PATVCGPKKSTNLVK | G4 | SIAIPTNFTISVTTE | H18 | QMAYRFNGIGVTQNV |
| E15 | GPKKSTNLVKNKCVN | G5 | TNFTISVTTEILPVS | H19 | FNGIGVTQNVLYENQ |
| E16 | TNLVKNKCVNFNFNG | G6 | SVTTEILPVSMTKTS | H20 | VTQNVLYENQKLIAN |
| E17 | NKCVNFNFNGLTGTG | G7 | ILPVSMTKTSVDCTM | H21 | LYENQKLIANQFNSA |
| E18 | FNFNGLTGTGVLTES | G8 | MTKTSVDCTMYICGD | H22 | KLIANQFNSAIGKIQ |
| E19 | LTGTGVLTESNKKFL | G9 | VDCTMYICGDSTECS | H23 | QFNSAIGKIQDSLSS |
| E20 | VLTESNKKFLPFQQF | G10 | YICGDSTECSNLLLQ | H24 | IGKIQDSLSSTASAL |
| E21 | NKKFLPFQQFGRDIA | G11 | STECSNLLLQYGSFC | I1 | DSLSSTASALGKLQD |
| E22 | PFQQFGRDIADTTDA | G12 | NLLLQYGSFCTQLNR | 12 | TASALGKLQDWNQN |
| E23 | GRDIADTTDAVRDPQ | G13 | YGSFCTQLNRALTGI | 13 | GKLQDVVNQNAQALN |
| E24 | DTTDAVRDPQTLEIL | G14 | TQLNRALTGIAVEQD | 14 | VVNQNAQALNTLVKQ |
| F1 | VRDPQTLEILDITPC | G15 | ALTGIAVEQDKNTQE | I5 | AQALNTLVKQLSSNF |
| 16 | TLVKQLSSNFGAISS | J20 | ELDKYFKNHTSPDVD | L10 | KKRWQLALSKGVHFV |
| 17 | LSSNFGAISSVLNDI | J21 | FKNHTSPDVDLGDIS | L11 | LALSKGVHFVCNLLL |
| 18 | GAISSVLNDILSRLD | J22 | SPDVDLGDISGINAS | L12 | GVHFVCNLLLLFVTV |
| 19 | VLNDILSRLDKVEAE | J23 | LGDISGINASVVNIQ | L13 | CNLLLLFVTVYSHLL |
| I10 | LSRLDKVEAEVQIDR | J24 | GINASVVNIQKEIDR | L14 | LFVTVYSHLLLVAAG |
| I11 | KVEAEVQIDRLITGR | K1 | VVNIQKEIDRLNEVA | L15 | YSHLLLVAAGLEAPF |
| 112 | VQIDRLITGRLQSLQ | K2 | KEIDRLNEVAKNLNE | L16 | LVAAGLEAPFLYLYA |
| 113 | LITGRLQSLQTYVTQ | K3 | LNEVAKNLNESLIDL | L17 | LEAPFLYLYALVYFL |
| 114 | LQSLQTYVTQQLIRA | K4 | KNLNESLIDLQELGK | L18 | LYLYALVYFLQSINF |
| 115 | TYVTQQLIRAAEIRA | K5 | SLIDLQELGKYEQYI | L19 | LVYFLQSINFVRIIM |
| 116 | QLIRAAEIRASANLA | K6 | QELGKYEQYIKWPWY | L20 | QSINFVRIIMRLWLC |
| 117 | AEIRASANLAATKMS | K7 | YEQYIKWPWYIWLGF | L21 | VRIIMRLWLCWKCRS |
| 118 | SANLAATKMSECVLG | K8 | KWPWYIWLGFIAGLI | L22 | RLWLCWKCRSKNPLL |
| 119 | ATKMSECVLGQSKRV | K9 | IWLGFIAGLIAIVMV | L23 | WKCRSKNPLLYDANY |
| 120 | ECVLGQSKRVDFCGK | K10 | IAGLIAIVMVTIMLC | L24 | KNPLLYDANYFLCWH |
| 121 | QSKRVDFCGKGYHLM | K11 | AIVMVTIMLCCMTSC | M1 | YDANYFLCWHTNCYD |
| 122 | DFCGKGYHLMSFPQS | K12 | TIMLCCMTSCCSCLK | M2 | FLCWHTNCYDYCIPY |
| 123 | GYHLMSFPQSAPHGV | K13 | CMTSCCSCLKGCCSC | M3 | TNCYDYCIPYNSVTS |
| 124 | SFPQSAPHGVVFLHV | K14 | CSCLKGCCSCGSCCK | M4 | YCIPYNSVTSSIVIT |
| J1 | APHGVVFLHVTYVPA | K15 | GCCSCGSCCKFDEDD | M5 | NSVTSSIVITSGDGT |
| J2 | VFLHVTYVPAQEKNF | K16 | GSCCKFDEDDSEPVL | M6 | SIVITSGDGTTSPIS |
| J3 | TYVPAQEKNFTTAPA | K17 | FDEDDSEPVLKGVKL | M7 | SGDGTTSPISEHDYQ |
| J4 | QEKNFTTAPAICHDG | K18 | DDSEPVLKGVKLHYT | M8 | TSPISEHDYQIGGYT |
| J5 | TTAPAICHDGKAHFP | K19 | | M9 | EHDYQIGGYTEKWES |
| J6 | ICHDGKAHFPREGVF | K20 | | M10 | IGGYTEKWESGVKDC |
| J7 | KAHFPREGVFVSNGT | K21 | MDLFMRIFTIGTVTL | M11 | EKWESGVKDCVVLHS |
| J8 | REGVFVSNGTHWFVT | K22 | RIFTIGTVTLKQGEI | M12 | GVKDCVVLHSYFTSD |
| J9 | VSNGTHWFVTQRNFY | K23 | GTVTLKQGEIKDATP | M13 | VVLHSYFTSDYYQLY |
| J10 | HWFVTQRNFYEPQII | K24 | KQGEIKDATPSDFVR | M14 | YFTSDYYQLYSTQLS |
| J11 | QRNFYEPQIITTDNT | L1 | KDATPSDFVRATATI | M15 | YYQLYSTQLSTDTGV |
| J12 | EPQIITTDNTFVSGN | L2 | SDFVRATATIPIQAS | M16 | STQLSTDTGVEHVTF |
| J13 | TTDNTFVSGNCDVVI | L3 | ATATIPIQASLPFGW | M17 | TDTGVEHVTFFIYNK |
| J14 | FVSGNCDVVIGIVNN | L4 | PIQASLPFGWLIVGV | M18 | EHVTFFIYNKIVDEP |
| J15 | CDVVIGIVNNTVYDP | L5 | LPFGWLIVGVALLAV | M19 | FIYNKIVDEPEEHVQ |
| J16 | GIVNNTVYDPLQPEL | L6 | LIVGVALLAVFQSAS | M20 | IVDEPEEHVQIHTID |
| J17 | TVYDPLQPELDSFKE | L7 | ALLAVFQSASKIITL | M21 | EEHVQIHTIDGSSGV |
| J18 | LQPELDSFKEELDKY | L8 | FQSASKIITLKKRWQ | M22 | IHTIDGSSGVVNPVM |
| J19 | DSFKEELDKYFKNHT | L9 | KIITLKKRWQLALSK | M23 | GSSGVVNPVMEPIYD |
| M24 | VNPVMEPIYDEPTTT | 014 | ATSRTLSYYKLGASQ | Q 4 | VKHVYQLRARSVSPK |
| N1 | EPIYDEPTTTTSVPL | 015 | LSYYKLGASQRVAGD | Q 5 | QLRARSVSPKLFIRQ |
| N2 | | 016 | LGASQRVAGDSGFAA | Q 6 | SVSPKLFIRQEEVQE |
| N3 | | 017 | RVAGDSGFAAYSRYR | Q 7 | LFIRQEEVQELYSPI |
| N4 | MADSNGTITVEELKK | 018 | SGFAAYSRYRIGNYK | Q 8 | EEVQELYSPIFLIVA |
| N5 | GTITVEELKKLLEQW | 019 | YSRYRIGNYKLNTDH | Q 9 | LYSPIFLIVAAIVFI |
| N6 | EELKKLLEQWNLVIG | 020 | IGNYKLNTDHSSSSD | Q10 | FLIVAAIVFITLCFT |
| N7 | LLEQWNLVIGFLFLT | 021 | LNTDHSSSSDNIALL | Q11 | AIVFITLCFTLKRKT |
| N8 | NLVIGFLFLTWICLL | 022 | TDHSSSSDNIALLVQ | Q12 | IVFITLCFTLKRKTE |
| N9 | FLFLTWICLLQFAYA | 023 | | Q13 | |
| N10 | WICLLQFAYANRNRF | 024 | | Q14 | |
| N11 | QFAYANRNRFLYIIK | P1 | MFHLVDFQVTIAEIL | Q15 | MKFLVFLGIITTVAA |
| N12 | NRNRFLYIIKLIFLW | P2 | DFQVTIAEILLIIMR | Q16 | FLGIITTVAAFHQEC |
| N13 | LYIIKLIFLWLLWPV | P3 | IAEILLIIMRTFKVS | Q17 | TTVAAFHQECSLQSC |
| N14 | LIFLWLLWPVTLACF | P4 | LIIMRTFKVSIWNLD | Q18 | FHQECSLQSCTQHQP |
| N15 | LLWPVTLACFVLAAV | P5 | TFKVSIWNLDYIINL | Q19 | SLQSCTQHQPYWDD |
| N16 | TLACFVLAAVYRINW | P6 | IWNLDYIINLIIKNL | Q20 | TQHQPYVVDDPCPIH |
| N17 | VLAAVYRINWITGGI | P7 | YIINLIIKNLSKSLT | Q21 | YVVDDPCPIHFYSKW |
| N18 | YRINWITGGIAIAMA | P8 | IIKNLSKSLTENKYS | Q22 | PCPIHFYSKWYIRVG |
| N19 | ITGGIAIAMACLVGL | P9 | SKSLTENKYSQLDEE | Q23 | FYSKWYIRVGARKSA |
| N20 | AIAMACLVGLMWLSY | P10 | ENKYSQLDEEQPMEI | Q24 | YIRVGARKSAPLIEL |
| N21 | CLVGLMWLSYFIASF | P11 | NKYSQLDEEQPMEID | R 1 | ARKSAPLIELCVDEA |
| N22 | MWLSYFIASFRLFAR | P12 | | R 2 | PLIELCVDEAGSKSP |
| N23 | FIASFRLFARTRSMW | P13 | | R 3 | CVDEAGSKSPIQYID |
| N24 | RLFARTRSMWSFNPE | P14 | MKIILFLALITLATC | R 4 | GSKSPIQYIDIGNYT |
| O1 | TRSMWSFNPETNILL | P15 | FLALITLATCELYHY | R 5 | IQYIDIGNYTVSCLP |
| 02 | SFNPETNILLNVPLH | P16 | TLATCELYHYQECVR | R 6 | IGNYTVSCLPFTINC |
| 03 | TNILLNVPLHGTILT | P17 | ELYHYQECVRGTTVL | R 7 | VSCLPFTINCQEPKL |
| 04 | NVPLHGTILTRPLLE | P18 | QECVRGTTVLLKEPC | R 8 | FTINCQEPKLGSLVV |
| 05 | GTILTRPLLESELVI | P19 | GTTVLLKEPCSSGTY | R 9 | QEPKLGSLVVRCSFY |
| 06 | RPLLESELVIGAVIL | P20 | LKEPCSSGTYEGNSP | R10 | GSLVVRCSFYEDFLE |
| 07 | SELVIGAVILRGHLR | P21 | SSGTYEGNSPFHPLA | R11 | RCSFYEDFLEYHDVR |
| 08 | GAVILRGHLRIAGHH | P22 | EGNSPFHPLADNKFA | R12 | EDFLEYHDVRVVLDF |
| 09 | RGHLRIAGHHLGRCD | P23 | FHPLADNKFALTCFS | R13 | DFLEYHDVRVVLDFI |
| O10 | IAGHHLGRCDIKDLP | P24 | DNKFALTCFSTQFAF | R14 | |
| O11 | LGRCDIKDLPKEITV | Q 1 | LTCFSTQFAFACPDG | R15 | |
| 012 | IKDLPKEITVATSRT | Q 2 | TQFAFACPDGVKHVY | R16 | MSDNGPQNQRNAPRI |
| 013 | KEITVATSRTLSYYK | Q 3 | ACPDGVKHVYQLRAR | R17 | PQNQRNAPRITFGGP |
| R18 | NAPRITFGGPSDSTG | T 5 | SSSRSRNSSRNSTPG | U16 | KTFPPTEPKKDKKKK |
| R19 | TFGGPSDSTGSNQNG | T 6 | RNSSRNSTPGSSRGT | U17 | TEPKKDKKKKADETQ |
| R20 | SDSTGSNQNGERSGA | T 7 | NSTPGSSRGTSPARM | U18 | DKKKKADETQALPQR |
| R21 | SNQNGERSGARSKQR | T 8 | SSRGTSPARMAGNGG | U19 | ADETQALPQRQKKQQ |
| R22 | ERSGARSKQRRPQGL | T 9 | SPARMAGNGGDAALA | U20 | ALPQRQKKQQTVTLL |
| R23 | RSKQRRPQGLPNNTA | T10 | AGNGGDAALALLLLD | U21 | QKKQQTVTLLPAADL |
| R24 | RPQGLPNNTASWFTA | T11 | DAALALLLLDRLNQL | U22 | TVTLLPAADLDDFSK |
| S 1 | PNNTASWFTALTQHG | T12 | LLLLDRLNQLESKMS | U23 | PAADLDDFSKQLQQS |
| S 2 | SWFTALTQHGKEDLK | T13 | RLNQLESKMSGKGQQ | U24 | DDFSKQLQQSMSSAD |
| S 3 | LTQHGKEDLKFPRGQ | T14 | ESKMSGKGQQQQGQT | V 1 | KQLQQSMSSADSTQA |
| S 4 | KEDLKFPRGQGVPIN | T15 | GKGQQQQGQTVTKKS | V 2 | |
| S 5 | FPRGQGVPINTNSSP | T16 | QQGQTVTKKSAAEAS | V 3 | |
| S 6 | GVPINTNSSPDDQIG | T17 | VTKKSAAEASKKPRQ | V 4 | MYSFVSEETGTLIVN |
| S 7 | TNSSPDDQIGYYRRA | T18 | AAEASKKPRQKRTAT | V 5 | SEETGTLIVNSVLLF |
| S 8 | DDQIGYYRRATRRIR | T19 | KKPRQKRTATKAYNV | V 6 | TLIVNSVLLFLAFVV |
| S 9 | YYRRATRRIRGGDGK | T20 | KRTATKAYNVTQAFG | V 7 | 5VLLFLAFVVFLLVT |
| S10 | TRRIRGGDGKMKDLS | T21 | KAYNVTQAFGRRGPE | V 8 | LAFVVFLLVTLAILT |
| S11 | GGDGKMKDLSPRWYF | T22 | TQAFGRRGPEQTQGN | V 9 | FLLVTLAILTALRLC |
| S12 | MKDLSPRWYFYYLGT | T23 | RRGPEQTQGNFGDQE | V 10 | LAILTALRLCAYCCN |
| S13 | PRWYFYYLGTGPEAG | T24 | QTQGNFGDQELIRQG | V 11 | ALRLCAYCCNIVNVS |
| S14 | YYLGTGPEAGLPYGA | U 1 | FGDQELIRQGTDYKH | V 12 | AYCCNIVNVSLVKPS |
| S15 | GPEAGLPYGANKDGI | U 2 | LIRQGTDYKHWPQIA | V 13 | IVNVSLVKPSFYVYS |
| S16 | LPYGANKDGIIWVAT | U 3 | TDYKHWPQIAQFAPS | V 14 | LVKPSFYVYSRVKNL |
| S17 | NKDGIIWVATEGALN | U 4 | WPQIAQFAPSASAFF | V 15 | FYVYSRVKNLNSSRV |
| S18 | IWVATEGALNTPKDH | U 5 | QFAPSASAFFGMSRI | V 16 | RVKNLNSSRVPDLLV |
| S19 | EGALNTPKDHIGTRN | U 6 | ASAFFGMSRIGMEVT | V 17 | |
| S20 | TPKDHIGTRNPANNA | U 7 | GMSRIGMEVTPSGTW | V 18 | |
| S21 | IGTRNPANNAAIVLQ | U 8 | GMEVTPSGTWLTYTG | V 19 | MGYINVFAFPFTIYS |
| S22 | PANNAAIVLQLPQGT | U 9 | PSGTWLTYTGAIKLD | V 20 | VFAFPFTIYSLLLCR |
| S23 | AIVLQLPQGTTLPKG | U10 | LTYTGAIKLDDKDPN | V 21 | FTIYSLLLCRMNSRN |
| S24 | LPQGTTLPKGFYAEG | U11 | AIKLDDKDPNFKDQV | V 22 | LLLCRMNSRNYIAQV |
| T 1 | TLPKGFYAEGSRGGS | U12 | DKDPNFKDQVILLNK | V 23 | MNSRNYIAQVDWNF |
| T 2 | FYAEGSRGGSQASSR | U13 | FKDQVILLNKHIDAY | V 24 | RNYIAQVDVVNFNLT |
| T 3 | SRGGSQASSRSSSRS | U14 | ILLNKHIDAYKTFPP | | |
| T 4 | QASSRSSSRSRNSSR | U15 | HIDAYKTFPPTEPKK | | |

**Table 20 - List of SARS-CoV-2 polyamino acids synthesized for mapping IgA-reactive epitopes from patient sera in Figure 14.**

| | | | | | |
|---|---|---|---|---|---|
| Spot | Polypeptide | B18 | GKQGNFKNLREFVFK | D12 | CFTNVYADSFVIRGD |
| A1 | IHLVNNESSEVIVHK | B19 | FKNLREFVFKNIDGY | D13 | YADSFVIRGDEVRQI |
| A2 | GYPKDGNAFNNLDRI | B20 | EFVFKNIDGYFKIYS | D14 | VIRGDEVRQIAPGQT |
| A3 | KEVPALTAVETGATN | B21 | NIDGYFKIYSKHTPI | D15 | EVRQIAPGQTGKIAD |
| A4 | YPYDVPDYAGYPYDV | B22 | FKIYSKHTPINLVRD | D16 | APGQTGKIADYNYKL |
| A5 | | B23 | KHTPINLVRDLPQGF | D17 | GKIADYNYKLPDDFT |
| A6 | MFVFLVLLPLVSSQC | B24 | NLVRDLPQGFSALEP | D18 | YNYKLPDDFTGCVIA |
| A7 | VLLPLVSSQCVNLTT | C1 | LPQGFSALEPLVDLP | D19 | PDDFTGCVIAWNSNN |
| A8 | VSSQCVNLTTRTQLP | C2 | SALEPLVDLPIGINI | D20 | GCVIAWNSNNLDSKV |
| A9 | VNLTTRTQLPPAYTN | C3 | LVDLPIGINITRFQT | D21 | WNSNNLDSKVGGNYN |
| A10 | RTQLPPAYTNSFTRG | C4 | IGINITRFQTLLALH | D22 | LDSKVGGNYNYLYRL |
| A11 | PAYTNSFTRGVYYPD | C5 | TRFQTLLALHRSYLT | D23 | GGNYNYLYRLFRKSN |
| A12 | SFTRGVYYPDKVFRS | C6 | LLALHRSYLTPGDSS | D24 | YLYRLFRKSNLKPFE |
| A13 | VYYPDKVFRSSVLHS | C7 | RSYLTPGDSSSGWTA | E1 | FRKSNLKPFERDIST |
| A14 | KVFRSSVLHSTQDLF | C8 | PGDSSSGWTAGAAAY | E2 | LKPFERDISTEIYQA |
| A15 | SVLHSTQDLFLPFFS | C9 | SGWTAGAAAYYVGYL | E3 | RDISTEIYQAGSTPC |
| A16 | TQDLFLPFFSNVTWF | C10 | GAAAYYVGYLQPRTF | E4 | EIYQAGSTPCNGVEG |
| A17 | LPFFSNVTWFHAIHV | C11 | YVGYLQPRTFLLKYN | E5 | GSTPCNGVEGFNCYF |
| A18 | NVTWFHAIHVSGTNG | C12 | QPRTFLLKYNENGTI | E6 | NGVEGFNCYFPLQSY |
| A19 | HAIHVSGTNGTKRFD | C13 | LLKYNENGTITDAVD | E7 | FNCYFPLQSYGFQPT |
| A20 | SGTNGTKRFDNPVLP | C14 | ENGTITDAVDCALDP | E8 | PLQSYGFQPTNGVGY |
| A21 | TKRFDNPVLPFNDGV | C15 | TDAVDCALDPLSETK | E9 | GFQPTNGVGYQPYRV |
| A22 | NPVLPFNDGVYFAST | C16 | CALDPLSETKCTLKS | E10 | NGVGYQPYRVVVLSF |
| A23 | FNDGVYFASTEKSNI | C17 | LSETKCTLKSFTVEK | E11 | QPYRVVVLSFELLHA |
| A24 | YFASTEKSNIIRGWI | C18 | CTLKSFTVEKGIYQT | E12 | VVLSFELLHAPATVC |
| B1 | EKSNIIRGWIFGTTL | C19 | FTVEKGIYQTSNFRV | E13 | ELLHAPATVCGPKKS |
| B2 | IRGWIFGTTLDSKTQ | C20 | GIYQTSNFRVQPTES | E14 | PATVCGPKKSTNLVK |
| B3 | FGTTLDSKTQSLLIV | C21 | SNFRVQPTESIVRFP | E15 | GPKKSTNLVKNKCVN |
| B4 | DSKTQSLLIVNNATN | C22 | QPTESIVRFPNITNL | E16 | TNLVKNKCVNFNFNG |
| B5 | SLLIVNNATNVVIKV | C23 | IVRFPNITNLCPFGE | E17 | NKCVNFNFNGLTGTG |
| B6 | NNATNVVIKVCEFQF | C24 | NITNLCPFGEVFNAT | E18 | FNFNGLTGTGVLTES |
| B7 | VVIKVCEFQFCNDPF | D1 | CPFGEVFNATRFASV | E19 | LTGTGVLTESNKKFL |
| B8 | CEFQFCNDPFLGVYY | D2 | VFNATRFASVYAWNR | E20 | VLTESNKKFLPFQQF |
| B9 | CNDPFLGVYYHKNNK | D3 | RFASVYAWNRKRISN | E21 | NKKFLPFQQFGRDIA |
| B10 | LGVYYHKNNKSWMES | D4 | YAWNRKRISNCVADY | E22 | PFQQFGRDIADTTDA |
| B11 | HKNNKSWMESEFRVY | D5 | KRISNCVADYSVLYN | E23 | GRDIADTTDAVRDPQ |
| B12 | SWMESEFRVYSSANN | D6 | CVADYSVLYNSASFS | E24 | DTTDAVRDPQTLEIL |
| B13 | EFRVYSSANNCTFEY | D7 | SVLYNSASFSTFKCY | F1 | VRDPQTLEILDITPC |
| B14 | SSANNCTFEYVSQPF | D8 | SASFSTFKCYGVSPT | F2 | TLEILDITPCSFGGV |
| B15 | CTFEYVSQPFLMDLE | D9 | TFKCYGVSPTKLNDL | F3 | DITPCSFGGVSVITP |
| B16 | VSQPFLMDLEGKQGN | D10 | GVSPTKLNDLCFTNV | F4 | SFGGVSVITPGTNTS |
| B17 | LMDLEGKQGNFKNLR | D11 | KLNDLCFTNVYADSF | F5 | SVITPGTNTSNQVAV |
| F6 | GTNTSNQVAVLYQDV | H2 | LLFNKVTLADAGFIK | 122 | DFCGKGYHLMSFPQS |
| F7 | NQVAVLYQDVNCTEV | H3 | VTLADAGFIKQYGDC | 123 | GYHLMSFPQSAPHGV |
| F8 | LYQDVNCTEVPVAIH | H4 | AGFIKQYGDCLGDIA | 124 | SFPQSAPHGVVFLHV |
| F9 | NCTEVPVAIHADQLT | H5 | QYGDCLGDIAARDLI | J1 | APHGVVFLHVTYVPA |
| F10 | PVAIHADQLTPTWRV | H6 | LGDIAARDLICAQKF | J2 | VFLHVTYVPAQEKNF |
| F11 | ADQLTPTWRVYSTGS | H7 | ARDLICAQKFNGLTV | J3 | TYVPAQEKNFTTAPA |
| F12 | PTWRVYSTGSNVFQT | H8 | CAQKFNGLTVLPPLL | J4 | QEKNFTTAPAICHDG |
| F13 | YSTGSNVFQTRAGCL | H9 | NGLTVLPPLLTDEMI | J5 | TTAPAICHDGKAHFP |
| F14 | NVFQTRAGCLIGAEH | H10 | LPPLLTDEMIAQYTS | J6 | ICHDGKAHFPREGVF |
| F15 | RAGCLIGAEHVNNSY | H11 | TDEMIAQYTSALLAG | J7 | KAHFPREGVFVSNGT |
| F16 | IGAEHVNNSYECDIP | H12 | AQYTSALLAGTITSG | J8 | REGVFVSNGTHWFVT |
| F17 | VNNSYECDIPIGAGI | H13 | ALLAGTITSGWTFGA | J9 | VSNGTHWFVTQRNFY |
| F18 | ECDIPIGAGICASYQ | H14 | TITSGWTFGAGAALQ | J10 | HWFVTQRNFYEPQII |
| F19 | IGAGICASYQTQTNS | H15 | WTFGAGAALQIPFAM | J11 | QRNFYEPQIITTDNT |
| F20 | CASYQTQTNSPRRAR | H16 | GAALQIPFAMQMAYR | J12 | EPQIITTDNTFVSGN |
| F21 | TQTNSPRRARSVASQ | H17 | IPFAMQMAYRFNGIG | J13 | TTDNTFVSGNCDVVI |
| F22 | PRRARSVASQSIIAY | H18 | QMAYRFNGIGVTQNV | J14 | FVSGNCDVVIGIVNN |
| F23 | SVASQSIIAYTMSLG | H19 | FNGIGVTQNVLYENQ | J15 | CDVVIGIVNNTVYDP |
| F24 | SIIAYTMSLGAENSV | H20 | VTQNVLYENQKLIAN | J16 | GIVNNTVYDPLQPEL |
| G1 | TMSLGAENSVAYSNN | H21 | LYENQKLIANQFNSA | J17 | TVYDPLQPELDSFKE |
| G2 | AENSVAYSNNSIAIP | H22 | KLIANQFNSAIGKIQ | J18 | LQPELDSFKEELDKY |
| G3 | AYSNNSIAIPTNFTI | H23 | QFNSAIGKIQDSLSS | J19 | DSFKEELDKYFKNHT |
| G4 | SIAIPTNFTISVTTE | H24 | IGKIQDSLSSTASAL | J20 | ELDKYFKNHTSPDVD |
| G5 | TNFTISVTTEILPVS | I1 | DSLSSTASALGKLQD | J21 | FKNHTSPDVDLGDIS |
| G6 | SVTTEILPVSMTKTS | 12 | TASALGKLQDVVNQN | J22 | SPDVDLGDISGINAS |
| G7 | ILPVSMTKTSVDCTM | 13 | GKLQDVVNQNAQALN | J23 | LGDISGINASVVNIQ |
| G8 | MTKTSVDCTMYICGD | 14 | VVNQNAQALNTLVKQ | J24 | GINASVVNIQKEIDR |
| G9 | VDCTMYICGDSTECS | I5 | AQALNTLVKQLSSNF | K1 | VVNIQKEIDRLNEVA |
| G10 | YICGDSTECSNLLLQ | 16 | TLVKQLSSNFGAISS | K2 | KEIDRLNEVAKNLNE |
| G11 | STECSNLLLQYGSFC | 17 | LSSNFGAISSVLNDI | K3 | LNEVAKNLNESLIDL |
| G12 | NLLLQYGSFCTQLNR | 18 | GAISSVLNDILSRLD | K4 | KNLNESLIDLQELGK |
| G13 | YGSFCTQLNRALTGI | 19 | VLNDILSRLDKVEAE | K5 | SLIDLQELGKYEQYI |
| G14 | TQLNRALTGIAVEQD | I10 | LSRLDKVEAEVQIDR | K6 | QELGKYEQYIKWPWY |
| G15 | ALTGIAVEQDKNTQE | I11 | KVEAEVQIDRLITGR | K7 | YEQYIKWPWYIWLGF |
| G16 | AVEQDKNTQEVFAQV | 112 | VQIDRLITGRLQSLQ | K8 | KWPWYIWLGFIAGLI |
| G17 | KNTQEVFAQVKQIYK | 113 | LITGRLQSLQTYVTQ | K9 | IWLGFIAGLIAIVMV |
| G18 | VFAQVKQIYKTPPIK | 114 | LQSLQTYVTQQLIRA | K10 | IAGLIAIVMVTIMLC |
| G19 | KQIYKTPPIKDFGGF | 115 | TYVTQQLIRAAEIRA | K11 | AIVMVTIMLCCMTSC |
| G20 | TPPIKDFGGFNFSQI | 116 | QLIRAAEIRASANLA | K12 | TIMLCCMTSCCSCLK |
| G21 | DFGGFNFSQILPDPS | 117 | AEIRASANLAATKMS | K13 | CMTSCCSCLKGCCSC |
| G22 | NFSQILPDPSKPSKR | 118 | SANLAATKMSECVLG | K14 | CSCLKGCCSCGSCCK |
| G23 | LPDPSKPSKRSFIED | 119 | ATKMSECVLGQSKRV | K15 | GCCSCGSCCKFDEDD |
| G24 | KPSKRSFIEDLLFNK | 120 | ECVLGQSKRVDFCGK | K16 | GSCCKFDEDDSEPVL |
| H1 | SFIEDLLFNKVTLAD | 121 | QSKRVDFCGKGYHLM | K17 | FDEDDSEPVLKGVKL |
| K18 | DDSEPVLKGVKLHYT | M14 | YFTSDYYQLYSTQLS | O10 | IAGHHLGRCDIKDLP |
| K19 | | M15 | YYQLYSTQLSTDTGV | O11 | LGRCDIKDLPKEITV |
| K20 | | M16 | STQLSTDTGVEHVTF | 012 | IKDLPKEITVATSRT |
| K21 | MDLFMRIFTIGTVTL | M17 | TDTGVEHVTFFIYNK | 013 | KEITVATSRTLSYYK |
| K22 | RIFTIGTVTLKQGEI | M18 | EHVTFFIYNKIVDEP | 014 | ATSRTLSYYKLGASQ |
| K23 | GTVTLKQGEIKDATP | M19 | FIYNKIVDEPEEHVQ | 015 | LSYYKLGASQRVAGD |
| K24 | KQGEIKDATPSDFVR | M20 | IVDEPEEHVQIHTID | 016 | LGASQRVAGDSGFAA |
| L1 | KDATPSDFVRATATI | M21 | EEHVQIHTIDGSSGV | 017 | RVAGDSGFAAYSRYR |
| L2 | SDFVRATATIPIQAS | M22 | IHTIDGSSGVVNPVM | 018 | SGFAAYSRYRIGNYK |
| L3 | ATATIPIQASLPFGW | M23 | GSSGVVNPVMEPIYD | 019 | YSRYRIGNYKLNTDH |
| L4 | PIQASLPFGWLIVGV | M24 | VNPVMEPIYDEPTTT | 020 | IGNYKLNTDHSSSSD |
| L5 | LPFGWLIVGVALLAV | N1 | EPIYDEPTTTTSVPL | 021 | LNTDHSSSSDNIALL |
| L6 | LIVGVALLAVFQSAS | N2 | | 022 | TDHSSSSDNIALLVQ |
| L7 | ALLAVFQSASKIITL | N3 | | 023 | |
| L8 | FQSASKIITLKKRWQ | N4 | MADSNGTITVEELKK | 024 | |
| L9 | KIITLKKRWQLALSK | N5 | GTITVEELKKLLEQW | P1 | MFHLVDFQVTIAEIL |
| L10 | KKRWQLALSKGVHFV | N6 | EELKKLLEQWNLVIG | P2 | DFQVTIAEILLIIMR |
| L11 | LALSKGVHFVCNLLL | N7 | LLEQWNLVIGFLFLT | P3 | IAEILLIIMRTFKVS |
| L12 | GVHFVCNLLLLFVTV | N8 | NLVIGFLFLTWICLL | P4 | LIIMRTFKVSIWNLD |
| L13 | CNLLLLFVTVYSHLL | N9 | FLFLTWICLLQFAYA | P5 | TFKVSIWNLDYIINL |
| L14 | LFVTVYSHLLLVAAG | N10 | WICLLQFAYANRNRF | P6 | IWNLDYIINLIIKNL |
| L15 | YSHLLLVAAGLEAPF | N11 | QFAYANRNRFLYIIK | P7 | YIINLIIKNLSKSLT |
| L16 | LVAAGLEAPFLYLYA | N12 | NRNRFLYIIKLIFLW | P8 | IIKNLSKSLTENKYS |
| L17 | LEAPFLYLYALVYFL | N13 | LYIIKLIFLWLLWPV | P9 | SKSLTENKYSQLDEE |
| L18 | LYLYALVYFLQSINF | N14 | LIFLWLLWPVTLACF | P10 | ENKYSQLDEEQPMEI |
| L19 | LVYFLQSINFVRIIM | N15 | LLWPVTLACFVLAAV | P11 | NKYSQLDEEQPMEID |
| L20 | QSINFVRIIMRLWLC | N16 | TLACFVLAAVYRINW | P12 | |
| L21 | VRIIMRLWLCWKCRS | N17 | VLAAVYRINWITGGI | P13 | |
| L22 | RLWLCWKCRSKNPLL | N18 | YRINWITGGIAIAMA | P14 | MKIILFLALITLATC |
| L23 | WKCRSKNPLLYDANY | N19 | ITGGIAIAMACLVGL | P15 | FLALITLATCELYHY |
| L24 | KNPLLYDANYFLCWH | N20 | AIAMACLVGLMWLSY | P16 | TLATCELYHYQECVR |
| M1 | YDANYFLCWHTNCYD | N21 | CLVGLMWLSYFIASF | P17 | ELYHYQECVRGTTVL |
| M2 | FLCWHTNCYDYCIPY | N22 | MWLSYFIASFRLFAR | P18 | QECVRGTTVLLKEPC |
| M3 | TNCYDYCIPYNSVTS | N23 | FIASFRLFARTRSMW | P19 | GTTVLLKEPCSSGTY |
| M4 | YCIPYNSVTSSIVIT | N24 | RLFARTRSMWSFNPE | P20 | LKEPCSSGTYEGNSP |
| M5 | NSVTSSIVITSGDGT | O1 | TRSMWSFNPETNILL | P21 | SSGTYEGNSPFHPLA |
| M6 | SIVITSGDGTTSPIS | 02 | SFNPETNILLNVPLH | P22 | EGNSPFHPLADNKFA |
| M7 | SGDGTTSPISEHDYQ | 03 | TNILLNVPLHGTILT | P23 | FHPLADNKFALTCFS |
| M8 | TSPISEHDYQIGGYT | 04 | NVPLHGTILTRPLLE | P24 | DNKFALTCFSTQFAF |
| M9 | EHDYQIGGYTEKWES | 05 | GTILTRPLLESELVI | Q 1 | LTCFSTQFAFACPDG |
| M10 | IGGYTEKWESGVKDC | 06 | RPLLESELVIGAVIL | Q 2 | TQFAFACPDGVKHVY |
| M11 | EKWESGVKDCVVLHS | 07 | SELVIGAVILRGHLR | Q 3 | ACPDGVKHVYQLRAR |
| M12 | GVKDCVVLHSYFTSD | 08 | GAVILRGHLRIAGHH | Q 4 | VKHVYQLRARSVSPK |
| M13 | VVLHSYFTSDYYQLY | 09 | RGHLRIAGHHLGRCD | Q 5 | QLRARSVSPKLFIRQ |
| Q 6 | SVSPKLFIRQEEVQE | S 2 | SWFTALTQHGKEDLK | T22 | TQAFGRRGPEQTQGN |
| Q 7 | LFIRQEEVQELYSPI | S 3 | LTQHGKEDLKFPRGQ | T23 | RRGPEQTQGNFGDQE |
| Q 8 | EEVQELYSPIFLIVA | S 4 | KEDLKFPRGQGVPIN | T24 | QTQGNFGDQELIRQG |
| Q 9 | LYSPIFLIVAAIVFI | S 5 | FPRGQGVPINTNSSP | U 1 | FGDQELIRQGTDYKH |
| Q10 | FLIVAAIVFITLCFT | S 6 | GVPINTNSSPDDQIG | U 2 | LIRQGTDYKHWPQIA |
| Q11 | AIVFITLCFTLKRKT | S 7 | TNSSPDDQIGYYRRA | U 3 | TDYKHWPQIAQFAPS |
| Q12 | IVFITLCFTLKRKTE | S 8 | DDQIGYYRRATRRIR | U 4 | WPQIAQFAPSASAFF |
| Q13 | | S 9 | YYRRATRRIRGGDGK | U 5 | QFAPSASAFFGMSRI |
| Q14 | | S10 | TRRIRGGDGKMKDLS | U 6 | ASAFFGMSRIGMEVT |
| Q15 | MKFLVFLGIITTVAA | S11 | GGDGKMKDLSPRWYF | U 7 | GMSRIGMEVTPSGTW |
| Q16 | FLGIITTVAAFHQEC | S12 | MKDLSPRWYFYYLGT | U 8 | GMEVTPSGTWLTYTG |
| Q17 | TTVAAFHQECSLQSC | S13 | PRWYFYYLGTGPEAG | U 9 | PSGTWLTYTGAIKLD |
| Q18 | FHQECSLQSCTQHQP | S14 | YYLGTGPEAGLPYGA | U10 | LTYTGAIKLDDKDPN |
| Q19 | SLQSCTQHQPYWDD | S15 | GPEAGLPYGANKDGI | U11 | AIKLDDKDPNFKDQV |
| Q20 | TQHQPYVVDDPCPIH | S16 | LPYGANKDGIIWVAT | U12 | DKDPNFKDQVILLNK |
| Q21 | YVVDDPCPIHFYSKW | S17 | NKDGIIWVATEGALN | U13 | FKDQVILLNKHIDAY |
| Q22 | PCPIHFYSKWYIRVG | S18 | IWVATEGALNTPKDH | U14 | ILLNKHIDAYKTFPP |
| Q23 | FYSKWYIRVGARKSA | S19 | EGALNTPKDHIGTRN | U15 | HIDAYKTFPPTEPKK |
| Q24 | YIRVGARKSAPLIEL | S20 | TPKDHIGTRNPANNA | U16 | KTFPPTEPKKDKKKK |
| R 1 | ARKSAPLIELCVDEA | S21 | IGTRNPANNAAIVLQ | U17 | TEPKKDKKKKADETQ |
| R 2 | PLIELCVDEAGSKSP | S22 | PANNAAIVLQLPQGT | U18 | DKKKKADETQALPQR |
| R 3 | CVDEAGSKSPIQYID | S23 | AIVLQLPQGTTLPKG | U19 | ADETQALPQRQKKQQ |
| R 4 | GSKSPIQYIDIGNYT | S24 | LPQGTTLPKGFYAEG | U20 | ALPQRQKKQQTVTLL |
| R 5 | IQYIDIGNYTVSCLP | T 1 | TLPKGFYAEGSRGGS | U21 | QKKQQTVTLLPAADL |
| R 6 | IGNYTVSCLPFTINC | T 2 | FYAEGSRGGSQASSR | U22 | TVTLLPAADLDDFSK |
| R 7 | VSCLPFTINCQEPKL | T 3 | SRGGSQASSRSSSRS | U23 | PAADLDDFSKQLQQS |
| R 8 | FTINCQEPKLGSLVV | T 4 | QASSRSSSRSRNSSR | U24 | DDFSKQLQQSMSSAD |
| R 9 | QEPKLGSLVVRCSFY | T 5 | SSSRSRNSSRNSTPG | V 1 | KQLQQSMSSADSTQA |
| R10 | GSLVVRCSFYEDFLE | T 6 | RNSSRNSTPGSSRGT | V 2 | |
| R11 | RCSFYEDFLEYHDVR | T 7 | NSTPGSSRGTSPARM | V 3 | |
| R12 | EDFLEYHDVRVVLDF | T 8 | SSRGTSPARMAGNGG | V 4 | MYSFVSEETGTLIVN |
| R13 | DFLEYHDVRVVLDFI | T 9 | SPARMAGNGGDAALA | V 5 | SEETGTLIVNSVLLF |
| R14 | | T10 | AGNGGDAALALLLLD | V 6 | TLIVNSVLLFLAFVV |
| R15 | | T11 | DAALALLLLDRLNQL | V 7 | SVLLFLAFVVFLLVT |
| R16 | MSDNGPQNQRNAPRI | T12 | LLLLDRLNQLESKMS | V 8 | LAFVVFLLVTLAILT |
| R17 | PQNQRNAPRITFGGP | T13 | RLNQLESKMSGKGQQ | V 9 | FLLVTLAILTALRLC |
| R18 | NAPRITFGGPSDSTG | T14 | ESKMSGKGQQQQGQT | V 10 | LAILTALRLCAYCCN |
| R19 | TFGGPSDSTGSNQNG | T15 | GKGQQQQGQTVTKKS | V 11 | ALRLCAYCCNIVNVS |
| R20 | SDSTGSNQNGERSGA | T16 | QQGQTVTKKSAAEAS | V 12 | AYCCNIVNVSLVKPS |
| R21 | SNQNGERSGARSKQR | T17 | VTKKSAAEASKKPRQ | V 13 | IVNVSLVKPSFYVYS |
| R22 | ERSGARSKQRRPQGL | T18 | AAEASKKPRQKRTAT | V 14 | LVKPSFYVYSRVKNL |
| R23 | RSKQRRPQGLPNNTA | T19 | KKPRQKRTATKAYNV | V 15 | FYVYSRVKNLNSSRV |
| R24 | RPQGLPNNTASWFTA | T20 | KRTATKAYNVTQAFG | V 16 | RVKNLNSSRVPDLLV |
| S 1 | PNNTASWFTALTQHG | T21 | KAYNVTQAFGRRGPE | V 17 | |
| V 18 | | | | | |
| V 19 | MGYINVFAFPFTIYS | | | | |
| V 20 | VFAFPFTIYSLLLCR | | | | |
| V 21 | FTIYSLLLCRMNSRN | | | | |
| V 22 | LLLCRMNSRNYIAQV | | | | |
| V 23 | MNSRNYIAQVDVVNF | | | | |
| V 24 | RNYIAQVDVVNFNLT | | | | |

### EXAMPLE 20 - Overview of libraries of polyamino acid libraries of SARS-CoV-2 on cellulose membranes

The libraries polypeptide libraries, described in Example 19, were prepared on cellulose membranes Amino-PEG500-UC540 cellulose membranes, according to the standard SPOT synthesis technique, using an Auto-Spot Robot ASP-222 Auto-Spot Robot according to the manufacturer's instructions.

Polyamino acids with a length of 15 residues and an overlap of 10 adjacent residues have been synthesized covering the entire length of the protein.

After synthesis, the free sites of the membranes were blocked with BSA (bovine albumin albumin prepared in TBS-T buffer (50 mM Tris, NaCl; 136 mM, 2 mM KCl; 0.05%, Tween-20; pH 7.4) for 90 min. Next, the membranes were incubated with patient serum (n=3; 1:100, diluted in TBS-T containing 0.75% BSA) and washed for 4 X with TBS-T. Subsequently, the membranes were incubated for 1.5 h with goat anti-IgM (mu, KPL), anti-IgG (H + L chain, Thermo Scientific) or human anti-IgA (α chain specific, Calbiochem) IgG antibodies (1:5000, prepared in TBS-T), and then washed with TBS-T and CBS (sodium citrate buffer containing 50 mM NaCl, pH 7.0). Then CDP-Star^{®} chemiluminescent substrate (0.25 mM) with Nitro-Block-II^{™} Enhancer (Applied Biosystems, USA) was added to complete the reaction.

The chemiluminescent signals were detected on the Odyssey FC equipment (LI-COR Bioscience) and the intensity of the signals quantified using TotalLab TL100 software (v 2009, Nonlinear Dynamics, USA). The data was analyzed with Microsoft Excel program, and only the spots that had signal intensity (SI) greater than or equal to 30% of the highest value obtained in the set of spots in the respective membranes were included in the characterization of the polyamino acids. As a negative control, the background signal intensity of each membrane was used.

### EXAMPLE 21 - Synthesis of branched polyamino acids from SARS-CoV-2

The multiple branched polyamino acids (SARS-X1-SARS-X8) were synthesized using the F-moc solid-phase polyamino acid synthesis strategy on a Schimadzu synthesizer, model PSS8, according to the manufacturer's instructions. Wang Kcore (two-lysine core, K4) resin (Novabiochem) was used as a solid support for the synthesis of branched polyamino acids.

The first amino acid to be coupled was the one located in the C-terminal portion of the polypeptide sequence and the last one located at the N-initial. After completion of all cycles of the synthesis of the branched polyamino acids, they were detached from the solid support by treatment with cleavage cocktail (trifluoracetic acid, triisopropylsilane, and ethandiol) according to standard procedure used in the state of the art for production of synthetic polyamino acids and deprotection of protecting groups of the amino acid side chains (Guy and Fields, Methods Emzymol 289, 67-83, 1997). For quality control of the synthesis, each polyamino acid was analyzed by HPLC and MALDI-TOF.

The synthetic polyamino acids XI, X2 and X5 of the SARS-CoV-2 protein S include SEQ ID NO: 100, 101 and 104, respectively. The synthetic polyamino acids X3 and X6 of the N protein of SARS-CoV-2 include the SEQ ID NO: 102 and 105, respectively. The synthetic polyamino acid X4 of SARS-CoV-2 protein E includes SEQ ID NO: 103. The synthetic polyamino acids X7 and X8 of the SARS-CoV-2 protein, encoded by the open reading window (ORF8) between the S and Se genes, include the SEQ ID NO: 106 and 107 respectively.

The genes encoding the X8 protein are found in small open reading frames (ORFs) between the S and Se genes. The genes encoding the ORF6, X4 and X5 proteins are found in the composition of the ORFs between the M and N genes.

A list of synthetic branched polypeptides is shown in **Table 21.**

**Table 21 - Synthetic branched peptides of SARS-CoV-2 proteins**

| **Code** | **Sequence** | **Protein** | **SEQ ID no:** |
|---|---|---|---|
| SARS-X1 | YFPLQSYGFQPTNGV | S | 100 |
| SARS- X2 | RSYTPGDSSSSSGWTAG | S | 101 |
| SARS- X3 | GKTFPPTEPKKDKKG | N | 102 |
| SARS-X4 | MYSFVSEETGTLIVN | E | 103 |
| SARS- X5 | PLQSYGFQPTNGVGY | S | 104 |
| SARS-X6 | GGMKDLSPRWYFGGG | N | 105 |
| SARS- X7 | GSKSPIQYIDGGGGG | ORF8 | 106 |
| SARS-X8 | YIRGARKSAPLIELG | ORF8 | 107 |

### EXAMPLE 22 - Human Serum Sample Groups

The 134 human serum samples were divided into five groups. The groups were:
- Group 0: ten healthy human serum samples obtained before 2016 from blood donor bank (HEMORIO) (sera #1 - #10);
- Group 1: twenty-six serum samples from "asymptomatic SARS patients", identified according to the WHO case definition (#1 - #26);
- Group 2: twenty-four serum samples from "suspected patients" (#27 - #50);
- Group 3: thirty-eight serum samples from "patients hospitalized for SARS (severe illness)" identified according to the WHO case definition (#51 - #88);
- Group 4: thirty-six serum samples from "patients immunoprotected for SARS" (#89 - #124). Sera #1 - #26, high body temperature with return to normal temperature, RT-PCR+ for SARS-CoV-2, SD (Standard diagnostic Inc.) rapid test for anti-SARS-CoV-2 antibodies negative.

#27 - #50: patients with RT-PCR+ diagnosis for SARS-CoV-2 and negative SD rapid test for anti-SARS-CoV-2 antibodies.
#51 - #88: hospitalized individuals with exacerbated signs and symptoms of SARS-CoV-2, rapid RT-PCR test +, SD for anti-SARS-CoV-2 antibody positive.
#89 - #124: immunoprotected individuals defined as recovered patient, hospitalized or not, who was or was not diagnosed with SARS-CoV-2 positive, sometimes without diagnosis by RT-PCR+, but showing characteristic symptoms.

### EXAMPLE 23 - Identification of SARS-CoV-2 related IgM polyamino acids

In order to identify potential polyamino acids that would be specifically recognized by anti-SARS-CoV-2 IgM antibodies, serum samples from infected patients were analyzed by the SARS-CoV-2 spot polypeptide library synthesis array that encompasses all regions of S, ORF3a, M, ORF6, ORF7, ORF8, N, E, ORF10 and control polyamino acids . Peptide arrays were used to detect the potential binding activity of human sera from infected individuals to polyamino acids. The peptide arrays covered peptide sequences 15 amino acids in length with 10 amino acids overlapping in adjacent spots. Such linear polyamino acids include the following SARS-CoV-2 proteins:
- spike protein (S): aa 1-1273 (spots A7-K19),
- ORF3a protein (ORF3): aa 1-275 (spots K22-N2),
- membrane glycoprotein (M): aa 1-222 (spots N5-O23),
- ORF6 protein (ORF6): aa 1-61 (spots P2-P12),
- ORF7 protein (ORF7): aa 1-121 (spots P15-Q13),
- ORF8 protein (ORF8): aa 1-121 (spots Q16-R17),
- nucleocapsid protein (N): aa 1-419 (spots R20-V17),
- envelope protein (E): aa 1-75 (spots W1-W13)
- ORF10 protein (ORF10): aa 1-38 (spots W15-W20)
- positive control polyamino acids : A1 and V5 ( *Clostridium tetani* precursor peptide), A2 and V6 ( *Clostridium tetani* precursor peptide), A3 and V7 (human poliovirus peptide), A4 and V8 (triple epitope of hemagglutinin)
- non-reactive spots as negative controls.

The serological immune response by human anti-IgM antibody to various SARS-CoV-2 synthetic polyamino acids (S, ORF3a, M, ORF6, ORF7, ORF8, N, E, ORF10) and control polyamino acids (Examples 19 and 20) were analyzed using the polyamino acids covalently synthesized on cellulose membrane (spot) and pool (n = 3) serum from patients #55, #60 and #74 (group 3), as can be seen in **Figure 12****,** supplemented by **Table 18.**

**Figures 15A to 15I** show the signal quantification of the results of the membrane spots from incubation with human sera revealed with goat anti-human IgM secondary antibody.

Human sera have been shown to be significantly reactive to polyamino acids from different viral proteins, as can be seen in Figures 15A to 151, when revealed by human anti-human IgM antibody demonstrating that a large number of different polyamino acids have great potential for diagnosing the disease, even in preliminary stages of infection.

### EXAMPLE 24 - Identification of SARS-related IgG epitopes

To identify potential epitopes that would be specifically recognized by anti-SARS-CoV-2 IgG antibodies, serum samples from infected patients were analyzed by the SARS-CoV-2 spot polyamino acid library synthesis array that encompasses all regions of S, ORF3a, M, ORF6, ORF7, ORF8, N, E, ORF10 and control polyamino acids.

Peptide arrays were used to detect the potential binding activity of human sera from infected individuals to the polyamino acids. The peptide arrays covered peptide sequences 15 amino acids in length with 10 amino acids overlapping in adjacent Spots. Such linear polyamino acids include the following SARS-CoV-2 proteins:
- ORF3a protein (OF3a): aa 1-275 (spots A7-C11),
- membrane glycoprotein (M): aa 1-222 (spots C14-E8),
- ORF6 protein (OF6): aa 1-61 (spots E11-E21),
- ORF7 protein (OF7 (: aa 1-121 (spots E24-F22),
- ORF8 protein (OF8): aa 1-121 (spots G1-G23),
- spike proteins (S): aa 1-1273 (spots H1-R13),
- nucleocapsid protein (N): aa 1-419 (spots R16-V1),
- envelope protein (E): aa 1-75 (spots W1-W13),
- ORF10 protein (OF10): aa 1-38 (spots W15-W20),
- positive control polypeptides: A1 and V4 ( *Clostridium tetani* precursor peptide), A2 and V5 ( *Clostridium tetani* precursor peptide), A3 and V6 (human poliovirus peptide), A4 and V7 (triple epitope of hemagglutinin)
- non-reacting spots as negative controls

The serological immune response by IgG antibodies to various SARS-CoV-2 synthetic polyamino acids (ORF3a, M, ORF6, ORF7, ORF8, S, N, E, ORF10) and control polyamino acids were analyzed using the peptides covalently synthesized in the cellulose membrane (spot) and pool (n = 3) serum from patients #55, #60 and #74 (group 3), as can be seen in **Figure 13****,** supplemented by **Table 19.**

**Figures 16A to 16H** show the signal quantification of the results of the membrane spots from incubation with human sera revealed with goat anti-human IgG secondary antibody.

Human sera have been shown to be significantly reactive to polyamino acids of different viral proteins, as can be seen in Figures 16A to 16H, when revealed by human anti-human IgG antibody demonstrating that a large number of different polyamino acids have great potential for diagnosing the disease, even in stages after the acute phase of infection.

### EXAMPLE 25 - Identification of polyamino acids IgA related to SARS

To identify potential polyamino acids that would be specifically recognized by anti-SARS-CoV-2 IgA antibodies, serum samples from infected patients were analyzed by the SARS-CoV-2 spot polyamino acid library synthesis array that encompasses all regions of S, ORF3a, M, ORF6, ORF7, ORF8, N, E, ORF10 and control polyamino acids.

Peptide arrays were used to detect the potential binding activity of human sera from infected individuals to polyamino acids. The peptide arrays covered peptide sequences 15 amino acids in length with 10 amino acids overlapping in adjacent spots. Such linear polyamino acids include the following SARS-CoV-2 proteins:
- spike protein (S): aa 1-1273 (spots A6-K18),
- ORF3a protein (ORF3): aa 1-275 (spots K21-N1),
- membrane glycoprotein (M): aa 1-222 (N4-O22),
- ORF6 protein (ORF6): aa 1-61 (P2-P12),
- ORF7 protein (ORF7): aa 1-121 (P15-Q13),
- ORF8 protein (ORF8): aa 1-121 (Q16-R17),
- nucleocapsid protein (N): aa 1-419 (spots R20-V17),
- Envelope protein (E): aa 1-75 (spots W1-W-13),
- ORF10 protein (ORF10): aa 1-38 (spots W15-W20),
- positive control polypeptides: A1 and V4 ( *Clostridium tetani* precursor peptide), A2 and V5 ( *Clostridium tetani* precursor peptide), A3 and V6 (human poliovirus peptide), A4 and V7 (triple epitope of hemagglutinin),
- Non-reactor spots as negative controls.

The B cell immune response by IgA antibodies to various synthetic polyamino acids of SARS-CoV-2 (ORF3a, M, ORF6, ORF7, ORF8, S, N, E, ORF10) and control polyamino acids were analyzed using the covalently synthesized peptides in the cellulose membrane (spot) and pool (n = 3) serum from patients #55, #60 and #74 (group 3), as can be seen in **Figure 14****,** supplemented by **Table 20.**

**Figures 17A** **to 171** show the signal quantification of the results of the membrane spots from incubation with human sera revealed with goat anti-human IgG secondary antibody.

The human sera were significantly reactive to the polyamino acids of different viral proteins, as can be seen in Figures 17A to 171, when revealed by human anti-human IgA antibody, demonstrating that a large number of different polyamino acids have great potential for the diagnosis of the disease by means of this class of antibody predominantly present in mucous membranes.

### EXAMPLE 26 - Enzyme-linked immunosorbent assay ELISA for detection of anti-SARS-CoV-2 antibodies

Enzyme-linked immunosorbent assay (ELISA) was used to screen for anti-SARS-CoV-2 antibody in patient sera. ELISA was performed by coating 96-well polystyrene plates with 1 µg/well of branched polyamino acids. For comparison of the results, the experiments of each group were performed in parallel and at the same time. To reduce the possible variation in the difference in performance between the tests, the reactivity index (RI) was employed, which was defined as the target's O.D.450 subtracted from the cutoff O.D.450. The primary human sera were diluted 100X in PBS/BSA 1% and the secondary antibodies goat anti-human IgG (Merck-Sigma), biotin-labeled goat anti-human IgG (Merck-Sigma) 8000x, followed by incubation with HRP-labeled high-sensitivity neutravidin (Thermo Fisher Scientific). The anti-IgA response was revealed with alkaline phosphatase-labeled goat anti-human Ig (KPL). TMB (3,3', 5,5' tetramethylbenzidine) was used as the substrate (Thermo Fisher Scientific), and the immune response was defined as significantly elevated when the reactivity index (IR) was greater than 1.

The results show that the branched polyamino acids SARS-X1 to SARS-X8 have differences in reactivity against anti-SARS-CoV2 antibodies and that these differences are related to the class of human antibody (IgM, IgG or IgA) detected and the status of the patient diagnosed with SARS-CoV2, as can be seen from the analysis of Figures 18 to 23.

Observing such differences allows the design of diagnostic tests that may provide more accurate or robust information than simply a positive or negative diagnosis for anti-SARS-CoV2 antibodies. Thus, a diagnostic test can, in addition to detecting IgM, IgG, or IgA antibodies, also be designed to indicate whether individuals should be hospitalized even in the absence of symptoms.

### EXAMPLE 27 - Development of receptacle proteins for SARS-CoV-2

The "Tx" receptacle protein has been genetically manipulated to harbor SARS-CoV-2 epitopes. Reactive epitopes for sera from SARS-CoV-2 infected individuals were selected for construction of eight Tx proteins: Ag-COVID19, Ag COVID19 (H), Tx-SARS2-IgM, Tx-SARS2-IgG, Tx-SARS2-G/M, Tx-SARS2-IgA, Tx-SARS2-Universal and Tx-SARS-G5 (No RBD).

The genes corresponding to the Ag-COVID19, Ag COVID19 (H), Tx-SARS2-IgM, Tx-SARS2-IgG, Tx-SARS2-G/M, Tx-SARS2-IgA, Tx-SARS2-Universal, and Tx-SARS-G5 (No RBD) proteins, herein called, respectively, Ag-COVID19 gene, Ag-COVID19 (H) gene, Tx-SARS2-IgM gene, Tx-SARS2-IgG gene, Tx-SARS2-G/M gene, Tx-SARS2-IgA gene, Tx-SARS2-Universal gene, and Tx-SARS-G5 (No RBD) gene, are described in SEQ ID NO nucleotide sequences: 108 to SEQ ID NO:115. The amino acid sequences corresponding to Ag-COVID19, Ag COVID19 (H), Tx-SARS2-IgM, Tx-SARS2-IgG, Tx-SARS2 -G/M, Tx-SARS2-IgA, Tx-SARS2-Universal and Tx-SARS-G5 (No RBD) proteins are described in SEQ ID NO 116 to 123, respectively.

From the SARS-CoV-2 epitope sequence mapping study, considering their diagnostic potential as clarified in Examples 19 to 26, the eight proteins described above harbored polyamino acids as shown in Tables 22 to 28.

**Table 22 - Ag-COVID19 and Ag COVID19 Proteins (H)**

| Polyamino acid sequences | Position in the protein | Original epitope protein | Sequence | SEQ ID no. |
|---|---|---|---|---|
| SARS-Co V-2 | 1^{a} | S | FERDISTEIYQAGST | 124 |
| SARS-Co V-2 | 4 | S | GSTPCNGVEGFNCYF | 125 |
| SARS-Co V-2 | 8 | S | NSNNLDSKVGGNYNY | 126 |
| SARS-Co V-2 | 10 | S | FERDISTEIYQAGST | 124 |
| SARS-Co V-2 | 11 | S | GSTPCNGVEGFNCYF | 125 |
| SARS-Co V-2 | 12 | S | YFPLQSYGFQPTNGV | 100 |
| SARS-Co V-2 | 13^{a} | S | YFPLQSYGFQPTNGV | 100 |
| SARS-Co V-2 | 13b | S | NSNNLDSKVGGNYNY | 126 |

**Table 23 - Tx-SARS2-IgM**

| Polyamino acid sequences | Position in the protein | Original epitope protein | Sequence | SEQ ID no. |
|---|---|---|---|---|
| SARS-Co V-2 | 1^{a} | ORF3a | GSSGVVNPVM | 127 |
| SARS-Co V-2 | 2 | N | NAPRITFGGPSDSTGS | 128 |
| SARS-Co V-2 | 4 | ORF3a | GSSGVVNPVM | 127 |
| SARS-Co V-2 | 5 | ORF8 | YIRVGARKSAPLIEL | 129 |
| SARS-Co V-2 | 6 | S | SLIDLQELGKYEQYI | 130 |
| SARS-Co V-2 | 8 | S | PFQQFGRDIADTTDA | 131 |
| SARS-Co V-2 | 10 | M | MWLSYFIASFRL | 132 |
| SARS-Co V-2 | 11 | S | RSYTPGDSSSGWTA | 101 |
| SARS-Co V-2 | 12 | ORF3a | IVDEP | 133 |
| SARS-Co V-2 | 13a | S | GFSALEPLVDLP | 134 |
| SARS-Co V-2 | 13b | N | KTFPPTEPKKDKK | 135 |

**Table 24 - Tx-SARS2-IgG**

| Polyamino acid sequences | Position in the protein | Original epitope protein | Sequence | SEQ ID no. |
|---|---|---|---|---|
| SARS-Co V-2 | 1a | S | LGVYHKNNKSWMESEFRVY | 136 |
| SARS-Co V-2 | 3 | S | FNCYFPLQSYGFQPT | 137 |
| SARS-Co V-2 | 4 | S | PLQSYGFQPT | 138 |
| SARS-Co V-2 | 5 | N | AGNGGDAALALLLLD | 139 |
| SARS-Co V-2 | 6 | S | RSYLTPGDSSS | 140 |
| SARS-Co V-2 | 8 | S | ADQLTPTWRV | 141 |
| SARS-Co V-2 | 10 | ORF3 | FIYNKIVDEP | 142 |
| SARS-Co V-2 | 11 | ORF3 | KNPLLYDANY | 143 |
| SARS-Co V-2 | 12 | N | RPQGLPNNTAS | 144 |
| SARS-Co V-2 | 13a | N | LAEILQKNLIRQGTDYKHWPQIA | 145 |
| SARS-Co V-2 | 13b | S | GKIADYNYKL | 146 |

**Table 25 - Tx-SARS2-G/M**

| Polyamino acid sequences | Position in the protein | Original epitope protein | Sequence | SEQ ID no. |
|---|---|---|---|---|
| SARS-Co V-2 | 1a | S | FNCYFPLQSYGFQPT | 137 |
| SARS-Co V-2 | 2 | N | RPQGLPNNTAS | 144 |
| SARS-Co V-2 | 3 | ORF3 | FIYNKIVDEP | 142 |
| SARS-Co V-2 | 4 | S | ADQLTPTWRV | 141 |
| SARS-Co V-2 | 5 | S | GKIADYNYKL | 146 |
| SARS-Co V-2 | 8 | ORF8 | YIRVGARKSAPLIEL | 129 |
| SARS-Co V-2 | 9 | ORF3a | IVDEP | 133 |
| SARS-Co V-2 | 10 | ORF3 | KNPLLYDANY | 143 |
| SARS-Co V-2 | 11 | N | KTFPPTEPKKDKK | 135 |
| SARS-Co V-2 | 12 | S | RSYLTPGDSSS | 140 |
| SARS-Co V-2 | 13a | ORF3a | GSSGVVNPVMEPIYD | 147 |
| SARS-Co V-2 | 13b | S | APGQTGKIADYNYKL | 148 |

**Table 26 - Tx-SARS2-IgA**

| Polyamino acid sequences | Position in the protein | Original epitope protein | Sequence | SEQ ID no. |
|---|---|---|---|---|
| SARS-Co V-2 | 1a | N | ALPQRQKKQQTVTLL | 149 |
| SARS-Co V-2 | 4 | ORF8 | GSKSPIQYID | 150 |
| SARS-Co V-2 | 5 | ORF8 | DFLEYHDVRVVLDF | 151 |
| SARS-Co V-2 | 6 | S | GINASVVNIQ | 152 |
| SARS-Co V-2 | 7 | N | QFAPSASAFF | 153 |
| SARS-Co V-2 | 8 | E | MYSFVSEETGTLIVN | 103 |
| SARS-Co V-2 | 11 | N | PSGTWLTYTG | 154 |
| SARS-Co V-2 | 12 | N | QFAPSASAFF | 153 |
| SARS-Co V-2 | 13a | S | ELDKY | 155 |
| SARS-Co V-2 | 13b | E | MYSFVSEETGTLIVN | 103 |

**Table 27 - Tx-SARS2-Universal**

| Polyamino acid sequences | Position in the protein | Original epitope protein | Sequence | SEQ ID no. |
|---|---|---|---|---|
| SARS-Co V-2 | 1a | S | PLQSYGFQPTNGVGY | 104 |
| SARS-Co V-2 | 4 | S | GIYQTSNFRV | 156 |
| SARS-Co V-2 | 5 | N | KAYNVTQAFGRRGPE | 157 |
| SARS-Co V-2 | 7 | S | GTNTSNQVAV | 158 |
| SARS-Co V-2 | 8 | S | NPVLPFNDGVYFAST | 159 |
| SARS-Co V-2 | 10 | s | YNYKLPDDFT | 160 |
| SARS-Co V-2 | 11 | ORF6 | MFHLVDFQVTIAEIL | 161 |
| SARS-Co V-2 | 12 | N | MKDLSPRWYF | 162 |
| SARS-Co V-2 | 13a | N | DAALALLLLD | 163 |
| SARS-Co V-2 | 13b | N | MKDLSPRWYF | 162 |

**Table 28 - Tx-SARS2-G5**

| Polyamino acid sequences | Position in the protein | Original epitope protein | Sequence | SEQ ID no. |
|---|---|---|---|---|
| SARS-Co V-2 | 1a | S | LGVYHKNNKSWMESEFRVY | 136 |
| SARS-Co V-2 | 3 | ORF3 | FIYNKIVDEP | 142 |
| SARS-Co V-2 | 4 | ORF3 | KNPLLYDANY | 143 |
| SARS-Co V-2 | 5 | N | AGNGGDAALALLLLD | 139 |
| SARS-Co V-2 | 6 | S | RSYLTPGDSSS | 140 |
| SARS-Co V-2 | 8 | S | ADQLTPTWRV | 141 |
| SARS-Co V-2 | 10 | ORF3 | FIYNKIVDEP | 142 |
| SARS-Co V-2 | 11 | ORF3 | KNPLLYDANY | 143 |
| SARS-Co V-2 | 12 | N | RPQGLPNNTAS | 144 |
| SARS-Co V-2 | 13a | N | LIRQGTDYKHWPQIA | 147 |
| SARS-Co V-2 | 13b | S | GKIADYNYKL | 146 |

### EXAMPLE 28 Expression of receptacle proteins with polyamino acids from SARS-CoV-2

The Ag-COVID19, Ag-COVID19 (H), Tx-SARS2-IgM, Tx-SARS2-IgG, Tx-SARS2-G/M, Tx-SARS2-IgA, Tx-SARS2-Universal and Tx-SARS-G5 (No RBD) proteins were expressed using pET24 plasmids harboring genes encoding each protein using restriction sites for the BamHI and XhoI enzymes. Each plasmid containing the gene for a specific protein was transferred to *E*. *coli* BL21 strains in order to promote the expression of the eight different proteins listed above.

The strain was grown overnight in LB medium and subsequently reseeded in the same medium, added kanamycin (30 µg/ml), on a shaker at 200rpm, until it reached an optical turbidity density of 0.6-0.8 (600 nm). The BL21 strain expresses T7 RNA polymerase when induced by isopropyl β-D-1-thiogalactopyranoside (IPTG). Then IPTG (q.s.p. 1 mM) is added to the culture and the same culture conditions are maintained for another 3 h at 37 °C.

The culture of each bacterial strain was subjected to centrifugation and the *pellet* resuspended in 10% CelLytic^{™} (Sigma, BR) in 150 mM NaCl and 50 mM Tris, pH 8.0.

Aliquots of the recombinant proteins (1 µg/well) were subjected to SDS-containing polyacrylamide gel electrophoresis (SDS- PAGE) (Laemmli, Nature 227: 680-685, 1970). Concentration *gels*(*stacking gel*) and separation gels(*running gel*) were prepared at an acrylamide concentration of 4% and 11%, respectively (table 5, below). Samples were prepared under denaturing conditions in 62.5 mM Tris-HCl buffer, pH 6.8, 2% SDS, 5% β-mercaptoethanol, 10% glycerol and boiled at 95 °C for 5 min (Hames BD, Gel electrophoresis of proteins: a practical approach. 3. Ed. Oxford. 1998). After electrophoresis, the proteins were detected by staining with comassie blue *Simply Blue R250* (ThermoFisher, BR). The marker *PageRuler Plus Prestained Standards* was used as a molecular weight reference (ThermoFisher, BR). **Figure 24****,** shows the band of Ag-COVID19 (column 3), Ag-COVID19 (H) (column 4), Tx-SARS2-IgM (column 5), Tx-SARS2-IgG (column 6), Tx-SARS2-G/M (column 7), Tx-SARS2-IgA (column 8), Tx-SARS2-Universal (column 9) and Tx-SARS-G5 (No RBD) (column 10). Column 1 shows the molecular weight marker: A) 250 kDa; B) 130 kDa; C) 100 kDa; D) 70 kDa; E) 55 kDa; F) 35 kDa and G) 25 kDa. Column 2 shows a total extract of uninduced bacteria.

Alternatively, the culture was also subjected to centrifugation and the *pellet* resuspended in urea buffer (100 mM NaH2PO4, 10 mM Tris-base, 8 M urea, pH 8.0). The solution was subjected to chromatography by a nickel affinity column (HisTrap) mL per minute, previously equlibrated in buffer A (50 mM Tris-HCl, pH 8.0, 100 mM NaCl and 5 mM imidazole). After binding, the resin was washed with 10 mL of buffer A. The protein was eluted in steps of buffer B (50 mM Tris-HCl, pH 8.0, 100 mM NaCl) with 75 mM, 200 mM, and 500 mM imidazole at a flow rate of 0.7 mL/min for 45 minutes. **Figure 25** shows the pattern corresponding to the Ag-COVID19 protein with the six histidine tail indicating the 200 mM eluted concentration. **Figure 26** shows the pattern corresponding to the affinity-purified SARS2-G5 protein by using 200 mM imidazole (the elution performed with 75 mM shows a contaminant).

The results show that the Tx receptacle protein can be used to create new and different proteins with great ease. Additionally, the expression of different receptacle proteins harboring different polyamino acids can be performed using the same expression protocol, generating great savings in inputs, time, and infrastructure. The inclusion of a six-histidine tail was shown to be a potential facilitator for purification at high purity levels.

### EXAMPLE 29 - Enzyme-linked immunosorbent assay (ELISA) for detecting anti-SARS-CoV-2 antibody using Ag-COVID19 and SARS2-G5 proteins

Enzyme-linked immunosorbent assay (ELISA) was used to screen for the presence of anti-SARS-CoV-2 antibody. The performance of Ag-COVID19 and SARS2-G5 proteins was evaluated against a panel of sera from individuals affected by SARS-CoV-2 virus infections.

ELISA was performed by coating 96-well polystyrene plates with 1 µg/well in solution (0.3 M Urea, pH8.0) of Ag-COVID19 protein**(****Figure 27****)** or Tx-SARS2-G5 protein**(Figure 28)** at 4 °^{C}for 12-18h. The wells were washed with saline-phosphate buffer (PBS) solution added Tween 20 (PBS-T, 10 mM sodium phosphate - Na3PO4, 150 mM sodium chloride - NaCl and 0.05% Tween-20, pH 7.4) and then incubated with 1× PBS buffer containing 5% (weight/volume) dehydrated skim milk for 2 h at 37 °C.

Then, the wells were washed three times with PBS-T buffer and incubated with human serum samples diluted 1:100 in PBS/BSA 1% for 1 h at 37°C. After the incubation period, the wells were washed three times with PBS-T and then incubated with biotin-labeled goat anti-human IgG antibody (Merck-Sigma) at a dilution of 1:8000 for 1 h at 37 °C. Subsequently, HRP-labeled high-sensitivity neutravidin (Thermo Fisher Scientific) was added. The wells were washed again three times with PBS-T buffer and TMB substrate (3.3', 5.5' tetramethylbenzidine, Thermo Fisher Scientific) was used. After 30 minutes and under shelter from light, the absorbance was measured in an ELISA plate reader at 405 nm.

The results show that Ag-COVID19 protein**(****Figure 27****)** and Tx-SARS2-G5 protein**(****Figure 28****)** have proven useful for detecting antibodies against SARS-CoV-2 by demonstrating excellent sensitivity and specificity indices. As can be seen from Figures 27 and 28, the proteins harboring polyamino acids from SARS-CoV-2 did not detect antibodies in sera from individuals collected prior to the SARS-CoV-2 pandemic, healthy or affected by diseases such as dengue, malaria, and syphilis. Differently, the proteins detected antibodies in sera from individuals diagnosed positive for SARS-CoV-2 (symptomatic or asymptomatic), hospitalized or already recovered patients.

### EXAMPLE 30 - Ag-COVID19 protein as vaccine composition

The Ag-COVID 19 protein was produced and purified according to the protocols described in this patent application. Three mice were subjected to inoculation with 10 µg of Ag-COVID19 protein in 25 µl of PBS suspended in Freud's complete adjuvant (25µl) on days 0, 14, 21 and 28. The negative control was performed using an animal inoculated with PBS. Blood samples from the animals were collected before each reinoculation and submitted to ELISA. Plasma was separated from the collected blood by centrifugation and subjected to serial dilution to perform antibody measurement (Figure 29). The results showed excellent antibody production against Ag-COVID19 after four weeks from the first injection.

### EXAMPLE 31 - Use of Ag-COVID19 protein for purification of anti SARS-CoV-2 antibodies.

Purification of anti-SARS-CoV-2 antibodies from sera of patients diagnosed with COVID19 was performed using the antibody affinity principle. Ag-COVID19 protein was conjugated to Sepharose ^{™} 4B activated with CNBr (GE Healthcare, USA). A 10 mL serum sample from a SARS-CoV-2 positive patient was diluted in 10 mL PBS and subjected to Sepharose-Ag-COVID19 for 1 hour. The mixture was then placed on a chromatography column. After the solution had passed through the column, 10 mL of PBS was added to the cormatographic system and then 5 mL of a 100 mM sodium citrate buffer at pH 4. Fractions of 0.5 ml were collected sequentially as they were recovered from the column and quantified for the presence of antibodies by spectrophotometry at 280 nm. The absorbance of each fraction was converted to protein concentration and plotted as a function of the volume of the fraction.

The results demonstrate that the Ag-COVID19 protein can be useful as an input for affinity purification of antibodies from patients previously infected with SARS-CoV-2 (Figure 30), indicating its importance in generating usable inputs for passive immunization to address the COVID-19 pandemic.

## Claims

1. Protein receptacle **characterized in that** it comprises a stable protein structure that supports, at different sites, the insertion of four or more exogenous polyamino acid sequences simultaneously.

2. Protein receptacle according to claim 1, **characterized in that** it comprises an amino acid sequence at least 90% identical to SEQ ID NO: 1.

3. Protein receptacle according to claim 1, **characterized in that** it comprises an amino acid sequence at least 90% identical to SEQ ID NO: 3.

4. Protein receptacle according to claim 1, **characterized in that** it comprises an amino acid sequence at least 90% identical to SEQ ID NO: 77.

5. Protein receptacle according to any one of claims 1 to 4, **characterized in that** it has insertion sites for exogenous polyamino acid sequences in protein loops facing the external medium.

6. Protein receptacle according to any one of claims 1 to 5, **characterized in that** the insertion of the exogenous polyamino acid sequences simultaneously does not interfere with the production conditions of the receptacle protein.

7. Protein receptacle according to any one of claims 2 to 4, **characterized in that** it contains exogenous polyamino acid sequences simultaneously for use in vaccine compositions, in diagnostics, or in the development of laboratory reagents.

8. Protein receptacle according to any of claims 2 to 4, **characterized in that** the exogenous polyamino acid sequences do not lose their immunogenic characteristics upon simultaneous insertion into the protein loops of the protein receptacle.

9. Protein receptacle according to claim 2, **characterized in that** it comprises the exogenous polyamino acid sequences SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15 and SEQ ID NO:16, simultaneously.

10. Protein receptacle according to claim 9, **characterized in that** it comprises the amino acid sequence shown in SEQ ID NO: 18.

11. Protein receptacle according to claim 3, **characterized in that** it comprises the exogenous polyamino acid sequences SEQ ID NO:21, SEQ ID NO:22, SEQ ID NO:23, SEQ ID NO:24, SEQ ID NO:25, SEQ ID NO:26, SEQ ID NO:27, SEQ ID NO:28 and SEQ ID NO:29, simultaneously.

12. Protein receptacle according to claim 11, **characterized in that** it comprises the amino acid sequence shown in SEQ ID NO. 20.

13. Protein receptacle according to claim 3, **characterized in that** it comprises multiple copies of the exogenous polyamino acid sequence simultaneously, SEQ ID NO:30.

14. Protein receptacle according to claim 13, **characterized in that** it comprises the amino acid sequence shown in SEQ ID^{no}. 31.

15. Protein receptacle according to claim 3, **characterized in that** it comprises the exogenous polyamino acid sequences SEQ ID NO:35, SEQ ID NO:36, SEQ ID NO:37, SEQ ID NO:38, SEQ ID NO:39 and SEQ ID NO:40, simultaneously.

16. Protein receptacle according to claim 15, **characterized in that** it comprises the amino acid sequence shown in SEQ ID NO. 33.

17. Protein receptacle according to claim 3, **characterized in that** it comprises the exogenous polyamino acid sequences SEQ ID NO: 41, SEQ ID NO: 42, SEQ ID NO: 43 and SEQ ID NO: 44, simultaneously.

18. Protein receptacle according to claim 17, **characterized in that** it comprises the amino acid sequence shown in SEQ ID NO:. 45.

19. Protein receptacle according to claim 3, **characterized in that** it comprises the exogenous polyamino acid sequences SEQ ID NO: 47, SEQ ID NO: 48, SEQ ID NO: 49 and SEQ ID NO: 50, simultaneously.

20. Protein receptacle according to claim 19, **characterized in that** it comprises the amino acid sequence shown in SEQ ID NO:. 51.

21. Protein receptacle according to claim 3, **characterized in that** it comprises the exogenous polyamino acid sequences SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 95 and SEQ ID NO: 96, simultaneously.

22. Protein receptacle according to claim 21, **characterized in that** it comprises the amino acid sequence shown in SEQ ID NO:. 64.

23. Protein receptacle according to claim 3, **characterized in that** it comprises the exogenous polyamino acid sequences SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74 and SEQ ID NO: 97, simultaneously.

24. Protein receptacle according to claim 23, **characterized in that** it comprises the amino acid sequence shown in SEQ ID NO:. 75.

25. Protein receptacle according to claim 4, **characterized in that** it comprises the exogenous polyamino acid sequences simultaneously SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87 and SEQ ID NO: 98.

26. Protein receptacle according to claim 25, **characterized in that** it comprises the amino acid sequence shown in SEQ ID NO: 88.

27. Protein receptacle according to claim 4, **characterized in that** it comprises the exogenous polyamino acid sequences SEQ ID NO: 7, SEQ ID NO: 9, SEQ ID NO: 10, SEQ ID NO: 12, SEQ ID NO: 14, SEQ ID NO: 15, SEQ ID NO: 16, SEQ ID NO: 92, SEQ ID NO: 93, SEQ ID NO: 94 and SEQ ID NO: 99, simultaneously.

28. Protein receptacle according to claim 27, **characterized in that** it comprises the amino acid sequence shown in SEQ ID NO: 90.

29. Protein receptacle according to claim 4, **characterized in that** it comprises exogenous polyamino acid sequences as defined in SEQ ID NO: 100, 124, 125 and 126, simultaneously; or SEQ ID NO:101, 127, 128, 129, 130, 131, 132, 133, 134 and 135, simultaneously; or SEQ ID NO: 136, 137, 138, 139, 140, 141, 142, simultaneously; or SEQ ID NO: 129, 133, 135, 137, 140, 141, 142, 143, 144, 146, 147, and 148, simultaneously; or SEQ ID NO: 103, 149, 150, 151, 152, 153, 154, 155, simultaneously; or SEQ ID NO: 104, 156, 157, 158, 159, 160, 161, 162, and 163, simultaneously; or SEQ ID NO: 136, 139, 140, 141, 142, 143, 144, 146 and 147, simultaneously.

30. Protein receptacle according to claim 29, **characterized in that** it comprises any of the amino acid sequences shown in SEQ ID NO: 116-123.

31. Polynucleotide, **characterized in that** it comprises_any one of SEQ ID NO: 2, 4, 78, 17, 19, 32, 34, 46, 52, 63, 76, 89, 91, 108-115 and their degenerate sequences, capable of generating, respectively, the polypeptides defined by SEQ ID NO: 1, 3, 77, 18, 20, 31, 33, 45, 51, 64, 75, 88, 90, 116-123.

32. Vector **characterized in that** it comprises_the polynucleotide as defined in claim 31.

33. Expression cassette **characterized in that** it comprises polynucleotide as defined in claim 31.

34. Cell **characterized in that** it comprises the vector as defined in claim 32 or the expression cassette as defined in claim 33.

35. Method for producing the protein receptacle **characterized in that** it introduces into competent cells of interest the polynucleotide as defined in claim 31; performing culture of the competent cells and performing isolation of the receptacle protein containing the exogenous polyamino acids of choice.

36. Method for producing the protein receptacle according to claim 35, **characterized in that** it is free from interference by the insertion of various exogenous polyamino acid sequences.

37. A method of pathogen identification or *in vitro* disease diagnosis **characterized in that** it uses the receptacle protein as defined in any of claims 1 to 30.

38. A method of pathogen identification or disease diagnosis according to claim 37, **characterized in that** it promotes the diagnosis of Chagas disease, rabies, pertussis, yellow fever, Oropouche virus infections, Mayaro virus infections, IgE hypersensitivity, *D. pteronyssinus* allergy, or COVID-19.

39. Use of the protein receptacle as defined in any one of claims 1 to 30, **characterized in that** it is a laboratory reagent.

40. Use of the protein receptacle as defined in any one of claims 1 to 30, **characterized in that** it is for the production of a vaccine composition for immunization against Chagas disease, rabies, pertussis, yellow fever, infections by the Oropouche, Mayaro viruses , hypersensitivity to IgE, allergy to D. pteronyssinus or COVID-19.

41. Diagnostic kit **characterized in that** it comprises the protein receptacle as defined in any one of claims 1 to 30.


SEQUENCE LISTING

<110>  FUNDAÇÃO OSWALDO CRUZ

<120>  PROTEIN RECEPTACLE, POLYNUCLEOTIDE, VECTOR, EXPRESSION
       CASSETTE, CELL, METHOD FOR PRODUCING THE RECEPTACLE, METHOD FOR
        PATOGEN IDENTIFICATION OR DISEASE DIAGNOSIS, USE OF
       THE RECEPTACLE, AND, DIAGNOSTIC KIT

<130>  EP136660-SZ382

<140> EP20859528.0
<141> 27-08-2020

<150> PCT/BR2020/050341
<151> 27-08-2020

<150>  BR102019017792-6
<151>  27-08-2019

<160>  163

<170>  PatentIn version 3.5

<210>  1
<211>  246
<212>  PRT
<213>  Artificial sequence

<200>
<223> Artificial sequence

<400>  1

Met Val Ala Ser Lys Gly Glu Glu Leu Phe Thr Gly Val Val Pro Ile
1               5                   10                  15

Leu Ile Glu Leu Asp Gly Asp Val Asn Gly His Lys Phe Phe Val Arg
            20                  25                  30

Gly Glu Gly Glu Gly Asp Ala Thr Ile Gly Lys Leu Ser Leu Lys Phe
        35                  40                  45

Ile Cys Thr Thr Gly Lys Leu Pro Val Pro Trp Pro Thr Leu Val Thr
    50                  55                  60

Thr Leu Thr Tyr Gly Val Gln Cys Phe Ser Arg Tyr Pro Asp His Met
65                  70                  75                  80

Lys Arg His Asp Phe Phe Lys Ser Ala Met Pro Glu Gly Tyr Val Gln
                85                  90                  95

Glu Arg Thr Ile Tyr Phe Lys Asp Val Asp Gly Thr Tyr Lys Thr Arg
            100                 105                 110

Ala Glu Val Lys Phe Glu Gly Thr Asp Thr Leu Val Asn Arg Ile Val
        115                 120                 125

Leu Lys Gly Ile Asp Phe Lys Glu Asp Gly Asn Ile Leu Lys Gly His
    130                 135                 140

Lys Leu Glu Tyr Asn Phe Asn Ser His Asn Val Tyr Ile Thr Ala Asp
145                 150                 155                 160

Lys Gln Lys Asn Gly Ile Lys Ala Asn Phe Thr Val Arg His Asn Val
                165                 170                 175

Glu Asp Gly Ser Val Gln Leu Ala Asp His Tyr Gln Gln Asn Thr Pro
            180                 185                 190

Ile Gly Asp Gly Pro Val Leu Leu Pro Asp Asn His Tyr Leu Ser Thr
        195                 200                 205

Gln Thr Ile Leu Leu Lys Asp Pro Asn Glu Leu Lys Arg Asp His Met
    210                 215                 220

Val Leu Leu Glu Tyr Val Thr Ala Ala Gly Ile Thr Leu Gly Met Asp
225                 230                 235                 240

Glu Leu Tyr Lys Glu Phe
                245

<210>  2
<211>  744
<212>  DNA
<213>  Artificial sequence

<200>
<223> Artificial sequence

<400>  2
catatggtgg ctagcaaagg tgaagaactg ttcaccggtg ttgttccgat cctgattgaa      60

ctggacggtg acgttaacgg tcacaaattc tttgttcgtg gtgaaggtga aggtgacgcg     120

accatcggta aactgagtct gaaattcatc tgcaccaccg gtaagcttcc ggttccgtgg     180

ccgaccctgg ttaccaccct gacctacggt gttcagtgct tctctcgtta cccggaccac     240

atgaaacgtc acgacttctt caaatctgcg atgccggaag gttacgttca ggaacgtacc     300

atctacttca aagacgtcga cggtacttac aaaacccgtg cggaagttaa attcgaaggt     360

accgacaccc tggttaaccg tatcgttctg aaaggcattg acttcaaaga agacggtaac     420

atccttaagg gtcacaaact ggaatacaac ttcaactctc acaacgttta catcaccgcg     480

gacaaacaga aaaacggtat caaagcgaac ttcaccgttc gtcacaacgt tgaagacgga     540

tccgttcagc tggcggacca ctaccagcag aacaccccga tcggtgacgg tccggttctg 600

ctgccggaca accactacct gtctacccag accattctgc tgaaagaccc gaacgagctc 660

aaacgtgacc acatggttct gctggaatat gttacggcgg cgggtatcac cctgggtatg 720

gacgaactgt acaaagaatt ctaa 744

<210> 3
<211> 246
<212> PRT
<213> Artificial sequence

<200>
<223> Artificial sequence

<400> 3

Met Val Ala Ser Lys Gly Glu Glu Leu Phe Thr Gly Val Val Pro Ile
1               5                   10                  15

Leu Val Glu Leu Asp Gly Asp Val Asn Gly His Lys Phe Ser Val Arg
            20                  25                  30

Gly Glu Gly Glu Gly Asp Ala Thr Ile Gly Lys Leu Thr Leu Lys Phe
        35                  40                  45

Ile Cys Thr Thr Gly Lys Leu Pro Val Pro Trp Pro Thr Leu Val Thr
    50                  55                  60

Thr Leu Thr Tyr Gly Val Gln Cys Phe Ala Arg Tyr Pro Asp His Met
65                  70                  75                  80

Lys Arg His Asp Phe Phe Lys Ser Ala Met Pro Glu Gly Tyr Val Gln
                85                  90                  95

Glu Arg Thr Ile Ser Phe Lys Asp Val Asp Gly Lys Tyr Lys Thr Arg
            100                 105                 110

Ala Val Val Lys Phe Glu Gly Thr Asp Thr Leu Val Asn Arg Ile Val
        115                 120                 125

Leu Lys Gly Thr Asp Phe Lys Glu Asp Gly Asn Ile Leu Lys Gly His
    130                 135                 140

Lys Leu Glu Tyr Asn Phe Asn Ser His Asn Val Tyr Ile Thr Ala Asp
145                 150                 155                 160

Lys Gln Lys Asn Gly Ile Lys Ala Asn Phe Thr Val Arg His Asn Val
                165                 170                 175

Glu Asp Gly Gly Val Gln Leu Ala Asp His Tyr Gln Gln Asn Thr Pro
            180                 185                 190

Ile Gly Asp Gly Pro Val Leu Leu Pro Asp Asn His Tyr Leu Ser Thr
        195                 200                 205

Gln Thr Val Leu Ser Lys Asp Pro Asn Glu Leu Lys Arg Asp His Met
    210                 215                 220

Val Leu Leu Glu Phe Val Thr Ala Ala Gly Ile Thr Leu Gly Met Asp
225                 230                 235                 240

Glu Leu Tyr Lys Glu Phe
                245

<210>  4
<211>  753
<212>  DNA
<213>  Artificial sequence

<200>
<223> Artificial sequence

<400>  4
catatggtgg ctagcaaggg cgaggagctg ttcaccggcg tcgtccccat cctggtcgag      60

ctggacggcg acgttaacgg ccacaagttc tccgtccggg gcgagggcga gggcgacgcc     120

accatcggca agctgaccct gaagttcatc tgcaccaccg gcaagcttcc cgtcccctgg     180

cccaccctgg tcaccaccct gacctatggc gtccagtgct tcgcccggta tcccgaccac     240

atgaagcggc acgacttctt caagtccgcc atgcccgagg gctatgtcca ggagcggacc     300

atctccttca aggacgtcga tggcaagtat aagacccggg ccgtcgtcaa gttcgagggt     360

accgacaccc tggtcaaccg gatcgtcctg aagggcaccg acttcaagga ggacggcaac     420

atccttaagg gccacaagct ggagtataac ttcaactccc acaacgtcta tatcaccgcg     480

gacaaacaga agaacggcat caaggccaac ttcaccgtcc ggcacaacgt cgaggacgga     540

tccgtccagc tggccgacca ctatcagcag aacaccccca tcggcgacgg tccggtcctg     600

ctgcccgaca accactatct gtccacccag accgtcctgt ccaaggaccc gaacgagctc     660

aaacgggacc acatggtcct gctggagttc gtcaccgccg ccggcatcac cctgggcatg     720

gacgagctgt ataaggaatt ctaatgactc gag                                  753

<210>  5
<211>  15
<212>  DNA
<213>  Artificial sequence

<200>
<223> Artificial sequence

<400> 5
catatggtgg ctagc 15

<210> 6
<211> 18
<212> DNA
<213> Artificial sequence

<200>
<223> Artificial sequence

<400> 6
gaattctaat gactcgag 18

<210> 7
<211> 17
<212> PRT
<213> Artificial sequence

<200>
<223> Artificial sequence

<400> 7

Lys Phe Ala Glu Leu Leu Glu Gln Gln Lys Asn Ala Gln Phe Pro Gly
1               5                   10                  15

Lys

<210> 8
<211> 7
<212> PRT
<213> Artificial sequence

<200>
<223> Artificial sequence

<400> 8

Lys Ala Ala Ala Ala Pro Ala
1               5

<210> 9
<211> 7
<212> PRT
<213> Artificial sequence

<200>
<223> Artificial sequence

<400> 9

Lys Ala Ala Ile Ala Pro Ala

1               5

<210>  10
<211>  15
<212>  PRT
<213>  Artificial sequence

<200>
<223> Artificial sequence


<400>  10

Gly Asp Lys Pro Ser Pro Phe Gly Gln Ala Ala Ala Ala Asp Lys
1               5                   10                  15


<210>  11
<211>  9
<212>  PRT
<213>  Artificial sequence

<200>
<223> Artificial sequence


<400>  11

Lys Gln Lys Ala Ala Glu Ala Thr Lys
1               5

<210>  12
<211>  10
<212>  PRT
<213>  Artificial sequence

<200>
<223> Artificial sequence


<400>  12

Ala Glu Pro Lys Pro Ala Glu Pro Lys Ser
1               5                   10

<210>  13
<211>  10
<212>  PRT
<213>  Artificial sequence

<200>
<223> Artificial sequence


<400>  13

Ala Glu Pro Lys Ser Ala Glu Pro Lys Pro
1               5                   10

<210>  14
<211>  15
<212>  PRT
<213>  Artificial sequence

<200>
<223> Artificial sequence

<400> 14

Gly Thr Ser Glu Glu Gly Ser Arg Gly Gly Ser Ser Met Pro Ser
1               5                   10                  15

<210> 15
<211> 11
<212> PRT
<213> Artificial sequence

<200>
<223> Artificial sequence

<400> 15

Ser Pro Phe Gly Gln Ala Ala Ala Gly Asp Lys
1               5                   10

<210> 16
<211> 9
<212> PRT
<213> Artificial sequence

<200>
<223> Artificial sequence

<400> 16

Lys Gln Arg Ala Ala Glu Ala Thr Lys
1               5

<210> 17
<211> 1128
<212> DNA
<213> Artificial sequence

<200>
<223> Artificial sequence

<400> 17
atgaaattcg cggaactgct ggaacagcag aaaaacgcgc agttcccggg taaagctagc      60

aaaggtgaag aactgttcac cggtgttgtt ccgatcctga ttgaactgga cggtgacgtt     120

aacggtcaca aattctttgt tcgtggtgaa ggtgaaggtg acgcgaccat cggtaaactg     180

agtctgaaat tcatctgcac caccggtaag cttaaagcgg cggcggcgcc ggcgaagctt     240

ccggttccgt ggccgaccct ggttaccacc ctgacctacg gtgttcagtg cttctctcgt     300

tacccggacc acatgaaacg tcacgacttc ttcaaatctg cgatgccgga aggttacgtt     360

caggaacgta ccatctactt caaagacgtc aaagcggcga tcgcgccggc ggacgtcgac     420

ggtacttaca aaacccgtgc ggaagttaaa ttcgaaggta ccggtgacaa accgtctccg     480

ttcggtcagg cggcggcggc ggacaaaggt accgacaccc tggttaaccg tatcgttctg 540

aaaggcattg acttcaaaga agacggtaac atccttaagc agaaagcggc ggaagcgacc 600

aaacttaagg gtcacaaact ggaatacaac ttcaactctc acaacgttta catcaccgcg 660

gacgcggaac cgaaaccggc ggaaccgaaa tctaccgcgg acaaacagaa aaacggtatc 720

aaagcgaact tcaccgttcg tcacaacgtt gaagacggat ccgctgaacc gaaatctgcg 780

gaaccgaaac cgggatccgt tcagctggcg gaccactacc agcagaacac cccgatcggt 840

gacggtccgg gcacctctga agaaggttct cgtggtggtt cttctatgcc gtctgacggt 900

ccggttctgc tgccggacaa ccactacctg tctacccaga ccattctgct gaaagacccg 960

aacgagctct ctccgttcgg tcaggcggcg gcgggtgaca aagagctcaa acgtgaccac 1020

atggttctgc tggaatatgt tacggcggcg ggtatcaccc tgggtatgga cgaactgtac 1080

aaagaattca aacagcgtgc ggcggaagcg accaaatgat gactcgag 1128

<210> 18
<211> 372
<212> PRT
<213> Artificial sequence

<200>
<223> Artificial sequence

<400> 18

Met Lys Phe Ala Glu Leu Leu Glu Gln Gln Lys Asn Ala Gln Phe Pro
1               5                   10                  15

Gly Lys Ala Ser Lys Gly Glu Glu Leu Phe Thr Gly Val Val Pro Ile
            20                  25                  30

Leu Ile Glu Leu Asp Gly Asp Val Asn Gly His Lys Phe Phe Val Arg
        35                  40                  45

Gly Glu Gly Glu Gly Asp Ala Thr Ile Gly Lys Leu Ser Leu Lys Phe
    50                  55                  60

Ile Cys Thr Thr Gly Lys Leu Lys Ala Ala Ala Ala Pro Ala Lys Leu
65                  70                  75                  80

Pro Val Pro Trp Pro Thr Leu Val Thr Thr Leu Thr Tyr Gly Val Gln
                85                  90                  95

Cys Phe Ser Arg Tyr Pro Asp His Met Lys Arg His Asp Phe Phe Lys
            100                 105                 110

Ser Ala Met Pro Glu Gly Tyr Val Gln Glu Arg Thr Ile Tyr Phe Lys
        115                 120                 125

Asp Val Lys Ala Ala Ile Ala Pro Ala Asp Val Asp Gly Thr Tyr Lys
    130                 135                 140

Thr Arg Ala Glu Val Lys Phe Glu Gly Thr Gly Asp Lys Pro Ser Pro
145                 150                 155                 160

Phe Gly Gln Ala Ala Ala Ala Asp Lys Gly Thr Asp Thr Leu Val Asn
                165                 170                 175

Arg Ile Val Leu Lys Gly Ile Asp Phe Lys Glu Asp Gly Asn Ile Leu
            180                 185                 190

Lys Gln Lys Ala Ala Glu Ala Thr Lys Leu Lys Gly His Lys Leu Glu
        195                 200                 205

Tyr Asn Phe Asn Ser His Asn Val Tyr Ile Thr Ala Asp Ala Glu Pro
    210                 215                 220

Lys Pro Ala Glu Pro Lys Ser Thr Ala Asp Lys Gln Lys Asn Gly Ile
225                 230                 235                 240

Lys Ala Asn Phe Thr Val Arg His Asn Val Glu Asp Gly Ser Ala Glu
                245                 250                 255

Pro Lys Ser Ala Glu Pro Lys Pro Gly Ser Val Gln Leu Ala Asp His
            260                 265                 270

Tyr Gln Gln Asn Thr Pro Ile Gly Asp Gly Pro Gly Thr Ser Glu Glu
        275                 280                 285

Gly Ser Arg Gly Gly Ser Ser Met Pro Ser Asp Gly Pro Val Leu Leu
    290                 295                 300

Pro Asp Asn His Tyr Leu Ser Thr Gln Thr Ile Leu Leu Lys Asp Pro
305                 310                 315                 320

Asn Glu Leu Ser Pro Phe Gly Gln Ala Ala Ala Gly Asp Lys Glu Leu
                325                 330                 335

Lys Arg Asp His Met Val Leu Leu Glu Tyr Val Thr Ala Ala Gly Ile
            340                 345                 350

Thr Leu Gly Met Asp Glu Leu Tyr Lys Glu Phe Lys Gln Arg Ala Ala
        355                 360                 365

Glu Ala Thr Lys
    370

<210>  19
<211>  1029
<212>  DNA
<213>  Artificial sequence

<200>
<223> Artificial sequence


<400>  19
atgctgcatg attttcgttc cgatgctagc aaaggtgaag aactgtttac cggtgttgtt      60

ccgattctgg ttgaactgga tggtgatgtt aatggccaca aattttcagt tcgtggtgaa     120

ggcgaaggtg atgcaaccat tggtaaactg accctgaaat ttatctgtac caccggtaag     180

cttccagttc cgtggccgac cctggttacc accctgacct atggtgttca gtgttttgca     240

cgttatccgg atcacatgaa acgccacgat tttttcaaaa gcgcaatgcc ggaaggttat     300

gttcaagaac gtaccattag ctttaaagac gtctgcaaac tgaaactgtg tggtgttctg     360

ggtctggacg tcgatggtaa atacaaaacc cgtgcagttg tgaaatttga gggtacctgt     420

aaactgaaac tgtgcggagt tagcggtctg ggtaccgata ccctggtgaa tcgtattgtt     480

ctgaaaggca ccgattttaa agaagatggc aacattctta agtgggttgc aatgcagacc     540

agcaatctta agggtcataa actggaatac aacttcaaca gccacaacgt gtatattacc     600

gcggatctgc acgattttca tagcgatacc gcggacaaac agaaaaatgg tattaaagcc     660

aattttaccg tgcggcataa tgttgaagat ggatcctgca aactgaaact gtgcggtgtg     720

cctggtctgg gatccgttca gctggcagat cattatcagc agaatacccc gattggtgac     780

ggtccggttg atgaacgtgg tctgtataaa gacggtccgg tgctgctgcc ggataatcat     840

tatctgagca cccagacagt tctgagcaaa gatccgaatg agctcctgca tgatctgcat     900

agtgatgagc tcaaacgtga tcacatggtt ctgctggaat ttgttaccgc agcaggtatt     960

accctgggta tggatgaact gtacaaagaa ttcaaaagtg tgcgcacctg gaacgaaatc    1020

taactcgag                                                            1029

<210>  20
<211>  340
<212>  PRT
<213>  Artificial sequence

<200>
<223> Artificial sequence


<400>  20

Met Leu His Asp Phe Arg Ser Asp Ala Ser Lys Gly Glu Glu Leu Phe
1               5                   10                  15

Thr Gly Val Val Pro Ile Leu Val Glu Leu Asp Gly Asp Val Asn Gly
                20                  25                  30

His Lys Phe Ser Val Arg Gly Glu Gly Glu Gly Asp Ala Thr Ile Gly
            35                  40                  45

Lys Leu Thr Leu Lys Phe Ile Cys Thr Thr Gly Lys Leu Pro Val Pro
    50                  55                  60

Trp Pro Thr Leu Val Thr Thr Leu Thr Tyr Gly Val Gln Cys Phe Ala
65                  70                  75                  80

Arg Tyr Pro Asp His Met Lys Arg His Asp Phe Phe Lys Ser Ala Met
                85                  90                  95

Pro Glu Gly Tyr Val Gln Glu Arg Thr Ile Ser Phe Lys Asp Val Cys
            100                 105                 110

Lys Leu Lys Leu Cys Gly Val Leu Gly Leu Asp Val Asp Gly Lys Tyr
        115                 120                 125

Lys Thr Arg Ala Val Val Lys Phe Glu Gly Thr Cys Lys Leu Lys Leu
    130                 135                 140

Cys Gly Val Ser Gly Leu Gly Thr Asp Thr Leu Val Asn Arg Ile Val
145                 150                 155                 160

Leu Lys Gly Thr Asp Phe Lys Glu Asp Gly Asn Ile Leu Lys Trp Val
                165                 170                 175

Ala Met Gln Thr Ser Asn Leu Lys Gly His Lys Leu Glu Tyr Asn Phe
            180                 185                 190

Asn Ser His Asn Val Tyr Ile Thr Ala Asp Leu His Asp Phe His Ser
        195                 200                 205

Asp Thr Ala Asp Lys Gln Lys Asn Gly Ile Lys Ala Asn Phe Thr Val
    210                 215                 220

Arg His Asn Val Glu Asp Gly Ser Cys Lys Leu Lys Leu Cys Gly Val
225                 230                 235                 240

Pro Gly Leu Gly Ser Val Gln Leu Ala Asp His Tyr Gln Gln Asn Thr
                245                 250                 255

Pro Ile Gly Asp Gly Pro Val Asp Glu Arg Gly Leu Tyr Lys Asp Gly

260                 265                 270

Pro Val Leu Leu Pro Asp Asn His Tyr Leu Ser Thr Gln Thr Val Leu
        275                 280                 285

Ser Lys Asp Pro Asn Glu Leu Leu His Asp Leu His Ser Asp Glu Leu
    290                 295                 300

Lys Arg Asp His Met Val Leu Leu Glu Phe Val Thr Ala Ala Gly Ile
305                 310                 315                 320

Thr Leu Gly Met Asp Glu Leu Tyr Lys Glu Phe Lys Ser Val Arg Thr
                325                 330                 335

Trp Asn Glu Ile
            340

<210>  21
<211>  11
<212>  PRT
<213>  Artificial sequence

<200>
<223> Artificial sequence

<400>  21

Cys Lys Leu Lys Leu Cys Gly Val Leu Gly Leu
1               5                   10

<210>  22
<211>  11
<212>  PRT
<213>  Artificial sequence

<200>
<223> Artificial sequence

<400>  22

Cys Lys Leu Lys Leu Cys Gly Cys Ser Gly Leu
1               5                   10

<210>  23
<211>  11
<212>  PRT
<213>  Artificial sequence

<200>
<223> Artificial sequence

<400>  23

Cys Lys Leu Lys Leu Cys Gly Val Pro Gly Leu

1                   5                   10

<210>  24
<211>  8
<212>  PRT
<213>  Artificial sequence

<200>
<223> Artificial sequence


<400>  24

Val Asp Glu Arg Gly Leu Tyr Lys
1               5


<210>  25
<211>  8
<212>  PRT
<213>  Artificial sequence

<200>
<223> Artificial sequence


<400>  25

Trp Val Ala Met Gln Thr Ser Asn
1               5


<210>  26
<211>  9
<212>  PRT
<213>  Artificial sequence

<200>
<223> Artificial sequence


<400>  26

Lys Ser Val Arg Thr Trp Asn Glu Ile
1               5


<210>  27
<211>  7
<212>  PRT
<213>  Artificial sequence

<200>
<223> Artificial sequence


<400>  27

Leu His Asp Phe His Ser Asp
1               5

<210>  28
<211>  7

<212>  PRT
<213>  Artificial sequence

<200>
<223> Artificial sequence


<400>  28

Leu His Asp Phe Arg Ser Asp
1               5

<210>  29
<211>  7
<212>  PRT
<213>  Artificial sequence

<200>
<223> Artificial sequence


<400>  29

Leu His Asp Leu His Ser Asp
1               5

<210>  30
<211>  13
<212>  PRT
<213>  Artificial sequence

<200>
<223> Artificial sequence


<400>  30

Asp Val Leu Phe Thr Trp Tyr Val Asp Gly Thr Glu Val
1               5                   10

<210>  31
<211>  344
<212>  PRT
<213>  Artificial sequence

<200>
<223> Artificial sequence


<400>  31

Met Asp Val Leu Phe Thr Trp Tyr Val Asp Gly Thr Glu Val Ala Ser
1               5                   10                  15


Lys Gly Glu Glu Leu Phe Thr Gly Val Val Pro Ile Leu Val Glu Leu
            20                  25                  30


Asp Gly Asp Val Asn Gly His Lys Phe Ser Val Arg Gly Glu Gly Glu
        35                  40                  45

Gly Asp Ala Thr Ile Gly Lys Leu Thr Leu Lys Phe Ile Cys Thr Thr
    50                  55                  60

Gly Lys Leu Pro Val Pro Trp Pro Thr Leu Val Thr Thr Leu Thr Tyr
65                  70                  75                  80

Gly Val Gln Cys Phe Ala Arg Tyr Pro Asp His Met Lys Arg His Asp
                85                  90                  95

Phe Phe Lys Ser Ala Met Pro Glu Gly Tyr Val Gln Glu Arg Thr Ile
            100                 105                 110

Ser Phe Lys Asp Val Asp Gly Lys Tyr Lys Thr Arg Ala Val Val Lys
        115                 120                 125

Phe Glu Gly Thr Asp Thr Leu Val Asn Arg Ile Val Leu Lys Gly Thr
    130                 135                 140

Asp Phe Lys Glu Asp Gly Asn Ile Leu Lys Asp Val Leu Phe Thr Trp
145                 150                 155                 160

Tyr Val Asp Gly Thr Glu Val Leu Lys Gly His Lys Leu Glu Tyr Asn
                165                 170                 175

Phe Asn Ser His Asn Val Tyr Ile Thr Ala Asp Val Leu Phe Thr Trp
            180                 185                 190

Tyr Val Asp Gly Thr Glu Val Gly Gly Ser Gly Thr Ala Asp Lys Gln
        195                 200                 205

Lys Asn Gly Ile Lys Ala Asn Phe Thr Val Arg His Asn Val Glu Asp
    210                 215                 220

Gly Ser Val Gln Leu Ala Asp His Tyr Gln Gln Asn Thr Pro Ile Gly
225                 230                 235                 240

Asp Gly Pro Gly Gly Ser Gly Asp Val Leu Phe Thr Trp Tyr Val Asp
                245                 250                 255

Gly Thr Glu Val Asp Gly Pro Val Leu Leu Pro Asp Asn His Tyr Leu
            260                 265                 270

Ser Thr Gln Thr Val Leu Ser Lys Asp Pro Asn Glu Leu Asp Val Leu
        275                 280                 285

Phe Thr Trp Tyr Val Asp Gly Thr Glu Val Gly Gly Ser Gly Glu Leu
    290                 295                 300

Lys Arg Asp His Met Val Leu Leu Glu Phe Val Thr Ala Ala Gly Ile
305                 310                 315                 320

Thr Leu Gly Met Asp Glu Leu Tyr Lys Glu Phe Asp Val Leu Phe Thr
                325                 330                 335

Trp Tyr Val Asp Gly Thr Glu Val
            340

<210>  32
<211>  1035
<212>  DNA
<213>  Artificial sequence

<200>
<223> Artificial sequence

<400>  32
atggatgttc tgtttacctg gtatgttgat ggcaccgaag tggctagcaa aggtgaagaa      60

ctgtttaccg gtgttgttcc gattctggtt gaactggatg gtgatgttaa tggccacaaa     120

ttttcagttc gtggtgaagg cgaaggtgat gcaaccattg gtaaactgac cctgaaattt     180

atctgtacca ccggtaagct tccagttccg tggccgaccc tggttaccac cctgacctat     240

ggtgttcagt gttttgcacg ttatccggat cacatgaaac gccacgattt tttcaaaagc     300

gcaatgccgg aaggttatgt tcaagaacgt accattagct tcaaagacgt cgatggtaaa     360

tacaaaaccc gtgccgttgt taaatttgaa ggtaccgata ccctggtgaa tcgtattgtt     420

ctgaaaggca ccgattttaa agaagatggc aacattctta aggacgtgct gttcacatgg     480

tatgtggacg gtacagaagt tcttaagggt cacaaactgg aatacaactt taacagccac     540

aacgtgtata ttaccgcgga cgtactgttt acgtggtacg tagacggaac ggaagttggt     600

ggtagcggca ccgcggacaa acagaaaaat ggtattaaag ccaattttac cgtgcggcat     660

aatgttgaag atggatccgt tcagctggca gatcattatc agcagaatac cccgattggt     720

gacggtccgg gtggttcagg tgatgtcctg ttcacttggt acgtcgatgg aaccgaggtg     780

gacggtccgg ttctgctgcc ggataatcat tatctgagca cccagaccgt tctgagcaaa     840

gatccgaatg agctcgatgt actgttcacg tggtatgtgg atgggactga agtgggtggt     900

agtggtgagc tcaaacgtga tcacatggtg ctgctggaat ttgttaccgc agcaggtatt     960

accctgggta tggatgaact gtataaagaa ttcgatgtgc tgtttacttg gtacgtggac    1020

gggactgagg tttaa                                                     1035

<210>  33
<211>  380
<212>  PRT
<213>  Artificial sequence

<200>
<223> Artificial sequence

<400> 33

Met Tyr Ile Glu Lys Asp Asp Ser Asp Ala Leu Lys Ala Leu Phe Ala
1               5                   10                  15

Ser Lys Gly Glu Glu Leu Phe Thr Gly Val Val Pro Ile Leu Val Glu
            20                  25                  30

Leu Asp Gly Asp Val Asn Gly His Lys Phe Ser Val Arg Gly Glu Gly
        35                  40                  45

Glu Gly Asp Ala Thr Ile Gly Lys Leu Thr Leu Lys Phe Ile Cys Thr
    50                  55                  60

Thr Gly Lys Leu Pro Val Pro Trp Pro Thr Leu Val Thr Thr Leu Thr
65                  70                  75                  80

Tyr Gly Val Gln Cys Phe Ala Arg Tyr Pro Asp His Met Lys Arg His
                85                  90                  95

Asp Phe Phe Lys Ser Ala Met Pro Glu Gly Tyr Val Gln Glu Arg Thr
            100                 105                 110

Ile Ser Phe Lys Asp Val Gly Asn Phe Met Val Leu Ser Val Asp Asp
        115                 120                 125

Val Asp Gly Lys Tyr Lys Thr Arg Ala Val Val Lys Phe Glu Gly Thr
    130                 135                 140

Gly Asn Phe Met Val Leu Ser Val Asp Asp Gly Thr Asp Thr Leu Val
145                 150                 155                 160

Asn Arg Ile Val Leu Lys Gly Thr Asp Phe Lys Glu Asp Gly Asn Ile
                165                 170                 175

Leu Lys Thr Ser Arg Pro Met Val Asp Leu Thr Phe Gly Gly Val Gln
            180                 185                 190

Leu Lys Gly His Lys Leu Glu Tyr Asn Phe Asn Ser His Asn Val Tyr
        195                 200                 205

Ile Thr Ala Asp Lys Thr Ser Arg Pro Met Val Asp Leu Thr Phe Gly
    210                 215                 220

Gly Val Gln Thr Ala Asp Lys Gln Lys Asn Gly Ile Lys Ala Asn Phe

225                 230                 235                 240

Thr Val Arg His Asn Val Glu Asp Gly Ser Ile Phe Asn Asp Val Pro
                245                 250                 255

Gln Arg Thr Thr Ser Thr Phe Asp Pro Gly Ser Val Gln Leu Ala Asp
            260                 265                 270

His Tyr Gln Gln Asn Thr Pro Ile Gly Asp Gly Pro Ile Phe Asn Asp
        275                 280                 285

Val Pro Gln Arg Thr Thr Ser Thr Phe Asp Pro Asp Gly Pro Val Leu
    290                 295                 300

Leu Pro Asp Asn His Tyr Leu Ser Thr Gln Thr Val Leu Ser Lys Asp
305                 310                 315                 320

Pro Asn Glu Leu Tyr Ser Asp Leu Phe Ser Lys Asn Leu Val Thr Glu
                325                 330                 335

Tyr Glu Leu Lys Arg Asp His Met Val Leu Leu Glu Phe Val Thr Ala
            340                 345                 350

Ala Gly Ile Thr Leu Gly Met Asp Glu Leu Tyr Lys Glu Phe Tyr Ile
        355                 360                 365

Glu Lys Asp Asp Ser Asp Ala Leu Lys Ala Leu Phe
    370                 375                 380

<210>  34
<211>  1149
<212>  DNA
<213>  Artificial sequence

<200>
<223> Artificial sequence


<400>  34
atgtacatcg agaaagacga tagcgacgca ctgaaagcac tgttcgctag caaaggtgaa        60

gaactgttta ccggtgttgt tccgattctg gttgaactgg atggtgatgt taatggccac       120

aaattttcag ttcgtggtga aggcgaaggt gatgcaacca ttggtaaact gaccctgaaa       180

tttatctgta ccaccggtaa gcttccagtt ccgtggccga ccctggttac caccctgacc       240

tatggtgttc agtgttttgc acgttatccg gatcacatga aacgccacga ttttttcaaa        300

agcgcaatgc cggaaggtta tgttcaagaa cgtaccatta gctttaaaga cgtcggcaat       360

tttatggttc tgagcgttga tgacgtcgac ggtaaataca aaacccgtgc agttgttaaa       420

tttgaaggta ccggtaactt tatggtgctg tcagttgatg atggtaccga taccctggtg       480

aatcgtattg ttctgaaagg caccgatttt aaagaagatg gcaacattct taagaccagc 540

cgtccgatgg ttgatctgac ctttggtggt gtgcagctta agggtcataa actggaatat 600

aatttcaaca gccacaacgt gtatatcacc gcggacaaaa cctcacgtcc tatggtggac 660

ctgacattcg gtggcgttca gaccgcggac aaacagaaaa atggtattaa agccaatttt 720

accgtgcggc ataatgttga ggacggatcc atttttaacg atgttccgca gcgtaccacc 780

agtacctttg atccgggatc cgttcagctg gcagatcatt atcagcagaa taccccgatt 840

ggtgacggtc cgatctttaa tgatgtgcct cagcgcacaa cctcaacctt cgatccggac 900

ggtccggttc tgctgccgga taatcattat ctgagcaccc agaccgttct gagcaaagat 960

ccgaatgagc tctatagcga cctgtttagc aaaaatctgg ttaccgaata tgagctcaaa 1020

cgtgatcaca tggtgctgct ggaatttgtt accgcagcag gtattaccct gggtatggat 1080

gaactgtata aagaattcta tatcgaaaaa gatgattccg atgccctgaa agccctgttt 1140

taactcgag 1149

<210> 35
<211> 14
<212> PRT
<213> Artificial sequence

<200>
<223> Artificial sequence


<400> 35

Tyr Ile Glu Lys Asp Asp Ser Asp Ala Leu Lys Ala Leu Phe
1               5                   10

<210> 36
<211> 10
<212> PRT
<213> Artificial sequence

<200>
<223> Artificial sequence


<400> 36

Gly Asn Phe Met Val Leu Ser Val Asp Asp
1               5                   10

<210> 37
<211> 15
<212> PRT
<213> Artificial sequence

<200>
<223> Artificial sequence


<400> 37

Lys Thr Ser Arg Pro Met Val Asp Leu Thr Phe Gly Gly Val Gln
1               5                   10                  15

<210>  38
<211>  15
<212>  PRT
<213>  Artificial sequence

<200>
<223> Artificial sequence


<400>  38

Ile Phe Asn Asp Val Pro Gln Arg Thr Thr Ser Thr Phe Asp Pro
1               5                   10                  15

<210>  39
<211>  14
<212>  PRT
<213>  Artificial sequence

<200>
<223> Artificial sequence


<400>  39

Leu Tyr Ser Asp Leu Phe Ser Lys Asn Leu Val Thr Glu Tyr
1               5                   10

<210>  40
<211>  14
<212>  PRT
<213>  Artificial sequence

<200>
<223> Artificial sequence


<400>  40

Tyr Ile Glu Lys Asp Asp Ser Asp Ala Leu Lys Ala Leu Phe
1               5                   10

<210>  41
<211>  10
<212>  PRT
<213>  Artificial sequence

<200>
<223> Artificial sequence


<400>  41

Lys Leu Ser Ala Thr Asp Trp Ser Ala Ile
1               5                   10

<210>  42
<211>  8
<212>  PRT

<213>  Artificial sequence

<200>
<223> Artificial sequence


<400>  42

Lys Pro Lys Pro Gln Pro Glu Lys
1               5

<210>  43
<211>  9
<212>  PRT
<213>  Artificial sequence

<200>
<223> Artificial sequence


<400>  43

Lys Lys Met Thr Pro Ser Asp Gln Ile
1               5

<210>  44
<211>  10
<212>  PRT
<213>  Artificial sequence

<200>
<223> Artificial sequence


<400>  44

Val Glu Leu Pro Trp Pro Leu Glu Thr Ile
1               5                   10

<210>  45
<211>  340
<212>  PRT
<213>  Artificial sequence

<200>
<223> Artificial sequence


<400>  45

Met Lys Leu Ser Ala Thr Asp Trp Ser Ala Ile Lys Gly Glu Glu Leu
1               5                   10                  15


Phe Thr Gly Val Val Pro Ile Leu Val Glu Leu Asp Gly Asp Val Asn
            20                  25                  30


Gly His Lys Phe Ser Val Arg Gly Glu Gly Glu Gly Asp Ala Thr Ile
        35                  40                  45


Gly Lys Leu Thr Leu Lys Phe Ile Cys Thr Thr Gly Lys Leu Pro Val

50                  55                  60

Pro Trp Pro Thr Leu Val Thr Thr Leu Thr Tyr Gly Val Gln Cys Phe
65                  70                  75                  80

Ala Arg Tyr Pro Asp His Met Lys Arg His Asp Phe Phe Lys Ser Ala
                85                  90                  95

Met Pro Glu Gly Tyr Val Gln Glu Arg Thr Ile Ser Phe Lys Asp Val
            100                 105                 110

Lys Pro Lys Pro Gln Pro Glu Lys Asp Val Asp Gly Lys Tyr Lys Thr
        115                 120                 125

Arg Ala Val Val Lys Phe Glu Gly Thr Lys Lys Met Thr Pro Ser Asp
    130                 135                 140

Gln Ile Gly Thr Asp Thr Leu Val Asn Arg Ile Val Leu Lys Gly Thr
145                 150                 155                 160

Asp Phe Lys Glu Asp Gly Asn Ile Leu Lys Val Glu Leu Pro Trp Pro
                165                 170                 175

Leu Glu Thr Ile Leu Lys Gly His Lys Leu Glu Tyr Asn Phe Asn Ser
            180                 185                 190

His Asn Val Tyr Ile Thr Ala Asp Lys Pro Lys Pro Gln Pro Glu Lys
        195                 200                 205

Thr Ala Asp Lys Gln Lys Asn Gly Ile Lys Ala Asn Phe Thr Val Arg
    210                 215                 220

His Asn Val Glu Asp Gly Ser Lys Leu Ser Ala Thr Asp Trp Ser Ala
225                 230                 235                 240

Ile Gly Ser Val Gln Leu Ala Asp His Tyr Gln Gln Asn Thr Pro Ile
                245                 250                 255

Gly Asp Gly Pro Val Glu Leu Pro Trp Pro Leu Glu Thr Ile Asp Gly
            260                 265                 270

Pro Val Leu Leu Pro Asp Asn His Tyr Leu Ser Thr Gln Thr Val Leu
        275                 280                 285

Ser Lys Asp Pro Asn Glu Leu Lys Lys Met Thr Pro Ser Asp Gln Ile
    290                 295                 300

Glu Leu Lys Arg Asp His Met Val Leu Leu Glu Phe Val Thr Ala Ala
305                 310                 315                 320

Gly Ile Thr Leu Gly Met Asp Glu Leu Tyr Lys Leu Ser Ala Thr Asp
                325                 330                 335

Trp Ser Ala Ile
            340

<210>  46
<211>  1023
<212>  DNA
<213>  Artificial sequence

<200>
<223> Artificial sequence

<400>  46
atgaaactga gcgcaaccga ttggagcgca attaaaggtg aagaactgtt taccggtgtt      60

gttccgattc tggttgaact ggatggtgat gttaatggcc acaaattttc agttcgtggt     120

gaaggcgaag gtgatgcaac cattggtaaa ctgaccctga aatttatctg taccaccggt     180

aagcttccag ttccgtggcc gaccctggtt accaccctga cctatggtgt tcagtgtttt     240

gcacgttatc cggatcacat gaaacgccac gattttttca aaagcgcaat gccggaaggt      300

tatgttcaag aacgtaccat tagcttcaaa gacgtcaaac cgaaaccgca gccggaaaaa     360

gacgtcgatg gtaaatacaa aacccgtgcc gttgttaaat ttgaaggtac caaaaaaatg     420

accccgagcg atcagattgg taccgatacc ctggtgaatc gtattgttct gaaaggcacc     480

gattttaaag aagatggcaa catccttaag gtggaactgc cgtggcctct ggaaaccatt     540

cttaagggtc ataaactgga atataatttc aacagccaca acgtgtatat caccgcggac     600

aaacctaaac ctcaacctga aaaaaccgcg gacaaacaga aaaatggcat caaagcaaat     660

tttaccgtgc gccataatgt tgaggatgga tccaaactgt cagccaccga ttggtcagca     720

attggatccg ttcagctggc agatcattat cagcagaata ccccgattgg tgacggtccg     780

gttgagctgc cttggccact ggaaacaatt gacggtccgg tgctgctgcc ggataatcat     840

tatctgagca cccagaccgt tctgagcaaa gatccgaatg agctcaaaaa aatgacaccg     900

tcagatcaga tcgagctcaa acgtgatcac atggttctgc tggaatttgt taccgcagca     960

ggtattaccc tgggtatgga tgaactgtat aaactgagtg cgacagactg gtctgcaatc    1020

taa                                                                  1023

<210>  47
<211>  9
<212>  PRT
<213>  Artificial sequence

<200>

<223> Artificial sequence

<400> 47

His Arg Ile Arg Leu Leu Leu Gln Ser
1               5

<210> 48
<211> 9
<212> PRT
<213> Artificial sequence

<200>
<223> Artificial sequence

<400> 48

Ser Tyr Arg Thr Gly Ala Glu Arg Val
1               5

<210> 49
<211> 9
<212> PRT
<213> Artificial sequence

<200>
<223> Artificial sequence

<400> 49

Asn Gly Val Lys Gln Thr Val Asp Val
1               5

<210> 50
<211> 9
<212> PRT
<213> Artificial sequence

<200>
<223> Artificial sequence

<400> 50

Gln Ser Arg Thr Leu Asp Ser Arg Asp
1               5

<210> 51
<211> 338
<212> PRT
<213> Artificial sequence

<200>
<223> Artificial sequence

<400> 51

Met His Arg Ile Arg Leu Leu Leu Gln Ser Lys Gly Glu Glu Leu Phe
1               5                   10                  15

Thr Gly Val Val Pro Ile Leu Val Glu Leu Asp Gly Asp Val Asn Gly
            20                  25                  30

His Lys Phe Ser Val Arg Gly Glu Gly Glu Gly Asp Ala Thr Ile Gly
        35                  40                  45

Lys Leu Thr Leu Lys Phe Ile Cys Thr Thr Gly Lys Leu Pro Val Pro
    50                  55                  60

Trp Pro Thr Leu Val Thr Thr Leu Thr Tyr Gly Val Gln Cys Phe Ala
65                  70                  75                  80

Arg Tyr Pro Asp His Met Lys Arg His Asp Phe Phe Lys Ser Ala Met
                85                  90                  95

Pro Glu Gly Tyr Val Gln Glu Arg Thr Ile Ser Phe Lys Asp Val Ser
            100                 105                 110

Tyr Arg Thr Gly Ala Glu Arg Val Asp Val Asp Gly Lys Tyr Lys Thr
        115                 120                 125

Arg Ala Val Val Lys Phe Glu Gly Thr Asn Gly Val Lys Gln Thr Val
    130                 135                 140

Asp Val Gly Thr Asp Thr Leu Val Asn Arg Ile Val Leu Lys Gly Thr
145                 150                 155                 160

Asp Phe Lys Glu Asp Gly Asn Ile Leu Lys Gln Ser Arg Thr Leu Asp
                165                 170                 175

Ser Arg Asp Leu Lys Gly His Lys Leu Glu Tyr Asn Phe Asn Ser His
            180                 185                 190

Asn Val Tyr Ile Thr Ala Asp Gln Ser Arg Thr Leu Asp Ser Arg Asp
        195                 200                 205

Thr Ala Asp Lys Gln Lys Asn Gly Ile Lys Ala Asn Phe Thr Val Arg
    210                 215                 220

His Asn Val Glu Asp Gly Ser His Arg Ile Arg Leu Leu Leu Gln Ser
225                 230                 235                 240

Gly Ser Val Gln Leu Ala Asp His Tyr Gln Gln Asn Thr Pro Ile Gly
                245                 250                 255

Asp Gly Pro Ser Tyr Arg Thr Gly Ala Glu Arg Val Asp Gly Pro Val

260                 265                 270

Leu Leu Pro Asp Asn His Tyr Leu Ser Thr Gln Thr Val Leu Ser Lys
        275                 280                 285

Asp Pro Asn Glu Leu Asn Gly Val Lys Gln Thr Val Asp Val Glu Leu
    290                 295                 300

Lys Arg Asp His Met Val Leu Leu Glu Phe Val Thr Ala Ala Gly Ile
305                 310                 315                 320

Thr Leu Gly Met Asp Glu Leu Tyr Lys His Arg Ile Arg Leu Leu Leu
                325                 330                 335

Gln Ser

<210>  52
<211>  1017
<212>  DNA
<213>  Artificial sequence

<200>
<223> Artificial sequence

<400>  52
atgcatcgta ttcgtctgct gctgcagagc aaaggtgaag aactgtttac cggtgttgtt        60

ccgattctgg ttgaactgga tggtgatgtt aatggccaca aattttcagt tcgtggtgaa       120

ggcgaaggtg atgcaaccat tggtaaactg accctgaaat ttatctgtac caccggtaag       180

cttccagttc cgtggccgac cctggttacc accctgacct atggtgttca gtgttttgca       240

cgttatccgg atcacatgaa acgccacgat tttttcaaaa gcgcaatgcc ggaaggttat       300

gttcaagaac gtaccattag ctttaaagac gtcagctatc gtaccggtgc agaacgtgtt       360

gacgtcgatg gtaaatacaa aacccgtgcc gttgttaaat ttgaaggtac caatggcgtt       420

aaacagaccg ttgatgttgg taccgatacc ctggtgaatc gtattgttct gaaaggcacc       480

gattttaaag aagatggcaa cattcttaag cagagccgta ccctggatag ccgtgatctt       540

aagggtcata aactggaata taatttcaac agccacaacg tgtatattac cgcggaccag       600

tcacgcaccc tggattcacg tgacaccgcg gacaaacaga aaaatggtat taaagccaat       660

tttaccgtgc ggcataatgt tgaagatgga tcccatcgta tccgcctgct gctgcaaagc       720

ggatccgttc agctggcaga tcattatcag cagaataccc cgattggtga cggtccgagt       780

tatcgcacag gtgccgaacg cgtggacggt ccggttctgc tgccggataa tcattatctg       840

agcacccaga ccgttctgag caaagatccg aatgagctca atggtgtgaa acaaacagtg       900

gatgtagagc tcaaacgtga tcacatggtg ctgctggaat ttgttaccgc agcaggtatt       960

accctgggta tggatgaact gtataaacac cgcattcggc tgctgctgca atcataa 1017

<210> 53
<211> 14
<212> PRT
<213> Artificial sequence

<200>
<223> Artificial sequence

<400> 53

Pro Tyr Thr Ser Arg Arg Ser Val Ala Ser Ile Val Gly Thr
1               5                   10

<210> 54
<211> 10
<212> PRT
<213> Artificial sequence

<200>
<223> Artificial sequence

<400> 54

Gln Tyr Tyr Asp Tyr Glu Asp Ala Thr Phe
1               5                   10

<210> 55
<211> 10
<212> PRT
<213> Artificial sequence

<200>
<223> Artificial sequence

<400> 55

Gly Pro Lys Gln Leu Thr Phe Glu Gly Lys
1               5                   10

<210> 56
<211> 10
<212> PRT
<213> Artificial sequence

<200>
<223> Artificial sequence

<400> 56

Asp Ala Thr Phe Glu Thr Tyr Ala Leu Thr
1               5                   10

<210> 57
<211> 9
<212> PRT
<213> Artificial sequence

<200>
<223> Artificial sequence

<400> 57

Leu Thr Val Glu Asp Ser Pro Tyr Pro
1               5

<210> 58
<211> 9
<212> PRT
<213> Artificial sequence

<200>
<223> Artificial sequence

<400> 58

Ala Leu Ala Thr Tyr Gln Ser Glu Tyr
1               5

<210> 59
<211> 14
<212> PRT
<213> Artificial sequence

<200>
<223> Artificial sequence

<400> 59

Pro Gly Ile Val Ile Pro Pro Lys Ala Leu Phe Thr Gln Gln
1               5                   10

<210> 60
<211> 9
<212> PRT
<213> Artificial sequence

<200>
<223> Artificial sequence

<400> 60

Ala Val Glu Ala Glu Arg Ala Gly Arg
1               5

<210> 61
<211> 9
<212> PRT
<213> Artificial sequence

<200>
<223> Artificial sequence

<400> 61

Thr Thr Thr Glu Tyr Ser Asn Ala Arg
1               5

<210>  62
<211>  14
<212>  PRT
<213>  Artificial sequence

<200>
<223> Artificial sequence


<400>  62

Glu Arg Ala Gly Glu Ala Met Val Leu Val Tyr Tyr Glu Ser
1               5                   10

<210>  63
<211>  1101
<212>  DNA
<213>  Artificial sequence

<200>
<223> Artificial sequence


<400>  63
atgccctaca cctcacgtcg cagcgtagct tcgattgttg gcacgtctta ttggaaaggg      60

tctcaatact atgactatga agatgcaact tttaaaggag aggagttgtt taccggcgtg     120

gtgccgatcc ttgtggagtt ggatggagac gttaacggtc acaagttttc agttcgcggt     180

gaaggcgaag gcgacgcgac tattggtaag cttaccttga agttcatctg tactacgggg     240

cctaagcagc ttacgttcga ggggaaatta cctgtcccct ggccaacatt ggtcacgaca     300

ttaacctatg gcgtgcagtg ttttgcgcgt taccccgatc acatgaagcg tcacgacttc     360

tttaagtccg cgatgccgga agggtatgtc caggaacgca cgatttcgtt caaagacgcg     420

acctttgaga cttacgcatt aaccgggggc tcgggcgagg cagccgcaaa agaagctgct     480

gctgacggca aatacaaaac tcgtgcggtc gtaaaattcg agggagacac tttagttaat     540

cgtattgtgc tgaaagggac tgacttcaag gaagatggaa acattttgac ggttgaggat     600

agcccctatc ctggccataa acttgagtac aacttcaatt cacataatgt ctacattaca     660

gcacttgcaa cgtatcagtc tgagtacgat aagcagaaga atggtatcaa ggctaatttt     720

acggtccgtc ataatgttga ggatgggagc gtgcagttag ctgaccatta tcaacaaaat     780

acgcccattg gggaccccgg catcgtgatt cccccaaaag cattattcac ccagcaagtg     840

cttttaccag acaaccatta cttgagcacc caaacggtgt taagcaagga ccccaatgag     900

aaagcagtgg aggcggaacg cgctggacgt gatcatatgg tacttcttga attcgtaacg     960

gcggccggga tcactttggg aatggacgag ttatataaag agcgtgcggg tgaggccatg    1020

gtgcttgtgt attacgagtc agaagccgcg aaggaggctg ccaagacaac aacggaatac    1080

agtaacgccc gtaaaaagta a                                                1101

<210>  64
<211>  366
<212>  PRT
<213>  Artificial sequence

<200>
<223> Artificial sequence

<400>  64

Met Pro Tyr Thr Ser Arg Arg Ser Val Ala Ser Ile Val Gly Thr Ser
1               5                   10                  15

Tyr Trp Lys Gly Ser Gln Tyr Tyr Asp Tyr Glu Asp Ala Thr Phe Lys
            20                  25                  30

Gly Glu Glu Leu Phe Thr Gly Val Val Pro Ile Leu Val Glu Leu Asp
        35                  40                  45

Gly Asp Val Asn Gly His Lys Phe Ser Val Arg Gly Glu Gly Glu Gly
    50                  55                  60

Asp Ala Thr Ile Gly Lys Leu Thr Leu Lys Phe Ile Cys Thr Thr Gly
65                  70                  75                  80

Pro Lys Gln Leu Thr Phe Glu Gly Lys Leu Pro Val Pro Trp Pro Thr
                85                  90                  95

Leu Val Thr Thr Leu Thr Tyr Gly Val Gln Cys Phe Ala Arg Tyr Pro
            100                 105                 110

Asp His Met Lys Arg His Asp Phe Phe Lys Ser Ala Met Pro Glu Gly
        115                 120                 125

Tyr Val Gln Glu Arg Thr Ile Ser Phe Lys Asp Ala Thr Phe Glu Thr
    130                 135                 140

Tyr Ala Leu Thr Gly Gly Ser Gly Glu Ala Ala Ala Lys Glu Ala Ala
145                 150                 155                 160

Ala Asp Gly Lys Tyr Lys Thr Arg Ala Val Val Lys Phe Glu Gly Asp
                165                 170                 175

Thr Leu Val Asn Arg Ile Val Leu Lys Gly Thr Asp Phe Lys Glu Asp
            180                 185                 190

Gly Asn Ile Leu Thr Val Glu Asp Ser Pro Tyr Pro Gly His Lys Leu
        195                 200                 205

Glu Tyr Asn Phe Asn Ser His Asn Val Tyr Ile Thr Ala Leu Ala Thr
    210                 215                 220

Tyr Gln Ser Glu Tyr Asp Lys Gln Lys Asn Gly Ile Lys Ala Asn Phe
225                 230                 235                 240

Thr Val Arg His Asn Val Glu Asp Gly Ser Val Gln Leu Ala Asp His
                245                 250                 255

Tyr Gln Gln Asn Thr Pro Ile Gly Asp Pro Gly Ile Val Ile Pro Pro
            260                 265                 270

Lys Ala Leu Phe Thr Gln Gln Val Leu Leu Pro Asp Asn His Tyr Leu
        275                 280                 285

Ser Thr Gln Thr Val Leu Ser Lys Asp Pro Asn Glu Lys Ala Val Glu
    290                 295                 300

Ala Glu Arg Ala Gly Arg Asp His Met Val Leu Leu Glu Phe Val Thr
305                 310                 315                 320

Ala Ala Gly Ile Thr Leu Gly Met Asp Glu Leu Tyr Lys Glu Arg Ala
                325                 330                 335

Gly Glu Ala Met Val Leu Val Tyr Tyr Glu Ser Glu Ala Ala Lys Glu
            340                 345                 350

Ala Ala Lys Thr Thr Thr Glu Tyr Ser Asn Ala Arg Lys Lys
        355                 360                 365

<210>  65
<211>  14
<212>  PRT
<213>  Artificial sequence

<200>
<223> Artificial sequence


<400>  65

Ser Pro Trp Ser Trp Pro Asp Leu Asp Leu Lys Pro Gly Ala
1               5                   10

<210>  66
<211>  14
<212>  PRT
<213>  Artificial sequence

<200>
<223> Artificial sequence

<400>  66

Asp Gly Asn Cys Asp Gly Arg Gly Lys Ser Thr Arg Ser Thr
1               5                   10

<210>  67
<211>  14
<212>  PRT
<213>  Artificial sequence

<200>
<223> Artificial sequence


<400>  67

Val Phe Ser Pro Gly Arg Lys Asn Gly Ser Phe Ile Ile Asp
1               5                   10

<210>  68
<211>  14
<212>  PRT
<213>  Artificial sequence

<200>
<223> Artificial sequence


<400>  68

His Val Gln Asp Cys Asp Glu Ser Val Leu Thr Arg Leu Glu
1               5                   10

<210>  69
<211>  14
<212>  PRT
<213>  Artificial sequence

<200>
<223> Artificial sequence


<400>  69

Asp Cys Asp Gly Ser Ile Leu Gly Ala Ala Val Asn Gly Lys
1               5                   10

<210>  70
<211>  9
<212>  PRT
<213>  Artificial sequence

<200>
<223> Artificial sequence


<400>  70

Phe Thr Thr Arg Val Tyr Met Asp Ala
1               5

<210>  71

<211>  14
<212>  PRT
<213>  Artificial sequence

<200>
<223> Artificial sequence


<400>  71

Arg Asp Ser Asp Asp Trp Leu Asn Lys Tyr Ser Tyr Tyr Pro
1               5                   10

<210>  72
<211>  14
<212>  PRT
<213>  Artificial sequence

<200>
<223> Artificial sequence


<400>  72

Glu Ser Glu Met Phe Met Pro Arg Ser Ile Gly Gly Pro Val
1               5                   10

<210>  73
<211>  14
<212>  PRT
<213>  Artificial sequence

<200>
<223> Artificial sequence


<400>  73

Ala Glu Ala Glu Met Val Ile His His Gln His Val Gln Asp
1               5                   10

<210>  74
<211>  19
<212>  PRT
<213>  Artificial sequence

<200>
<223> Artificial sequence


<400>  74

Leu Glu His Glu Met Trp Arg Ser Arg Ala Asp Glu Ile Asn Ala Ile
1               5                   10                  15


Phe Glu Glu

<210>  75
<211>  430
<212>  PRT
<213>  Artificial sequence

<200>
<223> Artificial sequence

<400> 75

Met Gly Ser Ser His His His His His His Ser Ser Gly Leu Val Pro
1               5                   10                  15

Arg Gly Ser His Met Ser Pro Trp Ser Trp Pro Asp Leu Asp Leu Lys
            20                  25                  30

Pro Gly Ala Lys Gly Glu Glu Leu Phe Thr Gly Val Val Pro Ile Leu
        35                  40                  45

Val Glu Leu Asp Gly Asp Val Asn Gly His Lys Phe Ser Val Arg Gly
    50                  55                  60

Glu Gly Glu Gly Asp Ala Thr Ile Gly Lys Leu Thr Leu Lys Phe Ile
65                  70                  75                  80

Cys Thr Thr Gly Lys Leu Pro Val Pro Trp Pro Thr Leu Val Thr Thr
                85                  90                  95

Leu Thr Tyr Gly Val Gln Cys Phe Ala Arg Tyr Pro Asp His Met Lys
            100                 105                 110

Arg His Asp Phe Phe Lys Ser Ala Met Pro Glu Gly Tyr Val Gln Glu
        115                 120                 125

Arg Thr Ile Ser Phe Lys Asp Gly Asn Cys Asp Gly Arg Gly Lys Ser
    130                 135                 140

Thr Arg Ser Thr Gly Gly Ser Gly Glu Ala Ala Ala Lys Glu Ala Ala
145                 150                 155                 160

Ala Lys Lys Tyr Lys Thr Arg Ala Val Val Lys Phe Glu Gly Val Phe
                165                 170                 175

Ser Pro Gly Arg Lys Asn Gly Ser Phe Ile Ile Asp Gly Gly Ser Gly
            180                 185                 190

Glu Ala Ala Ala Lys Glu Ala Ala Ala Lys Asp Thr Leu Val Asn Arg
        195                 200                 205

Ile Val Leu Lys Gly Thr Asp Phe Lys Glu Asp Gly Asn Ile Leu Gly
    210                 215                 220

His Lys His Val Gln Asp Cys Asp Glu Ser Val Leu Thr Arg Leu Glu

225                 230                 235                 240

Tyr Asn Phe Asn Ser His Asn Val Tyr Ile Thr Ala Asp Cys Asp Gly
                245                 250                 255

Ser Ile Leu Gly Ala Ala Val Asn Gly Lys Gln Lys Asn Gly Ile Lys
            260                 265                 270

Ala Asn Phe Thr Val Arg His Asn Val Glu Asp Phe Thr Thr Arg Val
        275                 280                 285

Tyr Met Asp Ala Gly Thr Ser Trp Lys Gly Gly Ser Val Gln Leu Ala
    290                 295                 300

Asp His Tyr Gln Gln Asn Thr Pro Ile Gly Asp Arg Asp Ser Asp Asp
305                 310                 315                 320

Trp Leu Asn Lys Tyr Ser Tyr Tyr Pro Val Leu Leu Pro Asp Asn His
                325                 330                 335

Tyr Leu Ser Thr Gln Thr Val Leu Ser Lys Asp Pro Asn Glu Ser Glu
            340                 345                 350

Met Phe Met Pro Arg Ser Ile Gly Gly Pro Val Lys Arg Asp His Met
        355                 360                 365

Val Leu Leu Glu Phe Val Thr Ala Ala Gly Ile Thr Leu Gly Met Asp
    370                 375                 380

Glu Leu Tyr Lys Leu Glu His Glu Met Trp Arg Ser Arg Ala Asp Glu
385                 390                 395                 400

Ile Asn Ala Ile Phe Glu Glu Thr Ser Tyr Trp Lys Gly Ser Ala Glu
                405                 410                 415

Ala Glu Met Val Ile His His Gln His Val Gln Asp Lys Lys
            420                 425                 430

<210>  76
<211>  1293
<212>  DNA
<213>  Artificial sequence

<200>
<223> Artificial sequence


<400>  76
atgggcagca gccatcatca tcatcatcac agcagcggcc tggtgccgcg cggcagccat        60

atgtccccat ggagttggcc tgaccttgat ttaaagcccg gtgctaaagg agaagaactg       120

ttcacagggg tcgttcccat cttagtggag ctggacggcg acgtgaacgg ccataaattc 180

agtgtgcgcg gggaaggaga aggggacgca acgattggta agttgacgct gaaatttatc 240

tgtacaactg gtaagcttcc agtgccgtgg ccgacgcttg tgacgacact tacttatgga 300

gtccagtgtt ttgcgcgtta tccagatcac atgaaacgcc acgacttctt taagtccgca 360

atgccggagg gctacgtgca ggaacgcaca atctcgttta aggacgggaa ctgcgatggc 420

cgtgggaaaa gtacacgctc aacgggcgga tcaggcgaag ccgcagcaaa agaggctgcg 480

gccaaaaaat ataaaactcg tgccgtagtg aaatttgagg gagtgttttc cccgggccgt 540

aagaacggca gttttatcat tgacggcggc tcaggagaag cggctgcaaa ggaggccgcc 600

gcgaaggaca ccttagtgaa ccgcattgtt ttgaaaggaa cggattttaa agaggacgga 660

aacattttag gacacaagca tgtccaagac tgcgatgaga gtgttcttac gcgtttagaa 720

tataatttca atagtcataa cgtatacatt acggctgatt gtgatggcag tatcttagga 780

gcggctgtta acgggaagca aaagaatgga atcaaagcca actttactgt tcgtcataat 840

gtcgaagact tcaccactcg tgtctacatg gatgccggta ctagctggaa aggcgggtcc 900

gttcagcttg cggatcatta ccaacaaaac actccgatcg gggaccgtga ctccgatgat 960

tggctgaaca agtattcata ctaccctgtg ctgctgcccg ataaccatta tttgagcaca 1020

cagaccgtgc tgtctaaaga tcctaatgaa tcagaaatgt ttatgcctcg ttcgatcggg 1080

ggacccgtta aacgcgatca catggtattg ttggaattcg ttacggcagc gggcatcacg 1140

cttggtatgg atgagttgta taaacttgag catgaaatgt ggcgctcgcg tgcagacgaa 1200

atcaatgcaa tttttgaaga gactagctat tggaagggta gtgcggaagc tgaaatggtc 1260

attcaccacc agcatgtcca ggacaaaaaa taa 1293

<210> 77
<211> 245
<212> PRT
<213> Artificial sequence

<200>
<223> Artificial sequence

<400> 77

Met Gly Ala His Ala Ser Val Ile Lys Pro Glu Met Lys Ile Lys Leu
1               5                   10                  15

Arg Met Glu Gly Ala Val Asn Gly His Lys Phe Val Ile Glu Gly Glu
            20                  25                  30

Gly Ile Gly Lys Pro Tyr Glu Gly Thr Gln Thr Leu Asp Leu Thr Val
        35                  40                  45

Glu Glu Gly Ala Pro Leu Pro Phe Ser Tyr Asp Ile Leu Thr Pro Ala
    50                  55                  60

Phe Gln Tyr Gly Asn Arg Ala Phe Thr Lys Tyr Pro Glu Asp Ile Pro
65                  70                  75                  80

Asp Tyr Phe Lys Gln Ala Phe Pro Glu Gly Tyr Ser Trp Glu Arg Ser
                85                  90                  95

Met Thr Tyr Glu Asp Gln Gly Ile Cys Ile Ala Thr Ser Asp Ile Thr
            100                 105                 110

Met Glu Gly Asp Cys Phe Phe Tyr Glu Ile Arg Phe Asp Gly Thr Asn
        115                 120                 125

Phe Pro Pro Asn Gly Pro Val Met Gln Lys Lys Thr Leu Lys Trp Glu
    130                 135                 140

Pro Ser Thr Glu Lys Met Tyr Val Glu Asp Gly Val Leu Lys Gly Asp
145                 150                 155                 160

Val Glu Met Ala Leu Leu Leu Glu Gly Gly Gly His Tyr Arg Cys Asp
                165                 170                 175

Phe Lys Thr Thr Tyr Lys Ala Lys Lys Asp Val Arg Leu Pro Asp Ala
            180                 185                 190

His Glu Val Asp His Arg Ile Glu Ile Leu Ser His Asp Lys Asp Tyr
        195                 200                 205

Asn Lys Val Arg Leu Tyr Glu His Ala Glu Ala Arg Tyr Ser Gly Gly
    210                 215                 220

Gly Ser Gly Gly Gly Ala Ser Gly Lys Pro Ile Pro Asn Pro Leu Leu
225                 230                 235                 240

Gly Leu Asp Ser Thr
                245

<210>  78
<211>  738
<212>  DNA
<213>  Artificial sequence

<200>
<223> Artificial sequence


<400>  78
atgggtgcgc acgcgtctgt tatcaaaccg gaaatgaaaa tcaaactgcg tatggaaggt        60

gcggttaacg gtcacaaatt cgttatcgaa ggtgaaggta tcggtaaacc gtacgaaggt     120

acccagaccc tggacctgac cgttgaagaa ggtgcgccgc tgccgttctc ttacgacatc     180

ctgaccccgg cgttccagta cggtaaccgt gcgttcacca aatacccgga agacatcccg     240

gactacttca aacaggcgtt cccggaaggt tactcttggg aacgttctat gacctacgaa     300

gaccagggta tctgcatcgc gacctctgac atcaccatgg aaggtgactg cttcttctac     360

gaaatccgtt tcgacggtac caacttcccg ccgaacggtc cggttatgca gaaaaaaacc     420

ctgaaatggg aaccgtctac cgaaaaaatg tacgttgaag acggtgttct gaaaggtgac     480

gttgaaatgg cgctgctgct ggaaggtggt ggtcactacc gttgcgactt caaaaccacc     540

tacaaagcga aaaaagacgt tcgtctgccg gacgcgcacg aagttgacca ccgtatcgaa     600

atcctgtctc acgacaaaga ctacaacaaa gttcgtctgt acgaacacgc ggaagcgcgt     660

tactctggtg gtggttctgg tggtggtgcg tctggtaaac cgatcccgaa cccgctgctg     720

ggtctggact ctacctaa                                                   738

<210>  79
<211>  20
<212>  PRT
<213>  Artificial sequence

<200>
<223> Artificial sequence


<400>  79

Asp Leu Arg Gln Met Arg Thr Val Thr Pro Ile Arg Met Gln Gly Gly
1               5                   10                  15


Cys Gly Ser Cys
            20

<210>  80
<211>  20
<212>  PRT
<213>  Artificial sequence

<200>
<223> Artificial sequence


<400>  80

Gly Cys Gly Ser Cys Trp Ala Phe Ser Gly Val Ala Ala Thr Glu Ser
1               5                   10                  15


Ala Tyr Leu Ala
            20

<210>  81
<211>  15

<212>  PRT
<213>  Artificial sequence

<200>
<223> Artificial sequence


<400>  81

Gln Glu Ser Tyr Tyr Arg Tyr Val Ala Arg Glu Gln Ser Cys Arg
1               5                   10                  15

<210>  82
<211>  15
<212>  PRT
<213>  Artificial sequence

<200>
<223> Artificial sequence


<400>  82

His Ala Val Asn Ile Val Gly Tyr Ser Asn Ala Gln Gly Val Asp
1               5                   10                  15

<210>  83
<211>  20
<212>  PRT
<213>  Artificial sequence

<200>
<223> Artificial sequence


<400>  83

Cys His Gly Ser Glu Pro Cys Ile Ile His Arg Gly Lys Pro Phe Gln
1               5                   10                  15


Leu Glu Ala Val
            20

<210>  84
<211>  20
<212>  PRT
<213>  Artificial sequence

<200>
<223> Artificial sequence


<400>  84

Tyr Asp Ile Lys Tyr Thr Trp Asn Val Pro Lys Ile Ala Pro Lys Ser
1               5                   10                  15


Glu Asn Val Val
            20

<210>  85

<211>  15
<212>  PRT
<213>  Artificial sequence

<200>
<223> Artificial sequence


<400>  85

Asn Thr Lys Thr Ala Lys Ile Glu Ile Lys Ala Ser Ile Asp Gly
1               5                   10                  15

<210>  86
<211>  15
<212>  PRT
<213>  Artificial sequence

<200>
<223> Artificial sequence


<400>  86

Gly Val Leu Ala Cys Ala Ile Ala Thr His Ala Lys Ile Arg Asp
1               5                   10                  15

<210>  87
<211>  20
<212>  PRT
<213>  Artificial sequence

<200>
<223> Artificial sequence


<400>  87

Pro Lys Asp Pro His Lys Phe Tyr Ile Cys Ser Asn Trp Glu Ala Val
1               5                   10                  15


His Lys Asp Cys
            20

<210>  88
<211>  366
<212>  PRT
<213>  Artificial sequence

<200>
<223> Artificial sequence


<400>  88

Met Pro Lys Asp Pro His Lys Phe Tyr Ile Cys Ser Asn Trp Glu Ala
1               5                   10                  15


Val His Lys Asp Cys Ser Val Ile Lys Pro Glu Met Lys Ile Lys Leu
            20                  25                  30

Arg Met Glu Gly Ala Val His Ala Val Asn Ile Val Gly Tyr Ser Asn
        35                  40                  45

Ala Gln Gly Val Asp His Lys Phe Val Ile Glu Gly Glu Gly Ile Gly
    50                  55                  60

Lys Pro Tyr Glu Gly Thr Gln Thr Leu Asp Leu Thr Val Glu Glu Gly
65                  70                  75                  80

Ala Pro Leu Pro Phe Ser Tyr Asp Ile Leu Thr Pro Ala Phe Gln Tyr
                85                  90                  95

Gly Asn Arg Ala Phe Thr Lys Tyr Pro Glu Asp Ile Pro Asp Tyr Phe
            100                 105                 110

Lys Gln Ala Phe Pro Glu Gly Tyr Ser Trp Glu Arg Ser Met Thr Tyr
        115                 120                 125

Gly Val Leu Ala Cys Ala Ile Ala Thr His Ala Lys Ile Arg Asp Gly
    130                 135                 140

Ile Cys Ile Ala Thr Ser Asp Ile Thr Met Glu Tyr Asp Ile Lys Tyr
145                 150                 155                 160

Thr Trp Asn Val Pro Lys Ile Ala Pro Lys Ser Glu Asn Val Val Cys
                165                 170                 175

Phe Phe Tyr Glu Ile Arg Phe Asp Gly Thr Gln Glu Ser Tyr Tyr Arg
            180                 185                 190

Tyr Val Ala Arg Glu Gln Ser Cys Arg Thr Leu Lys Trp Glu Pro Ser
        195                 200                 205

Thr Glu Lys Met Tyr Val Glu Asp Gly Val Leu Lys Gly Asp Val Glu
    210                 215                 220

Met Ala Leu Leu Leu Cys His Gly Ser Glu Pro Cys Ile Ile His Arg
225                 230                 235                 240

Gly Lys Pro Phe Gln Leu Glu Ala Val His Tyr Arg Cys Asp Phe Lys
                245                 250                 255

Thr Thr Tyr Lys Ala Gly Cys Gly Ser Cys Trp Ala Phe Ser Gly Val
            260                 265                 270

Ala Ala Thr Glu Ser Ala Tyr Leu Ala His Glu Val Asp His Arg Ile
        275                 280                 285

Glu Ile Leu Ser His Asp Lys Asp Tyr Asn Lys Val Arg Leu Tyr Glu
    290                 295                 300

His Ala Glu Ala Arg Tyr Ser Gly Gly Ser Gly Asp Leu Arg Gln Met
305                 310                 315                 320

Arg Thr Val Thr Pro Ile Arg Met Gln Gly Gly Cys Gly Ser Cys Gly
                325                 330                 335

Gly Ser Gly Asn Thr Lys Thr Ala Lys Ile Glu Ile Lys Ala Ser Ile
            340                 345                 350

Asp Gly Leu Asp Ser Thr His His His His His His Lys Lys
        355                 360                 365

<210>  89
<211>  1101
<212>  DNA
<213>  Artificial sequence

<200>
<223> Artificial sequence

<400>  89
   atgccgaaag atccccacaa gttctatatt tgctcaaact gggaggccgt gcacaaagat      60
   tgttctgtta tcaagccaga gatgaaaatc aaactgcgca tggagggggc tgtccatgcg     120
   gtaaacattg tgggttactc aaatgcgcag ggagtggacc ataagtttgt catcgaaggc     180
   gagggcatcg gtaaacctta tgaaggcact cagactttgg atttaacggt cgaagaagga     240
   gcgcctttgc cattttcgta tgatatctta accccggcgt ttcaatacgg aaatcgcgcg     300
   ttcacaaagt atcccgaaga catccccgat tacttcaagc aagcttttcc agaaggctac     360
   agctgggagc gttcaatgac gtatggagtc cttgcatgtg ctatcgccac acatgctaaa     420
   atccgcgatg gtatttgtat tgccacgtca gatatcacaa tggaatacga tatcaagtac     480
   acttggaacg tgccaaaaat cgctcccaag tcagaaaacg tggtgtgttt cttctatgag     540
   attcgttttg atggtaccca agagtcttat tatcgttatg ttgcacgtga acaaagttgt     600
   cgtacgttga agtgggagcc ttctacagaa aaaatgtatg tggaggacgg cgtgcttaaa     660
   ggagacgtgg agatggcact gcttttgtgt catgggtccg aaccgtgtat tattcatcgc     720
   ggaaaaccct ttcaattgga agcggtccac taccgctgcg attttaagac cacatataaa     780
   gctggatgcg gttcctgctg ggcgttctcc ggtgtagctg ccactgaatc ggcttacttg     840
   gcgcacgaag tcgatcatcg tatcgagatt ttatcgcacg acaaggatta caacaaagtc     900
   cgcttatatg agcacgcaga agcacgctac tctggtggca gtggtgactt acgtcagatg     960
   cgtacagtga cacctattcg tatgcaagga ggatgtggta gctgtggagg atcaggcaac 1020
   accaagacgg cgaaaattga aattaaagca tccattgatg gacttgattc gacgcaccac 1080
   caccatcatc acaaaaagta g 1101

<210> 90
<211> 333
<212> PRT
<213> Artificial sequence

<200>
<223> Artificial sequence

<400> 90

Met Gly Ala His Ala Ser Val Ile Lys Phe Ala Glu Leu Leu Glu Gln
1               5                   10                  15

Gln Lys Asn Ala Gln Phe Pro Gly Lys Pro Glu Met Lys Ile Lys Leu
            20                  25                  30

Arg Met Glu Gly Ala Val Asn Gly His Lys Phe Val Ile Glu Gly Glu
        35                  40                  45

Gly Ile Gly Lys Pro Tyr Glu Gly Thr Gln Thr Leu Asp Leu Thr Val
    50                  55                  60

Glu Glu Asp Ser Ser Ala His Ser Thr Pro Ser Thr Pro Ala Tyr Asp
65                  70                  75                  80

Ile Leu Thr Pro Ala Phe Gln Tyr Gly Asn Arg Ala Phe Thr Lys Tyr
                85                  90                  95

Pro Glu Asp Ile Pro Asp Tyr Phe Lys Gln Ala Phe Pro Glu Gly Tyr
            100                 105                 110

Ser Trp Glu Arg Ser Met Thr Tyr Glu Asp Gln Gly Ile Cys Ile Ala
        115                 120                 125

Thr Ser Asp Ile Thr Met Glu Gly Asp Lys Pro Ser Pro Phe Gly Gln
    130                 135                 140

Ala Ala Ala Ala Asp Lys Cys Phe Phe Tyr Glu Ile Arg Phe Asp Gly
145                 150                 155                 160

Thr Phe Gly Gln Ala Ala Ala Gly Asp Lys Pro Ser Thr Leu Lys Trp
                165                 170                 175

Glu Pro Ser Thr Glu Lys Met Tyr Val Glu Ala Glu Pro Lys Pro Ala
            180                 185                 190

Glu Pro Lys Ser Val Leu Lys Gly Asp Val Glu Met Ala Leu Leu Leu
        195                 200                 205

Thr Ser Ser Thr Pro Pro Ser Gly Thr Glu Asn Lys Pro Ala Thr Gly
    210                 215                 220

His Tyr Arg Cys Asp Phe Lys Thr Thr Tyr Lys Ala Gly Thr Ser Glu
225                 230                 235                 240

Glu Gly Ser Arg Gly Gly Ser Ser Met Pro Ser His Glu Val Asp His
                245                 250                 255

Arg Ile Glu Ile Leu Ser His Ser Pro Phe Gly Gln Ala Ala Ala Gly
            260                 265                 270

Asp Lys Lys Val Arg Leu Tyr Glu His Ala Glu Ala Arg Tyr Ser Gly
        275                 280                 285

Gly Gly Ser Gly Lys Ala Ala Ile Ala Pro Ala Gly Gly Ala Ser Gly
    290                 295                 300

Lys Gln Arg Ala Ala Glu Ala Thr Lys Pro Ile Pro Asn Pro Leu Leu
305                 310                 315                 320

Gly Leu Asp Ser Thr His His His His His His Lys Lys
                325                 330

<210>  91
<211>  1002
<212>  PRT
<213>  Artificial sequence

<200>
<223> Artificial sequence


<400>  91

Ala Thr Gly Gly Gly Thr Gly Cys Gly Cys Ala Cys Gly Cys Ala Ala
1               5                   10                  15

Gly Cys Gly Thr Gly Ala Thr Thr Ala Ala Gly Thr Thr Thr Gly Cys
            20                  25                  30

Gly Gly Ala Ala Cys Thr Thr Thr Thr Ala Gly Ala Ala Cys Ala Gly
        35                  40                  45

Cys Ala Gly Ala Ala Ala Ala Ala Cys Gly Cys Ala Cys Ala Gly Thr
    50                  55                  60

Thr Cys Cys Cys Gly Gly Gly Gly Ala Ala Ala Cys Cys Cys Gly Ala
65                  70                  75                  80

Ala Ala Thr Gly Ala Ala Ala Ala Thr Cys Ala Ala Ala Cys Thr Thr
                85                  90                  95

Cys Gly Cys Ala Thr Gly Gly Ala Gly Gly Gly Gly Gly Cys Gly Gly
            100                 105                 110

Thr Ala Ala Ala Cys Gly Gly Cys Cys Ala Cys Ala Ala Gly Thr Thr
        115                 120                 125

Thr Gly Thr Thr Ala Thr Cys Gly Ala Gly Gly Gly Gly Gly Ala Ala
    130                 135                 140

Gly Gly Thr Ala Thr Cys Gly Gly Ala Ala Ala Gly Cys Cys Thr Thr
145                 150                 155                 160

Ala Cys Gly Ala Gly Gly Gly Ala Ala Cys Gly Cys Ala Gly Ala Cys
                165                 170                 175

Thr Thr Thr Gly Gly Ala Cys Thr Thr Ala Ala Cys Ala Gly Thr Ala
            180                 185                 190

Gly Ala Gly Gly Ala Ala Gly Ala Cys Thr Cys Ala Thr Cys Thr Gly
        195                 200                 205

Cys Ala Cys Ala Thr Thr Cys Ala Ala Cys Cys Cys Cys Gly Thr Cys
    210                 215                 220

Thr Ala Cys Thr Cys Cys Gly Gly Cys Ala Thr Ala Thr Gly Ala Thr
225                 230                 235                 240

Ala Thr Thr Thr Thr Ala Ala Cys Thr Cys Cys Thr Gly Cys Gly Thr
                245                 250                 255

Thr Thr Cys Ala Ala Thr Ala Thr Gly Gly Gly Ala Ala Cys Cys Gly
            260                 265                 270

Thr Gly Cys Ala Thr Thr Thr Ala Cys Thr Ala Ala Ala Thr Ala Cys
        275                 280                 285

Cys Cys Ala Gly Ala Gly Gly Ala Cys Ala Thr Cys Cys Cys Thr Gly
    290                 295                 300

Ala Thr Thr Ala Thr Thr Thr Cys Ala Ala Ala Cys Ala Gly Gly Cys
305                 310                 315                 320

Thr Thr Thr Thr Cys Cys Gly Gly Ala Ala Gly Gly Thr Thr Ala Cys
                325                 330                 335

Thr Cys Ala Thr Gly Gly Gly Ala Gly Cys Gly Cys Thr Cys Thr Ala
            340                 345                 350

Thr Gly Ala Cys Ala Thr Ala Thr Gly Ala Ala Gly Ala Thr Cys Ala
        355                 360                 365

Ala Gly Gly Ala Ala Thr Cys Thr Gly Thr Ala Thr Thr Gly Cys Cys
    370                 375                 380

Ala Cys Thr Thr Cys Gly Gly Ala Cys Ala Thr Cys Ala Cys Cys Ala
385                 390                 395                 400

Thr Gly Gly Ala Gly Gly Gly Ala Gly Ala Cys Ala Ala Gly Cys Cys
                405                 410                 415

Gly Ala Gly Cys Cys Cys Ala Thr Thr Thr Gly Gly Thr Cys Ala Ala
            420                 425                 430

Gly Cys Ala Gly Cys Thr Gly Cys Cys Gly Cys Ala Gly Ala Thr Ala
        435                 440                 445

Ala Ala Thr Gly Thr Thr Thr Thr Thr Thr Cys Thr Ala Thr Gly Ala
    450                 455                 460

Ala Ala Thr Thr Cys Gly Thr Thr Thr Cys Gly Ala Cys Gly Gly Gly
465                 470                 475                 480

Ala Cys Ala Thr Thr Thr Gly Gly Ala Cys Ala Ala Gly Cys Gly Gly
                485                 490                 495

Cys Thr Gly Cys Ala Gly Gly Cys Gly Ala Cys Ala Ala Gly Cys Cys
            500                 505                 510

Thr Ala Gly Cys Ala Cys Gly Thr Thr Gly Ala Ala Gly Thr Gly Gly
        515                 520                 525

Gly Ala Gly Cys Cys Ala Ala Gly Cys Ala Cys Cys Gly Ala Ala Ala
    530                 535                 540

Ala Gly Ala Thr Gly Thr Ala Cys Gly Thr Thr Gly Ala Gly Gly Cys
545                 550                 555                 560

Thr Gly Ala Ala Cys Cys Gly Ala Ala Gly Cys Cys Ala Gly Cys Gly

565                 570                 575

Gly Ala Gly Cys Cys Gly Ala Ala Ala Thr Cys Ala Gly Thr Cys Thr
            580                 585                 590

Thr Ala Ala Ala Gly Gly Gly Gly Gly Ala Thr Gly Thr Thr Gly Ala
        595                 600                 605

Ala Ala Thr Gly Gly Cys Thr Thr Thr Gly Cys Thr Thr Cys Thr Gly
    610                 615                 620

Ala Cys Gly Ala Gly Cys Ala Gly Cys Ala Cys Gly Cys Cys Ala Cys
625                 630                 635                 640

Cys Ala Ala Gly Thr Gly Gly Cys Ala Cys Ala Gly Ala Ala Ala Ala
                645                 650                 655

Cys Ala Ala Ala Cys Cys Cys Gly Cys Cys Ala Cys Ala Gly Gly Ala
            660                 665                 670

Cys Ala Thr Thr Ala Thr Cys Gly Cys Thr Gly Cys Gly Ala Thr Thr
        675                 680                 685

Thr Thr Ala Ala Gly Ala Cys Thr Ala Cys Ala Thr Ala Thr Ala Ala
    690                 695                 700

Gly Gly Cys Thr Gly Gly Thr Ala Cys Cys Thr Cys Thr Gly Ala Gly
705                 710                 715                 720

Gly Ala Gly Gly Gly Gly Thr Cys Thr Cys Gly Cys Gly Gly Ala Gly
                725                 730                 735

Gly Thr Ala Gly Thr Ala Gly Cys Ala Thr Gly Cys Cys Gly Thr Cys
            740                 745                 750

Ala Cys Ala Cys Gly Ala Gly Gly Thr Thr Gly Ala Cys Cys Ala Cys
        755                 760                 765

Cys Gly Cys Ala Thr Thr Gly Ala Gly Ala Thr Cys Thr Thr Ala Thr
    770                 775                 780

Cys Thr Cys Ala Cys Thr Cys Cys Cys Cys Thr Thr Thr Thr Gly Gly
785                 790                 795                 800

Thr Cys Ala Gly Gly Cys Thr Gly Cys Ala Gly Cys Thr Gly Gly Gly
                805                 810                 815

Gly Ala Thr Ala Ala Gly Ala Ala Gly Gly Thr Gly Cys Gly Thr Cys
                820                 825                 830

Thr Thr Thr Ala Thr Gly Ala Gly Cys Ala Cys Gly Cys Gly Gly Ala
            835                 840                 845

Gly Gly Cys Cys Cys Gly Thr Thr Ala Cys Thr Cys Thr Gly Gly Thr
    850                 855                 860

Gly Gly Ala Gly Gly Cys Ala Gly Thr Gly Gly Gly Ala Ala Ala Gly
865                 870                 875                 880

Cys Gly Gly Cys Ala Ala Thr Thr Gly Cys Cys Cys Cys Cys Gly Cys
                885                 890                 895

Ala Gly Gly Cys Gly Gly Cys Gly Cys Gly Thr Cys Ala Gly Gly Gly
            900                 905                 910

Ala Ala Ala Cys Ala Ala Cys Gly Cys Gly Cys Cys Gly Cys Thr Gly
        915                 920                 925

Ala Gly Gly Cys Gly Ala Cys Gly Ala Ala Ala Cys Cys Gly Ala Thr
    930                 935                 940

Cys Cys Cys Gly Ala Ala Cys Cys Cys Gly Cys Thr Gly Thr Thr Gly
945                 950                 955                 960

Gly Gly Ala Cys Thr Thr Gly Ala Cys Ala Gly Thr Ala Cys Cys Cys
                965                 970                 975

Ala Cys Cys Ala Thr Cys Ala Thr Cys Ala Cys Cys Ala Cys Cys Ala
            980                 985                 990

Cys Ala Ala Gly Ala Ala Ala Thr  Ala Gly
        995                 1000

<210>  92
<211>  12
<212>  PRT
<213>  Artificial sequence

<200>
<223> Artificial sequence


<400>  92

Asp Ser Ser Ala His Ser Thr Pro Ser Thr Pro Ala
1               5                   10

<210>  93
<211>  11

<212>  PRT
<213>  Artificial sequence

<200>
<223> Artificial sequence


<400>  93

Phe Gly Gln Ala Ala Ala Gly Asp Lys Pro Ser
1               5                   10

<210>  94
<211>  16
<212>  PRT
<213>  Artificial sequence

<200>
<223> Artificial sequence


<400>  94

Thr Ser Ser Thr Pro Pro Ser Gly Thr Glu Asn Lys Pro Ala Thr Gly
1               5                   10                  15

<210>  95
<211>  6
<212>  PRT
<213>  Artificial sequence

<200>
<223> Artificial sequence


<400>  95

Ser Tyr Trp Lys Gly Ser
1               5

<210>  96
<211>  8
<212>  PRT
<213>  Artificial sequence

<200>
<223> Artificial sequence


<400>  96

Glu Ala Ala Lys Glu Ala Ala Lys
1               5

<210>  97
<211>  7
<212>  PRT
<213>  Artificial sequence

<200>
<223> Artificial sequence

<400>  97

Thr Ser Tyr Trp Lys Gly Ser
1               5

<210>  98
<211>  4
<212>  PRT
<213>  Artificial sequence

<200>
<223> Artificial sequence


<400>  98

Gly Gly Ser Gly
1

<210>  99
<211>  5
<212>  PRT
<213>  Artificial sequence

<200>
<223> Artificial sequence


<400>  99

Gly Gly Ala Ser Gly
1               5

<210>  100
<211>  15
<212>  PRT
<213>  Artificial sequence

<200>
<223> Artificial sequence


<400>  100

Tyr Phe Pro Leu Gln Ser Tyr Gly Phe Gln Pro Thr Asn Gly Val
1               5                   10                  15

<210>  101
<211>  15
<212>  PRT
<213>  Artificial sequence

<200>
<223> Artificial sequence


<400>  101

Arg Ser Tyr Thr Pro Gly Asp Ser Ser Ser Gly Trp Thr Ala Gly
1               5                   10                  15

<210>  102
<211>  15

<212>  PRT
<213>  Artificial sequence

<200>
<223> Artificial sequence



<400>  102

Gly Lys Thr Phe Pro Pro Thr Glu Pro Lys Lys Asp Lys Lys Gly
1               5                   10                  15

<210>  103
<211>  15
<212>  PRT
<213>  Artificial sequence

<200>
<223> Artificial sequence



<400>  103

Met Tyr Ser Phe Val Ser Glu Glu Thr Gly Thr Leu Ile Val Asn
1               5                   10                  15

<210>  104
<211>  15
<212>  PRT
<213>  Artificial sequence

<200>
<223> Artificial sequence



<400>  104

Pro Leu Gln Ser Tyr Gly Phe Gln Pro Thr Asn Gly Val Gly Tyr
1               5                   10                  15

<210>  105
<211>  15
<212>  PRT
<213>  Artificial sequence

<200>
<223> Artificial sequence



<400>  105

Gly Gly Met Lys Asp Leu Ser Pro Arg Trp Tyr Phe Gly Gly Gly
1               5                   10                  15

<210>  106
<211>  15
<212>  PRT
<213>  Artificial sequence

<200>
<223> Artificial sequence

<400>  106

Gly Ser Lys Ser Pro Ile Gln Tyr Ile Asp Gly Gly Gly Gly Gly
1               5                   10                  15

<210>  107
<211>  15
<212>  PRT
<213>  Artificial sequence

<200>
<223> Artificial sequence


<400>  107

Tyr Ile Arg Gly Ala Arg Lys Ser Ala Pro Leu Ile Glu Leu Gly
1               5                   10                  15

<210>  108
<211>  1041
<212>  DNA
<213>  Artificial sequence

<200>
<223> Artificial sequence


<400>  108
atggttaatt atctcggagc acacgctttc gagcgcgaca tctcaacgga aatttatcaa        60

gccggttcaa ccatgaagat caagctccgt atggaagggg cggtcaacgg tcacaaattc       120

gtcatcgaag gggaaggcat tggcaagcca tacgaaggga cacagacttt ggacctcact       180

gtggaagaag gttcatctgg tgaagcggcg aaggaagcgg ccaagggaag cacaccatgc       240

aacggcgtgg aaggtttcaa ttgctacttt tacgacatcc ttaccccggc gttccaatat       300

ggtaaccgtg ccttcacgaa gtacccagaa gatatccctg attactttaa gcaggcattt       360

cctgaaggtt attcgtggga gcgcagtatg acctatgagg accaaggtat ttgtatcgcg       420

acgagcgaca ttaccatgga aggggactgt ttcttctacg agattcgctt cgatggaact       480

aattcgaata acctggacag taaagttggt ggcaactaca actatacctt aaaatgggag       540

ccaagtacag aaaagatgta cgttgaagac ggggtgttga agggtgacgt ggaaatggca       600

ttactcttgt ttgagcgcga catttcaacc gagatttacc aagccgggtc gacaggcggg       660

tccggaacaa gctactggaa ggggagtcac tatcgctgtg attttaagac gacctacaaa       720

gctggtagca ctccatgtaa cggagtagag gggttcaact gctactttca tgaagtagat       780

caccgtattg agattttatc acactacttt cctctgcaat cgtatggctt ccaacccacg       840

aatggcgttg gtagtagcgg cgaagccgcc aaggaagcag ccaagaaggt gcgcctgtac       900

gagcacgccg aggcgtactt tcctctccag tcctatggat tccaacctac caatggagtt       960

ggcggctcgg gcggcggtgc ttccgggaat tcgaacaact tagatagcaa ggtgggtggg      1020

aactataatt atctcgagta g                                              1041

<210>  109
<211>  1059
<212>  DNA
<213>  Artificial sequence

<200>
<223> Artificial sequence


<400>  109
atggttaatt atctcggagc acacgctttc gagcgcgaca tctcaacgga aatttatcaa      60

gccggttcaa ccatgaagat caagctccgt atggaagggg cggtcaacgg tcacaaattc     120

gtcatcgaag gggaaggcat tggcaagcca tacgaaggga cacagacttt ggacctcact     180

gtggaagaag gttcatctgg tgaagcggcg aaggaagcgg ccaagggaag cacaccatgc     240

aacggcgtgg aaggtttcaa ttgctacttt tacgacatcc ttaccccggc gttccaatat     300

ggtaaccgtg ccttcacgaa gtacccagaa gatatccctg attactttaa gcaggcattt     360

cctgaaggtt attcgtggga gcgcagtatg acctatgagg accaaggtat ttgtatcgcg     420

acgagcgaca ttaccatgga aggggactgt ttcttctacg agattcgctt cgatggaact     480

aattcgaata acctggacag taaagttggt ggcaactaca actatacctt aaaatgggag     540

ccaagtacag aaaagatgta cgttgaagac ggggtgttga agggtgacgt ggaaatggca     600

ttactcttgt ttgagcgcga catttcaacc gagatttacc aagccgggtc gacaggcggg     660

tccggaacaa gctactggaa ggggagtcac tatcgctgtg attttaagac gacctacaaa     720

gctggtagca ctccatgtaa cggagtagag gggttcaact gctactttca tgaagtagat     780

caccgtattg agattttatc acactacttt cctctgcaat cgtatggctt ccaacccacg     840

aatggcgttg gtagtagcgg cgaagccgcc aaggaagcag ccaagaaggt gcgcctgtac     900

gagcacgccg aggcgtactt tcctctccag tcctatggat tccaacctac caatggagtt     960

ggcggctcgg gcggcggtgc ttccgggaat tcgaacaact tagatagcaa ggtgggtggg    1020

aactataatt atctcgagca ccaccaccac caccactga                           1059

<210>  110
<211>  1128
<212>  DNA
<213>  Artificial sequence

<200>
<223> Artificial sequence


<400>  110
atggtaaatt atatagggtc aagtggagtt gtcaacccgg tgatgggtgg ctcgggtggt      60

ccagcgccag cgccgatgaa aattaagttg cgtatggaag gggcggttaa tgctccgcgt     120

atcaccttcg gcggtcctag cgatagcacc ggcggtggtt ctggcgaagc cgcgaagacg     180

agctactgga agggttcgca taaattcgtg attgagggtg agggcattgg taaaccgtat 240

gagggtacgc agaccttaga tctgaccgtt gaggaaggtt ctagcggcgt cgttaacccg 300

gttatgtatg atattttgac cccggcgttc caatatggaa accgcgcatt caccaaatac 360

ccggaatata ttcgtgttgg tgcccgtaaa agcgctccgc tgatcgagct gggttattcc 420

tgggagcgct ccatgaccta cggctccctg atcgacctgc aagagttggg taagtacgag 480

caatacatcg aggcagccgc taaagaagcc gcagcgaagg gcggcgcttc tggcatctgc 540

attgcgacca gcgacatcac catggagggc gactgttttt tctatgaaat ccgcttcgat 600

ggcaccggtc cgtttcagca gtttggacgt gatatcgcgg ataccacgga tgcgaccctg 660

aagtgggagc cgtccactga aaaaatgtac gtcgaggacg gtgtgctgaa gggtgacgtt 720

gaaatggcgc tgctccttgg tggctccggc acgtcatact ggaaaggttc tatgtggctg 780

tcttacttca tcgccagctt tcgtctgcat tatagatgcg attttaaaac tacgtacaaa 840

gcacgcagct acaccccggg cgactccagc agcggctgga ccgcacatga agtggatcat 900

cgtattgaga tcttaagtca cattgtggac gaaccgggcg gtgcgagcgg caaggtgcgt 960

ctgtatgagc acgcggaagc gccggcgccg gctccgggtt ttagcgcatt ggaaccgctg 1020

gtagacctgc cgggcggcag cggtggtggt gcgagcggga agaccttccc gcctacagaa 1080

ccgaaaaagg acaaaaaggg tcatcaccac caccaccaca agaagtaa 1128

<210> 111
<211> 1074
<212> DNA
<213> Artificial sequence

<200>
<223> Artificial sequence

<400> 111
atggtaaatt acatactagg agtttatcac aagaacaaca aaagctggat ggagagcgaa 60

ttccgcgttt atccggcacc ggcgcctatg aaaatcaagc tgcgtatgga aggtgcagtt 120

aatggtcata aattcgtgat tgagggtgaa ggcattggca aaccaggtgg ttcaggtgag 180

gctgcgaagt tcaactgcta ttttccgctg cagagctacg gctttcagcc gacgggtacg 240

cagaccctgg atctgacggt cgaggaaccg ttgcaatcgt acggtttcca accgacctat 300

gacatcctga ccccggcgtt ccagtatggc aaccgcgcgt tcaccaaata cccggaagcc 360

ggtaacggtg gcgatgccgc cctcgctctg ttactgttag atggctacag ctgggaacgt 420

agcatgacgt acggccgttc ctacctgacc ccgggtgaca gcagctccgg tggcgcgtcg 480

ggtggtatct gcattgcgac cagtgatatt acgatggagg gtgactgttt tttttatgag 540

atccgctttg atggtaccgc ggaccagttg accccaactt ggcgtgtgac ccttaagtgg 600

gagccgagca ccgaaaaaat gtatgtagag gatggggtgc tgaagggcga cgttgaaatg 660

gcattgttgt tgtttatcta taataaaatc gtggacgaac cgggtggcag cggtacttct 720

tactggaaag gttcccatta tcgttgcgac ttcaaaacca cctacaaagc aaagaacccg 780

ttgctatacg atgcgaatta ccatgaagtt gaccatcgta ttgaaattct gagccacggc 840

ggctctggag gcgaggctgc taagggtaga cctcaaggtc tgccgaataa caccgcatcc 900

aaagttcgtc tgtacgagca cgcggaggcg ctggcggaga tcctgcaaaa aaacctgatc 960

cgccagggta cagattataa gcattggccg caaattgcgg gcggctccgg tggcggcaag 1020

atcgccgact acaattacaa gctgggccat caccaccacc accacaagaa gtaa 1074

<210> 112
<211> 1119
<212> DNA
<213> Artificial sequence

<200>
<223> Artificial sequence

<400> 112
atggtaaatt atatatttaa ctgttacttc ccgctgcaga gctacggctt ccagccgacc 60

aatggtgtga tgaaaatcaa actgcgtatg gaaggcgcag ttcgtccgca gggtctgccg 120

aataacaccg caagcggcgg ttccggtggc gaggcggcga agcataagtt cgtgattgaa 180

ggtgagggta ttggtaagcc ggaagctgcg aaagaagctg ctaagggctc tggcttcatc 240

tataacaaaa tcgttgatga gccgggtgcg ggtacgcaga ccctggatct gactgtagag 300

gaagcggacc aactgacccc gacctggcgt gtgggctatg acatcttgac tccggcgttt 360

caatatggta atagagcatt caccaaatac ccggagggca agatcgccga ctacaactac 420

aagttgggtg gctacagctg ggaacgctct atgacctacg aagatcaagg tatttgtatt 480

gcgaccagcg atatcaccat ggagggtgac tgcttttttt atgaaatccg ctttgatggt 540

acatacattc gcgtgggtgc gcgtaaaagc gctccgctga ttgagctgac gctgaaatgg 600

gagccgagca ccgaaaaaat gtatgtcgag atcgtggacg aaccgggagg gtccggtggt 660

gttctgaagg gcgacgttga gatggcactg ttgttgaaga acccgttatt gtacgatgca 720

aattacgggg gttcgggcac ctcctattgg aaaggtcatt atcgctgcga tttcaaaacc 780

acctataaag cgaagacctt tccgccgacg gagcctaaaa aagataaaaa gcacgaagtt 840

gaccatcgta ttgagatcct gagccatgaa gcggctaagg gcggtgccag cggccgtagc 900

tacctcactc cgggtgacag ctctagcaaa gttcgtctgt atgagcacgc agaagccccg 960

gcgccagcgc caggctcgtc cggtgtcgtg aacccggtca tggagccgat ttacgacggc 1020

ggctccggcg gtgcgccagg ccagacggga aagatcgccg actataacta caagcttggt 1080

gcgtcaggca aacaccatca ccaccaccac aagaagtaa 1119

<210>  113
<211>  975
<212>  DNA
<213>  Artificial sequence

<200>
<223> Artificial sequence


<400>  113
atggtcaatt atatcgcgct cccacaacgc caaaagaagc agcagaccgt gacgctgttg      60

ccggcaccgg cacccatgaa gattaagtta cgtatggaag gagcggtcaa tgggcacaag     120

tttgtcatcg agggcgaagg aatcgggaaa ccttacgaag ggacccaaac attagatctg     180

accgttgaag agggaagcaa gagcccaatc caatacattg attacgatat tcttacgcct     240

gcatttcagt acggcaatcg tgctttcaca aagtaccctg aagacttctt ggagtatcac     300

gacgtgcgcg tagttttaga cttcgggtat tcttgggaac gtagtatgac gtatggtgga     360

gctagcggtg gtatcaatgc aagtgttgtc aacatccagg gaatctgcat cgccacctcg     420

gatatcacta tggaaggcgg gtccggggaa gctgcgaagc aattcgcccc ctcggctagt     480

gccttcttct gcttcttcta cgaaatccgt tttgacggta ccatgtatag cttcgtaagc     540

gaagaaacgg gcacgctgat tgtcaatact ctgaagtggg agccgtctac ggaaaagatg     600

tatgtagagg acggcgtact gaagggcgac gtcgagatgg cgcttctgtt agaaggcggc     660

ggtcattacc gttgcgattt taagacaacg tataaggcac catccggtac ttggttaact     720

tacacaggcc acgaggtcga ccatcgtatc gagatcttat cacatggcgg ttccggcggc     780

gaagcggcca aggggcagtt cgcgcctagt gcatcggctt tcttcaaggt tcgcctctat     840

gaacacgccg aggcaggcgg tgcatctgga gagctggaca aatatggcgg ttcgggcggc     900

ggtatgtaca gtttcgtatc ggaagagaca ggaactttaa ttgtgaatca ccatcaccac     960

caccataaga agtaa                                                      975

<210>  114
<211>  996
<212>  DNA
<213>  Artificial sequence

<200>
<223> Artificial sequence


<400>  114
atggtgaatt acatcccgct ccaatcatac ggatttcagc caacgaatgg ggtcgggtac      60

cctgcgccag cacccgcaat gaaaatcaag cttcgtatgg aaggtgcagt taatggacac     120

aagttcgtga tcgagggcga gggcattggc aagccctatg aaggacccaa aactttggac     180

ttgacggtag aagagggtat ctatcagacc agtaattttc gtgtctacga catcttgact      240

ccggctttcc aatacgggaa ccgcgcgttc actaagtacc ctgagaaggc gtacaatgtg 300

acccaagcat tcgggcgtcg tggccctgaa ggctatagct gggaacgttc tatgacgtac 360

gaggatcaag gtatctgtat tgcgactagc gatattacaa tggaaggcac caatacatct 420

aatcaagtgg cggtgggtgg aagcggtgag gcagcaaagt gcttctttta tgagatccgc 480

ttcgatggta ctaacccggt cctgcccttt aacgatggag tttatttcgc ctcaaccaca 540

ttgaaatggg agccctcaac tgaaaagatg tatgttgaag acggcgtttt gaagggtgac 600

gtagagatgg cacttctgct gtacaactac aagcttcccg atgacttcac tggcggcagc 660

ggtacaagtt attggaaggg ttcacattat cgctgtgatt tcaagacaac ctataaagca 720

atgttccatc tggtagactt tcaagttacg attgctgaga tccttcatga ggttgatcac 780

cgtatcgaaa tcctttctca cggcggaagc ggcggagagg cggccaaggg tatgaaggac 840

ttgagccctc gctggtattt caaggtccgc ttatacgagc acgccgaggc cgacgctgcc 900

cttgcgctcc tgttattaga cggcggaagc ggtggtggca tgaaggattt atcccctcgc 960

tggtacttcc atcaccatca ccaccacaag aagtga 996

<210> 115
<211> 1059
<212> DNA
<213> Artificial sequence

<200>
<223> Artificial sequence

<400> 115
atggtgaatt atatcttagg ggtgtaccac aagaataaca agtcatggat ggagtcggaa 60

tttcgtgtgt acccggcccc ggctcccatg aagatcaagt tacgcatgga aggcgccgtt 120

aacggccaca aattcgtgat cgagggcgaa ggaatcggta agccaggtgg ctcgggtgaa 180

gccgccaagt tcatttacaa taagatcgtt gatgaaccag gaacacaaac tttagatctt 240

accgtagagg agaagaaccc gcttctttac gacgccaatt actatgacat tttaacgccg 300

gccttccaat acggcaatcg cgcttttacg aagtatccgg aagcgggtaa cggcggcgat 360

gcagccttag cattattatt actggacggt tattcgtggg agcgttcaat gacgtatggc 420

cgtagttact tgacccccgg agacagtagc tctggtggtg ccagcggcgg gatctgtatt 480

gcaactagtg atatcaccat ggaaggcgac tgcttcttct atgagattcg tttcgatggg 540

accgccgatc aactgacccc tacctggcgt gtaacattga agtgggagcc aagcactgag 600

aagatgtacg tggaagacgg cgttctgaag ggcgacgtgg agatggcctt gttattattc 660

atctacaata agatcgtgga cgagccgggt ggtagcggta catcctactg gaagggttcg 720

cattaccgtt gtgatttcaa gacaacctat aaggctaaga atccgttatt gtatgacgca 780

aattaccatg aggtggacca ccgtatcgaa atcctttccc atggcggaag cggtggtgag 840

gcggcgaagg gtcgcccgca gggactgccg aataatacgg cgtctaaggt tcgcttatac 900

gaacacgctg aggccctcgc cgagatcctg caaaagaact taatccgcca aggaaccgac 960

tataagcact ggccgcaaat cgccggtggc tccggtggtg gtaagatcgc agactacaac 1020

tataagctcg gtcatcatca tcatcaccat aagaaataa 1059

<210> 116
<211> 346
<212> PRT
<213> Artificial sequence

<200>
<223> Artificial sequence

<400> 116

Met Val Asn Tyr Leu Gly Ala His Ala Phe Glu Arg Asp Ile Ser Thr
1               5                   10                  15

Glu Ile Tyr Gln Ala Gly Ser Thr Met Lys Ile Lys Leu Arg Met Glu
            20                  25                  30

Gly Ala Val Asn Gly His Lys Phe Val Ile Glu Gly Glu Gly Ile Gly
        35                  40                  45

Lys Pro Tyr Glu Gly Thr Gln Thr Leu Asp Leu Thr Val Glu Glu Gly
    50                  55                  60

Ser Ser Gly Glu Ala Ala Lys Glu Ala Ala Lys Gly Ser Thr Pro Cys
65                  70                  75                  80

Asn Gly Val Glu Gly Phe Asn Cys Tyr Phe Tyr Asp Ile Leu Thr Pro
                85                  90                  95

Ala Phe Gln Tyr Gly Asn Arg Ala Phe Thr Lys Tyr Pro Glu Asp Ile
            100                 105                 110

Pro Asp Tyr Phe Lys Gln Ala Phe Pro Glu Gly Tyr Ser Trp Glu Arg
        115                 120                 125

Ser Met Thr Tyr Glu Asp Gln Gly Ile Cys Ile Ala Thr Ser Asp Ile
    130                 135                 140

Thr Met Glu Gly Asp Cys Phe Phe Tyr Glu Ile Arg Phe Asp Gly Thr
145                 150                 155                 160

Asn Ser Asn Asn Leu Asp Ser Lys Val Gly Gly Asn Tyr Asn Tyr Thr
                165                 170                 175

Leu Lys Trp Glu Pro Ser Thr Glu Lys Met Tyr Val Glu Asp Gly Val
            180                 185                 190

Leu Lys Gly Asp Val Glu Met Ala Leu Leu Leu Phe Glu Arg Asp Ile
        195                 200                 205

Ser Thr Glu Ile Tyr Gln Ala Gly Ser Thr Gly Gly Ser Gly Thr Ser
    210                 215                 220

Tyr Trp Lys Gly Ser His Tyr Arg Cys Asp Phe Lys Thr Thr Tyr Lys
225                 230                 235                 240

Ala Gly Ser Thr Pro Cys Asn Gly Val Glu Gly Phe Asn Cys Tyr Phe
                245                 250                 255

His Glu Val Asp His Arg Ile Glu Ile Leu Ser His Tyr Phe Pro Leu
            260                 265                 270

Gln Ser Tyr Gly Phe Gln Pro Thr Asn Gly Val Gly Ser Ser Gly Glu
        275                 280                 285

Ala Ala Lys Glu Ala Ala Lys Lys Val Arg Leu Tyr Glu His Ala Glu
    290                 295                 300

Ala Tyr Phe Pro Leu Gln Ser Tyr Gly Phe Gln Pro Thr Asn Gly Val
305                 310                 315                 320

Gly Gly Ser Gly Gly Gly Ala Ser Gly Asn Ser Asn Asn Leu Asp Ser
                325                 330                 335

Lys Val Gly Gly Asn Tyr Asn Tyr Leu Glu
            340                 345

<210> 117
<211> 352
<212> PRT
<213> Artificial sequence

<200>
<223> Artificial sequence

<400> 117

Met Val Asn Tyr Leu Gly Ala His Ala Phe Glu Arg Asp Ile Ser Thr
1               5                   10                  15

Glu Ile Tyr Gln Ala Gly Ser Thr Met Lys Ile Lys Leu Arg Met Glu
            20                  25                  30

Gly Ala Val Asn Gly His Lys Phe Val Ile Glu Gly Glu Gly Ile Gly
        35                  40                  45

Lys Pro Tyr Glu Gly Thr Gln Thr Leu Asp Leu Thr Val Glu Glu Gly
    50                  55                  60

Ser Ser Gly Glu Ala Ala Lys Glu Ala Ala Lys Gly Ser Thr Pro Cys
65                  70                  75                  80

Asn Gly Val Glu Gly Phe Asn Cys Tyr Phe Tyr Asp Ile Leu Thr Pro
                85                  90                  95

Ala Phe Gln Tyr Gly Asn Arg Ala Phe Thr Lys Tyr Pro Glu Asp Ile
            100                 105                 110

Pro Asp Tyr Phe Lys Gln Ala Phe Pro Glu Gly Tyr Ser Trp Glu Arg
        115                 120                 125

Ser Met Thr Tyr Glu Asp Gln Gly Ile Cys Ile Ala Thr Ser Asp Ile
    130                 135                 140

Thr Met Glu Gly Asp Cys Phe Phe Tyr Glu Ile Arg Phe Asp Gly Thr
145                 150                 155                 160

Asn Ser Asn Asn Leu Asp Ser Lys Val Gly Gly Asn Tyr Asn Tyr Thr
                165                 170                 175

Leu Lys Trp Glu Pro Ser Thr Glu Lys Met Tyr Val Glu Asp Gly Val
            180                 185                 190

Leu Lys Gly Asp Val Glu Met Ala Leu Leu Leu Phe Glu Arg Asp Ile
        195                 200                 205

Ser Thr Glu Ile Tyr Gln Ala Gly Ser Thr Gly Gly Ser Gly Thr Ser
    210                 215                 220

Tyr Trp Lys Gly Ser His Tyr Arg Cys Asp Phe Lys Thr Thr Tyr Lys
225                 230                 235                 240

Ala Gly Ser Thr Pro Cys Asn Gly Val Glu Gly Phe Asn Cys Tyr Phe
                245                 250                 255

His Glu Val Asp His Arg Ile Glu Ile Leu Ser His Tyr Phe Pro Leu
            260                 265                 270

Gln Ser Tyr Gly Phe Gln Pro Thr Asn Gly Val Gly Ser Ser Gly Glu
        275                 280                 285

Ala Ala Lys Glu Ala Ala Lys Lys Val Arg Leu Tyr Glu His Ala Glu
    290                 295                 300

Ala Tyr Phe Pro Leu Gln Ser Tyr Gly Phe Gln Pro Thr Asn Gly Val
305                 310                 315                 320

Gly Gly Ser Gly Gly Gly Ala Ser Gly Asn Ser Asn Asn Leu Asp Ser
                325                 330                 335

Lys Val Gly Gly Asn Tyr Asn Tyr Leu Glu His His His His His His
            340                 345                 350

<210>  118
<211>  375
<212>  PRT
<213>  Artificial sequence

<200>
<223> Artificial sequence

<400>  118

Met Val Asn Tyr Ile Gly Ser Ser Gly Val Val Asn Pro Val Met Gly
1               5                   10                  15

Gly Ser Gly Gly Pro Ala Pro Ala Pro Met Lys Ile Lys Leu Arg Met
            20                  25                  30

Glu Gly Ala Val Asn Ala Pro Arg Ile Thr Phe Gly Gly Pro Ser Asp
        35                  40                  45

Ser Thr Gly Gly Gly Ser Gly Glu Ala Ala Lys Thr Ser Tyr Trp Lys
    50                  55                  60

Gly Ser His Lys Phe Val Ile Glu Gly Glu Gly Ile Gly Lys Pro Tyr
65                  70                  75                  80

Glu Gly Thr Gln Thr Leu Asp Leu Thr Val Glu Glu Gly Ser Ser Gly
                85                  90                  95

Val Val Asn Pro Val Met Tyr Asp Ile Leu Thr Pro Ala Phe Gln Tyr
            100                 105                 110

Gly Asn Arg Ala Phe Thr Lys Tyr Pro Glu Tyr Ile Arg Val Gly Ala
        115                 120                 125

Arg Lys Ser Ala Pro Leu Ile Glu Leu Gly Tyr Ser Trp Glu Arg Ser
    130                 135                 140

Met Thr Tyr Gly Ser Leu Ile Asp Leu Gln Glu Leu Gly Lys Tyr Glu
145                 150                 155                 160

Gln Tyr Ile Glu Ala Ala Ala Lys Glu Ala Ala Ala Lys Gly Gly Ala
                165                 170                 175

Ser Gly Ile Cys Ile Ala Thr Ser Asp Ile Thr Met Glu Gly Asp Cys
            180                 185                 190

Phe Phe Tyr Glu Ile Arg Phe Asp Gly Thr Gly Pro Phe Gln Gln Phe
        195                 200                 205

Gly Arg Asp Ile Ala Asp Thr Thr Asp Ala Thr Leu Lys Trp Glu Pro
    210                 215                 220

Ser Thr Glu Lys Met Tyr Val Glu Asp Gly Val Leu Lys Gly Asp Val
225                 230                 235                 240

Glu Met Ala Leu Leu Leu Gly Gly Ser Gly Thr Ser Tyr Trp Lys Gly
                245                 250                 255

Ser Met Trp Leu Ser Tyr Phe Ile Ala Ser Phe Arg Leu His Tyr Arg
            260                 265                 270

Cys Asp Phe Lys Thr Thr Tyr Lys Ala Arg Ser Tyr Thr Pro Gly Asp
        275                 280                 285

Ser Ser Ser Gly Trp Thr Ala His Glu Val Asp His Arg Ile Glu Ile
    290                 295                 300

Leu Ser His Ile Val Asp Glu Pro Gly Gly Ala Ser Gly Lys Val Arg
305                 310                 315                 320

Leu Tyr Glu His Ala Glu Ala Pro Ala Pro Ala Pro Gly Phe Ser Ala
                325                 330                 335

Leu Glu Pro Leu Val Asp Leu Pro Gly Gly Ser Gly Gly Gly Ala Ser
            340                 345                 350

Gly Lys Thr Phe Pro Pro Thr Glu Pro Lys Lys Asp Lys Lys Gly His
        355                 360                 365

His His His His His Lys Lys
    370                 375

<210>  119
<211>  357
<212>  PRT
<213>  Artificial sequence

<200>
<223> Artificial sequence

<400> 119

Met Val Asn Tyr Ile Leu Gly Val Tyr His Lys Asn Asn Lys Ser Trp
1               5                   10                  15

Met Glu Ser Glu Phe Arg Val Tyr Pro Ala Pro Ala Pro Met Lys Ile
            20                  25                  30

Lys Leu Arg Met Glu Gly Ala Val Asn Gly His Lys Phe Val Ile Glu
        35                  40                  45

Gly Glu Gly Ile Gly Lys Pro Gly Gly Ser Gly Glu Ala Ala Lys Phe
    50                  55                  60

Asn Cys Tyr Phe Pro Leu Gln Ser Tyr Gly Phe Gln Pro Thr Gly Thr
65                  70                  75                  80

Gln Thr Leu Asp Leu Thr Val Glu Glu Pro Leu Gln Ser Tyr Gly Phe
                85                  90                  95

Gln Pro Thr Tyr Asp Ile Leu Thr Pro Ala Phe Gln Tyr Gly Asn Arg
            100                 105                 110

Ala Phe Thr Lys Tyr Pro Glu Ala Gly Asn Gly Gly Asp Ala Ala Leu
        115                 120                 125

Ala Leu Leu Leu Leu Asp Gly Tyr Ser Trp Glu Arg Ser Met Thr Tyr
    130                 135                 140

Gly Arg Ser Tyr Leu Thr Pro Gly Asp Ser Ser Ser Gly Gly Ala Ser
145                 150                 155                 160

Gly Gly Ile Cys Ile Ala Thr Ser Asp Ile Thr Met Glu Gly Asp Cys
                165                 170                 175

Phe Phe Tyr Glu Ile Arg Phe Asp Gly Thr Ala Asp Gln Leu Thr Pro
            180                 185                 190

Thr Trp Arg Val Thr Leu Lys Trp Glu Pro Ser Thr Glu Lys Met Tyr
        195                 200                 205

Val Glu Asp Gly Val Leu Lys Gly Asp Val Glu Met Ala Leu Leu Leu
    210                 215                 220

Phe Ile Tyr Asn Lys Ile Val Asp Glu Pro Gly Gly Ser Gly Thr Ser
225                 230                 235                 240

Tyr Trp Lys Gly Ser His Tyr Arg Cys Asp Phe Lys Thr Thr Tyr Lys
                245                 250                 255

Ala Lys Asn Pro Leu Leu Tyr Asp Ala Asn Tyr His Glu Val Asp His
            260                 265                 270

Arg Ile Glu Ile Leu Ser His Gly Gly Ser Gly Gly Glu Ala Ala Lys
        275                 280                 285

Gly Arg Pro Gln Gly Leu Pro Asn Asn Thr Ala Ser Lys Val Arg Leu
    290                 295                 300

Tyr Glu His Ala Glu Ala Leu Ala Glu Ile Leu Gln Lys Asn Leu Ile
305                 310                 315                 320

Arg Gln Gly Thr Asp Tyr Lys His Trp Pro Gln Ile Ala Gly Gly Ser
                325                 330                 335

Gly Gly Gly Lys Ile Ala Asp Tyr Asn Tyr Lys Leu Gly His His His
            340                 345                 350

His His His Lys Lys
        355

<210>  120
<211>  372
<212>  PRT
<213>  Artificial sequence

<200>
<223> Artificial sequence

<400>  120

Met Val Asn Tyr Ile Phe Asn Cys Tyr Phe Pro Leu Gln Ser Tyr Gly
1               5                   10                  15

Phe Gln Pro Thr Asn Gly Val Met Lys Ile Lys Leu Arg Met Glu Gly
            20                  25                  30

Ala Val Arg Pro Gln Gly Leu Pro Asn Asn Thr Ala Ser Gly Gly Ser
        35                  40                  45

Gly Gly Glu Ala Ala Lys His Lys Phe Val Ile Glu Gly Glu Gly Ile
    50                  55                  60

Gly Lys Pro Glu Ala Ala Lys Glu Ala Ala Lys Gly Ser Gly Phe Ile

65                  70                  75                  80

Tyr Asn Lys Ile Val Asp Glu Pro Gly Ala Gly Thr Gln Thr Leu Asp
                85                  90                  95

Leu Thr Val Glu Glu Ala Asp Gln Leu Thr Pro Thr Trp Arg Val Gly
            100                 105                 110

Tyr Asp Ile Leu Thr Pro Ala Phe Gln Tyr Gly Asn Arg Ala Phe Thr
        115                 120                 125

Lys Tyr Pro Glu Gly Lys Ile Ala Asp Tyr Asn Tyr Lys Leu Gly Gly
    130                 135                 140

Tyr Ser Trp Glu Arg Ser Met Thr Tyr Glu Asp Gln Gly Ile Cys Ile
145                 150                 155                 160

Ala Thr Ser Asp Ile Thr Met Glu Gly Asp Cys Phe Phe Tyr Glu Ile
                165                 170                 175

Arg Phe Asp Gly Thr Tyr Ile Arg Val Gly Ala Arg Lys Ser Ala Pro
            180                 185                 190

Leu Ile Glu Leu Thr Leu Lys Trp Glu Pro Ser Thr Glu Lys Met Tyr
        195                 200                 205

Val Glu Ile Val Asp Glu Pro Gly Gly Ser Gly Gly Val Leu Lys Gly
    210                 215                 220

Asp Val Glu Met Ala Leu Leu Leu Lys Asn Pro Leu Leu Tyr Asp Ala
225                 230                 235                 240

Asn Tyr Gly Gly Ser Gly Thr Ser Tyr Trp Lys Gly His Tyr Arg Cys
                245                 250                 255

Asp Phe Lys Thr Thr Tyr Lys Ala Lys Thr Phe Pro Pro Thr Glu Pro
            260                 265                 270

Lys Lys Asp Lys Lys His Glu Val Asp His Arg Ile Glu Ile Leu Ser
        275                 280                 285

His Glu Ala Ala Lys Gly Gly Ala Ser Gly Arg Ser Tyr Leu Thr Pro
    290                 295                 300

Gly Asp Ser Ser Ser Lys Val Arg Leu Tyr Glu His Ala Glu Ala Pro
305                 310                 315                 320

Ala Pro Ala Pro Gly Ser Ser Gly Val Val Asn Pro Val Met Glu Pro
                325                 330                 335

Ile Tyr Asp Gly Gly Ser Gly Gly Ala Pro Gly Gln Thr Gly Lys Ile
            340                 345                 350

Ala Asp Tyr Asn Tyr Lys Leu Gly Ala Ser Gly Lys His His His His
        355                 360                 365

His His Lys Lys
    370

<210>  121
<211>  324
<212>  PRT
<213>  Artificial sequence

<200>
<223> Artificial sequence

<400>  121

Met Val Asn Tyr Ile Ala Leu Pro Gln Arg Gln Lys Lys Gln Gln Thr
1               5                   10                  15

Val Thr Leu Leu Pro Ala Pro Ala Pro Met Lys Ile Lys Leu Arg Met
            20                  25                  30

Glu Gly Ala Val Asn Gly His Lys Phe Val Ile Glu Gly Glu Gly Ile
        35                  40                  45

Gly Lys Pro Tyr Glu Gly Thr Gln Thr Leu Asp Leu Thr Val Glu Glu
    50                  55                  60

Gly Ser Lys Ser Pro Ile Gln Tyr Ile Asp Tyr Asp Ile Leu Thr Pro
65                  70                  75                  80

Ala Phe Gln Tyr Gly Asn Arg Ala Phe Thr Lys Tyr Pro Glu Asp Phe
                85                  90                  95

Leu Glu Tyr His Asp Val Arg Val Val Leu Asp Phe Gly Tyr Ser Trp
            100                 105                 110

Glu Arg Ser Met Thr Tyr Gly Gly Ala Ser Gly Gly Ile Asn Ala Ser
        115                 120                 125

Val Val Asn Ile Gln Gly Ile Cys Ile Ala Thr Ser Asp Ile Thr Met
    130                 135                 140

Glu Gly Gly Ser Gly Glu Ala Ala Lys Gln Phe Ala Pro Ser Ala Ser

145                 150                 155                 160

Ala Phe Phe Cys Phe Phe Tyr Glu Ile Arg Phe Asp Gly Thr Met Tyr
                165                 170                 175

Ser Phe Val Ser Glu Glu Thr Gly Thr Leu Ile Val Asn Thr Leu Lys
            180                 185                 190

Trp Glu Pro Ser Thr Glu Lys Met Tyr Val Glu Asp Gly Val Leu Lys
        195                 200                 205

Gly Asp Val Glu Met Ala Leu Leu Leu Glu Gly Gly Gly His Tyr Arg
    210                 215                 220

Cys Asp Phe Lys Thr Thr Tyr Lys Ala Pro Ser Gly Thr Trp Leu Thr
225                 230                 235                 240

Tyr Thr Gly His Glu Val Asp His Arg Ile Glu Ile Leu Ser His Gly
                245                 250                 255

Gly Ser Gly Gly Glu Ala Ala Lys Gly Gln Phe Ala Pro Ser Ala Ser
            260                 265                 270

Ala Phe Phe Lys Val Arg Leu Tyr Glu His Ala Glu Ala Gly Gly Ala
        275                 280                 285

Ser Gly Glu Leu Asp Lys Tyr Gly Gly Ser Gly Gly Gly Met Tyr Ser
    290                 295                 300

Phe Val Ser Glu Glu Thr Gly Thr Leu Ile Val Asn His His His His
305                 310                 315                 320

His His Lys Lys

<210>  122
<211>  331
<212>  PRT
<213>  Artificial sequence

<200>
<223> Artificial sequence


<400>  122

Met Val Asn Tyr Ile Pro Leu Gln Ser Tyr Gly Phe Gln Pro Thr Asn
1               5                   10                  15

Gly Val Gly Tyr Pro Ala Pro Ala Pro Ala Met Lys Ile Lys Leu Arg
            20                  25                  30

Met Glu Gly Ala Val Asn Gly His Lys Phe Val Ile Glu Gly Glu Gly
        35                  40                  45
Ile Gly Lys Pro Tyr Glu Gly Thr Gln Thr Leu Asp Leu Thr Val Glu
    50                  55                  60
Glu Gly Ile Tyr Gln Thr Ser Asn Phe Arg Val Tyr Asp Ile Leu Thr
65                  70                  75                  80
Pro Ala Phe Gln Tyr Gly Asn Arg Ala Phe Thr Lys Tyr Pro Glu Lys
                85                  90                  95
Ala Tyr Asn Val Thr Gln Ala Phe Gly Arg Arg Gly Pro Glu Gly Tyr
            100                 105                 110
Ser Trp Glu Arg Ser Met Thr Tyr Glu Asp Gln Gly Ile Cys Ile Ala
        115                 120                 125
Thr Ser Asp Ile Thr Met Glu Gly Thr Asn Thr Ser Asn Gln Val Ala
    130                 135                 140
Val Gly Gly Ser Gly Glu Ala Ala Lys Cys Phe Phe Tyr Glu Ile Arg
145                 150                 155                 160
Phe Asp Gly Thr Asn Pro Val Leu Pro Phe Asn Asp Gly Val Tyr Phe
                165                 170                 175
Ala Ser Thr Thr Leu Lys Trp Glu Pro Ser Thr Glu Lys Met Tyr Val
            180                 185                 190
Glu Asp Gly Val Leu Lys Gly Asp Val Glu Met Ala Leu Leu Leu Tyr
        195                 200                 205
Asn Tyr Lys Leu Pro Asp Asp Phe Thr Gly Gly Ser Gly Thr Ser Tyr
    210                 215                 220
Trp Lys Gly Ser His Tyr Arg Cys Asp Phe Lys Thr Thr Tyr Lys Ala
225                 230                 235                 240
Met Phe His Leu Val Asp Phe Gln Val Thr Ile Ala Glu Ile Leu His
                245                 250                 255
Glu Val Asp His Arg Ile Glu Ile Leu Ser His Gly Gly Ser Gly Gly
            260                 265                 270
Glu Ala Ala Lys Gly Met Lys Asp Leu Ser Pro Arg Trp Tyr Phe Lys
        275                 280                 285

Val Arg Leu Tyr Glu His Ala Glu Ala Asp Ala Ala Leu Ala Leu Leu
    290                 295                 300

Leu Leu Asp Gly Gly Ser Gly Gly Gly Met Lys Asp Leu Ser Pro Arg
305                 310                 315                 320

Trp Tyr Phe His His His His His His Lys Lys
                325                 330

<210>  123
<211>  352
<212>  PRT
<213>  Artificial sequence

<200>
<223> Artificial sequence

<400>  123

Met Val Asn Tyr Ile Leu Gly Val Tyr His Lys Asn Asn Lys Ser Trp
1               5                   10                  15

Met Glu Ser Glu Phe Arg Val Tyr Pro Ala Pro Ala Pro Met Lys Ile
            20                  25                  30

Lys Leu Arg Met Glu Gly Ala Val Asn Gly His Lys Phe Val Ile Glu
        35                  40                  45

Gly Glu Gly Ile Gly Lys Pro Gly Gly Ser Gly Glu Ala Ala Lys Phe
    50                  55                  60

Ile Tyr Asn Lys Ile Val Asp Glu Pro Gly Thr Gln Thr Leu Asp Leu
65                  70                  75                  80

Thr Val Glu Glu Lys Asn Pro Leu Leu Tyr Asp Ala Asn Tyr Tyr Asp
                85                  90                  95

Ile Leu Thr Pro Ala Phe Gln Tyr Gly Asn Arg Ala Phe Thr Lys Tyr
            100                 105                 110

Pro Glu Ala Gly Asn Gly Gly Asp Ala Ala Leu Ala Leu Leu Leu Leu
        115                 120                 125

Asp Gly Tyr Ser Trp Glu Arg Ser Met Thr Tyr Gly Arg Ser Tyr Leu
    130                 135                 140

Thr Pro Gly Asp Ser Ser Ser Gly Gly Ala Ser Gly Gly Ile Cys Ile
145                 150                 155                 160

Ala Thr Ser Asp Ile Thr Met Glu Gly Asp Cys Phe Phe Tyr Glu Ile
                165                 170                 175

Arg Phe Asp Gly Thr Ala Asp Gln Leu Thr Pro Thr Trp Arg Val Thr
            180                 185                 190

Leu Lys Trp Glu Pro Ser Thr Glu Lys Met Tyr Val Glu Asp Gly Val
        195                 200                 205

Leu Lys Gly Asp Val Glu Met Ala Leu Leu Leu Phe Ile Tyr Asn Lys
    210                 215                 220

Ile Val Asp Glu Pro Gly Gly Ser Gly Thr Ser Tyr Trp Lys Gly Ser
225                 230                 235                 240

His Tyr Arg Cys Asp Phe Lys Thr Thr Tyr Lys Ala Lys Asn Pro Leu
                245                 250                 255

Leu Tyr Asp Ala Asn Tyr His Glu Val Asp His Arg Ile Glu Ile Leu
            260                 265                 270

Ser His Gly Gly Ser Gly Gly Glu Ala Ala Lys Gly Arg Pro Gln Gly
        275                 280                 285

Leu Pro Asn Asn Thr Ala Ser Lys Val Arg Leu Tyr Glu His Ala Glu
    290                 295                 300

Ala Leu Ala Glu Ile Leu Gln Lys Asn Leu Ile Arg Gln Gly Thr Asp
305                 310                 315                 320

Tyr Lys His Trp Pro Gln Ile Ala Gly Gly Ser Gly Gly Gly Lys Ile
                325                 330                 335

Ala Asp Tyr Asn Tyr Lys Leu Gly His His His His His His Lys Lys
            340                 345                 350

<210>  124
<211>  15
<212>  PRT
<213>  Artificial sequence

<200>
<223> Artificial sequence


<400>  124

Phe Glu Arg Asp Ile Ser Thr Glu Ile Tyr Gln Ala Gly Ser Thr
1               5                   10                  15

<210>  125

<211>  15
<212>  PRT
<213>  Artificial sequence

<200>
<223> Artificial sequence


<400>  125

Gly Ser Thr Pro Cys Asn Gly Val Glu Gly Phe Asn Cys Tyr Phe
1               5                   10                  15

<210>  126
<211>  15
<212>  PRT
<213>  Artificial sequence

<200>
<223> Artificial sequence


<400>  126

Asn Ser Asn Asn Leu Asp Ser Lys Val Gly Gly Asn Tyr Asn Tyr
1               5                   10                  15

<210>  127
<211>  10
<212>  PRT
<213>  Artificial sequence

<200>
<223> Artificial sequence


<400>  127

Gly Ser Ser Gly Val Val Asn Pro Val Met
1               5                   10

<210>  128
<211>  16
<212>  PRT
<213>  Artificial sequence

<200>
<223> Artificial sequence


<400>  128

Asn Ala Pro Arg Ile Thr Phe Gly Gly Pro Ser Asp Ser Thr Gly Ser
1               5                   10                  15

<210>  129
<211>  15
<212>  PRT
<213>  Artificial sequence

<200>
<223> Artificial sequence

<400>  129

Tyr Ile Arg Val Gly Ala Arg Lys Ser Ala Pro Leu Ile Glu Leu
1               5                   10                  15

<210>  130
<211>  15
<212>  PRT
<213>  Artificial sequence

<200>
<223> Artificial sequence


<400>  130

Ser Leu Ile Asp Leu Gln Glu Leu Gly Lys Tyr Glu Gln Tyr Ile
1               5                   10                  15

<210>  131
<211>  15
<212>  PRT
<213>  Artificial sequence

<200>
<223> Artificial sequence


<400>  131

Pro Phe Gln Gln Phe Gly Arg Asp Ile Ala Asp Thr Thr Asp Ala
1               5                   10                  15

<210>  132
<211>  12
<212>  PRT
<213>  Artificial sequence

<200>
<223> Artificial sequence


<400>  132

Met Trp Leu Ser Tyr Phe Ile Ala Ser Phe Arg Leu
1               5                   10

<210>  133
<211>  5
<212>  PRT
<213>  Artificial sequence

<200>
<223> Artificial sequence


<400>  133

Ile Val Asp Glu Pro
1               5

<210>  134

<211>  12
<212>  PRT
<213>  Artificial sequence

<200>
<223> Artificial sequence


<400>  134

Gly Phe Ser Ala Leu Glu Pro Leu Val Asp Leu Pro
1               5                   10

<210>  135
<211>  13
<212>  PRT
<213>  Artificial sequence

<200>
<223> Artificial sequence


<400>  135

Lys Thr Phe Pro Pro Thr Glu Pro Lys Lys Asp Lys Lys
1               5                   10

<210>  136
<211>  19
<212>  PRT
<213>  Artificial sequence

<200>
<223> Artificial sequence


<400>  136

Leu Gly Val Tyr His Lys Asn Asn Lys Ser Trp Met Glu Ser Glu Phe
1               5                   10                  15


Arg Val Tyr

<210>  137
<211>  15
<212>  PRT
<213>  Artificial sequence

<200>
<223> Artificial sequence


<400>  137

Phe Asn Cys Tyr Phe Pro Leu Gln Ser Tyr Gly Phe Gln Pro Thr
1               5                   10                  15

<210>  138
<211>  10
<212>  PRT
<213>  Artificial sequence

<200>
<223> Artificial sequence

<400> 138

Pro Leu Gln Ser Tyr Gly Phe Gln Pro Thr
1               5                   10

<210> 139
<211> 15
<212> PRT
<213> Artificial sequence

<200>
<223> Artificial sequence

<400> 139

Ala Gly Asn Gly Gly Asp Ala Ala Leu Ala Leu Leu Leu Leu Asp
1               5                   10                  15

<210> 140
<211> 11
<212> PRT
<213> Artificial sequence

<200>
<223> Artificial sequence

<400> 140

Arg Ser Tyr Leu Thr Pro Gly Asp Ser Ser Ser
1               5                   10

<210> 141
<211> 10
<212> PRT
<213> Artificial sequence

<200>
<223> Artificial sequence

<400> 141

Ala Asp Gln Leu Thr Pro Thr Trp Arg Val
1               5                   10

<210> 142
<211> 10
<212> PRT
<213> Artificial sequence

<200>
<223> Artificial sequence

<400> 142

Phe Ile Tyr Asn Lys Ile Val Asp Glu Pro

1               5                   10

<210>  143
<211>  10
<212>  PRT
<213>  Artificial sequence

<200>
<223> Artificial sequence


<400>  143

Lys Asn Pro Leu Leu Tyr Asp Ala Asn Tyr
1               5                   10

<210>  144
<211>  11
<212>  PRT
<213>  Artificial sequence

<200>
<223> Artificial sequence


<400>  144

Arg Pro Gln Gly Leu Pro Asn Asn Thr Ala Ser
1               5                   10

<210>  145
<211>  23
<212>  PRT
<213>  Artificial sequence

<200>
<223> Artificial sequence


<400>  145

Leu Ala Glu Ile Leu Gln Lys Asn Leu Ile Arg Gln Gly Thr Asp Tyr
1               5                   10                  15


Lys His Trp Pro Gln Ile Ala
            20

<210>  146
<211>  10
<212>  PRT
<213>  Artificial sequence

<200>
<223> Artificial sequence


<400>  146

Gly Lys Ile Ala Asp Tyr Asn Tyr Lys Leu
1               5                   10

<210>  147

<211>  15
<212>  PRT
<213>  Artificial sequence

<200>
<223> Artificial sequence


<400>  147

Gly Ser Ser Gly Val Val Asn Pro Val Met Glu Pro Ile Tyr Asp
1               5                   10                  15

<210>  148
<211>  15
<212>  PRT
<213>  Artificial sequence

<200>
<223> Artificial sequence


<400>  148

Ala Pro Gly Gln Thr Gly Lys Ile Ala Asp Tyr Asn Tyr Lys Leu
1               5                   10                  15

<210>  149
<211>  15
<212>  PRT
<213>  Artificial sequence

<200>
<223> Artificial sequence


<400>  149

Ala Leu Pro Gln Arg Gln Lys Lys Gln Gln Thr Val Thr Leu Leu
1               5                   10                  15

<210>  150
<211>  10
<212>  PRT
<213>  Artificial sequence

<200>
<223> Artificial sequence


<400>  150

Gly Ser Lys Ser Pro Ile Gln Tyr Ile Asp
1               5                   10

<210>  151
<211>  14
<212>  PRT
<213>  Artificial sequence

<200>
<223> Artificial sequence

<400>  151

Asp Phe Leu Glu Tyr His Asp Val Arg Val Val Leu Asp Phe
1               5                   10

<210>  152
<211>  10
<212>  PRT
<213>  Artificial sequence

<200>
<223> Artificial sequence



<400>  152

Gly Ile Asn Ala Ser Val Val Asn Ile Gln
1               5                   10

<210>  153
<211>  10
<212>  PRT
<213>  Artificial sequence

<200>
<223> Artificial sequence



<400>  153

Gln Phe Ala Pro Ser Ala Ser Ala Phe Phe
1               5                   10

<210>  154
<211>  10
<212>  PRT
<213>  Artificial sequence

<200>
<223> Artificial sequence



<400>  154

Pro Ser Gly Thr Trp Leu Thr Tyr Thr Gly
1               5                   10

<210>  155
<211>  5
<212>  PRT
<213>  Artificial sequence

<200>
<223> Artificial sequence



<400>  155

Glu Leu Asp Lys Tyr
1               5

<210>  156

<211>  10
<212>  PRT
<213>  Artificial sequence

<200>
<223> Artificial sequence


<400>  156

Gly Ile Tyr Gln Thr Ser Asn Phe Arg Val
1               5                   10

<210>  157
<211>  15
<212>  PRT
<213>  Artificial sequence

<200>
<223> Artificial sequence


<400>  157

Lys Ala Tyr Asn Val Thr Gln Ala Phe Gly Arg Arg Gly Pro Glu
1               5                   10                  15

<210>  158
<211>  10
<212>  PRT
<213>  Artificial sequence

<200>
<223> Artificial sequence


<400>  158

Gly Thr Asn Thr Ser Asn Gln Val Ala Val
1               5                   10

<210>  159
<211>  15
<212>  PRT
<213>  Artificial sequence

<200>
<223> Artificial sequence


<400>  159

Asn Pro Val Leu Pro Phe Asn Asp Gly Val Tyr Phe Ala Ser Thr
1               5                   10                  15

<210>  160
<211>  10
<212>  PRT
<213>  Artificial sequence

<200>
<223> Artificial sequence

<400>  160

Tyr Asn Tyr Lys Leu Pro Asp Asp Phe Thr
1               5                   10

<210>  161
<211>  15
<212>  PRT
<213>  Artificial sequence

<200>
<223> Artificial sequence


<400>  161

Met Phe His Leu Val Asp Phe Gln Val Thr Ile Ala Glu Ile Leu
1               5                   10                  15

<210>  162
<211>  10
<212>  PRT
<213>  Artificial sequence

<200>
<223> Artificial sequence


<400>  162

Met Lys Asp Leu Ser Pro Arg Trp Tyr Phe
1               5                   10

<210>  163
<211>  10
<212>  PRT
<213>  Artificial sequence

<200>
<223> Artificial sequence


<400>  163

Asp Ala Ala Leu Ala Leu Leu Leu Leu Asp
1               5                   10